(19) Europäisches Patentamt
European Patent Office
Office européen des brevets

(11) EP 4 601 449 A2

(12) **EUROPÄISCHE PATENTANMELDUNG**

(43) Veröffentlichungstag:
**13.08.2025 Patentblatt 2025/33**

(21) Anmeldenummer: **25186173.8**

(22) Anmeldetag: **24.09.2019**

(51) Internationale Patentklassifikation (IPC):
***H10K 85/60** (2023.01)*

(52) Gemeinsame Patentklassifikation (CPC):
**C09K 11/06; C07B 59/001; C07C 13/32; C07C 13/615; C07C 13/68; C07C 13/72; C07C 211/54; C07C 211/61; C07D 209/86; C07D 209/94; C07D 251/24; C07D 307/91; C07D 307/93; C07D 333/76; C07D 403/04;** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.09.2018 EP 18197197**

(62) Dokumentnummer(n) der früheren Anmeldung(en) nach Art. 76 EPÜ:
**23152882.9 / 4 190 880**
**19779418.3 / 3 856 868**

(71) Anmelder: **Merck Patent GmbH**
**64293 Darmstadt (DE)**

(72) Erfinder: **Stoessel, Philipp**
**64293 Darmstadt (DE)**

(74) Vertreter: **Merck Patent Association**
**Merck Patent GmbH**
**64271 Darmstadt (DE)**

Bemerkungen:
Diese Anmeldung ist am 30-06-2025 als Teilanmeldung zu der unter INID-Code 62 erwähnten Anmeldung eingereicht worden.

(54) **VERBINDUNGEN, DIE IN EINER ORGANISCHEN ELEKTRONISCHEN VORRICHTUNG ALS AKTIVE VERBINDUNGEN EINSETZBAR SIND**

(57) Die vorliegende Erfindung beschreibt Verbindungen, die in einer organischen elektronischen Vorrichtung als aktive Verbindung einsetzbar sind, insbesondere zur Verwendung in elektronische Vorrichtungen. Die Erfindung betrifft ferner ein Verfahren zur Herstellung der erfindungsgemäßen Verbindungen sowie elektronische Vorrichtungen, enthaltend diese.

EP 4 601 449 A2

(52) Gemeinsame Patentklassifikation (CPC): (Forts.)
**C07D 403/14; C07D 405/04; C07D 487/04; C07F 7/0807; C07F 15/0033; H10K 85/40; H10K 85/622; H10K 85/626; H10K 85/633; H10K 85/636; H10K 85/654; H10K 85/6572; H10K 85/6574; H10K 85/6576;** C07C 2603/18; C07C 2603/42; C07C 2603/74; C07C 2603/86; C07C 2603/94; H10K 50/11; H10K 2101/10; H10K 2101/90; Y02E 10/549

## Beschreibung

Die vorliegende Erfindung beschreibt Verbindungen, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer. Ferner sind organische Elektrolumineszenzvorrichtungen bekannt, die fluoreszierende Emitter oder Emitter umfassen, die TADF (thermally activated delayed fluorescence) zeigen.

Die Eigenschaften organischer elektrolumineszierender Vorrichtungen werden nicht nur durch die eingesetzten Emitter bestimmt. Hier sind insbesondere auch die anderen verwendeten Materialien, wie Host-/Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden insbesondere zur Herstellung von Metallkomplexen, die Phosphoreszenz zeigen, Verbindungen mit bi- oder tricyclischen Ringsystemen eingesetzt. Diese Verbindungen dienen insbesondere als Ligand in den entsprechenden Komplexen. Zu diesem Stand der Technik gehören insbesondere die Druckschriften WO 2014/094960 A1, WO 2014/094961 A1, WO 2015/104045 A1, WO 2015/117718 A1 und WO 2016/124304. Matrixmaterialien, Elektronentransportmaterialien, Lochtransportmaterialien, fluoreszierende Emitter oder Emitter, die TADF (thermally activated delayed fluorescence) zeigen, werden in diesen Dokumenten nicht dargelegt.

Ferner sind aus der Druckschrift WO 2015/036078 heterocyclische Verbindungen bekannt, die unter anderem als Matrixmaterialien, Elektronentransportmaterialien oder Lochtransportmaterialien eingesetzt werden können. Die meisten dieser Verbindungen umfassen ein bicyclisches Ringsystem, wobei dieses an eine Pyridin- oder Pyridazinstruktur kondensiert ist, an die wiederum ein aromatisches oder heteroaromatisches Ringsystem kondensiert ist.

Generell besteht bei diesen Materialien, beispielsweise für die Verwendung als Matrixmaterialien, Lochleitermaterialien oder Elektronentransportmaterialien noch Verbesserungsbedarf, insbesondere in Bezug auf die Lebensdauer, aber auch in Bezug auf die Effizienz und die Betriebsspannung der Vorrichtung. Ferner sollten die Verbindungen eine hohe Farbreinheit aufweisen.

Eine weitere Aufgabe der vorliegenden Erfindung ist die Bereitstellung von Verbindungen, welche sich für den Einsatz in einer organischen elektronischen Vorrichtung, insbesondere in einer organischen Elektrolumineszenzvorrichtung, als fluoreszierende Emitter oder Emitter eignen, die TADF (thermally activated delayed fluorescence) zeigen, und welche bei Verwendung in dieser Vorrichtung zu guten Device-Eigenschaften führen, sowie die Bereitstellung der entsprechenden elektronischen Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung, eignen und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung, Verbindungen zur Verfügung zu stellen, die zu hoher Lebensdauer, guter Effizienz und geringer Betriebsspannung führen. Gerade auch die Eigenschaften der Matrixmaterialien, der Lochleitermaterialien oder der Elektronentransportmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs eignen.

Weiterhin sollten die Verbindungen, insbesondere bei ihrem Einsatz als Matrixmaterialien, als Lochleitermaterialien oder als Elektronentransportmaterialien in organischen Elektrolumineszenzvorrichtung zu Vorrichtungen führen, die eine ausgezeichnete Farbreinheit aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen

Weiterhin sollten die elektronischen Vorrichtungen für viele Zwecke eingesetzt oder angepasst werden können.

Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausfürhungsformen sind daher Gegenstand der vorliegenden Erfindung.

Gegenstand der vorliegenden Erfindung ist daher eine Verbindung, die einer organischen elektronischen Vorrichtung als aktive Verbindung einsetzbar ist, dadurch gekennzeichnet, dass die Verbindung mindestens ein aromatisches oder heteroaromatisches Ringsystem mit 5 bis 60 Kohlenstoffaromen aufweist, welches an ein aliphatisches polycyclisches Ringsystem mit mindestens 3 Ringen kondensiert ist.

Bevorzugt kann vorgesehen sein, dass der größte Cyclus des aliphatischen polycyclischen Ringsystems mit mindestens 3 Ringen höchstens 14, vorzugsweise höchstens 12, besonders bevorzugt höchstens 10 und speziell bevorzugt höchstens 7 Atome im entsprechenden Ring aufweist.

Ferner kann vorgesehen sein, dass der Ring, über den das aliphatische polycyclische Ringsystem mit mindestens 3 Ringen an das aromatische oder heteroaromatische Ringsystem mit 5 bis 60 Kohlenstoffaromen kondensiert ist, sechs Ringatome und mindestens zwei nicht benachbarte Stickstoffatome umfasst.

Darüber hinaus kann vorgesehen sein, dass der Ring, über den das aliphatische polycyclische Ringsystem mit mindestens 3 Ringen an das aromatische oder heteroaromatische Ringsystem mit 5 bis 60 Kohlenstoffaromen kondensiert ist, sechs Ringatome und kein Stickstoffatom umfasst.

Weiterhin kann vorgesehen sein, dass der Ring, über den das aliphatische polycyclische Ringsystem mit mindestens 3 Ringen an das aromatische oder heteroaromatische Ringsystem mit 5 bis 60 Kohlenstoffaromen kondensiert ist, sechs Ringatome und mindestens ein Stickstoffatom umfasst und an diesen Ring kein weiteres Ringsystem kondensiert ist.

Aktive Verbindungen sind generell die organischen oder anorganischen Materialien, welche beispielsweise in einer organischen elektronischen Vorrichtung, insbesondere in einer organischen Elektrolumineszenzvorrichtung zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Die Verbindung, die einer organischen elektronischen Vorrichtung als aktive Verbindung einsetzbar ist, kann bevorzugt ausgewählt sein aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Excitonenblockiermaterialien, Elektroneninjektionsmaterialien, Lochleitermaterialien, Lochinjektionsmaterialien, n-Dotanden, p-Dotanden, Wide-Band-Gap-Materialien, Elektronenblockiermaterialien und/oder Lochblockiermaterialien. Hierbei sind fluoreszierenden Emittern, Emittern, die TADF (thermally activated delayed fluorescence) zeigen, Hostmaterialien, Elektronentransportmaterialien, Excitonenblockiermaterialien, Elektroneninjektionsmaterialien, Lochleitermaterialien, Lochinjektionsmaterialien, n-Dotanden, p-Dotanden, Wide-Band-Gap-Materialien, Elektronenblockiermaterialien und/oder Lochblockiermaterialien bevorzugt.

In einer bevorzugten Ausgestaltung können die erfindungsgemäßen Verbindungen mindestens eine Struktur der Formeln (I) bis (XVIII) umfassen

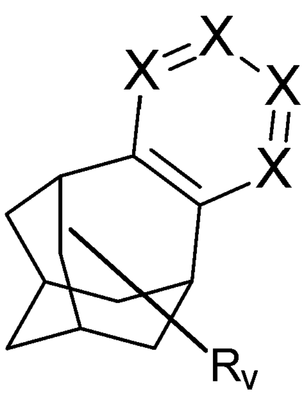

Formel (I)

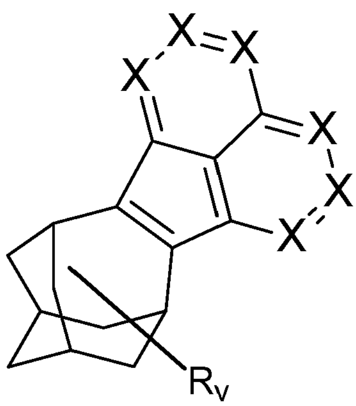

Formel (II)

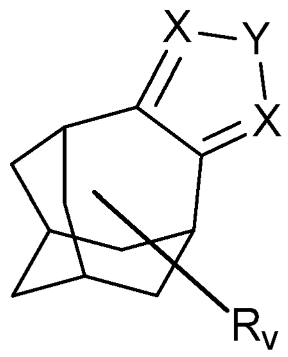

Formel (III)

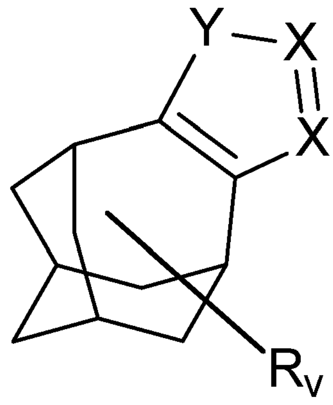

Formel (IV)

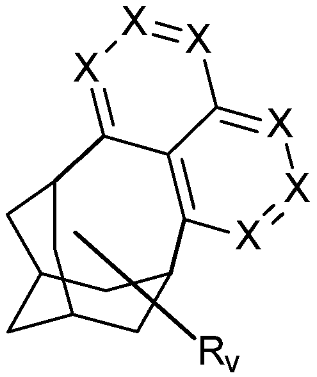

Formel (V)

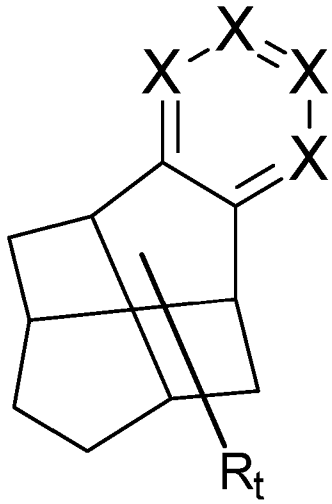

Formel (VI)

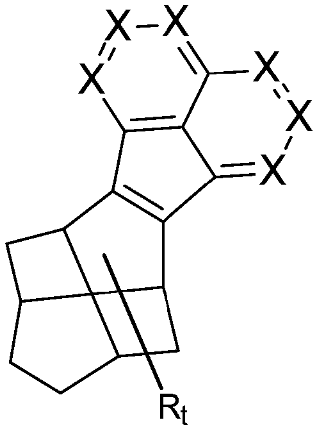

Formel (VII)

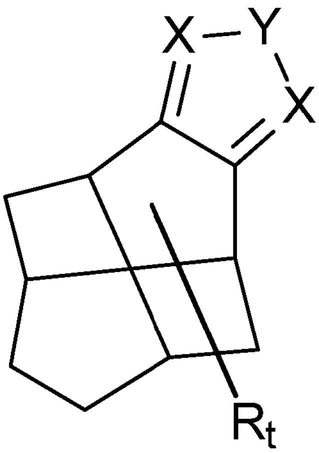

Formel (VIII)

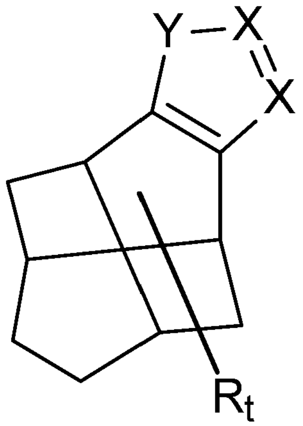

Formel (IX)

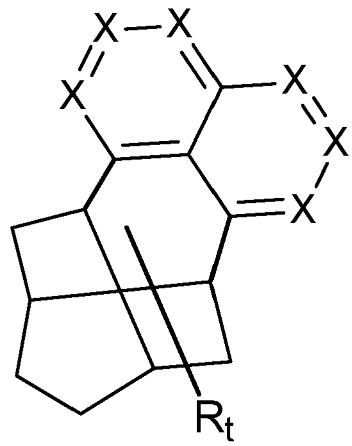

Formel (X)

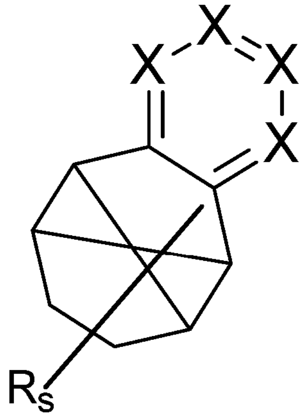

Formel (XI)

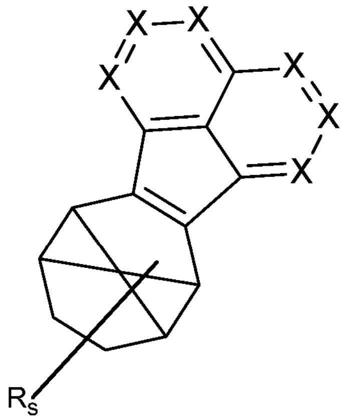

Formel (XII)

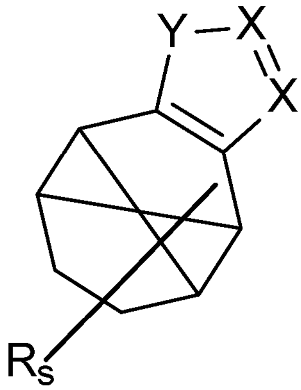

Formel (XIII)

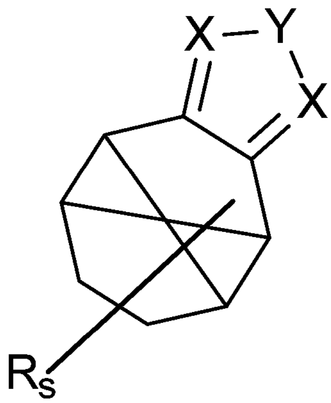

Formel (XIV)

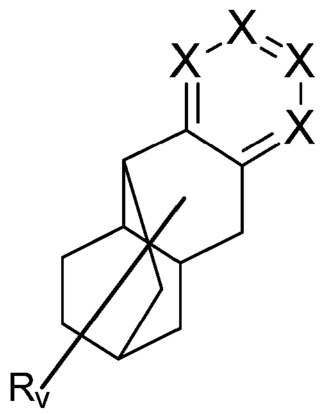

Formel (XV)

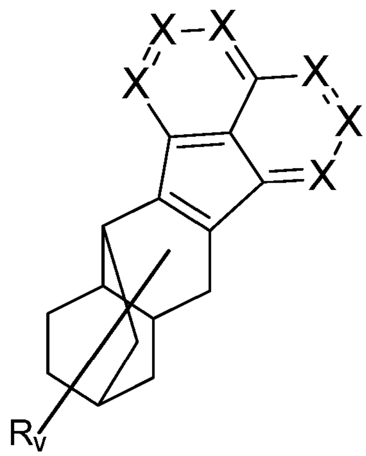

Formel (XVI)

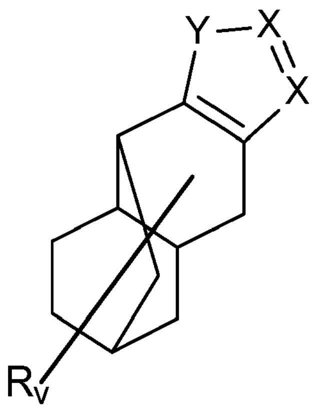

Formel (XVII)

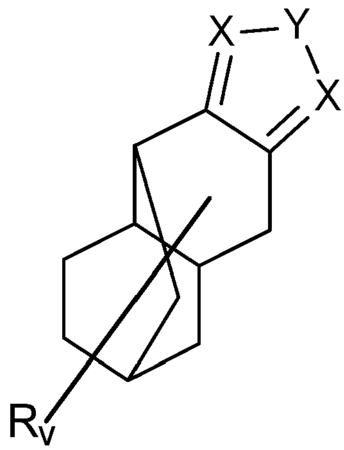

Formel (XVIII)

wobei für die verwendeten Symbole gilt:

Y ist bei jedem Auftreten gleich oder verschieden O, S, $C(R)_2$, CArR, $C(Ar)_2$, $Si(Ar)_2$, SiArR oder $Si(R)_2$, NR oder NAr, bevorzugt O, S, NAr, besonders bevorzugt NAr;

X ist bei jedem Auftreten gleich oder verschieden N oder CR, vorzugsweise CR;

R ist bei jedem Auftreten gleich oder verschieden H, D, OH, F, Cl, Br, I, CN, $NO_2$, $N(Ar)_2$, $N(R^1)_2$, $C(=O)N(Ar)_2$, $C(=O)N(R^1)_2$, $Si(Ar)_3$, $Si(R^1)_3$, $B(OAr)_2$, $B(OR^1)_2$, C(=O)Ar, $C(=O)R^1$, $P(=O)(Ar)_2$, $P(=O)(R^1)_2$, S(=O)Ar, S(=O)R', $S(=O)_2Ar$, $S(=O)_2R^1$, $OSO_2Ar$, $OSO_2R^1$, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten $R^1$ substituiert sein kann, wobei eine oder mehrere nicht benachbarte $CH_2$-Gruppen durch $R^1C=CR^1$, C=C, $Si(R^1)_2$, C=O, $NR^1$, O, S oder $CONR^1$ ersetzt sein können, oder ein aromatisches oder heteroaromatisches Ringsystem mit 5 bis 40 aromatischen Ringatomen, das jeweils durch einen oder mehrere Reste $R^1$ substituiert sein kann, oder eine Aryloxy- oder Heteroaryloxygruppe mit 5 bis 40 aromatischen Ringatomen, die durch einen oder mehrere Reste $R^1$ substituiert sein kann; dabei können zwei Reste R auch miteinander ein mono- oder polycyclisches, aliphatisches oder aromatisches oder heteroaromatisches Ringsystem bilden;

Ar ist bei jedem Auftreten gleich oder verschieden ein aromatisches oder heteroaromatisches Ringsystem mit 5

bis 60 aromatischen Ringatomen, das mit einem oder mehreren Resten $R^1$ substituiert sein kann, dabei können zwei Reste Ar, welche an dasselbe Si-Atom, N-Atom, P-Atom oder B-Atom binden, auch durch eine Einfachbindung oder eine Brücke, ausgewählt aus $B(R^1)$, $C(R^1)_2$, $Si(R^1)_2$, C=O, $C=NR^1$, $C=C(R^1)_2$, O, S, S=O, $SO_2$, $N(R^1)$, $P(R^1)$ und $P(=O)R^1$, miteinander verbrückt sein;

$R^1$ ist bei jedem Auftreten gleich oder verschieden H, D, OH, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenyl- oder Alkinylgruppe mit 2 bis 40 C-Atomen, die jeweils mit einem oder mehreren Resten $R^2$ substituiert sein kann, wobei eine oder mehrere nicht benachbarte $CH_2$-Gruppen durch - $R^2C=CR^2$-, -C≡C-, $Si(R^2)_2$, $Ge(R^2)_2$, $Sn(R^2)_2$, C=O, C=S, C=Se, $C=NR^2$, -C(=O)O-, $-C(=O)NR^2$-, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere, vorzugsweise benachbarte Reste $R^1$ miteinander ein mono- oder polycyclisches, aliphatisches oder aromatisches oder heteroaromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren, vorzugsweise nicht-aromatischen Resten $R^2$ substituiert sein kann, dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, $NO_2$, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3$-, -C≡C-, $Si(R^3)_2$, $Ge(R^3)_2$, $Sn(R^3)_2$, C=O, C=S, $C=NR^3$, -C(=O)O-, $-C(=O)NR^3$-, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere, vorzugsweise benachbarte Substituenten $R^2$ auch miteinander ein mono- oder polycyclisches, aliphatisches oder aromatisches oder heteroaromatisches Ringsystem bilden;

$R^3$ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehrere, vorzugsweise benachbarte Substituenten $R^3$ auch miteinander ein mono- oder polycyclisches, aliphatisches oder aromatisches oder heteroaromatisches Ringsystem bilden;

der Index s ist 0, 1, 2, 3, 4, 5 oder 6, vorzugsweise 0, 1, 2, 3, oder 4, besonders bevorzugt 0, 1 oder 2;

der Index t ist 0, 1, 2, 3, 4, 5, 6, 7 oder 8, vorzugsweise 0, 1, 2, 3, oder 4, besonders bevorzugt 0, 1 oder 2;

der Index v ist 0, 1, 2, 3, 4, 5, 6, 7, 8 oder 9, vorzugsweise 0, 1, 2, 3, oder 4, besonders bevorzugt 0, 1 oder 2.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

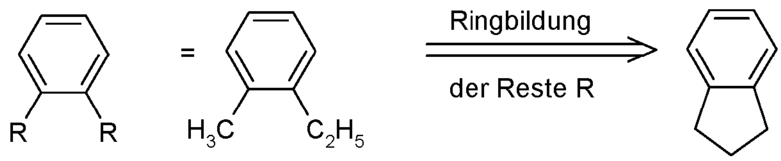


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

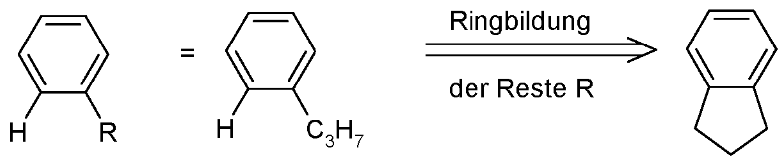


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Falls zwei oder mehrere, vorzugsweise benachbarte Reste R, $R^1$, $R^2$ und/oder $R^3$ miteinander ein Ringsystem bilden, so kann ein monocyclisches oder polycyclisches, aliphatisches, aromatisches oder heteroaromatisches Ringsystem entstehen.

Eine Arylgruppe im Sinne dieser Erfindung enthält 6 bis 60 C-Atome, vorzugsweise 6 bis 40 C-Atome, besonders bevorzugt 6 bis 30 C-Atome, eine Heteroarylgruppe im Sinne dieser Erfindung enthält 2 bis 60 C-Atome, vorzugsweise 2 bis 40 C-Atome, besonders bevorzugt 2 bis 30 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome, besonders bevorzugt 6 bis 30 C-Atome im Ringsystem. Ein heteroaromatisches Ringsystem im Sinne dieser Erfindung enthält 1 bis 60 C, vorzugsweise 1 bis 40 C-Atome, besonders bevorzugt 1 bis 30 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60, vorzugsweise 5 - 40 aromatischen Ringatomen, besonders bevorzugt 5 bis 30 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ferner sind Verbindungen mit Strukturen der Formeln (I) bis (XVIII) bevorzugt, in denen höchstens zwei Gruppen X pro Ring für N, bevorzugt alle Gruppen X pro Ring für CR stehen, stehen und bevorzugt mindestens eine, besonders bevorzugt mindestens zwei der Gruppen X pro Ring ausgewählt sind aus C-H und C-D.

In einer weiteren Ausgestaltung sind Verbindungen mit Strukturen der Formeln (I) bis (XVIII) bevorzugt, in denen zwei Gruppen X pro Ring für N stehen, wobei diese Gruppen X nicht benachbart sind.

Darüber hinaus sind Verbindungen mit Strukturen gemäß Formeln (I) bis (XVIII) bevorzugt, in denen nicht mehr als vier, vorzugsweise nicht mehr als zwei Gruppen X für N stehen, und besonders bevorzugt alle Gruppen X für CR stehen, wobei vorzugsweise höchstens vier, besonders bevorzugt höchstens drei und speziell bevorzugt höchstens zwei der Gruppen CR, für die X steht, ungleich der Gruppe CH ist.

In einer weiteren Ausgestaltung sind Verbindungen mit Strukturen der Formeln (I) bis (XVIII) bevorzugt, in denen zwei Gruppen X für N stehen, wobei diese Gruppen X nicht benachbart sind.

Vorzugsweise können die erfindungsgemäßen Verbindungen mindestens eine Struktur der Formeln (Ia) bis (XVIIIa) umfassen

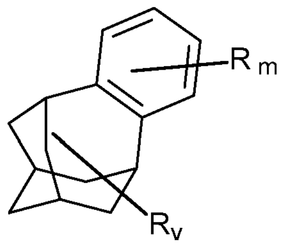

Formel (Ia)

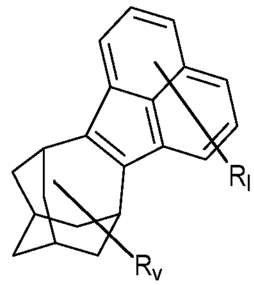

Formel (IIa)

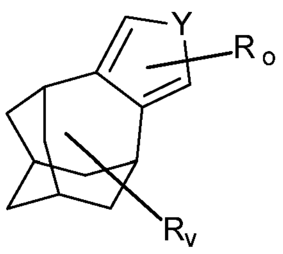

Formel (IIIa)

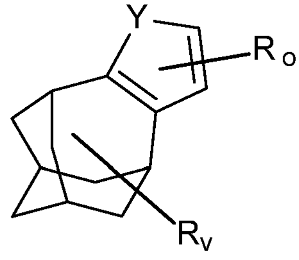

Formel (IVa)

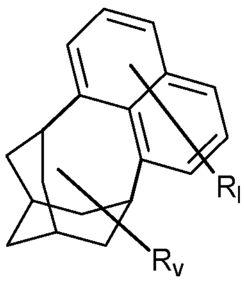

Formel (Va)

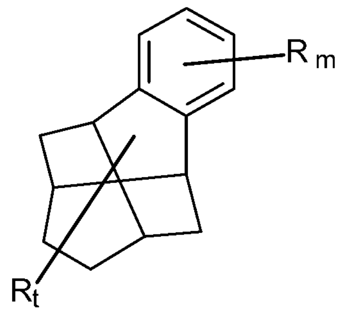

Formel (VIa)

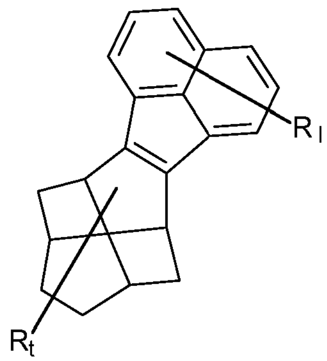

Formel (VIIa)

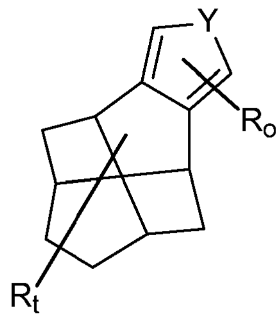

Formel (VIIIa)

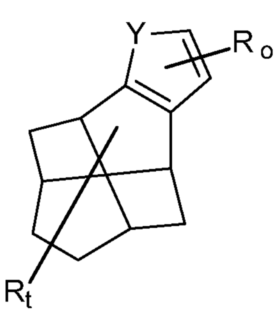

Formel (IXa)

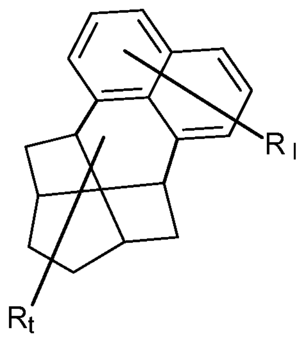

Formel (Xa)

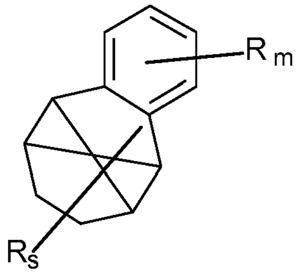

Formel (XIa)

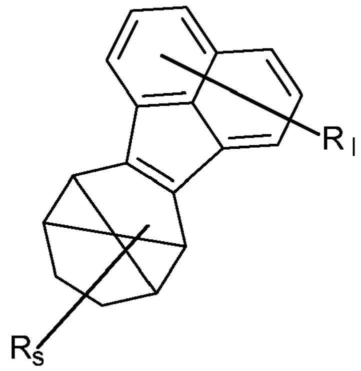

Formel (XIIa)

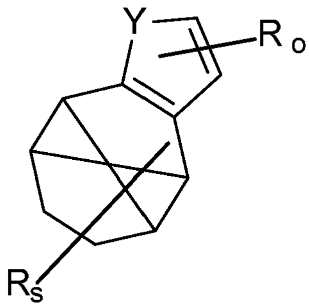

Formel (XIII)

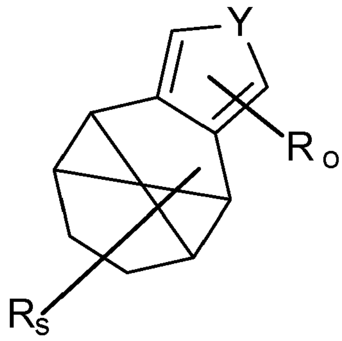

Formel (XIV)

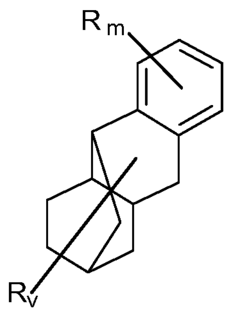

Formel (XVa)

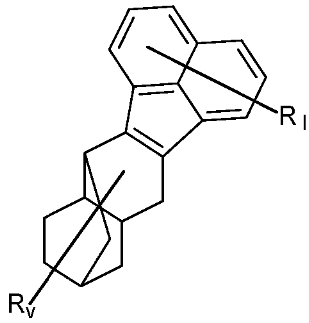

Formel (XVIa)

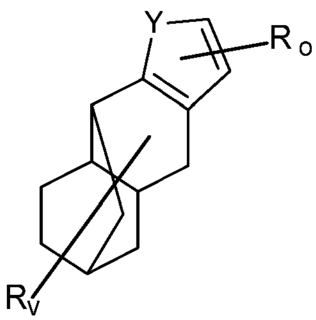

Formel (XVIIa)

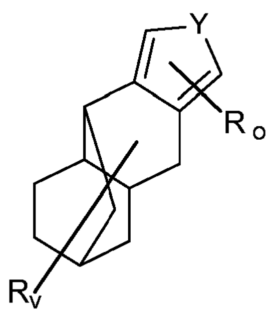

Formel (XVIIIa)

wobei die Symbole Y, R, v, t und s die zuvor, insbesondere für Formeln (I) bis (XVIII) genannte Bedeutung aufweisen, der Index o 0, 1 oder 2, vorzugsweise 0 oder 1 ist, der Index n 0, 1, 2, oder 3, vorzugsweise 0, 1 oder 2 ist und der Index m 0, 1, 2, 3 oder 4, vorzugsweise 0, 1 oder 2 ist, und der Index l 0, 1, 2, 3, 4, 5 oder 6, vorzugsweise 0, 1 oder 2 ist, wobei Y bevorzugt O, S, NR oder NAr, besonders bevorzugt NAr ist.

Ferner kann in den Strukturen gemäß den Formeln (Ia) bis (XVIIIa) vorgesehen sein, dass die Summe der Indices v, t, s, o, n, m und l höchstens 6, vorzugsweise höchstens 4 und besonders bevorzugt höchstens 2 beträgt.

Wenn X für CR steht bzw. wenn die aromatische und/oder heteroaromatische Gruppen durch Substituenten R substituiert sind, dann sind diese Substituenten R bevorzugt gewählt aus der Gruppe bestehend aus H, D, F, CN, $N(Ar)_2$, C(=O)Ar, $P(=O)(Ar)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^1$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^1$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^1$ substituiert sein kann; dabei können optional zwei Substituenten R, die vorzugsweise an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann, wobei die Gruppe Ar die zuvor, insbesondere für Formeln (I) bis (XVIII) genannte Bedeutung aufweist.

Besonders bevorzugt sind diese Substituenten R ausgewählt aus der Gruppe bestehend aus H, D, F, CN, $N(Ar)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^1$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei Ar die zuvor dargelegte Bedeutung aufweisen kann.

Ganz besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^1$ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^1$ substituiert sein können, bevorzugt aber unsubstituiert sind.

Weiterhin kann vorgesehen sein, dass die Substitutenten R des heteroaromatischen Ringssystems gemäß den Formeln (I) bis (XVIII) und/oder (Ia) bis (XVIIIa) mit den Ringatomen des aromatischen oder heteroaromatischen Ringssystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^1$, $R^2$, $R^3$ ein, die an die Reste $R^1$ gebunden sein können.

In einer weiterhin bevorzugten Ausführungsform kann vorgesehen sein, dass die Verbindung, die in einer organischen elektronischen Vorrichtung als aktive Verbindung einsetzbar ist, mindestens zwei, vorzugsweise mindestens drei aliphatische polycyclische Ringsysteme mit mindestens 3 Ringen umfasst.

Ferner kann vorgesehen sein, dass das aromatische oder heteroaromatische Ringsystem mit 5 bis 60 Kohlenstoffaromen, an welches an ein aliphatisches polycyclisches Ringsystem mit mindestens 3 Ringen kondensiert ist, ausgewählt ist aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imidazolyl, Benzimidazolyl, Benzoxazolyl, Benzthiazolyl, 1-, 2-, 3- oder 4-Carbazolyl, Indenocarbazolyl, 1- oder 2-Naphthyl, Anthracenyl, vorzugsweise 9-Anthracenyl, Phenanthrenyl und/oder Triphenylenyl, die jeweils durch einen oder mehrere Reste R und/oder $R^1$ substituiert sein können, wobei Phenyl, Spirobifluoren-, Fluoren-, Dibenzofuran-, Dibenzothiophen-, Anthracen-, Phenanthren-, Triphenylen-Gruppen besonders bevorzugt sind.

In einer weiterhin bevorzugten Ausführungsform kann vorgesehen sein, dass das aliphatische polycyclische Ringsystem mit mindestens 3 Ringen welches an ein aromatisches oder heteroaromatisches Ringsystem mit 5 bis 60 Kohlenstoffaromen kondensiert ist, eine Teilstruktur der Formeln (N-1) bis (N-6) bildet

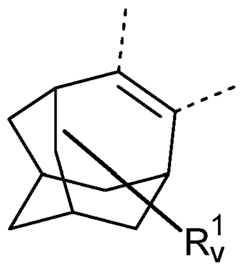

Formel (N-1)

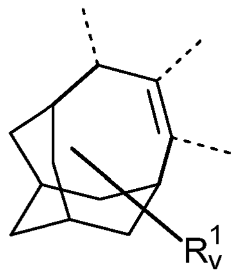

Formel (N-2)

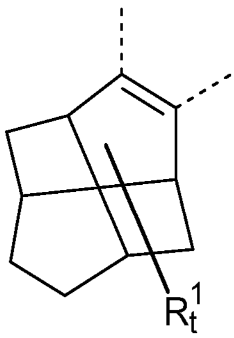

Formel (N-3)

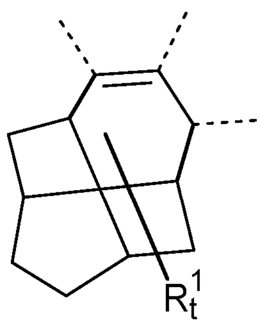

Formel (N-4)

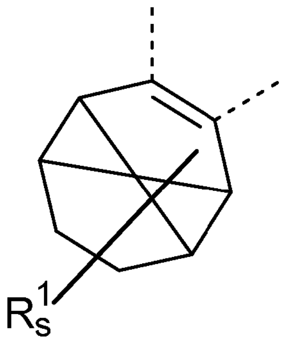

Formel (N-5)

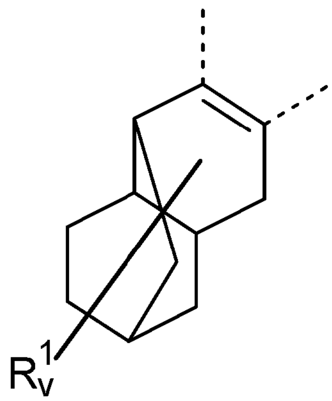

Formel (N-6)

wobei die Symbole $R^1$, v, t und s die zuvor, insbesondere für Formeln (I) bis (XVIII) genannte Bedeutung aufweisen und die gestrichelten Linien die Verknüpfungen des aromatischen oder des heteroaromatischen Ringsystems mit 5 bis 60 Kohlenstoffaromen darstellen, an welches das aliphatische polycyclische Ringsystem mit mindestens 3 Ringen kondensiert ist. Hierbei kann die in den Strukturen gemäß Formeln (N-1) bis (N-6) dargestellte Doppelbindung als Teil des aromatischen oder heteroaromatischen Ringsystems mit 5 bis 60 Kohlenstoffaromen angesehen werden, an welches die Struktur gemäß einer der Formeln (N-1) bis (N-6) kondensiert ist.

Ferner kann vorgesehen sein, dass das aromatische oder heteroaromatische Ringsystem mit 5 bis 60 Kohlenstoffaromen, an welches an ein aliphatisches polycyclisches Ringsystem mit mindestens 3 Ringen kondensiert ist, eine Teilstruktur der Formeln (Ar-1) bis (Ar-66) bildet

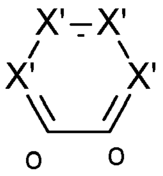
(Ar-1)

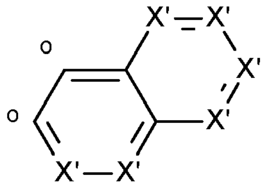
(Ar-2)

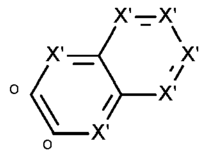
(Ar-3)

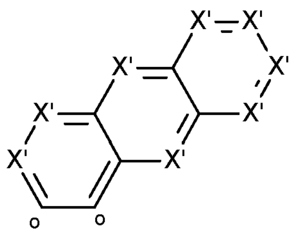
(Ar-4)

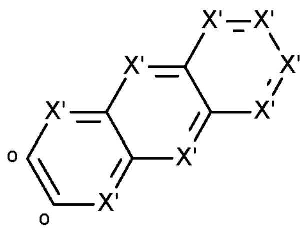
(Ar-5)

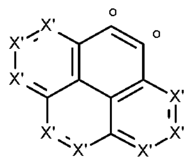
(Ar-6)

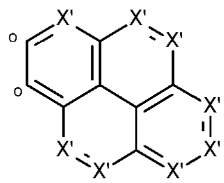
(Ar-7)

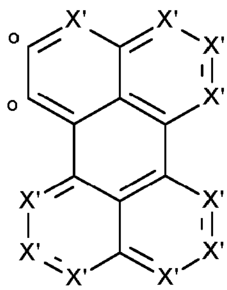
(Ar-8)

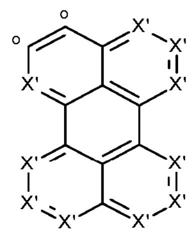
(Ar-9)

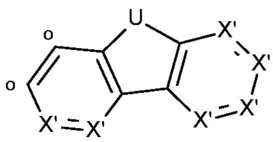
(Ar-10)

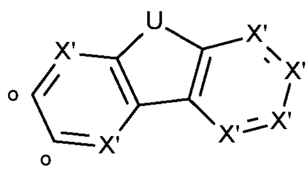
(Ar-11)

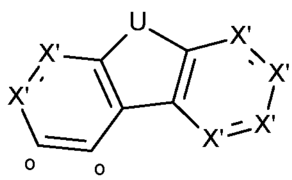
(Ar-12)

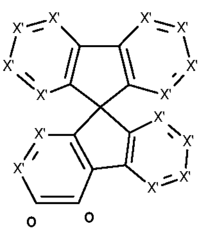
(Ar-13)

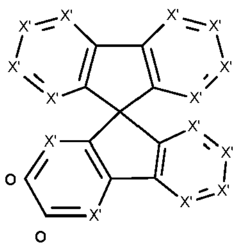
(Ar-14)

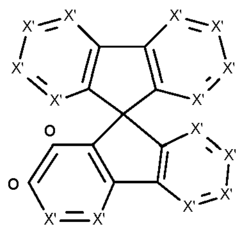
(Ar-15)

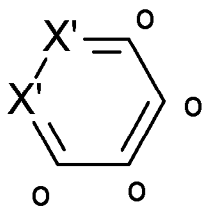
(Ar-16)

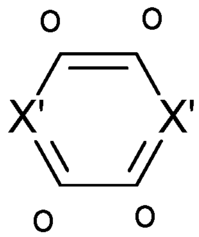
(Ar-17)

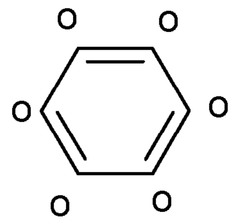
(Ar-18)

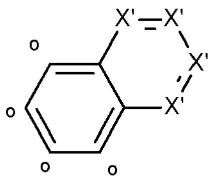
(Ar-19)

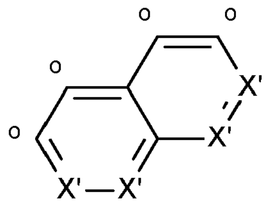
(Ar-20)

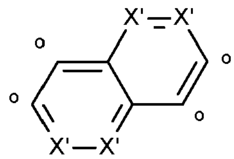
(Ar-21)

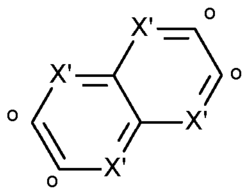
(Ar-22)

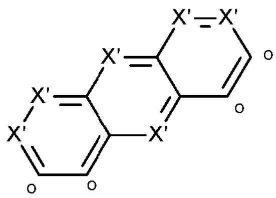
(Ar-23)

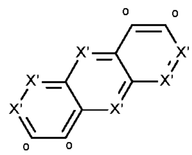
(Ar-24)

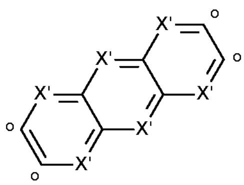
(Ar-25)

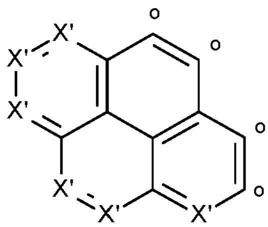
(Ar-26)

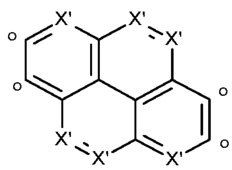
(Ar-27)

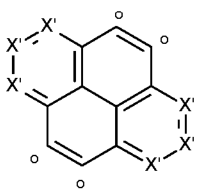
(Ar-28)

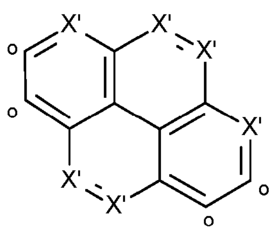
(Ar-29)

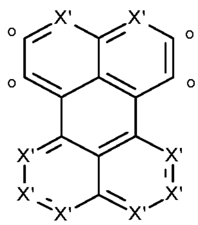
(Ar-30)

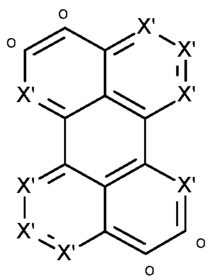
(Ar-31)

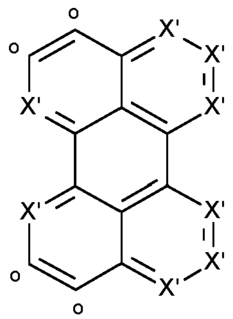
(Ar-32)

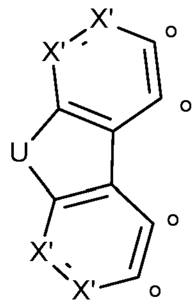
(Ar-33)

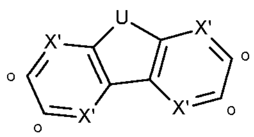
(Ar-34)

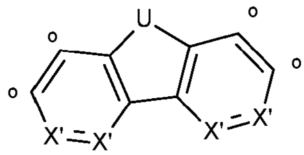
(Ar-35)

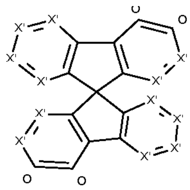
(Ar-36)

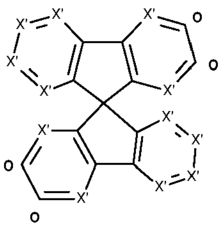
(Ar-37)

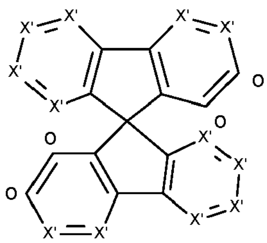
(Ar-38)

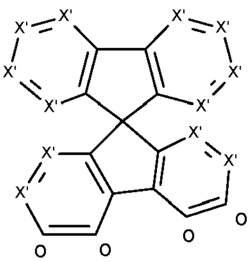
(Ar-39)

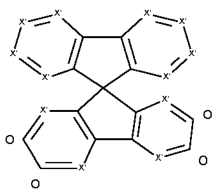
(Ar-40)

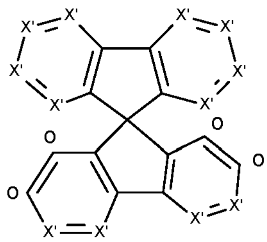
(Ar-41)

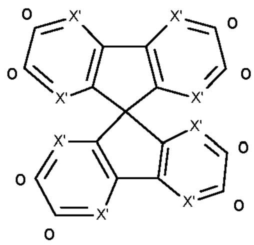
(Ar-42)

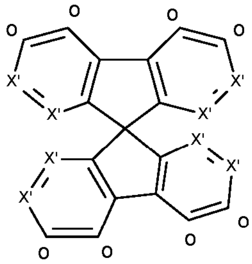
(Ar-43)

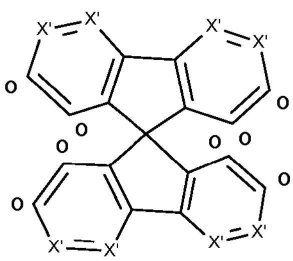
(Ar-44)

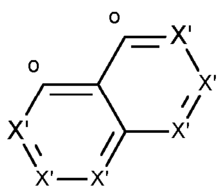
(Ar-45)

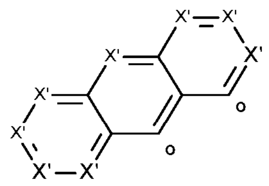
(Ar-46)

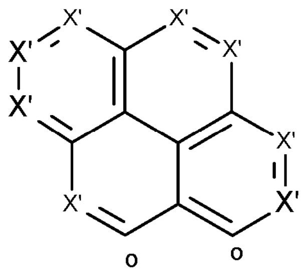
(Ar-47)

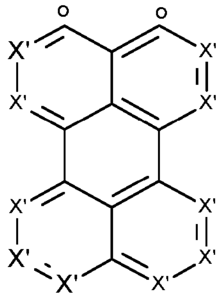
(Ar-48)

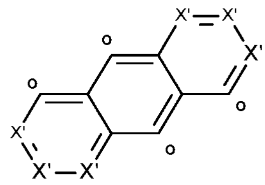
(Ar-49)

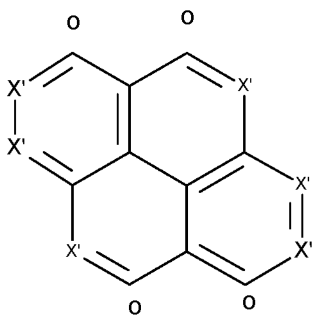
(Ar-50)

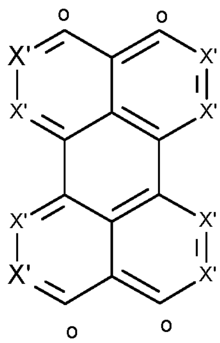
(Ar-51)

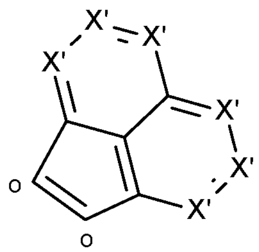
(Ar-52)

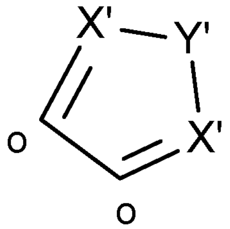
(Ar-53)

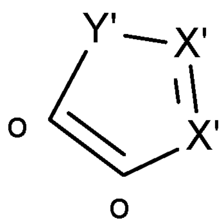
(Ar-54)

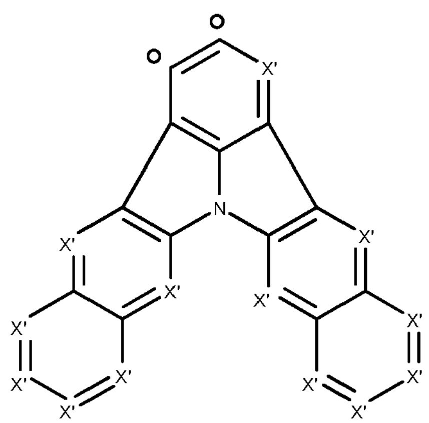
(Ar-55)

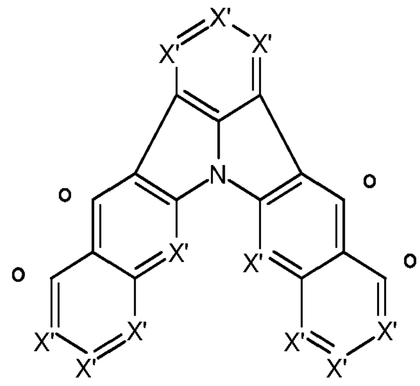

(Ar-56)

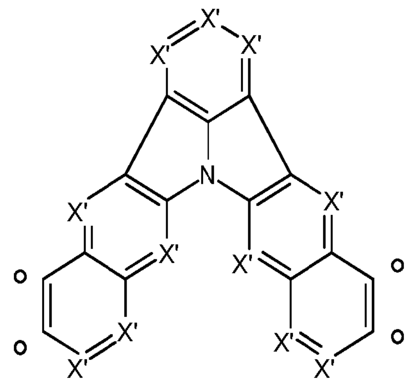

(Ar-57)

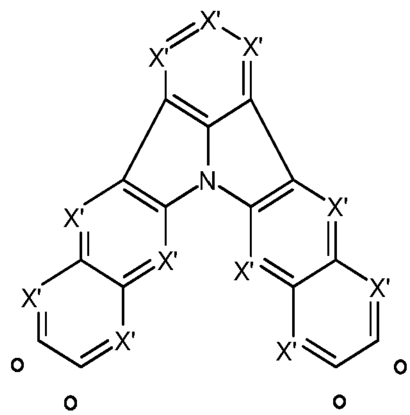

(Ar-58)

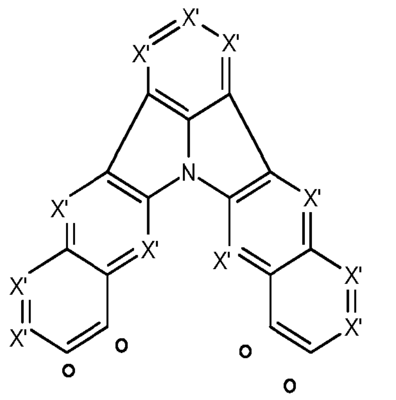

(Ar-59)

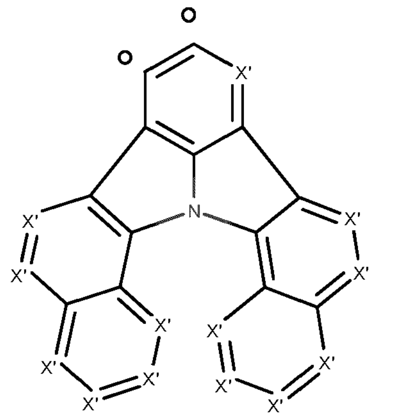

(Ar-60)

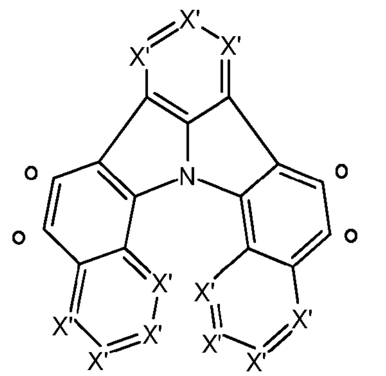

(Ar-61)

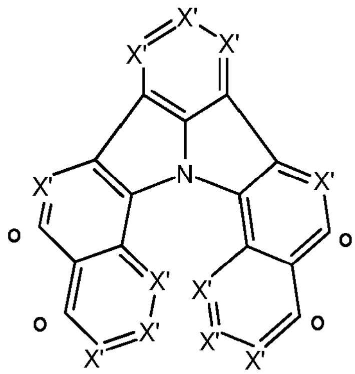

(Ar-62)

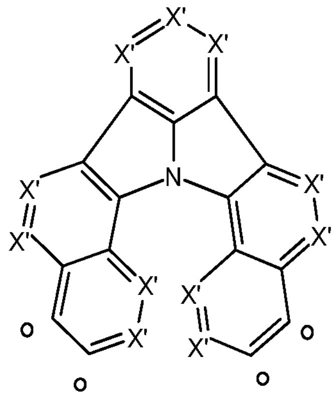

(Ar-63)

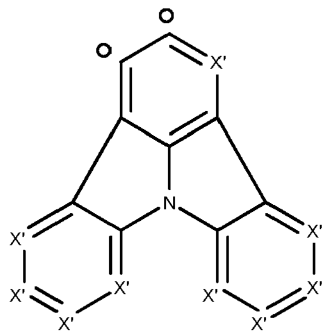

(Ar-64)

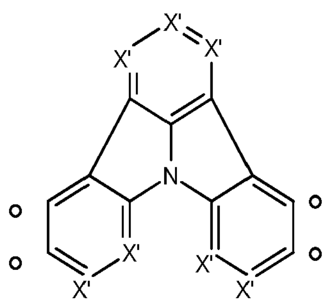

(Ar-65)

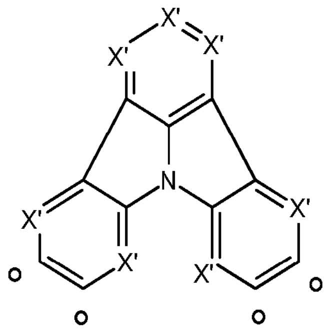

(Ar-66)

wobei X' N oder $CR^1$, vorzugsweise $CR^1$ ist, Y' ausgewählt ist aus O, S, $C(R^1)_2$, $Si(R^1)_2$, N $R^1$ oder $NAr^1$, bevorzugt O, S, $NAr^1$, besonders bevorzugt $NAr^1$, U ausgewählt ist aus O, S, $C(R^1)_2$, $N(R^1)$, $B(R^1)$, $Si(R^1)_2$, C=O, S=O, $SO_2$, $P(R^1)$ und $P(=O)R^1$, wobei $R^1$ die zuvor, insbesondere für Formeln (I) bis (XVIII) genannte Bedeutung hat und das aliphatische polycyclische Ringsystem mit mindestens 3 Ringen jeweils an den durch o gekennzeichneten Positionen an das aromatische oder heteroaromatische Ringsystem mit 5 bis 60 Kohlenstoffaromen unter Bildung eines Ringes bindet. Hierbei sind Strukturen der Formeln (Ar-2) bis (Ar-66) bevorzugt und Strukturen der Formeln (Ar-4) bis (Ar-15) und (Ar-23) bis (Ar-44) besonders bevorzugt.

Ferner sind Verbindungen mit Teilstrukturen der Formeln (Ar-1) bis (Ar-66) bevorzugt, in denen höchstens zwei Gruppen X' pro Ring für N, bevorzugt alle Gruppen X' pro Ring für $CR^1$ stehen, stehen und bevorzugt mindestens eine, besonders bevorzugt mindestens zwei der Gruppen X' pro Ring ausgewählt sind aus C-H und C-D.

In einer weiteren Ausgestaltung sind Verbindungen mit Strukturen der Formeln (I) bis (XVIII) bevorzugt, in denen zwei Gruppen X' pro Ring für N stehen, wobei diese Gruppen X' nicht benachbart sind.

Darüber hinaus sind Verbindungen mit Teilstrukturen der Formeln (Ar-1) bis (Ar-66) bevorzugt, in denen nicht mehr als vier, vorzugsweise nicht mehr als zwei Gruppen X' für N stehen, und besonders bevorzugt alle Gruppen X' für $CR^1$ stehen, wobei vorzugsweise höchstens vier, besonders bevorzugt höchstens drei und speziell bevorzugt höchstens zwei der Gruppen $CR^1$, für die X' steht, ungleich der Gruppe CH ist.

In einer weiteren Ausgestaltung sind Verbindungen mit Teilstrukturen der Formeln (Ar-1) bis (Ar-66) bevorzugt, in denen zwei Gruppen X' für N stehen, wobei diese Gruppen X nicht benachbart sind.

In noch einer weiteren Ausgestaltung sind Verbindungen mit Teilstrukturen der Formeln (Ar-55) bis (Ar-66) bevorzugt, in denen vorzugsweise maximal zwei Gruppen X' für N stehen.

Gemäß einer bevorzugten Ausführungsform sind unter anderem die Kombinationen gemäß der folgenden Tabelle bevorzugt

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| N-1 | Ar-1 | 0 | 0 bis 4, vorzugsweise 1 oder 2, bevorzugt 1 |
| N-3 | Ar-1 | 0 | 0 bis 4, vorzugsweise 1 oder 2, bevorzugt 1 |
| N-5 | Ar-1 | 0 | 0 bis 4, vorzugsweise 1 oder 2, bevorzugt 1 |
| N-6 | Ar-1 | 0 | 0 bis 4, vorzugsweise 1 oder 2, bevorzugt 1 |
| N-1 | Ar-1 | 2 | 0 bis 2, vorzugsweise 1 oder 2, bevorzugt 1 |
| N-3 | Ar-1 | 2 | 0 bis 2, vorzugsweise 1 oder 2, bevorzugt 1 |
| N-5 | Ar-1 | 2 | 0 bis 2, vorzugsweise 1 oder 2, bevorzugt 1 |
| N-6 | Ar-1 | 2 | 0 bis 2, vorzugsweise 1 oder 2, bevorzugt 1 |
| N-1 | Ar-2 | 0 | 0 bis 4, vorzugsweise 1 oder 2, bevorzugt 1 |
| N-3 | Ar-2 | 0 | 0 bis 4, vorzugsweise 1 oder 2, bevorzugt 1 |
| N-5 | Ar-2 | 0 | 0 bis 4, vorzugsweise 1 oder 2, bevorzugt 1 |
| N-6 | Ar-2 | 0 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| N-1 | Ar-2 | 2 | 0 bis 2, vorzugsweise 1 oder 2, bevorzugt 1 |
| N-3 | Ar-2 | 2 | 0 bis 2, vorzugsweise 1 oder 2, bevorzugt 1 |
| N-5 | Ar-2 | 2 | 0 bis 2, vorzugsweise 1 oder 2, bevorzugt 1 |
| N-6 | Ar-2 | 2 | 0 bis 2, vorzugsweise 1 oder 2, bevorzugt 1 |
| N-1 | Ar-3 | 0 | 0 bis 4, vorzugsweise 1 oder 2, bevorzugt 1 |
| N-3 | Ar-3 | 0 | 0 bis 4, vorzugsweise 1 oder 2, bevorzugt 1 |
| N-5 | Ar-3 | 0 | 0 bis 4, vorzugsweise 1 oder 2, bevorzugt 1 |
| N-6 | Ar-3 | 0 | 0 bis 4, vorzugsweise 1 oder 2, bevorzugt 1 |
| N-1 | Ar-3 | 2 | 0 bis 2, vorzugsweise 1 oder 2, bevorzugt 1 |
| N-3 | Ar-3 | 2 | 0 bis 2, vorzugsweise 1 oder 2, bevorzugt 1 |
| N-5 | Ar-3 | 2 | 0 bis 2, vorzugsweise 1 oder 2, bevorzugt 1 |
| N-6 | Ar-3 | 2 | 0 bis 2, vorzugsweise 1 oder 2, bevorzugt 1 |
| N-1 | Ar-4 | 0 | 0 bis 4, vorzugsweise 1, 2 oder 3, bevorzugt 2 |
| N-3 | Ar-4 | 0 | 0 bis 4, vorzugsweise 1, 2 oder 3, bevorzugt 2 |
| N-5 | Ar-4 | 0 | 0 bis 4, vorzugsweise 1, 2 oder 3, bevorzugt 2 |
| N-6 | Ar-4 | 0 | 0 bis 4, vorzugsweise 1, 2 oder 3, bevorzugt 2 |
| N-1 | Ar-4 | 2 | 0 bis 2, vorzugsweise 1 oder 2, bevorzugt 2 |
| N-3 | Ar-4 | 2 | 0 bis 2, vorzugsweise 1 oder 2, bevorzugt 2 |
| N-5 | Ar-4 | 2 | 0 bis 2, vorzugsweise 1 oder 2, bevorzugt 2 |
| N-6 | Ar-4 | 2 | 0 bis 2, vorzugsweise 1 oder 2, bevorzugt 2 |
| N-1 | Ar-5 | 0 | 0 bis 4, vorzugsweise 1, 2 oder 3, bevorzugt 2 |
| N-3 | Ar-5 | 0 | 0 bis 4, vorzugsweise 1, 2 oder 3, bevorzugt 2 |
| N-5 | Ar-5 | 0 | 0 bis 4, vorzugsweise 1, 2 oder 3, bevorzugt 2 |
| N-6 | Ar-5 | 0 | 0 bis 4, vorzugsweise 1, 2 oder 3, bevorzugt 2 |
| N-1 | Ar-5 | 2 | 0 bis 2, vorzugsweise 1 oder 2, bevorzugt 2 |
| N-3 | Ar-5 | 2 | 0 bis 2, vorzugsweise 1 oder 2, bevorzugt 2 |
| N-5 | Ar-5 | 2 | 0 bis 2, vorzugsweise 1 oder 2, bevorzugt 2 |
| N-6 | Ar-5 | 2 | 0 bis 2, vorzugsweise 1 oder 2, bevorzugt 2 |
| N-1 | Ar-6 | 0 | 0 bis 4, vorzugsweise 1 oder 2, bevorzugt 1 |
| N-3 | Ar-6 | 0 | 0 bis 4, vorzugsweise 1 oder 2, bevorzugt 1 |
| N-5 | Ar-6 | 0 | 0 bis 4, vorzugsweise 1 oder 2, bevorzugt 1 |
| N-6 | Ar-6 | 0 | 0 bis 4, vorzugsweise 1 oder 2, bevorzugt 1 |
| N-1 | Ar-6 | 2 | 0 bis 2, vorzugsweise 1 oder 2, bevorzugt 1 |
| N-3 | Ar-6 | 2 | 0 bis 2, vorzugsweise 1 oder 2, bevorzugt 1 |
| N-5 | Ar-6 | 2 | 0 bis 2, vorzugsweise 1 oder 2, bevorzugt 1 |
| N-6 | Ar-6 | 2 | 0 bis 2, vorzugsweise 1 oder 2, bevorzugt 1 |
| N-1 | Ar-7 | 0 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| N-3 | Ar-7 | 0 | 0 bis 4, vorzugsweise 1 oder 2, bevorzugt 1 |
| N-5 | Ar-7 | 0 | 0 bis 4, vorzugsweise 1 oder 2, bevorzugt 1 |
| N-6 | Ar-7 | 0 | 0 bis 4, vorzugsweise 1 oder 2, bevorzugt 1 |
| N-1 | Ar-7 | 2 | 0 bis 2, vorzugsweise 1 oder 2, bevorzugt 1 |
| N-3 | Ar-7 | 2 | 0 bis 2, vorzugsweise 1 oder 2, bevorzugt 1 |
| N-5 | Ar-7 | 2 | 0 bis 2, vorzugsweise 1 oder 2, bevorzugt 1 |
| N-6 | Ar-7 | 2 | 0 bis 2, vorzugsweise 1 oder 2, bevorzugt 1 |
| N-1 | Ar-8 | 0 | 0 bis 4, vorzugsweise 1 oder 2, bevorzugt 1 |
| N-3 | Ar-8 | 0 | 0 bis 4, vorzugsweise 1 oder 2, bevorzugt 1 |
| N-5 | Ar-8 | 0 | 0 bis 4, vorzugsweise 1 oder 2, bevorzugt 1 |
| N-6 | Ar-8 | 0 | 0 bis 4, vorzugsweise 1 oder 2, bevorzugt 1 |
| N-1 | Ar-8 | 2 | 0 bis 2, vorzugsweise 1 oder 2, bevorzugt 1 |
| N-3 | Ar-8 | 2 | 0 bis 2, vorzugsweise 1 oder 2, bevorzugt 1 |
| N-5 | Ar-8 | 2 | 0 bis 2, vorzugsweise 1 oder 2, bevorzugt 1 |
| N-6 | Ar-8 | 2 | 0 bis 2, vorzugsweise 1 oder 2, bevorzugt 1 |
| N-1 | Ar-9 | 0 | 0 bis 4, vorzugsweise 1 oder 2, bevorzugt 1 |
| N-3 | Ar-9 | 0 | 0 bis 4, vorzugsweise 1 oder 2, bevorzugt 1 |
| N-5 | Ar-9 | 0 | 0 bis 4, vorzugsweise 1 oder 2, bevorzugt 1 |
| N-6 | Ar-9 | 0 | 0 bis 4, vorzugsweise 1 oder 2, bevorzugt 1 |
| N-1 | Ar-9 | 2 | 0 bis 2, vorzugsweise 1 oder 2, bevorzugt 1 |
| N-3 | Ar-9 | 2 | 0 bis 2, vorzugsweise 1 oder 2, bevorzugt 1 |
| N-5 | Ar-9 | 2 | 0 bis 2, vorzugsweise 1 oder 2, bevorzugt 1 |
| N-6 | Ar-9 | 2 | 0 bis 2, vorzugsweise 1 oder 2, bevorzugt 1 |
| N-1 | Ar-10 | 0 | 0 bis 4, vorzugsweise 1 oder 2, bevorzugt 1 |
| N-3 | Ar-10 | 0 | 0 bis 4, vorzugsweise 1 oder 2, bevorzugt 1 |
| N-5 | Ar-10 | 0 | 0 bis 4, vorzugsweise 1 oder 2, bevorzugt 1 |
| N-6 | Ar-10 | 0 | 0 bis 4, vorzugsweise 1 oder 2, bevorzugt 1 |
| N-1 | Ar-10 | 2 | 0 bis 2, vorzugsweise 1 oder 2, bevorzugt 1 |
| N-3 | Ar-10 | 2 | 0 bis 2, vorzugsweise 1 oder 2, bevorzugt 1 |
| N-5 | Ar-10 | 2 | 0 bis 2, vorzugsweise 1 oder 2, bevorzugt 1 |
| N-6 | Ar-10 | 2 | 0 bis 2, vorzugsweise 1 oder 2, bevorzugt 1 |
| N-1 | Ar-11 | 0 | 0 bis 4, vorzugsweise 1 oder 2, bevorzugt 1 |
| N-3 | Ar-11 | 0 | 0 bis 4, vorzugsweise 1 oder 2, bevorzugt 1 |
| N-5 | Ar-11 | 0 | 0 bis 4, vorzugsweise 1 oder 2, bevorzugt 1 |
| N-6 | Ar-11 | 0 | 0 bis 4, vorzugsweise 1 oder 2, bevorzugt 1 |
| N-1 | Ar-11 | 2 | 0 bis 2, vorzugsweise 1 oder 2, bevorzugt 1 |
| N-3 | Ar-11 | 2 | 0 bis 2, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| N-5 | Ar-11 | 2 | 0 bis 2, vorzugsweise 1 oder 2, bevorzugt 1 |
| N-6 | Ar-11 | 2 | 0 bis 2, vorzugsweise 1 oder 2, bevorzugt 1 |
| N-1 | Ar-12 | 0 | 0 bis 4, vorzugsweise 1 oder 2, bevorzugt 1 |
| N-3 | Ar-12 | 0 | 0 bis 4, vorzugsweise 1 oder 2, bevorzugt 1 |
| N-5 | Ar-12 | 0 | 0 bis 4, vorzugsweise 1 oder 2, bevorzugt 1 |
| N-6 | Ar-12 | 0 | 0 bis 4, vorzugsweise 1 oder 2, bevorzugt 1 |
| N-1 | Ar-12 | 2 | 0 bis 2, vorzugsweise 1 oder 2, bevorzugt 1 |
| N-3 | Ar-12 | 2 | 0 bis 2, vorzugsweise 1 oder 2, bevorzugt 1 |
| N-5 | Ar-12 | 2 | 0 bis 2, vorzugsweise 1 oder 2, bevorzugt 1 |
| N-6 | Ar-12 | 2 | 0 bis 2, vorzugsweise 1 oder 2, bevorzugt 1 |
| N-1 | Ar-13 | 0 | 0 bis 4, vorzugsweise 1 oder 2, bevorzugt 1 |
| N-3 | Ar-13 | 0 | 0 bis 4, vorzugsweise 1 oder 2, bevorzugt 1 |
| N-5 | Ar-13 | 0 | 0 bis 4, vorzugsweise 1 oder 2, bevorzugt 1 |
| N-6 | Ar-13 | 0 | 0 bis 4, vorzugsweise 1 oder 2, bevorzugt 1 |
| N-1 | Ar-13 | 2 | 0 bis 2, vorzugsweise 1 oder 2, bevorzugt 1 |
| N-3 | Ar-13 | 2 | 0 bis 2, vorzugsweise 1 oder 2, bevorzugt 1 |
| N-5 | Ar-13 | 2 | 0 bis 2, vorzugsweise 1 oder 2, bevorzugt 1 |
| N-6 | Ar-13 | 2 | 0 bis 2, vorzugsweise 1 oder 2, bevorzugt 1 |
| N-1 | Ar-14 | 0 | 0 bis 4, vorzugsweise 1 oder 2, bevorzugt 1 |
| N-3 | Ar-14 | 0 | 0 bis 4, vorzugsweise 1 oder 2, bevorzugt 1 |
| N-5 | Ar-14 | 0 | 0 bis 4, vorzugsweise 1 oder 2, bevorzugt 1 |
| N-6 | Ar-14 | 0 | 0 bis 4, vorzugsweise 1 oder 2, bevorzugt 1 |
| N-1 | Ar-14 | 2 | 0 bis 2, vorzugsweise 1 oder 2, bevorzugt 1 |
| N-3 | Ar-14 | 2 | 0 bis 2, vorzugsweise 1 oder 2, bevorzugt 1 |
| N-5 | Ar-14 | 2 | 0 bis 2, vorzugsweise 1 oder 2, bevorzugt 1 |
| N-6 | Ar-14 | 2 | 0 bis 2, vorzugsweise 1 oder 2, bevorzugt 1 |
| N-1 | Ar-15 | 0 | 0 bis 4, vorzugsweise 1 oder 2, bevorzugt 1 |
| N-3 | Ar-15 | 0 | 0 bis 4, vorzugsweise 1 oder 2, bevorzugt 1 |
| N-5 | Ar-15 | 0 | 0 bis 4, vorzugsweise 1 oder 2, bevorzugt 1 |
| N-6 | Ar-15 | 0 | 0 bis 4, vorzugsweise 1 oder 2, bevorzugt 1 |
| N-1 | Ar-15 | 2 | 0 bis 2, vorzugsweise 1 oder 2, bevorzugt 1 |
| N-3 | Ar-15 | 2 | 0 bis 2, vorzugsweise 1 oder 2, bevorzugt 1 |
| N-5 | Ar-15 | 2 | 0 bis 2, vorzugsweise 1 oder 2, bevorzugt 1 |
| N-6 | Ar-15 | 2 | 0 bis 2, vorzugsweise 1 oder 2, bevorzugt 1 |
| 2 * N-1 | Ar-16 | 0 | 0 bis 2, vorzugsweise 1 oder 2, bevorzugt 1 |
| 2 * N-3 | Ar-16 | 0 | 0 bis 2, vorzugsweise 1 oder 2, bevorzugt 1 |
| 2 * N-5 | Ar-16 | 0 | 0 bis 2, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-6 | Ar-16 | 0 | 0 bis 2, vorzugsweise 1 oder 2, bevorzugt 1 |
| 2 * N-1 | Ar-17 | 0 | 0 bis 2, vorzugsweise 1 oder 2, bevorzugt 1 |
| 2 * N-3 | Ar-17 | 0 | 0 bis 2, vorzugsweise 1 oder 2, bevorzugt 1 |
| 2 * N-5 | Ar-17 | 0 | 0 bis 2, vorzugsweise 1 oder 2, bevorzugt 1 |
| 2 * N-6 | Ar-17 | 0 | 0 bis 2, vorzugsweise 1 oder 2, bevorzugt 1 |
| 3 * N-1 | Ar-18 | 0 | 0 |
| 3 * N-3 | Ar-18 | 0 | 0 |
| 3 * N-5 | Ar-18 | 0 | 0 |
| 3 * N-6 | Ar-18 | 0 | 0 |
| 2 * N-1 | Ar-19 | 0 | 0 bis 4, vorzugsweise 1 oder 2, bevorzugt 1 |
| 2 * N-3 | Ar-19 | 0 | 0 bis 4, vorzugsweise 1 oder 2, bevorzugt 1 |
| 2 * N-5 | Ar-19 | 0 | 0 bis 4, vorzugsweise 1 oder 2, bevorzugt 1 |
| 2 * N-6 | Ar-19 | 0 | 0 bis 4, vorzugsweise 1 oder 2, bevorzugt 1 |
| 2 * N-1 | Ar-19 | 2 | 0 bis 4, vorzugsweise 1 oder 2, bevorzugt 1 |
| 2 * N-3 | Ar-19 | 2 | 0 bis 4, vorzugsweise 1 oder 2, bevorzugt 1 |
| 2 * N-5 | Ar-19 | 2 | 0 bis 4, vorzugsweise 1 oder 2, bevorzugt 1 |
| 2 * N-6 | Ar-19 | 2 | 0 bis 4, vorzugsweise 1 oder 2, bevorzugt 1 |
| 2 * N-1 | Ar-20 | 0 | 0 bis 4, vorzugsweise 1 oder 2, bevorzugt 1 |
| 2 * N-3 | Ar-20 | 0 | 0 bis 4, vorzugsweise 1 oder 2, bevorzugt 1 |
| 2 * N-5 | Ar-20 | 0 | 0 bis 4, vorzugsweise 1 oder 2, bevorzugt 1 |
| 2 * N-6 | Ar-20 | 0 | 0 bis 4, vorzugsweise 1 oder 2, bevorzugt 1 |
| 2 * N-1 | Ar-20 | 2 | 0 bis 4, vorzugsweise 1 oder 2, bevorzugt 1 |
| 2 * N-3 | Ar-20 | 2 | 0 bis 4, vorzugsweise 1 oder 2, bevorzugt 1 |
| 2 * N-5 | Ar-20 | 2 | 0 bis 4, vorzugsweise 1 oder 2, bevorzugt 1 |
| 2 * N-6 | Ar-20 | 2 | 0 bis 4, vorzugsweise 1 oder 2, bevorzugt 1 |
| 2 * N-1 | Ar-21 | 0 | 0 bis 4, vorzugsweise 1 oder 2, bevorzugt 1 |
| 2 * N-3 | Ar-21 | 0 | 0 bis 4, vorzugsweise 1 oder 2, bevorzugt 1 |
| 2 * N-5 | Ar-21 | 0 | 0 bis 4, vorzugsweise 1 oder 2, bevorzugt 1 |
| 2 * N-6 | Ar-21 | 0 | 0 bis 4, vorzugsweise 1 oder 2, bevorzugt 1 |
| 2 * N-1 | Ar-21 | 2 | 0 bis 4, vorzugsweise 1 oder 2, bevorzugt 1 |
| 2 * N-3 | Ar-21 | 2 | 0 bis 4, vorzugsweise 1 oder 2, bevorzugt 1 |
| 2 * N-5 | Ar-21 | 2 | 0 bis 4, vorzugsweise 1 oder 2, bevorzugt 1 |
| 2 * N-6 | Ar-21 | 2 | 0 bis 4, vorzugsweise 1 oder 2, bevorzugt 1 |
| 2 * N-1 | Ar-22 | 0 | 0 bis 4, vorzugsweise 1 oder 2, bevorzugt 1 |
| 2 * N-3 | Ar-22 | 0 | 0 bis 4, vorzugsweise 1 oder 2, bevorzugt 1 |
| 2 * N-5 | Ar-22 | 0 | 0 bis 4, vorzugsweise 1 oder 2, bevorzugt 1 |
| 2 * N-6 | Ar-22 | 0 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-1 | Ar-22 | 2 | 0 bis 4, vorzugsweise 1 oder 2, bevorzugt 1 |
| 2 * N-3 | Ar-22 | 2 | 0 bis 4, vorzugsweise 1 oder 2, bevorzugt 1 |
| 2 * N-5 | Ar-22 | 2 | 0 bis 4, vorzugsweise 1 oder 2, bevorzugt 1 |
| 2 * N-6 | Ar-22 | 2 | 0 bis 4, vorzugsweise 1 oder 2, bevorzugt 1 |
| 2 * N-1 | Ar-23 | 0 | 0 bis 4, vorzugsweise 1, 2 oder 3, bevorzugt 2 |
| 2 * N-3 | Ar-23 | 0 | 0 bis 4, vorzugsweise 1, 2 oder 3, bevorzugt 2 |
| 2 * N-5 | Ar-23 | 0 | 0 bis 4, vorzugsweise 1, 2 oder 3, bevorzugt 2 |
| 2 * N-6 | Ar-23 | 0 | 0 bis 4, vorzugsweise 1, 2 oder 3, bevorzugt 2 |
| 2 * N-1 | Ar-23 | 2 | 0 bis 2, vorzugsweise 1 oder 2, bevorzugt 2 |
| 2 * N-3 | Ar-23 | 2 | 0 bis 2, vorzugsweise 1 oder 2, bevorzugt 2 |
| 2 * N-5 | Ar-23 | 2 | 0 bis 2, vorzugsweise 1 oder 2, bevorzugt 2 |
| 2 * N-6 | Ar-23 | 2 | 0 bis 2, vorzugsweise 1 oder 2, bevorzugt 2 |
| 2 * N-1 | Ar-24 | 0 | 0 bis 4, vorzugsweise 1, 2 oder 3, bevorzugt 2 |
| 2 * N-3 | Ar-24 | 0 | 0 bis 4, vorzugsweise 1, 2 oder 3, bevorzugt 2 |
| 2 * N-5 | Ar-24 | 0 | 0 bis 4, vorzugsweise 1, 2 oder 3, bevorzugt 2 |
| 2 * N-6 | Ar-24 | 0 | 0 bis 4, vorzugsweise 1, 2 oder 3, bevorzugt 2 |
| 2 * N-1 | Ar-24 | 2 | 0 bis 2, vorzugsweise 1 oder 2, bevorzugt 2 |
| 2 * N-3 | Ar-24 | 2 | 0 bis 2, vorzugsweise 1 oder 2, bevorzugt 2 |
| 2 * N-5 | Ar-24 | 2 | 0 bis 2, vorzugsweise 1 oder 2, bevorzugt 2 |
| 2 * N-6 | Ar-24 | 2 | 0 bis 2, vorzugsweise 1 oder 2, bevorzugt 2 |
| 2 * N-1 | Ar-25 | 0 | 0 bis 4, vorzugsweise 1, 2 oder 3, bevorzugt 2 |
| 2 * N-3 | Ar-25 | 0 | 0 bis 4, vorzugsweise 1, 2 oder 3, bevorzugt 2 |
| 2 * N-5 | Ar-25 | 0 | 0 bis 4, vorzugsweise 1, 2 oder 3, bevorzugt 2 |
| 2 * N-6 | Ar-25 | 0 | 0 bis 4, vorzugsweise 1, 2 oder 3, bevorzugt 2 |
| 2 * N-1 | Ar-25 | 2 | 0 bis 2, vorzugsweise 1 oder 2, bevorzugt 2 |
| 2 * N-3 | Ar-25 | 2 | 0 bis 2, vorzugsweise 1 oder 2, bevorzugt 2 |
| 2 * N-5 | Ar-25 | 2 | 0 bis 2, vorzugsweise 1 oder 2, bevorzugt 2 |
| 2 * N-6 | Ar-25 | 2 | 0 bis 2, vorzugsweise 1 oder 2, bevorzugt 2 |
| 2 * N-1 | Ar-26 | 0 | 0 bis 4, vorzugsweise 1 oder 2, bevorzugt 1 |
| 2 * N-3 | Ar-26 | 0 | 0 bis 4, vorzugsweise 1 oder 2, bevorzugt 1 |
| 2 * N-5 | Ar-26 | 0 | 0 bis 4, vorzugsweise 1 oder 2, bevorzugt 1 |
| 2 * N-6 | Ar-26 | 0 | 0 bis 4, vorzugsweise 1 oder 2, bevorzugt 1 |
| 2 * N-1 | Ar-26 | 2 | 0 bis 4, vorzugsweise 1 oder 2, bevorzugt 1 |
| 2 * N-3 | Ar-26 | 2 | 0 bis 4, vorzugsweise 1 oder 2, bevorzugt 1 |
| 2 * N-5 | Ar-26 | 2 | 0 bis 4, vorzugsweise 1 oder 2, bevorzugt 1 |
| 2 * N-6 | Ar-26 | 2 | 0 bis 4, vorzugsweise 1 oder 2, bevorzugt 1 |
| 2 * N-1 | Ar-27 | 0 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-3 | Ar-27 | 0 | 0 bis 4, vorzugsweise 1 oder 2, bevorzugt 1 |
| 2 * N-5 | Ar-27 | 0 | 0 bis 4, vorzugsweise 1 oder 2, bevorzugt 1 |
| 2 * N-6 | Ar-27 | 0 | 0 bis 4, vorzugsweise 1 oder 2, bevorzugt 1 |
| 2 * N-1 | Ar-27 | 2 | 0 bis 4, vorzugsweise 1 oder 2, bevorzugt 1 |
| 2 * N-3 | Ar-27 | 2 | 0 bis 4, vorzugsweise 1 oder 2, bevorzugt 1 |
| 2 * N-5 | Ar-27 | 2 | 0 bis 4, vorzugsweise 1 oder 2, bevorzugt 1 |
| 2 * N-6 | Ar-27 | 2 | 0 bis 4, vorzugsweise 1 oder 2, bevorzugt 1 |
| 2 * N-1 | Ar-28 | 0 | 0 bis 4, vorzugsweise 1 oder 2, bevorzugt 1 |
| 2 * N-3 | Ar-28 | 0 | 0 bis 4, vorzugsweise 1 oder 2, bevorzugt 1 |
| 2 * N-5 | Ar-28 | 0 | 0 bis 4, vorzugsweise 1 oder 2, bevorzugt 1 |
| 2 * N-6 | Ar-28 | 0 | 0 bis 4, vorzugsweise 1 oder 2, bevorzugt 1 |
| 2 * N-1 | Ar-28 | 2 | 0 bis 4, vorzugsweise 1 oder 2, bevorzugt 1 |
| 2 * N-3 | Ar-28 | 2 | 0 bis 4, vorzugsweise 1 oder 2, bevorzugt 1 |
| 2 * N-5 | Ar-28 | 2 | 0 bis 4, vorzugsweise 1 oder 2, bevorzugt 1 |
| 2 * N-6 | Ar-28 | 2 | 0 bis 4, vorzugsweise 1 oder 2, bevorzugt 1 |
| 2 * N-1 | Ar-29 | 0 | 0 bis 4, vorzugsweise 1 oder 2, bevorzugt 1 |
| 2 * N-3 | Ar-29 | 0 | 0 bis 4, vorzugsweise 1 oder 2, bevorzugt 1 |
| 2 * N-5 | Ar-29 | 0 | 0 bis 4, vorzugsweise 1 oder 2, bevorzugt 1 |
| 2 * N-6 | Ar-29 | 0 | 0 bis 4, vorzugsweise 1 oder 2, bevorzugt 1 |
| 2 * N-1 | Ar-29 | 2 | 0 bis 4, vorzugsweise 1 oder 2, bevorzugt 1 |
| 2 * N-3 | Ar-29 | 2 | 0 bis 4, vorzugsweise 1 oder 2, bevorzugt 1 |
| 2 * N-5 | Ar-29 | 2 | 0 bis 4, vorzugsweise 1 oder 2, bevorzugt 1 |
| 2 * N-6 | Ar-29 | 2 | 0 bis 4, vorzugsweise 1 oder 2, bevorzugt 1 |
| 2 * N-1 | Ar-30 | 0 | 0 bis 4, vorzugsweise 1 oder 2, bevorzugt 1 |
| 2 * N-3 | Ar-30 | 0 | 0 bis 4, vorzugsweise 1 oder 2, bevorzugt 1 |
| 2 * N-5 | Ar-30 | 0 | 0 bis 4, vorzugsweise 1 oder 2, bevorzugt 1 |
| 2 * N-6 | Ar-30 | 0 | 0 bis 4, vorzugsweise 1 oder 2, bevorzugt 1 |
| 2 * N-1 | Ar-30 | 2 | 0 bis 4, vorzugsweise 1 oder 2, bevorzugt 1 |
| 2 * N-3 | Ar-30 | 2 | 0 bis 4, vorzugsweise 1 oder 2, bevorzugt 1 |
| 2 * N-5 | Ar-30 | 2 | 0 bis 4, vorzugsweise 1 oder 2, bevorzugt 1 |
| 2 * N-6 | Ar-30 | 2 | 0 bis 4, vorzugsweise 1 oder 2, bevorzugt 1 |
| 2 * N-1 | Ar-31 | 0 | 0 bis 4, vorzugsweise 1 oder 2, bevorzugt 1 |
| 2 * N-3 | Ar-31 | 0 | 0 bis 4, vorzugsweise 1 oder 2, bevorzugt 1 |
| 2 * N-5 | Ar-31 | 0 | 0 bis 4, vorzugsweise 1 oder 2, bevorzugt 1 |
| 2 * N-6 | Ar-31 | 0 | 0 bis 4, vorzugsweise 1 oder 2, bevorzugt 1 |
| 2 * N-1 | Ar-31 | 2 | 0 bis 4, vorzugsweise 1 oder 2, bevorzugt 1 |
| 2 * N-3 | Ar-31 | 2 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-5 | Ar-31 | 2 | 0 bis 4, vorzugsweise 1 oder 2, bevorzugt 1 |
| 2 * N-6 | Ar-31 | 2 | 0 bis 4, vorzugsweise 1 oder 2, bevorzugt 1 |
| 2 * N-1 | Ar-32 | 0 | 0 bis 4, vorzugsweise 1 oder 2, bevorzugt 1 |
| 2 * N-3 | Ar-32 | 0 | 0 bis 4, vorzugsweise 1 oder 2, bevorzugt 1 |
| 2 * N-5 | Ar-32 | 0 | 0 bis 4, vorzugsweise 1 oder 2, bevorzugt 1 |
| 2 * N-6 | Ar-32 | 0 | 0 bis 4, vorzugsweise 1 oder 2, bevorzugt 1 |
| 2 * N-1 | Ar-32 | 2 | 0 bis 4, vorzugsweise 1 oder 2, bevorzugt 1 |
| 2 * N-3 | Ar-32 | 2 | 0 bis 4, vorzugsweise 1 oder 2, bevorzugt 1 |
| 2 * N-5 | Ar-32 | 2 | 0 bis 4, vorzugsweise 1 oder 2, bevorzugt 1 |
| 2 * N-6 | Ar-32 | 2 | 0 bis 4, vorzugsweise 1 oder 2, bevorzugt 1 |
| 2 * N-1 | Ar-33 | 0 | 0 bis 4, vorzugsweise 1 oder 2, bevorzugt 1 |
| 2 * N-3 | Ar-33 | 0 | 0 bis 4, vorzugsweise 1 oder 2, bevorzugt 1 |
| 2 * N-5 | Ar-33 | 0 | 0 bis 4, vorzugsweise 1 oder 2, bevorzugt 1 |
| 2 * N-6 | Ar-33 | 0 | 0 bis 4, vorzugsweise 1 oder 2, bevorzugt 1 |
| 2 * N-1 | Ar-33 | 2 | 0 bis 4, vorzugsweise 1 oder 2, bevorzugt 1 |
| 2 * N-3 | Ar-33 | 2 | 0 bis 4, vorzugsweise 1 oder 2, bevorzugt 1 |
| 2 * N-5 | Ar-33 | 2 | 0 bis 4, vorzugsweise 1 oder 2, bevorzugt 1 |
| 2 * N-6 | Ar-33 | 2 | 0 bis 4, vorzugsweise 1 oder 2, bevorzugt 1 |
| 2 * N-1 | Ar-34 | 0 | 0 bis 4, vorzugsweise 1 oder 2, bevorzugt 1 |
| 2 * N-3 | Ar-34 | 0 | 0 bis 4, vorzugsweise 1 oder 2, bevorzugt 1 |
| 2 * N-5 | Ar-34 | 0 | 0 bis 4, vorzugsweise 1 oder 2, bevorzugt 1 |
| 2 * N-6 | Ar-34 | 0 | 0 bis 4, vorzugsweise 1 oder 2, bevorzugt 1 |
| 2 * N-1 | Ar-34 | 2 | 0 bis 4, vorzugsweise 1 oder 2, bevorzugt 1 |
| 2 * N-3 | Ar-34 | 2 | 0 bis 4, vorzugsweise 1 oder 2, bevorzugt 1 |
| 2 * N-5 | Ar-34 | 2 | 0 bis 4, vorzugsweise 1 oder 2, bevorzugt 1 |
| 2 * N-6 | Ar-34 | 2 | 0 bis 4, vorzugsweise 1 oder 2, bevorzugt 1 |
| 2 * N-1 | Ar-35 | 0 | 0 bis 4, vorzugsweise 1 oder 2, bevorzugt 1 |
| 2 * N-3 | Ar-35 | 0 | 0 bis 4, vorzugsweise 1 oder 2, bevorzugt 1 |
| 2 * N-5 | Ar-35 | 0 | 0 bis 4, vorzugsweise 1 oder 2, bevorzugt 1 |
| 2 * N-6 | Ar-35 | 0 | 0 bis 4, vorzugsweise 1 oder 2, bevorzugt 1 |
| 2 * N-1 | Ar-35 | 2 | 0 bis 4, vorzugsweise 1 oder 2, bevorzugt 1 |
| 2 * N-3 | Ar-35 | 2 | 0 bis 4, vorzugsweise 1 oder 2, bevorzugt 1 |
| 2 * N-5 | Ar-35 | 2 | 0 bis 4, vorzugsweise 1 oder 2, bevorzugt 1 |
| 2 * N-6 | Ar-35 | 2 | 0 bis 4, vorzugsweise 1 oder 2, bevorzugt 1 |
| 2 * N-1 | Ar-36 | 0 | 0 bis 4, vorzugsweise 1 oder 2, bevorzugt 1 |
| 2 * N-3 | Ar-36 | 0 | 0 bis 4, vorzugsweise 1 oder 2, bevorzugt 1 |
| 2 * N-5 | Ar-36 | 0 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-6 | Ar-36 | 0 | 0 bis 4, vorzugsweise 1 oder 2, bevorzugt 1 |
| 2 * N-1 | Ar-36 | 2 | 0 bis 4, vorzugsweise 1 oder 2, bevorzugt 1 |
| 2 * N-3 | Ar-36 | 2 | 0 bis 4, vorzugsweise 1 oder 2, bevorzugt 1 |
| 2 * N-5 | Ar-36 | 2 | 0 bis 4, vorzugsweise 1 oder 2, bevorzugt 1 |
| 2 * N-6 | Ar-36 | 2 | 0 bis 4, vorzugsweise 1 oder 2, bevorzugt 1 |
| 2 * N-1 | Ar-36 | 0 | 0 bis 4, vorzugsweise 1 oder 2, bevorzugt 1 |
| 2 * N-3 | Ar-36 | 0 | 0 bis 4, vorzugsweise 1 oder 2, bevorzugt 1 |
| 2 * N-5 | Ar-36 | 0 | 0 bis 4, vorzugsweise 1 oder 2, bevorzugt 1 |
| 2 * N-6 | Ar-36 | 0 | 0 bis 4, vorzugsweise 1 oder 2, bevorzugt 1 |
| 2 * N-1 | Ar-36 | 2 | 0 bis 4, vorzugsweise 1 oder 2, bevorzugt 1 |
| 2 * N-3 | Ar-36 | 2 | 0 bis 4, vorzugsweise 1 oder 2, bevorzugt 1 |
| 2 * N-5 | Ar-36 | 2 | 0 bis 4, vorzugsweise 1 oder 2, bevorzugt 1 |
| 2 * N-6 | Ar-36 | 2 | 0 bis 4, vorzugsweise 1 oder 2, bevorzugt 1 |
| 2 * N-1 | Ar-37 | 0 | 0 bis 4, vorzugsweise 1 oder 2, bevorzugt 1 |
| 2 * N-3 | Ar-37 | 0 | 0 bis 4, vorzugsweise 1 oder 2, bevorzugt 1 |
| 2 * N-5 | Ar-37 | 0 | 0 bis 4, vorzugsweise 1 oder 2, bevorzugt 1 |
| 2 * N-6 | Ar-37 | 0 | 0 bis 4, vorzugsweise 1 oder 2, bevorzugt 1 |
| 2 * N-1 | Ar-37 | 2 | 0 bis 4, vorzugsweise 1 oder 2, bevorzugt 1 |
| 2 * N-3 | Ar-37 | 2 | 0 bis 4, vorzugsweise 1 oder 2, bevorzugt 1 |
| 2 * N-5 | Ar-37 | 2 | 0 bis 4, vorzugsweise 1 oder 2, bevorzugt 1 |
| 2 * N-6 | Ar-37 | 2 | 0 bis 4, vorzugsweise 1 oder 2, bevorzugt 1 |
| 2 * N-1 | Ar-38 | 0 | 0 bis 4, vorzugsweise 1 oder 2, bevorzugt 1 |
| 2 * N-3 | Ar-38 | 0 | 0 bis 4, vorzugsweise 1 oder 2, bevorzugt 1 |
| 2 * N-5 | Ar-38 | 0 | 0 bis 4, vorzugsweise 1 oder 2, bevorzugt 1 |
| 2 * N-6 | Ar-38 | 0 | 0 bis 4, vorzugsweise 1 oder 2, bevorzugt 1 |
| 2 * N-1 | Ar-38 | 2 | 0 bis 4, vorzugsweise 1 oder 2, bevorzugt 1 |
| 2 * N-3 | Ar-38 | 2 | 0 bis 4, vorzugsweise 1 oder 2, bevorzugt 1 |
| 2 * N-5 | Ar-38 | 2 | 0 bis 4, vorzugsweise 1 oder 2, bevorzugt 1 |
| 2 * N-6 | Ar-38 | 2 | 0 bis 4, vorzugsweise 1 oder 2, bevorzugt 1 |
| 2 * N-1 | Ar-39 | 0 | 0 bis 4, vorzugsweise 1 oder 2, bevorzugt 1 |
| 2 * N-3 | Ar-39 | 0 | 0 bis 4, vorzugsweise 1 oder 2, bevorzugt 1 |
| 2 * N-5 | Ar-39 | 0 | 0 bis 4, vorzugsweise 1 oder 2, bevorzugt 1 |
| 2 * N-6 | Ar-39 | 0 | 0 bis 4, vorzugsweise 1 oder 2, bevorzugt 1 |
| 2 * N-1 | Ar-39 | 2 | 0 bis 4, vorzugsweise 1 oder 2, bevorzugt 1 |
| 2 * N-3 | Ar-39 | 2 | 0 bis 4, vorzugsweise 1 oder 2, bevorzugt 1 |
| 2 * N-5 | Ar-39 | 2 | 0 bis 4, vorzugsweise 1 oder 2, bevorzugt 1 |
| 2 * N-6 | Ar-39 | 2 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 2 * N-1 | Ar-40 | 0 | 0 bis 4, vorzugsweise 1 oder 2, bevorzugt 1 |
| 2 * N-3 | Ar-40 | 0 | 0 bis 4, vorzugsweise 1 oder 2, bevorzugt 1 |
| 2 * N-5 | Ar-40 | 0 | 0 bis 4, vorzugsweise 1 oder 2, bevorzugt 1 |
| 2 * N-6 | Ar-40 | 0 | 0 bis 4, vorzugsweise 1 oder 2, bevorzugt 1 |
| 2 * N-1 | Ar-40 | 2 | 0 bis 4, vorzugsweise 1 oder 2, bevorzugt 1 |
| 2 * N-3 | Ar-40 | 2 | 0 bis 4, vorzugsweise 1 oder 2, bevorzugt 1 |
| 2 * N-5 | Ar-40 | 2 | 0 bis 4, vorzugsweise 1 oder 2, bevorzugt 1 |
| 2 * N-6 | Ar-40 | 2 | 0 bis 4, vorzugsweise 1 oder 2, bevorzugt 1 |
| 2 * N-1 | Ar-41 | 0 | 0 bis 4, vorzugsweise 1 oder 2, bevorzugt 1 |
| 2 * N-3 | Ar-41 | 0 | 0 bis 4, vorzugsweise 1 oder 2, bevorzugt 1 |
| 2 * N-5 | Ar-41 | 0 | 0 bis 4, vorzugsweise 1 oder 2, bevorzugt 1 |
| 2 * N-6 | Ar-41 | 0 | 0 bis 4, vorzugsweise 1 oder 2, bevorzugt 1 |
| 2 * N-1 | Ar-41 | 2 | 0 bis 4, vorzugsweise 1 oder 2, bevorzugt 1 |
| 2 * N-3 | Ar-41 | 2 | 0 bis 4, vorzugsweise 1 oder 2, bevorzugt 1 |
| 2 * N-5 | Ar-41 | 2 | 0 bis 4, vorzugsweise 1 oder 2, bevorzugt 1 |
| 2 * N-6 | Ar-41 | 2 | 0 bis 4, vorzugsweise 1 oder 2, bevorzugt 1 |
| 4 * N-1 | Ar-42 | 0 | 0 bis 4, vorzugsweise 1 oder 2, bevorzugt 1 |
| 4 * N-3 | Ar-42 | 0 | 0 bis 4, vorzugsweise 1 oder 2, bevorzugt 1 |
| 4 * N-5 | Ar-42 | 0 | 0 bis 4, vorzugsweise 1 oder 2, bevorzugt 1 |
| 4 * N-6 | Ar-42 | 0 | 0 bis 4, vorzugsweise 1 oder 2, bevorzugt 1 |
| 4 * N-1 | Ar-42 | 2 | 0 bis 4, vorzugsweise 1 oder 2, bevorzugt 1 |
| 4 * N-3 | Ar-42 | 2 | 0 bis 4, vorzugsweise 1 oder 2, bevorzugt 1 |
| 4 * N-5 | Ar-42 | 2 | 0 bis 4, vorzugsweise 1 oder 2, bevorzugt 1 |
| 4 * N-6 | Ar-42 | 2 | 0 bis 4, vorzugsweise 1 oder 2, bevorzugt 1 |
| 4 * N-1 | Ar-43 | 0 | 0 bis 4, vorzugsweise 1 oder 2, bevorzugt 1 |
| 4 * N-3 | Ar-43 | 0 | 0 bis 4, vorzugsweise 1 oder 2, bevorzugt 1 |
| 4 * N-5 | Ar-43 | 0 | 0 bis 4, vorzugsweise 1 oder 2, bevorzugt 1 |
| 4 * N-6 | Ar-43 | 0 | 0 bis 4, vorzugsweise 1 oder 2, bevorzugt 1 |
| 4 * N-1 | Ar-43 | 2 | 0 bis 4, vorzugsweise 1 oder 2, bevorzugt 1 |
| 4 * N-3 | Ar-43 | 2 | 0 bis 4, vorzugsweise 1 oder 2, bevorzugt 1 |
| 4 * N-5 | Ar-43 | 2 | 0 bis 4, vorzugsweise 1 oder 2, bevorzugt 1 |
| 4 * N-6 | Ar-43 | 2 | 0 bis 4, vorzugsweise 1 oder 2, bevorzugt 1 |
| 4 * N-1 | Ar-44 | 0 | 0 bis 4, vorzugsweise 1 oder 2, bevorzugt 1 |
| 4 * N-3 | Ar-44 | 0 | 0 bis 4, vorzugsweise 1 oder 2, bevorzugt 1 |
| 4 * N-5 | Ar-44 | 0 | 0 bis 4, vorzugsweise 1 oder 2, bevorzugt 1 |
| 4 * N-6 | Ar-44 | 0 | 0 bis 4, vorzugsweise 1 oder 2, bevorzugt 1 |
| 4 * N-1 | Ar-44 | 2 | 0 bis 4, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| 4 * N-3 | Ar-44 | 2 | 0 bis 4, vorzugsweise 1 oder 2, bevorzugt 1 |
| 4 * N-5 | Ar-44 | 2 | 0 bis 4, vorzugsweise 1 oder 2, bevorzugt 1 |
| 4 * N-6 | Ar-44 | 2 | 0 bis 4, vorzugsweise 1 oder 2, bevorzugt 1 |
| N-2 | Ar-45 | 0 | 0 bis 4, vorzugsweise 1 oder 2, bevorzugt 1 |
| N-4 | Ar-45 | 0 | 0 bis 4, vorzugsweise 1 oder 2, bevorzugt 1 |
| N-2 | Ar-45 | 2 | 0 bis 2, vorzugsweise 1 oder 2, bevorzugt 1 |
| N-4 | Ar-45 | 2 | 0 bis 2, vorzugsweise 1 oder 2, bevorzugt 1 |
| N-2 | Ar-46 | 0 | 0 bis 4, vorzugsweise 1 oder 2, bevorzugt 1 |
| N-4 | Ar-46 | 0 | 0 bis 4, vorzugsweise 1 oder 2, bevorzugt 1 |
| N-2 | Ar-46 | 2 | 0 bis 2, vorzugsweise 1 oder 2, bevorzugt 1 |
| N-4 | Ar-46 | 2 | 0 bis 2, vorzugsweise 1 oder 2, bevorzugt 1 |
| N-2 | Ar-47 | 0 | 0 bis 4, vorzugsweise 1 oder 2, bevorzugt 1 |
| N-4 | Ar-47 | 0 | 0 bis 4, vorzugsweise 1 oder 2, bevorzugt 1 |
| N-2 | Ar-47 | 2 | 0 bis 2, vorzugsweise 1 oder 2, bevorzugt 1 |
| N-4 | Ar-47 | 2 | 0 bis 2, vorzugsweise 1 oder 2, bevorzugt 1 |
| N-2 | Ar-48 | 0 | 0 bis 4, vorzugsweise 1 oder 2, bevorzugt 1 |
| N-4 | Ar-48 | 0 | 0 bis 4, vorzugsweise 1 oder 2, bevorzugt 1 |
| N-2 | Ar-48 | 2 | 0 bis 2, vorzugsweise 1 oder 2, bevorzugt 1 |
| N-4 | Ar-48 | 2 | 0 bis 2, vorzugsweise 1 oder 2, bevorzugt 1 |
| 2 * N-2 | Ar-49 | 0 | 0 bis 4, vorzugsweise 1 oder 2, bevorzugt 1 |
| 2 * N-4 | Ar-49 | 0 | 0 bis 4, vorzugsweise 1 oder 2, bevorzugt 1 |
| 2 * N-2 | Ar-49 | 2 | 0 bis 4, vorzugsweise 1 oder 2, bevorzugt 1 |
| 2 * N-4 | Ar-49 | 2 | 0 bis 4, vorzugsweise 1 oder 2, bevorzugt 1 |
| 2 * N-2 | Ar-50 | 0 | 0 bis 4, vorzugsweise 1 oder 2, bevorzugt 1 |
| 2 * N-4 | Ar-50 | 0 | 0 bis 4, vorzugsweise 1 oder 2, bevorzugt 1 |
| 2 * N-2 | Ar-50 | 2 | 0 bis 4, vorzugsweise 1 oder 2, bevorzugt 1 |
| 2 * N-4 | Ar-50 | 2 | 0 bis 4, vorzugsweise 1 oder 2, bevorzugt 1 |
| 2 * N-2 | Ar-51 | 0 | 0 bis 4, vorzugsweise 1 oder 2, bevorzugt 1 |
| 2 * N-4 | Ar-51 | 0 | 0 bis 4, vorzugsweise 1 oder 2, bevorzugt 1 |
| 2 * N-2 | Ar-51 | 2 | 0 bis 4, vorzugsweise 1 oder 2, bevorzugt 1 |
| 2 * N-4 | Ar-51 | 2 | 0 bis 4, vorzugsweise 1 oder 2, bevorzugt 1 |
| N-1 | Ar-52 | 0 | 0 bis 4, vorzugsweise 1 oder 2, bevorzugt 1 |
| N-3 | Ar-52 | 0 | 0 bis 4, vorzugsweise 1 oder 2, bevorzugt 1 |
| N-5 | Ar-52 | 0 | 0 bis 4, vorzugsweise 1 oder 2, bevorzugt 1 |
| N-6 | Ar-52 | 0 | 0 bis 4, vorzugsweise 1 oder 2, bevorzugt 1 |
| N-1 | Ar-52 | 2 | 0 bis 2, vorzugsweise 1 oder 2, bevorzugt 1 |
| N-3 | Ar-52 | 2 | 0 bis 2, vorzugsweise 1 oder 2, bevorzugt 1 |

(fortgesetzt)

| Teilstruktur der Formel | Teilstruktur der Formel | Anzahl der Gruppen X', die für N stehen, wobei diese Gruppen X nicht benachbart sind | Anzahl der Gruppen X', die für $CR^1$ stehen, wobei die Gruppe $CR^1$ ungleich der Gruppe CH ist |
|---|---|---|---|
| N-5 | Ar-52 | 2 | 0 bis 2, vorzugsweise 1 oder 2, bevorzugt 1 |
| N-6 | Ar-52 | 2 | 0 bis 2, vorzugsweise 1 oder 2, bevorzugt 1 |
| N-1 | Ar-53 | 0 | 0 bis 4, vorzugsweise 1 oder 2, bevorzugt 1 |
| N-3 | Ar-53 | 0 | 0 bis 4, vorzugsweise 1 oder 2, bevorzugt 1 |
| N-5 | Ar-53 | 0 | 0 bis 4, vorzugsweise 1 oder 2, bevorzugt 1 |
| N-6 | Ar-53 | 0 | 0 bis 4, vorzugsweise 1 oder 2, bevorzugt 1 |
| N-1 | Ar-53 | 2 | 0 bis 2, vorzugsweise 1 oder 2, bevorzugt 1 |
| N-3 | Ar-53 | 2 | 0 bis 2, vorzugsweise 1 oder 2, bevorzugt 1 |
| N-5 | Ar-53 | 2 | 0 bis 2, vorzugsweise 1 oder 2, bevorzugt 1 |
| N-6 | Ar-53 | 2 | 0 bis 2, vorzugsweise 1 oder 2, bevorzugt 1 |
| N-1 | Ar-54 | 0 | 0 bis 4, vorzugsweise 1, 2 oder 3, bevorzugt 2 |
| N-3 | Ar-54 | 0 | 0 bis 4, vorzugsweise 1, 2 oder 3, bevorzugt 2 |
| N-5 | Ar-54 | 0 | 0 bis 4, vorzugsweise 1, 2 oder 3, bevorzugt 2 |
| N-6 | Ar-54 | 0 | 0 bis 4, vorzugsweise 1, 2 oder 3, bevorzugt 2 |
| N-1 | Ar-54 | 2 | 0 bis 2, vorzugsweise 1 oder 2, bevorzugt 2 |
| N-3 | Ar-54 | 2 | 0 bis 2, vorzugsweise 1 oder 2, bevorzugt 2 |
| N-5 | Ar-54 | 2 | 0 bis 2, vorzugsweise 1 oder 2, bevorzugt 2 |
| N-6 | Ar-54 | 2 | 0 bis 2, vorzugsweise 1 oder 2, bevorzugt 2 |

Die Gruppen X', die für $CR^1$ stehen, wobei die Gruppe $CR^1$ ungleich der Gruppe CH ist, sind bevorzugt ausgewählt aus Lochleitergruppen und/oder Elektronenleitergruppen, wobei die Elektronenleitergruppen vorzugsweise mindestens 2 Stickstoffatome in einem Sechsring oder in zwei kondensierten Sechsringen umfassen, besonders bevorzugt ausgewählt sind aus Triazinen oder Pyrimidinen. Ferner sind Gruppen bevorzugt, die eine TADF (thermally activated delayed fluorescence) fördern, je nach Einsatzzweck der erfindungsgemäßen Verbindungen.

Von den zuvor dargelegten Verbindungen sind insbesondere Verbindungen bevorzugt, die kondensierte, besonders bevorzugt flächig kondensierte aromatische oder heteroaromatische Ringsysteme aufweisen, wie zum Beispiel Verbindungen mit Teilstrukturen der Formeln (Ar-2) bis (Ar-12), (Ar-19) bis (Ar-35) und (Ar-45) bis (Ar-54), besonders bevorzugt (Ar-4) bis (Ar-9), (Ar-23) bis (Ar-32) und (Ar-46) bis (Ar-54), speziell bevorzugt (Ar-4), (Ar-5), (Ar-23) bis (Ar-25), (Ar-46) und (Ar-49).

Gemäß einer weiteren Ausführungsform der vorliegenden Erfindung sind Verbindungen bevorzugt, die Fluoren-, Dibenzofuran, Dibenzothiofuran, Carbazol, Spirobifluoren und ähnlich Strukturen umfassen, besonders, wie zum Beispiel Verbindungen mit Teilstrukturen der Formeln (Ar-10) bis (Ar-15) und (Ar-33) bis (Ar-44), besonders bevorzugt (Ar-13) bis (Ar-15) und (Ar-36) bis (Ar-44).

Zur Klarheit der obigen Kombinationen ist festzuhalten, dass beispielsweise die Kombination von einer Teilstruktur N-1 mit einer Teilstruktur Ar-1 eine Verbindung gemäß Formel (I) ergibt. Ähnlich ergibt beispielsweise die Kombination von einer Teilstruktur N-2 mit einer Teilstruktur Ar-45 eine Strukture gemäß Formel (V). Ähnliches gilt für die weiteren Kombinationen.

Ferner kann vorgesehen sein, dass das aromatische oder heteroaromatische Ringsystem mit 5 bis 60 Kohlenstoffaromen, an welches an ein aliphatisches polycyclisches Ringsystem mit mindestens 3 Ringen kondensiert ist, eine Teilstruktur der Formeln (Ar'-1) bis (Ar'-65) bildet

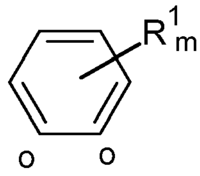
(Ar'-1)

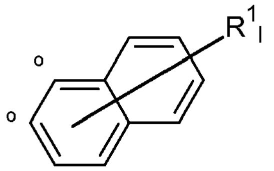
(Ar'-2)

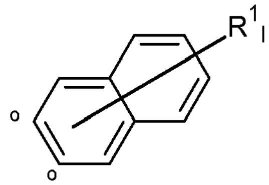
(Ar'-3)

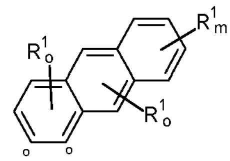
(Ar'-4)

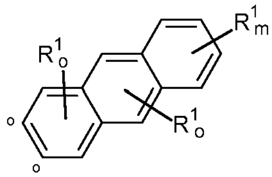
(Ar'-5)

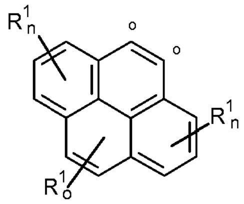
(Ar'-6)

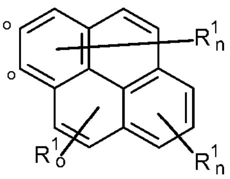
(Ar'-7)

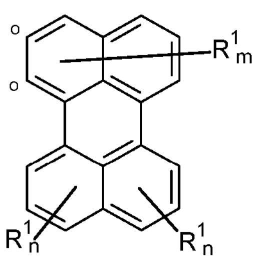
(Ar'-8)

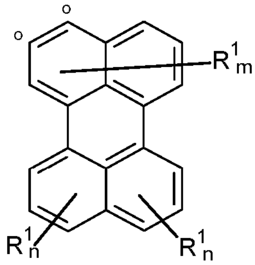
(Ar'-9)

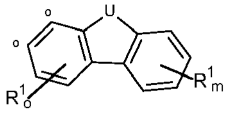
(Ar'-10)

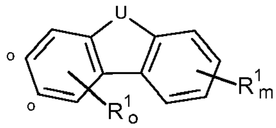
(Ar'-11)

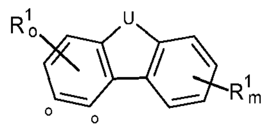
(Ar'-12)

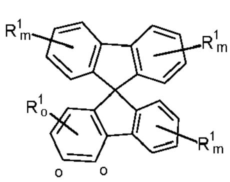
(Ar'-13)

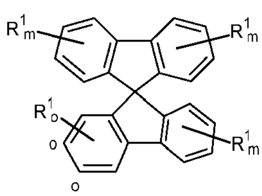
(Ar'-14)

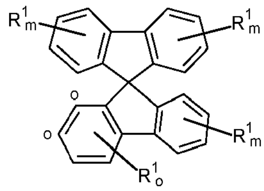
(Ar'-15)

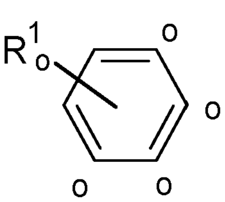
(Ar'-16)

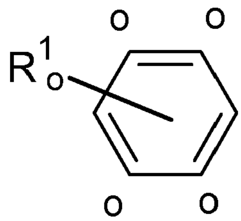
(Ar'-17)

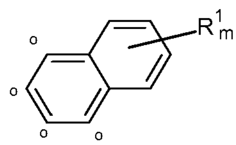
(Ar'-18)

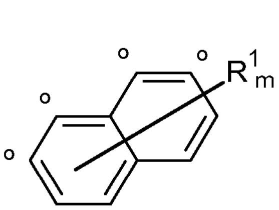

(Ar'-19)

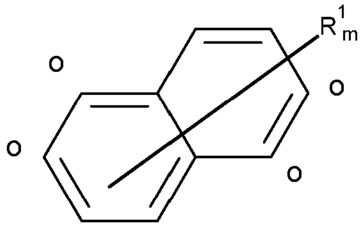

(Ar'-20)

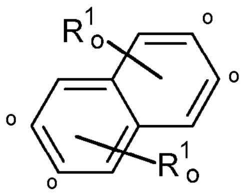

(Ar'-21)

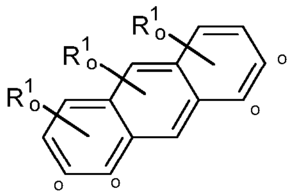

(Ar'-22)

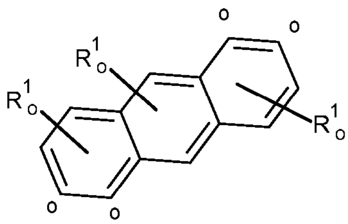

(Ar'-23)

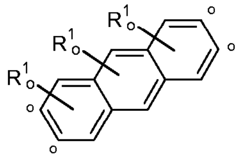

(Ar'-24)

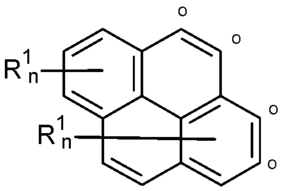

(Ar'-25)

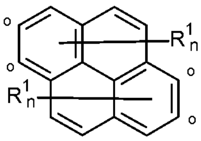

(Ar'-26)

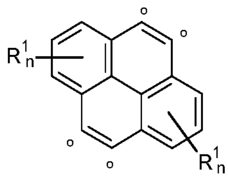

(Ar'-27)

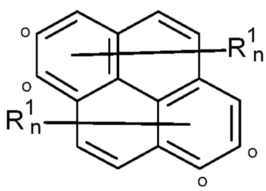

(Ar'-28)

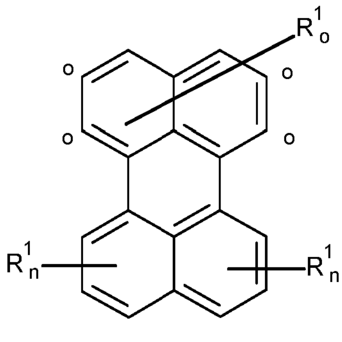

(Ar'-29)

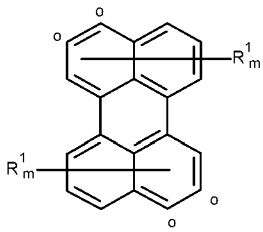

(Ar'-30)

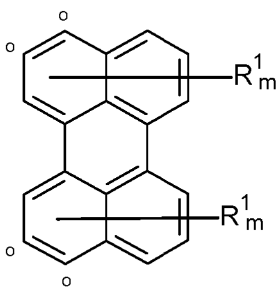

(Ar'-31)

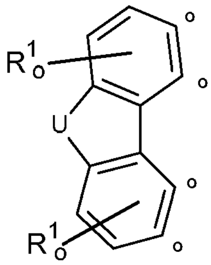

(Ar'-32)

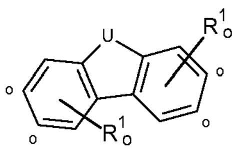

(Ar'-33)

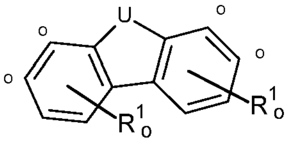
(Ar'-34)

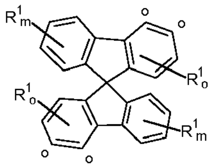
(Ar'-35)

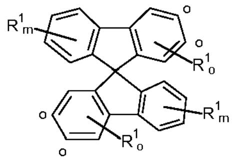
(Ar'-36)

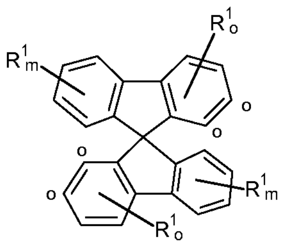
(Ar'-37)

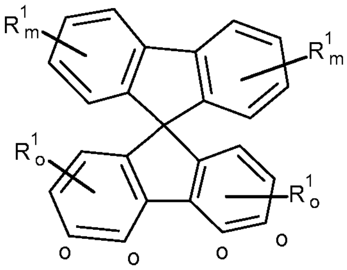
(Ar'-38)

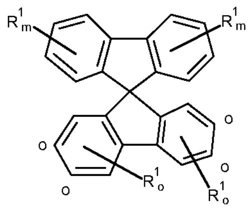
(Ar'-39)

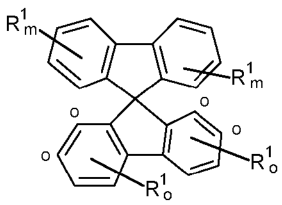
(Ar'-40)

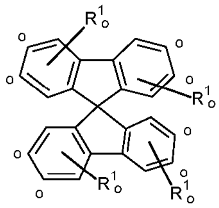
(Ar'-41)

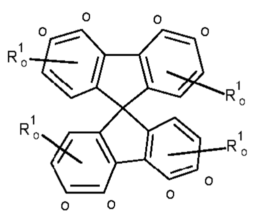
(Ar'-42)

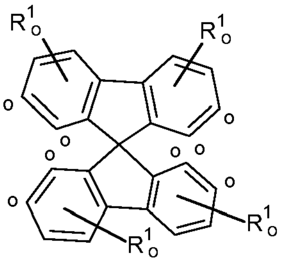
(Ar'-43)

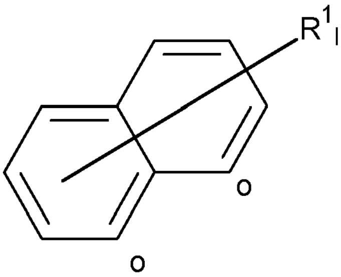
(Ar'-44)

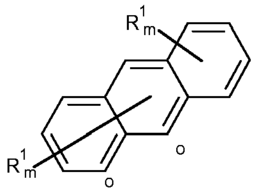
(Ar'-45)

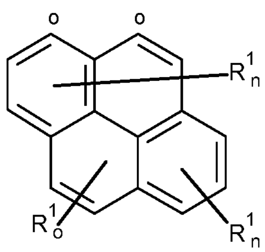
(Ar'-46)

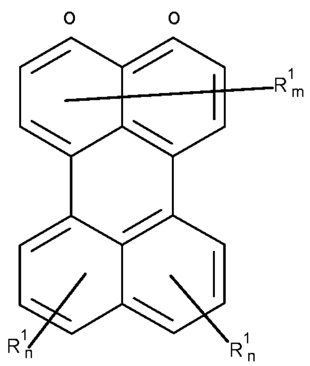
(Ar'-47)

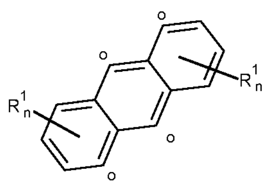
(Ar'-48)

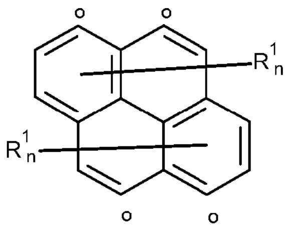
(Ar'-49)

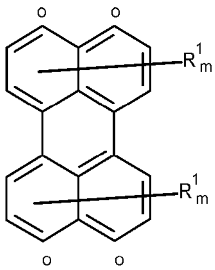
(Ar'-50)

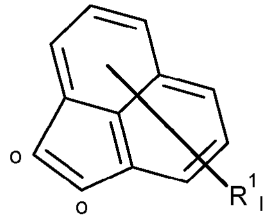
(Ar'-51)

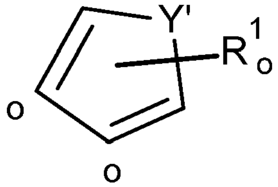
(Ar'-52)

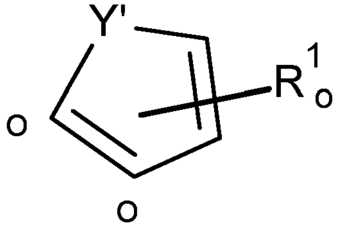
(Ar'-53)

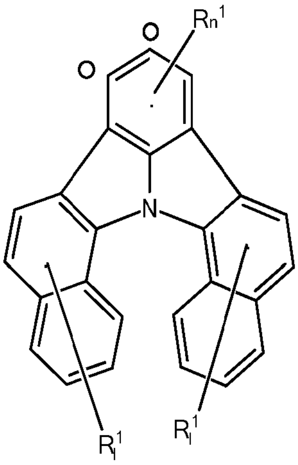
(Ar'-54)

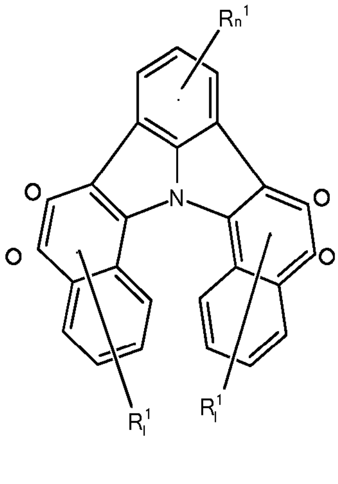
(Ar'-55)

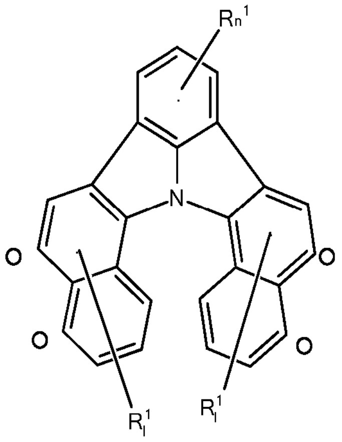

(Ar'-56)

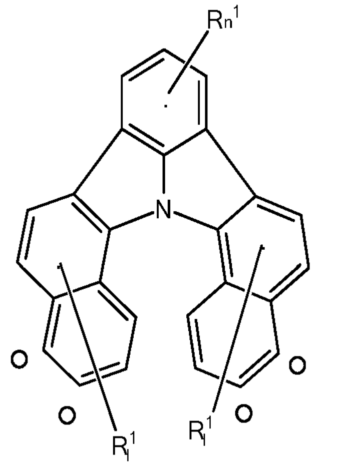

(Ar'-57)

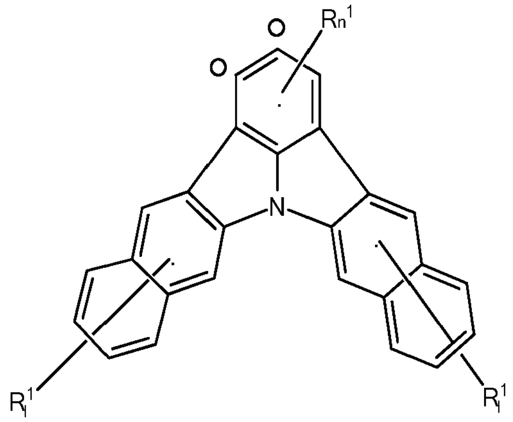

(Ar'-58)

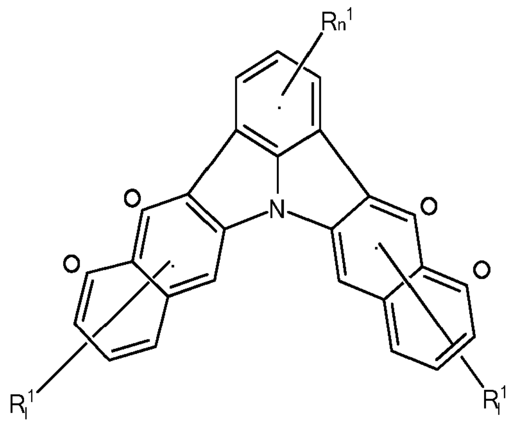

(Ar'-59)

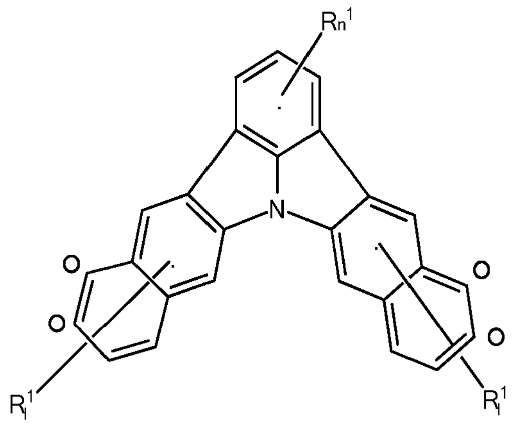

(Ar'-60)

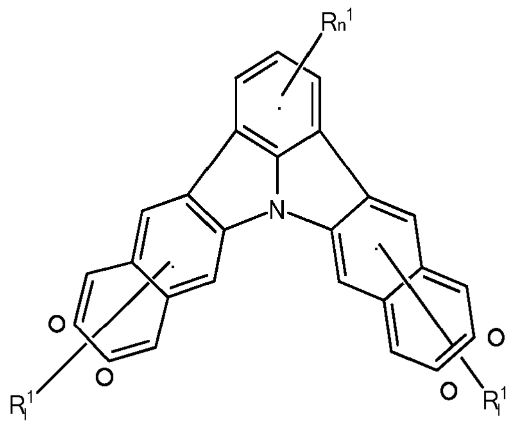

(Ar'-61)

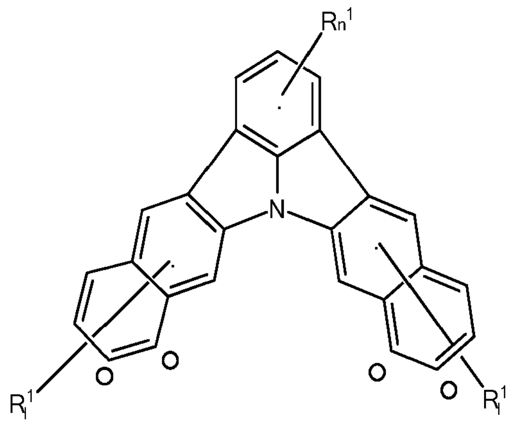

(Ar'-62)

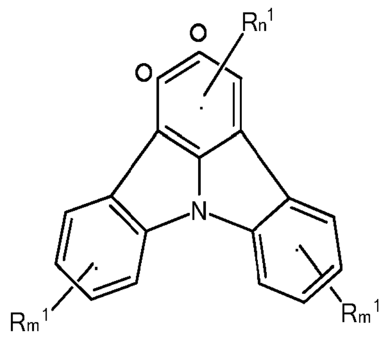

(Ar'-63)

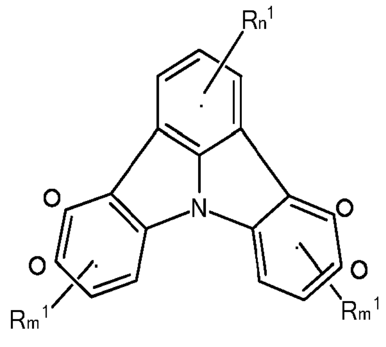

(Ar'-64)

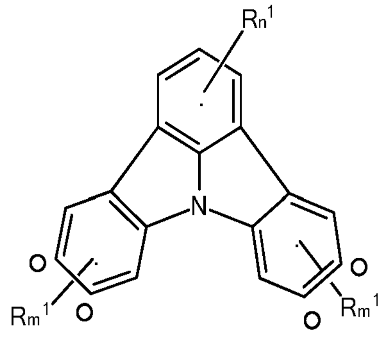

(Ar'-65)

wobei $R^1$ die zuvor, insbesondere für Formeln (I) bis (XVIII) genannte Bedeutung hat und die Symbole Y' und U die zuvor, insbesondere für Formeln (Ar-1) bis (Ar-65) genannte Bedeutung aufweisen, der Index o 0, 1 oder 2, vorzugsweise 0 oder 1 ist, der Index n 0, 1, 2, oder 3, vorzugsweise 0, 1 oder 2 ist und der Index m 0, 1, 2, 3 oder 4, vorzugsweise 0, 1 oder 2 ist, und der Index l 0, 1, 2, 3, 4, 5 oder 6, vorzugsweise 0, 1 oder 2 ist, und das aliphatische polycyclische Ringsystem mit mindestens 3 Ringen jeweils an den durch o gekennzeichneten Positionen an das aromatische oder heteroaromatische Ringsystem mit 5 bis 60 Kohlenstoffaromen unter Bildung eines Ringes bindet. Hierbei sind Strukturen der Formeln (Ar'-2) bis (Ar'-53) bevorzugt und Strukturen der Formeln (Ar'-4) bis (Ar'-15) und (Ar'-22) bis (Ar'-43) besonders bevorzugt.

Ferner kann in den Teilstrukturen gemäß den Formeln (Ar'-1) bis (Ar'-65) vorgesehen sein, dass die Summe der Indices o, n, m und l höchstens 6, vorzugsweise höchstens 4 und besonders bevorzugt höchstens 2 beträgt.

Ferner bevorzugt sind die Teilstrukturen gemäß den Formeln (Ar'-54) bis (Ar'-65).

Gemäß einer bevorzugten Ausführungsform sind unter anderem die Kombinationen gemäß der folgenden Tabelle bevorzugt

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-1 | Ar'-1 | 0 bis 6, vorzugsweise 0 bis 3, bevorzugt 0 oder 1 | 0 bis 4, vorzugsweise 1 oder 2, bevorzugt 1 |
| N-3 | Ar'-1 | 0 bis 6, vorzugsweise 0 bis 3, bevorzugt 0 oder 1 | 0 bis 4, vorzugsweise 1 oder 2, bevorzugt 1 |
| N-5 | Ar'-1 | 0 bis 6, vorzugsweise 0 bis 3, bevorzugt 0 oder 1 | 0 bis 4, vorzugsweise 1 oder 2, bevorzugt 1 |
| N-6 | Ar'-1 | 0 bis 6, vorzugsweise 0 bis 3, bevorzugt 0 oder 1 | 0 bis 4, vorzugsweise 1 oder 2, bevorzugt 1 |
| N-1 | Ar'-1 | 0 | 0 bis 2, vorzugsweise 1 oder 2, bevorzugt 1 |
| N-3 | Ar'-1 | 0 | 0 bis 2, vorzugsweise 1 oder 2, bevorzugt 1 |
| N-5 | Ar'-1 | 0 | 0 bis 2, vorzugsweise 1 oder 2, bevorzugt 1 |
| N-6 | Ar'-1 | 0 | 0 bis 2, vorzugsweise 1 oder 2, bevorzugt 1 |
| N-1 | Ar'-2 | 0 bis 6, vorzugsweise 0 bis 3, bevorzugt 0 oder 1 | 0 bis 4, vorzugsweise 1 oder 2, bevorzugt 1 |
| N-3 | Ar'-2 | 0 bis 6, vorzugsweise 0 bis 3, bevorzugt 0 oder 1 | 0 bis 4, vorzugsweise 1 oder 2, bevorzugt 1 |
| N-5 | Ar'-2 | 0 bis 6, vorzugsweise 0 bis 3, bevorzugt 0 oder 1 | 0 bis 4, vorzugsweise 1 oder 2, bevorzugt 1 |
| N-6 | Ar'-2 | 0 bis 6, vorzugsweise 0 bis 3, bevorzugt 0 oder 1 | 0 bis 4, vorzugsweise 1 oder 2, bevorzugt 1 |
| N-1 | Ar'-2 | 0 | 0 bis 2, vorzugsweise 1 oder 2, bevorzugt 1 |
| N-3 | Ar'-2 | 0 | 0 bis 2, vorzugsweise 1 oder 2, bevorzugt 1 |
| N-5 | Ar'-2 | 0 | 0 bis 2, vorzugsweise 1 oder 2, bevorzugt 1 |
| N-6 | Ar'-2 | 0 | 0 bis 2, vorzugsweise 1 oder 2, bevorzugt 1 |
| N-1 | Ar'-3 | 0 bis 6, vorzugsweise 0 bis 3, bevorzugt 0 oder 1 | 0 bis 4, vorzugsweise 1 oder 2, bevorzugt 1 |
| N-3 | Ar'-3 | 0 bis 6, vorzugsweise 0 bis 3, bevorzugt 0 oder 1 | 0 bis 4, vorzugsweise 1 oder 2, bevorzugt 1 |
| N-5 | Ar'-3 | 0 bis 6, vorzugsweise 0 bis 3, bevorzugt 0 oder 1 | 0 bis 4, vorzugsweise 1 oder 2, bevorzugt 1 |
| N-6 | Ar'-3 | 0 bis 6, vorzugsweise 0 bis 3, bevorzugt 0 oder 1 | 0 bis 4, vorzugsweise 1 oder 2, bevorzugt 1 |
| N-1 | Ar'-3 | 0 | 0 bis 2, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-3 | Ar'-3 | 0 | 0 bis 2, vorzugsweise 1 oder 2, bevorzugt 1 |
| N-5 | Ar'-3 | 0 | 0 bis 2, vorzugsweise 1 oder 2, bevorzugt 1 |
| N-6 | Ar'-3 | 0 | 0 bis 2, vorzugsweise 1 oder 2, bevorzugt 1 |
| N-1 | Ar'-4 | 0 bis 6, vorzugsweise 0 bis 3, bevorzugt 0 oder 1 | 0 bis 4, vorzugsweise 1 bis 3, bevorzugt 2 |
| N-3 | Ar'-4 | 0 bis 6, vorzugsweise 0 bis 3, bevorzugt 0 oder 1 | 0 bis 4, vorzugsweise 1 bis 3, bevorzugt 2 |
| N-5 | Ar'-4 | 0 bis 6, vorzugsweise 0 bis 3, bevorzugt 0 oder 1 | 0 bis 4, vorzugsweise 1 bis 3, bevorzugt 2 |
| N-6 | Ar'-4 | 0 bis 6, vorzugsweise 0 bis 3, bevorzugt 0 oder 1 | 0 bis 4, vorzugsweise 1 bis 3, bevorzugt 2 |
| N-1 | Ar'-4 | 0 | 0 bis 3, vorzugsweise 1, 2 oder 3, bevorzugt 2 |
| N-3 | Ar'-4 | 0 | 0 bis 3, vorzugsweise 1, 2 oder 3, bevorzugt 2 |
| N-5 | Ar'-4 | 0 | 0 bis 3, vorzugsweise 1, 2 oder 3, bevorzugt 2 |
| N-6 | Ar'-4 | 0 | 0 bis 3, vorzugsweise 1, 2 oder 3, bevorzugt 2 |
| N-1 | Ar'-5 | 0 bis 6, vorzugsweise 0 bis 3, bevorzugt 0 oder 1 | 0 bis 4, vorzugsweise 1 bis 3, bevorzugt 2 |
| N-3 | Ar'-5 | 0 bis 6, vorzugsweise 0 bis 3, bevorzugt 0 oder 1 | 0 bis 4, vorzugsweise 1 bis 3, bevorzugt 2 |
| N-5 | Ar'-5 | 0 bis 6, vorzugsweise 0 bis 3, bevorzugt 0 oder 1 | 0 bis 4, vorzugsweise 1 bis 3, bevorzugt 2 |
| N-6 | Ar'-5 | 0 bis 6, vorzugsweise 0 bis 3, bevorzugt 0 oder 1 | 0 bis 4, vorzugsweise 1 bis 3, bevorzugt 2 |
| N-1 | Ar'-5 | 0 | 0 bis 3, vorzugsweise 1, 2 oder 3, bevorzugt 2 |
| N-3 | Ar'-5 | 0 | 0 bis 3, vorzugsweise 1, 2 oder 3, bevorzugt 2 |
| N-5 | Ar'-5 | 0 | 0 bis 3, vorzugsweise 1, 2 oder 3, bevorzugt 2 |
| N-6 | Ar'-5 | 0 | 0 bis 3, vorzugsweise 1, 2 oder 3, bevorzugt 2 |
| N-1 | Ar'-6 | 0 bis 6, vorzugsweise 0 bis 3, bevorzugt 0 oder 1 | 0 bis 4, vorzugsweise 1 oder 2, bevorzugt 1 |
| N-3 | Ar'-6 | 0 bis 6, vorzugsweise 0 bis 3, bevorzugt 0 oder 1 | 0 bis 4, vorzugsweise 1 oder 2, bevorzugt 1 |
| N-5 | Ar'-6 | 0 bis 6, vorzugsweise 0 bis 3, bevorzugt 0 oder 1 | 0 bis 4, vorzugsweise 1 oder 2, bevorzugt 1 |
| N-6 | Ar'-6 | 0 bis 6, vorzugsweise 0 bis 3, bevorzugt 0 oder 1 | 0 bis 4, vorzugsweise 1 oder 2, bevorzugt 1 |
| N-1 | Ar'-6 | 0 | 0 bis 2, vorzugsweise 1 oder 2, bevorzugt 1 |
| N-3 | Ar'-6 | 0 | 0 bis 2, vorzugsweise 1 oder 2, bevorzugt 1 |
| N-5 | Ar'-6 | 0 | 0 bis 2, vorzugsweise 1 oder 2, bevorzugt 1 |
| N-6 | Ar'-6 | 0 | 0 bis 2, vorzugsweise 1 oder 2, bevorzugt 1 |
| N-1 | Ar'-7 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-3 | Ar'-7 | 0 bis 6, vorzugsweise 0 bis 3, bevorzugt 0 oder 1 | 0 bis 4, vorzugsweise 1 oder 2, bevorzugt 1 |
| N-5 | Ar'-7 | 0 bis 6, vorzugsweise 0 bis 3, bevorzugt 0 oder 1 | 0 bis 4, vorzugsweise 1 oder 2, bevorzugt 1 |
| N-6 | Ar'-7 | 0 bis 6, vorzugsweise 0 bis 3, bevorzugt 0 oder 1 | 0 bis 4, vorzugsweise 1 oder 2, bevorzugt 1 |
| N-1 | Ar'-7 | 0 | 0 bis 2, vorzugsweise 1 oder 2, bevorzugt 1 |
| N-3 | Ar'-7 | 0 | 0 bis 2, vorzugsweise 1 oder 2, bevorzugt 1 |
| N-5 | Ar'-7 | 0 | 0 bis 2, vorzugsweise 1 oder 2, bevorzugt 1 |
| N-6 | Ar'-7 | 0 | 0 bis 2, vorzugsweise 1 oder 2, bevorzugt 1 |
| N-1 | Ar'-8 | 0 bis 6, vorzugsweise 0 bis 3, bevorzugt 0 oder 1 | 0 bis 4, vorzugsweise 1 oder 2, bevorzugt 1 |
| N-3 | Ar'-8 | 0 bis 6, vorzugsweise 0 bis 3, bevorzugt 0 oder 1 | 0 bis 4, vorzugsweise 1 oder 2, bevorzugt 1 |
| N-5 | Ar'-8 | 0 bis 6, vorzugsweise 0 bis 3, bevorzugt 0 oder 1 | 0 bis 4, vorzugsweise 1 oder 2, bevorzugt 1 |
| N-6 | Ar'-8 | 0 bis 6, vorzugsweise 0 bis 3, bevorzugt 0 oder 1 | 0 bis 4, vorzugsweise 1 oder 2, bevorzugt 1 |
| N-1 | Ar'-8 | 0 | 0 bis 2, vorzugsweise 1 oder 2, bevorzugt 1 |
| N-3 | Ar'-8 | 0 | 0 bis 2, vorzugsweise 1 oder 2, bevorzugt 1 |
| N-5 | Ar'-8 | 0 | 0 bis 2, vorzugsweise 1 oder 2, bevorzugt 1 |
| N-6 | Ar'-8 | 0 | 0 bis 2, vorzugsweise 1 oder 2, bevorzugt 1 |
| N-1 | Ar'-9 | 0 bis 6, vorzugsweise 0 bis 3, bevorzugt 0 oder 1 | 0 bis 4, vorzugsweise 1 oder 2, bevorzugt 1 |
| N-3 | Ar'-9 | 0 bis 6, vorzugsweise 0 bis 3, bevorzugt 0 oder 1 | 0 bis 4, vorzugsweise 1 oder 2, bevorzugt 1 |
| N-5 | Ar'-9 | 0 bis 6, vorzugsweise 0 bis 3, bevorzugt 0 oder 1 | 0 bis 4, vorzugsweise 1 oder 2, bevorzugt 1 |
| N-6 | Ar'-9 | 0 bis 6, vorzugsweise 0 bis 3, bevorzugt 0 oder 1 | 0 bis 4, vorzugsweise 1 oder 2, bevorzugt 1 |
| N-1 | Ar'-9 | 0 | 0 bis 2, vorzugsweise 1 oder 2, bevorzugt 1 |
| N-3 | Ar'-9 | 0 | 0 bis 2, vorzugsweise 1 oder 2, bevorzugt 1 |
| N-5 | Ar'-9 | 0 | 0 bis 2, vorzugsweise 1 oder 2, bevorzugt 1 |
| N-6 | Ar'-9 | 0 | 0 bis 2, vorzugsweise 1 oder 2, bevorzugt 1 |
| N-1 | Ar'-10 | 0 bis 6, vorzugsweise 0 bis 3, bevorzugt 0 oder 1 | 0 bis 4, vorzugsweise 1 oder 2, bevorzugt 1 |
| N-3 | Ar'-10 | 0 bis 6, vorzugsweise 0 bis 3, bevorzugt 0 oder 1 | 0 bis 4, vorzugsweise 1 oder 2, bevorzugt 1 |
| N-5 | Ar'-10 | 0 bis 6, vorzugsweise 0 bis 3, bevorzugt 0 oder 1 | 0 bis 4, vorzugsweise 1 oder 2, bevorzugt 1 |
| N-6 | Ar'-10 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-1 | Ar'-10 | 0 | 0 bis 2, vorzugsweise 1 oder 2, bevorzugt 1 |
| N-3 | Ar'-10 | 0 | 0 bis 2, vorzugsweise 1 oder 2, bevorzugt 1 |
| N-5 | Ar'-10 | 0 | 0 bis 2, vorzugsweise 1 oder 2, bevorzugt 1 |
| N-6 | Ar'-10 | 0 | 0 bis 2, vorzugsweise 1 oder 2, bevorzugt 1 |
| N-1 | Ar'-11 | 0 bis 6, vorzugsweise 0 bis 3, bevorzugt 0 oder 1 | 0 bis 4, vorzugsweise 1 oder 2, bevorzugt 1 |
| N-3 | Ar'-11 | 0 bis 6, vorzugsweise 0 bis 3, bevorzugt 0 oder 1 | 0 bis 4, vorzugsweise 1 oder 2, bevorzugt 1 |
| N-5 | Ar'-11 | 0 bis 6, vorzugsweise 0 bis 3, bevorzugt 0 oder 1 | 0 bis 4, vorzugsweise 1 oder 2, bevorzugt 1 |
| N-6 | Ar'-11 | 0 bis 6, vorzugsweise 0 bis 3, bevorzugt 0 oder 1 | 0 bis 4, vorzugsweise 1 oder 2, bevorzugt 1 |
| N-1 | Ar'-11 | 0 | 0 bis 2, vorzugsweise 1 oder 2, bevorzugt 1 |
| N-3 | Ar'-11 | 0 | 0 bis 2, vorzugsweise 1 oder 2, bevorzugt 1 |
| N-5 | Ar'-11 | 0 | 0 bis 2, vorzugsweise 1 oder 2, bevorzugt 1 |
| N-6 | Ar'-11 | 0 | 0 bis 2, vorzugsweise 1 oder 2, bevorzugt 1 |
| N-1 | Ar'-12 | 0 bis 6, vorzugsweise 0 bis 3, bevorzugt 0 oder 1 | 0 bis 4, vorzugsweise 1 oder 2, bevorzugt 1 |
| N-3 | Ar'-12 | 0 bis 6, vorzugsweise 0 bis 3, bevorzugt 0 oder 1 | 0 bis 4, vorzugsweise 1 oder 2, bevorzugt 1 |
| N-5 | Ar'-12 | 0 bis 6, vorzugsweise 0 bis 3, bevorzugt 0 oder 1 | 0 bis 4, vorzugsweise 1 oder 2, bevorzugt 1 |
| N-6 | Ar'-12 | 0 bis 6, vorzugsweise 0 bis 3, bevorzugt 0 oder 1 | 0 bis 4, vorzugsweise 1 oder 2, bevorzugt 1 |
| N-1 | Ar'-12 | 0 | 0 bis 2, vorzugsweise 1 oder 2, bevorzugt 1 |
| N-3 | Ar'-12 | 0 | 0 bis 2, vorzugsweise 1 oder 2, bevorzugt 1 |
| N-5 | Ar'-12 | 0 | 0 bis 2, vorzugsweise 1 oder 2, bevorzugt 1 |
| N-6 | Ar'-12 | 0 | 0 bis 2, vorzugsweise 1 oder 2, bevorzugt 1 |
| N-1 | Ar'-13 | 0 bis 6, vorzugsweise 0 bis 3, bevorzugt 0 oder 1 | 0 bis 4, vorzugsweise 1 oder 2, bevorzugt 1 |
| N-3 | Ar'-13 | 0 bis 6, vorzugsweise 0 bis 3, bevorzugt 0 oder 1 | 0 bis 4, vorzugsweise 1 oder 2, bevorzugt 1 |
| N-5 | Ar'-13 | 0 bis 6, vorzugsweise 0 bis 3, bevorzugt 0 oder 1 | 0 bis 4, vorzugsweise 1 oder 2, bevorzugt 1 |
| N-6 | Ar'-13 | 0 bis 6, vorzugsweise 0 bis 3, bevorzugt 0 oder 1 | 0 bis 4, vorzugsweise 1 oder 2, bevorzugt 1 |
| N-1 | Ar'-13 | 0 | 0 bis 2, vorzugsweise 1 oder 2, bevorzugt 1 |
| N-3 | Ar'-13 | 0 | 0 bis 2, vorzugsweise 1 oder 2, bevorzugt 1 |
| N-5 | Ar'-13 | 0 | 0 bis 2, vorzugsweise 1 oder 2, bevorzugt 1 |
| N-6 | Ar'-13 | 0 | 0 bis 2, vorzugsweise 1 oder 2, bevorzugt 1 |
| N-1 | Ar'-14 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-3 | Ar'-14 | 0 bis 6, vorzugsweise 0 bis 3, bevorzugt 0 oder 1 | 0 bis 4, vorzugsweise 1 oder 2, bevorzugt 1 |
| N-5 | Ar'-14 | 0 bis 6, vorzugsweise 0 bis 3, bevorzugt 0 oder 1 | 0 bis 4, vorzugsweise 1 oder 2, bevorzugt 1 |
| N-6 | Ar'-14 | 0 bis 6, vorzugsweise 0 bis 3, bevorzugt 0 oder 1 | 0 bis 4, vorzugsweise 1 oder 2, bevorzugt 1 |
| N-1 | Ar'-14 | 0 | 0 bis 2, vorzugsweise 1 oder 2, bevorzugt 1 |
| N-3 | Ar'-14 | 0 | 0 bis 2, vorzugsweise 1 oder 2, bevorzugt 1 |
| N-5 | Ar'-14 | 0 | 0 bis 2, vorzugsweise 1 oder 2, bevorzugt 1 |
| N-6 | Ar'-14 | 0 | 0 bis 2, vorzugsweise 1 oder 2, bevorzugt 1 |
| N-1 | Ar'-15 | 0 bis 6, vorzugsweise 0 bis 3, bevorzugt 0 oder 1 | 0 bis 4, vorzugsweise 1 oder 2, bevorzugt 1 |
| N-3 | Ar'-15 | 0 bis 6, vorzugsweise 0 bis 3, bevorzugt 0 oder 1 | 0 bis 4, vorzugsweise 1 oder 2, bevorzugt 1 |
| N-5 | Ar'-15 | 0 bis 6, vorzugsweise 0 bis 3, bevorzugt 0 oder 1 | 0 bis 4, vorzugsweise 1 oder 2, bevorzugt 1 |
| N-6 | Ar'-15 | 0 bis 6, vorzugsweise 0 bis 3, bevorzugt 0 oder 1 | 0 bis 4, vorzugsweise 1 oder 2, bevorzugt 1 |
| N-1 | Ar'-15 | 0 | 0 bis 2, vorzugsweise 1 oder 2, bevorzugt 1 |
| N-3 | Ar'-15 | 0 | 0 bis 2, vorzugsweise 1 oder 2, bevorzugt 1 |
| N-5 | Ar'-15 | 0 | 0 bis 2, vorzugsweise 1 oder 2, bevorzugt 1 |
| N-6 | Ar'-15 | 0 | 0 bis 2, vorzugsweise 1 oder 2, bevorzugt 1 |
| 2 * N-1 | Ar'-16 | 0 bis 6, vorzugsweise 0 bis 3, bevorzugt 0 oder 1 | 0 bis 2, vorzugsweise 1 oder 2, bevorzugt 1 |
| 2 * N-3 | Ar'-16 | 0 bis 6, vorzugsweise 0 bis 3, bevorzugt 0 oder 1 | 0 bis 2, vorzugsweise 1 oder 2, bevorzugt 1 |
| 2 * N-5 | Ar'-16 | 0 bis 6, vorzugsweise 0 bis 3, bevorzugt 0 oder 1 | 0 bis 2, vorzugsweise 1 oder 2, bevorzugt 1 |
| 2 * N-6 | Ar'-16 | 0 bis 6, vorzugsweise 0 bis 3, bevorzugt 0 oder 1 | 0 bis 2, vorzugsweise 1 oder 2, bevorzugt 1 |
| 2 * N-1 | Ar'-16 | 0 | 0 bis 2, vorzugsweise 1 oder 2, bevorzugt 1 |
| 2 * N-3 | Ar'-16 | 0 | 0 bis 2, vorzugsweise 1 oder 2, bevorzugt 1 |
| 2 * N-5 | Ar'-16 | 0 | 0 bis 2, vorzugsweise 1 oder 2, bevorzugt 1 |
| 2 * N-6 | Ar'-16 | 0 | 0 bis 2, vorzugsweise 1 oder 2, bevorzugt 1 |
| 2 * N-1 | Ar'-17 | 0 bis 6, vorzugsweise 0 bis 3, bevorzugt 0 oder 1 | 0 bis 2, vorzugsweise 1 oder 2, bevorzugt 1 |
| 2 * N-3 | Ar'-17 | 0 bis 6, vorzugsweise 0 bis 3, bevorzugt 0 oder 1 | 0 bis 2, vorzugsweise 1 oder 2, bevorzugt 1 |
| 2 * N-5 | Ar'-17 | 0 bis 6, vorzugsweise 0 bis 3, bevorzugt 0 oder 1 | 0 bis 2, vorzugsweise 1 oder 2, bevorzugt 1 |
| 2 * N-6 | Ar'-17 | 0 bis 6, vorzugsweise 0 bis 3, bevorzugt 0 oder 1 | 0 bis 2, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-1 | Ar'-17 | 0 | 0 bis 2, vorzugsweise 1 oder 2, bevorzugt 1 |
| 2 * N-3 | Ar'-17 | 0 | 0 bis 2, vorzugsweise 1 oder 2, bevorzugt 1 |
| 2 * N-5 | Ar'-17 | 0 | 0 bis 2, vorzugsweise 1 oder 2, bevorzugt 1 |
| 2 * N-6 | Ar'-17 | 0 | 0 bis 2, vorzugsweise 1 oder 2, bevorzugt 1 |
| 2 * N-1 | Ar'-18 | 0 bis 6, vorzugsweise 0 bis 3, bevorzugt 0 oder 1 | 0 bis 4, vorzugsweise 1 oder 2, bevorzugt 1 |
| 2 * N-3 | Ar'-18 | 0 bis 6, vorzugsweise 0 bis 3, bevorzugt 0 oder 1 | 0 bis 4, vorzugsweise 1 oder 2, bevorzugt 1 |
| 2 * N-5 | Ar'-18 | 0 bis 6, vorzugsweise 0 bis 3, bevorzugt 0 oder 1 | 0 bis 4, vorzugsweise 1 oder 2, bevorzugt 1 |
| 2 * N-6 | Ar'-18 | 0 bis 6, vorzugsweise 0 bis 3, bevorzugt 0 oder 1 | 0 bis 4, vorzugsweise 1 oder 2, bevorzugt 1 |
| 2 * N-1 | Ar'-18 | 0 | 0 bis 2, vorzugsweise 1 oder 2, bevorzugt 1 |
| 2 * N-3 | Ar'-18 | 0 | 0 bis 2, vorzugsweise 1 oder 2, bevorzugt 1 |
| 2 * N-5 | Ar'-18 | 0 | 0 bis 2, vorzugsweise 1 oder 2, bevorzugt 1 |
| 2 * N-6 | Ar'-18 | 0 | 0 bis 2, vorzugsweise 1 oder 2, bevorzugt 1 |
| 2 * N-1 | Ar'-19 | 0 bis 6, vorzugsweise 0 bis 3, bevorzugt 0 oder 1 | 0 bis 4, vorzugsweise 1 oder 2, bevorzugt 1 |
| 2 * N-3 | Ar'-19 | 0 bis 6, vorzugsweise 0 bis 3, bevorzugt 0 oder 1 | 0 bis 4, vorzugsweise 1 oder 2, bevorzugt 1 |
| 2 * N-5 | Ar'-19 | 0 bis 6, vorzugsweise 0 bis 3, bevorzugt 0 oder 1 | 0 bis 4, vorzugsweise 1 oder 2, bevorzugt 1 |
| 2 * N-6 | Ar'-19 | 0 bis 6, vorzugsweise 0 bis 3, bevorzugt 0 oder 1 | 0 bis 4, vorzugsweise 1 oder 2, bevorzugt 1 |
| 2 * N-1 | Ar'-19 | 0 | 0 bis 2, vorzugsweise 1 oder 2, bevorzugt 1 |
| 2 * N-3 | Ar'-19 | 0 | 0 bis 2, vorzugsweise 1 oder 2, bevorzugt 1 |
| 2 * N-5 | Ar'-19 | 0 | 0 bis 2, vorzugsweise 1 oder 2, bevorzugt 1 |
| 2 * N-6 | Ar'-19 | 0 | 0 bis 2, vorzugsweise 1 oder 2, bevorzugt 1 |
| 2 * N-1 | Ar'-20 | 0 bis 6, vorzugsweise 0 bis 3, bevorzugt 0 oder 1 | 0 bis 4, vorzugsweise 1 oder 2, bevorzugt 1 |
| 1 2 * N-3 | Ar'-20 | 0 bis 6, vorzugsweise 0 bis 3, bevorzugt 0 oder 1 | 0 bis 4, vorzugsweise 1 oder 2, bevorzugt 1 |
| 2 * N-5 | Ar'-20 | 0 bis 6, vorzugsweise 0 bis 3, bevorzugt 0 oder 1 | 0 bis 4, vorzugsweise 1 oder 2, bevorzugt 1 |
| 2 * N-6 | Ar'-20 | 0 bis 6, vorzugsweise 0 bis 3, bevorzugt 0 oder 1 | 0 bis 4, vorzugsweise 1 oder 2, bevorzugt 1 |
| 2 * N-1 | Ar'-20 | 0 | 0 bis 2, vorzugsweise 1 oder 2, bevorzugt 1 |
| 2 * N-3 | Ar'-20 | 0 | 0 bis 2, vorzugsweise 1 oder 2, bevorzugt 1 |
| 2 * N-5 | Ar'-20 | 0 | 0 bis 2, vorzugsweise 1 oder 2, bevorzugt 1 |
| 2 * N-6 | Ar'-20 | 0 | 0 bis 2, vorzugsweise 1 oder 2, bevorzugt 1 |
| 2 * N-1 | Ar'-21 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-3 | Ar'-21 | 0 bis 6, vorzugsweise 0 bis 3, bevorzugt 0 oder 1 | 0 bis 4, vorzugsweise 1 oder 2, bevorzugt 1 |
| 2 * N-5 | Ar'-21 | 0 bis 6, vorzugsweise 0 bis 3, bevorzugt 0 oder 1 | 0 bis 4, vorzugsweise 1 oder 2, bevorzugt 1 |
| 2 * N-6 | Ar'-21 | 0 bis 6, vorzugsweise 0 bis 3, bevorzugt 0 oder 1 | 0 bis 4, vorzugsweise 1 oder 2, bevorzugt 1 |
| 2 * N-1 | Ar'-21 | 0 | 0 bis 2, vorzugsweise 1 oder 2, bevorzugt 1 |
| 2 * N-3 | Ar'-21 | 0 | 0 bis 2, vorzugsweise 1 oder 2, bevorzugt 1 |
| 2 * N-5 | Ar'-21 | 0 | 0 bis 2, vorzugsweise 1 oder 2, bevorzugt 1 |
| 2 * N-6 | Ar'-21 | 0 | 0 bis 2, vorzugsweise 1 oder 2, bevorzugt 1 |
| 2 * N-1 | Ar'-22 | 0 bis 6, vorzugsweise 0 bis 3, bevorzugt 0 oder 1 | 0 bis 4, vorzugsweise 1 bis 3, bevorzugt 2 |
| 2 * N-3 | Ar'-22 | 0 bis 6, vorzugsweise 0 bis 3, bevorzugt 0 oder 1 | 0 bis 4, vorzugsweise 1 bis 3, bevorzugt 2 |
| 2 * N-5 | Ar'-22 | 0 bis 6, vorzugsweise 0 bis 3, bevorzugt 0 oder 1 | 0 bis 4, vorzugsweise 1 bis 3, bevorzugt 2 |
| 2 * N-6 | Ar'-22 | 0 bis 6, vorzugsweise 0 bis 3, bevorzugt 0 oder 1 | 0 bis 4, vorzugsweise 1 bis 3, bevorzugt 2 |
| 2 * N-1 | Ar'-22 | 0 | 0 bis 3, vorzugsweise 1, 2 oder 3, bevorzugt 2 |
| 2 * N-3 | Ar'-22 | 0 | 0 bis 3, vorzugsweise 1, 2 oder 3, bevorzugt 2 |
| 2 * N-5 | Ar'-22 | 0 | 0 bis 3, vorzugsweise 1, 2 oder 3, bevorzugt 2 |
| 2 * N-6 | Ar'-22 | 0 | 0 bis 3, vorzugsweise 1, 2 oder 3, bevorzugt 2 |
| 2 * N-1 | Ar'-23 | 0 bis 6, vorzugsweise 0 bis 3, bevorzugt 0 oder 1 | 0 bis 4, vorzugsweise 1 bis 3, bevorzugt 2 |
| 2 * N-3 | Ar'-23 | 0 bis 6, vorzugsweise 0 bis 3, bevorzugt 0 oder 1 | 0 bis 4, vorzugsweise 1 bis 3, bevorzugt 2 |
| 2 * N-5 | Ar'-23 | 0 bis 6, vorzugsweise 0 bis 3, bevorzugt 0 oder 1 | 0 bis 4, vorzugsweise 1 bis 3, bevorzugt 2 |
| 2 * N-6 | Ar'-23 | 0 bis 6, vorzugsweise 0 bis 3, bevorzugt 0 oder 1 | 0 bis 4, vorzugsweise 1 bis 3, bevorzugt 2 |
| 2 * N-1 | Ar'-23 | 0 | 0 bis 3, vorzugsweise 1, 2 oder 3, bevorzugt 2 |
| 2 * N-3 | Ar'-23 | 0 | 0 bis 3, vorzugsweise 1, 2 oder 3, bevorzugt 2 |
| 2 * N-5 | Ar'-23 | 0 | 0 bis 3, vorzugsweise 1, 2 oder 3, bevorzugt 2 |
| 2 * N-6 | Ar'-23 | 0 | 0 bis 3, vorzugsweise 1, 2 oder 3, bevorzugt 2 |
| 2 * N-1 | Ar'-24 | 0 bis 6, vorzugsweise 0 bis 3, bevorzugt 0 oder 1 | 0 bis 4, vorzugsweise 1 bis 3, bevorzugt 2 |
| 2 * N-3 | Ar'-24 | 0 bis 6, vorzugsweise 0 bis 3, bevorzugt 0 oder 1 | 0 bis 4, vorzugsweise 1 bis 3, bevorzugt 2 |
| 2 * N-5 | Ar'-24 | 0 bis 6, vorzugsweise 0 bis 3, bevorzugt 0 oder 1 | 0 bis 4, vorzugsweise 1 bis 3, bevorzugt 2 |
| 2 * N-6 | Ar'-24 | 0 bis 6, vorzugsweise 0 bis 3, bevorzugt 0 oder 1 | 0 bis 4, vorzugsweise 1 bis 3, bevorzugt 2 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-1 | Ar'-24 | 0 | 0 bis 3, vorzugsweise 1, 2 oder 3, bevorzugt 2 |
| 2 * N-3 | Ar'-24 | 0 | 0 bis 3, vorzugsweise 1, 2 oder 3, bevorzugt 2 |
| 2 * N-5 | Ar'-24 | 0 | 0 bis 3, vorzugsweise 1, 2 oder 3, bevorzugt 2 |
| 2 * N-6 | Ar'-24 | 0 | 0 bis 3, vorzugsweise 1, 2 oder 3, bevorzugt 2 |
| 2 * N-1 | Ar'-25 | 0 bis 6, vorzugsweise 0 bis 3, bevorzugt 0 oder 1 | 0 bis 4, vorzugsweise 1 oder 2, bevorzugt 1 |
| 2 * N-3 | Ar'-25 | 0 bis 6, vorzugsweise 0 bis 3, bevorzugt 0 oder 1 | 0 bis 4, vorzugsweise 1 oder 2, bevorzugt 1 |
| 2 * N-5 | Ar'-25 | 0 bis 6, vorzugsweise 0 bis 3, bevorzugt 0 oder 1 | 0 bis 4, vorzugsweise 1 oder 2, bevorzugt 1 |
| 2 * N-6 | Ar'-25 | 0 bis 6, vorzugsweise 0 bis 3, bevorzugt 0 oder 1 | 0 bis 4, vorzugsweise 1 oder 2, bevorzugt 1 |
| 2 * N-1 | Ar'-25 | 0 | 0 bis 2, vorzugsweise 1 oder 2, bevorzugt 1 |
| 2 * N-3 | Ar'-25 | 0 | 0 bis 2, vorzugsweise 1 oder 2, bevorzugt 1 |
| 2 * N-5 | Ar'-25 | 0 | 0 bis 2, vorzugsweise 1 oder 2, bevorzugt 1 |
| 2 * N-6 | Ar'-25 | 0 | 0 bis 2, vorzugsweise 1 oder 2, bevorzugt 1 |
| 2 * N-1 | Ar'-26 | 0 bis 6, vorzugsweise 0 bis 3, bevorzugt 0 oder 1 | 0 bis 4, vorzugsweise 1 oder 2, bevorzugt 1 |
| 2 * N-3 | Ar'-26 | 0 bis 6, vorzugsweise 0 bis 3, bevorzugt 0 oder 1 | 0 bis 4, vorzugsweise 1 oder 2, bevorzugt 1 |
| 2 * N-5 | Ar'-26 | 0 bis 6, vorzugsweise 0 bis 3, bevorzugt 0 oder 1 | 0 bis 4, vorzugsweise 1 oder 2, bevorzugt 1 |
| 2 * N-6 | Ar'-26 | 0 bis 6, vorzugsweise 0 bis 3, bevorzugt 0 oder 1 | 0 bis 4, vorzugsweise 1 oder 2, bevorzugt 1 |
| 2 * N-1 | Ar'-26 | 0 | 0 bis 2, vorzugsweise 1 oder 2, bevorzugt 1 |
| 2 * N-4 | Ar'-26 | 0 | 0 bis 2, vorzugsweise 1 oder 2, bevorzugt 1 |
| 2 * N-5 | Ar'-26 | 0 | 0 bis 2, vorzugsweise 1 oder 2, bevorzugt 1 |
| 2 * N-6 | Ar'-26 | 0 | 0 bis 2, vorzugsweise 1 oder 2, bevorzugt 1 |
| 2 * N-1 | Ar'-27 | 0 bis 6, vorzugsweise 0 bis 3, bevorzugt 0 oder 1 | 0 bis 4, vorzugsweise 1 oder 2, bevorzugt 1 |
| 2 * N-3 | Ar'-27 | 0 bis 6, vorzugsweise 0 bis 3, bevorzugt 0 oder 1 | 0 bis 4, vorzugsweise 1 oder 2, bevorzugt 1 |
| 2 * N-5 | Ar'-27 | 0 bis 6, vorzugsweise 0 bis 3, bevorzugt 0 oder 1 | 0 bis 4, vorzugsweise 1 oder 2, bevorzugt 1 |
| 2 * N-6 | Ar'-27 | 0 bis 6, vorzugsweise 0 bis 3, bevorzugt 0 oder 1 | 0 bis 4, vorzugsweise 1 oder 2, bevorzugt 1 |
| 2 * N-1 | Ar'-27 | 0 | 0 bis 2, vorzugsweise 1 oder 2, bevorzugt 1 |
| 2 * N-3 | Ar'-27 | 0 | 0 bis 2, vorzugsweise 1 oder 2, bevorzugt 1 |
| 2 * N-5 | Ar'-27 | 0 | 0 bis 2, vorzugsweise 1 oder 2, bevorzugt 1 |
| 2 * N-6 | Ar'-27 | 0 | 0 bis 2, vorzugsweise 1 oder 2, bevorzugt 1 |
| 2 * N-1 | Ar'-28 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-3 | Ar'-28 | 0 bis 6, vorzugsweise 0 bis 3, bevorzugt 0 oder 1 | 0 bis 4, vorzugsweise 1 oder 2, bevorzugt 1 |
| 2 * N-5 | Ar'-28 | 0 bis 6, vorzugsweise 0 bis 3, bevorzugt 0 oder 1 | 0 bis 4, vorzugsweise 1 oder 2, bevorzugt 1 |
| 2 * N-6 | Ar'-28 | 0 bis 6, vorzugsweise 0 bis 3, bevorzugt 0 oder 1 | 0 bis 4, vorzugsweise 1 oder 2, bevorzugt 1 |
| 2 * N-1 | Ar'-28 | 0 | 0 bis 2, vorzugsweise 1 oder 2, bevorzugt 1 |
| 2 * N-3 | Ar'-28 | 0 | 0 bis 2, vorzugsweise 1 oder 2, bevorzugt 1 |
| 2 * N-5 | Ar'-28 | 0 | 0 bis 2, vorzugsweise 1 oder 2, bevorzugt 1 |
| 2 * N-6 | Ar'-28 | 0 | 0 bis 2, vorzugsweise 1 oder 2, bevorzugt 1 |
| 2 * N-1 | Ar'-29 | 0 bis 6, vorzugsweise 0 bis 3, bevorzugt 0 oder 1 | 0 bis 4, vorzugsweise 1 oder 2, bevorzugt 1 |
| 2 * N-3 | Ar'-29 | 0 bis 6, vorzugsweise 0 bis 3, bevorzugt 0 oder 1 | 0 bis 4, vorzugsweise 1 oder 2, bevorzugt 1 |
| 2 * N-5 | Ar'-29 | 0 bis 6, vorzugsweise 0 bis 3, bevorzugt 0 oder 1 | 0 bis 4, vorzugsweise 1 oder 2, bevorzugt 1 |
| 2 * N-6 | Ar'-29 | 0 bis 6, vorzugsweise 0 bis 3, bevorzugt 0 oder 1 | 0 bis 4, vorzugsweise 1 oder 2, bevorzugt 1 |
| 2 * N-1 | Ar'-29 | 0 | 0 bis 2, vorzugsweise 1 oder 2, bevorzugt 1 |
| 2 * N-3 | Ar'-29 | 0 | 0 bis 2, vorzugsweise 1 oder 2, bevorzugt 1 |
| 2 * N-5 | Ar'-29 | 0 | 0 bis 2, vorzugsweise 1 oder 2, bevorzugt 1 |
| 2 * N-6 | Ar'-29 | 0 | 0 bis 2, vorzugsweise 1 oder 2, bevorzugt 1 |
| 2 * N-1 | Ar'-30 | 0 bis 6, vorzugsweise 0 bis 3, bevorzugt 0 oder 1 | 0 bis 4, vorzugsweise 1 oder 2, bevorzugt 1 |
| 2 * N-3 | Ar'-30 | 0 bis 6, vorzugsweise 0 bis 3, bevorzugt 0 oder 1 | 0 bis 4, vorzugsweise 1 oder 2, bevorzugt 1 |
| 2 * N-5 | Ar'-30 | 0 bis 6, vorzugsweise 0 bis 3, bevorzugt 0 oder 1 | 0 bis 4, vorzugsweise 1 oder 2, bevorzugt 1 |
| 2 * N-6 | Ar'-30 | 0 bis 6, vorzugsweise 0 bis 3, bevorzugt 0 oder 1 | 0 bis 4, vorzugsweise 1 oder 2, bevorzugt 1 |
| 2 * N-1 | Ar'-30 | 0 | 0 bis 2, vorzugsweise 1 oder 2, bevorzugt 1 |
| 2 * N-3 | Ar'-30 | 0 | 0 bis 2, vorzugsweise 1 oder 2, bevorzugt 1 |
| 2 * N-5 | Ar'-30 | 0 | 0 bis 2, vorzugsweise 1 oder 2, bevorzugt 1 |
| 2 * N-6 | Ar'-30 | 0 | 0 bis 2, vorzugsweise 1 oder 2, bevorzugt 1 |
| 2 * N-1 | Ar'-31 | 0 bis 6, vorzugsweise 0 bis 3, bevorzugt 0 oder 1 | 0 bis 4, vorzugsweise 1 oder 2, bevorzugt 1 |
| 2 * N-3 | Ar'-31 | 0 bis 6, vorzugsweise 0 bis 3, bevorzugt 0 oder 1 | 0 bis 4, vorzugsweise 1 oder 2, bevorzugt 1 |
| 2 * N-5 | Ar'-31 | 0 bis 6, vorzugsweise 0 bis 3, bevorzugt 0 oder 1 | 0 bis 4, vorzugsweise 1 oder 2, bevorzugt 1 |
| 2 * N-6 | Ar'-31 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-1 | Ar'-31 | 0 | 0 bis 2, vorzugsweise 1 oder 2, bevorzugt 1 |
| 2 * N-3 | Ar'-31 | 0 | 0 bis 2, vorzugsweise 1 oder 2, bevorzugt 1 |
| 2 * N-5 | Ar'-31 | 0 | 0 bis 2, vorzugsweise 1 oder 2, bevorzugt 1 |
| 2 * N-6 | Ar'-31 | 0 | 0 bis 2, vorzugsweise 1 oder 2, bevorzugt 1 |
| 2 * N-1 | Ar'-32 | 0 bis 6, vorzugsweise 0 bis 3, bevorzugt 0 oder 1 | 0 bis 4, vorzugsweise 1 oder 2, bevorzugt 1 |
| 2 * N-3 | Ar'-32 | 0 bis 6, vorzugsweise 0 bis 3, bevorzugt 0 oder 1 | 0 bis 4, vorzugsweise 1 oder 2, bevorzugt 1 |
| 2 * N-5 | Ar'-32 | 0 bis 6, vorzugsweise 0 bis 3, bevorzugt 0 oder 1 | 0 bis 4, vorzugsweise 1 oder 2, bevorzugt 1 |
| 2 * N-6 | Ar'-32 | 0 bis 6, vorzugsweise 0 bis 3, bevorzugt 0 oder 1 | 0 bis 4, vorzugsweise 1 oder 2, bevorzugt 1 |
| 2 * N-1 | Ar'-32 | 0 | 0 bis 2, vorzugsweise 1 oder 2, bevorzugt 1 |
| 2 * N-3 | Ar'-32 | 0 | 0 bis 2, vorzugsweise 1 oder 2, bevorzugt 1 |
| 2 * N-5 | Ar'-32 | 0 | 0 bis 2, vorzugsweise 1 oder 2, bevorzugt 1 |
| 2 * N-6 | Ar' -32 | 0 | 0 bis 2, vorzugsweise 1 oder 2, bevorzugt 1 |
| 2 * N-1 | Ar'-33 | 0 bis 6, vorzugsweise 0 bis 3, bevorzugt 0 oder 1 | 0 bis 4, vorzugsweise 1 oder 2, bevorzugt 1 |
| 2 * N-3 | Ar' -33 | 0 bis 6, vorzugsweise 0 bis 3, bevorzugt 0 oder 1 | 0 bis 4, vorzugsweise 1 oder 2, bevorzugt 1 |
| 2 * N-5 | Ar'-33 | 0 bis 6, vorzugsweise 0 bis 3, bevorzugt 0 oder 1 | 0 bis 4, vorzugsweise 1 oder 2, bevorzugt 1 |
| 2 * N-6 | Ar'-33 | 0 bis 6, vorzugsweise 0 bis 3, bevorzugt 0 oder 1 | 0 bis 4, vorzugsweise 1 oder 2, bevorzugt 1 |
| 2 * N-1 | Ar'-33 | 0 | 0 bis 2, vorzugsweise 1 oder 2, bevorzugt 1 |
| 2 * N-3 | Ar'-33 | 0 | 0 bis 2, vorzugsweise 1 oder 2, bevorzugt 1 |
| 2 * N-5 | Ar'-33 | 0 | 0 bis 2, vorzugsweise 1 oder 2, bevorzugt 1 |
| 2 * N-6 | Ar'-33 | 0 | 0 bis 2, vorzugsweise 1 oder 2, bevorzugt 1 |
| 2 * N-1 | Ar'-34 | 0 bis 6, vorzugsweise 0 bis 3, bevorzugt 0 oder 1 | 0 bis 4, vorzugsweise 1 oder 2, bevorzugt 1 |
| 2 * N-3 | Ar'-34 | 0 bis 6, vorzugsweise 0 bis 3, bevorzugt 0 oder 1 | 0 bis 4, vorzugsweise 1 oder 2, bevorzugt 1 |
| 2 * N-5 | Ar'-34 | 0 bis 6, vorzugsweise 0 bis 3, bevorzugt 0 oder 1 | 0 bis 4, vorzugsweise 1 oder 2, bevorzugt 1 |
| 2 * N-6 | Ar'-34 | 0 bis 6, vorzugsweise 0 bis 3, bevorzugt 0 oder 1 | 0 bis 4, vorzugsweise 1 oder 2, bevorzugt 1 |
| 2 * N-1 | Ar'-34 | 0 | 0 bis 2, vorzugsweise 1 oder 2, bevorzugt 1 |
| 2 * N-3 | Ar'-34 | 0 | 0 bis 2, vorzugsweise 1 oder 2, bevorzugt 1 |
| 2 * N-5 | Ar'-34 | 0 | 0 bis 2, vorzugsweise 1 oder 2, bevorzugt 1 |
| 2 * N-6 | Ar'-34 | 0 | 0 bis 2, vorzugsweise 1 oder 2, bevorzugt 1 |
| 2 * N-1 | Ar'-35 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-3 | Ar'-35 | 0 bis 6, vorzugsweise 0 bis 3, bevorzugt 0 oder 1 | 0 bis 4, vorzugsweise 1 oder 2, bevorzugt 1 |
| 2 * N-5 | Ar'-35 | 0 bis 6, vorzugsweise 0 bis 3, bevorzugt 0 oder 1 | 0 bis 4, vorzugsweise 1 oder 2, bevorzugt 1 |
| 2 * N-6 | Ar'-35 | 0 bis 6, vorzugsweise 0 bis 3, bevorzugt 0 oder 1 | 0 bis 4, vorzugsweise 1 oder 2, bevorzugt 1 |
| 2 * N-1 | Ar'-35 | 0 | 0 bis 2, vorzugsweise 1 oder 2, bevorzugt 1 |
| 2 * N-3 | Ar'-35 | 0 | 0 bis 2, vorzugsweise 1 oder 2, bevorzugt 1 |
| 2 * N-5 | Ar'-35 | 0 | 0 bis 2, vorzugsweise 1 oder 2, bevorzugt 1 |
| 2 * N-6 | Ar'-35 | 0 | 0 bis 2, vorzugsweise 1 oder 2, bevorzugt 1 |
| 2 * N-1 | Ar'-36 | 0 bis 6, vorzugsweise 0 bis 3, bevorzugt 0 oder 1 | 0 bis 4, vorzugsweise 1 oder 2, bevorzugt 1 |
| 2 * N-3 | Ar'-36 | 0 bis 6, vorzugsweise 0 bis 3, bevorzugt 0 oder 1 | 0 bis 4, vorzugsweise 1 oder 2, bevorzugt 1 |
| 2 * N-5 | Ar'-36 | 0 bis 6, vorzugsweise 0 bis 3, bevorzugt 0 oder 1 | 0 bis 4, vorzugsweise 1 oder 2, bevorzugt 1 |
| 2 * N-6 | Ar'-36 | 0 bis 6, vorzugsweise 0 bis 3, bevorzugt 0 oder 1 | 0 bis 4, vorzugsweise 1 oder 2, bevorzugt 1 |
| 2 * N-1 | Ar'-36 | 0 | 0 bis 2, vorzugsweise 1 oder 2, bevorzugt 1 |
| 2 * N-3 | Ar'-36 | 0 | 0 bis 2, vorzugsweise 1 oder 2, bevorzugt 1 |
| 2 * N-5 | Ar'-36 | 0 | 0 bis 2, vorzugsweise 1 oder 2, bevorzugt 1 |
| 2 * N-6 | Ar'-36 | 0 | 0 bis 2, vorzugsweise 1 oder 2, bevorzugt 1 |
| 2 * N-1 | Ar'-36 | 0 bis 6, vorzugsweise 0 bis 3, bevorzugt 0 oder 1 | 0 bis 4, vorzugsweise 1 oder 2, bevorzugt 1 |
| 2 * N-3 | Ar'-36 | 0 bis 6, vorzugsweise 0 bis 3, bevorzugt 0 oder 1 | 0 bis 4, vorzugsweise 1 oder 2, bevorzugt 1 |
| 2 * N-5 | Ar'-36 | 0 bis 6, vorzugsweise 0 bis 3, bevorzugt 0 oder 1 | 0 bis 4, vorzugsweise 1 oder 2, bevorzugt 1 |
| 2 * N-6 | Ar' -36 | 0 bis 6, vorzugsweise 0 bis 3, bevorzugt 0 oder 1 | 0 bis 4, vorzugsweise 1 oder 2, bevorzugt 1 |
| 2 * N-1 | Ar'-36 | 0 | 0 bis 2, vorzugsweise 1 oder 2, bevorzugt 1 |
| 2 * N-3 | Ar'-36 | 0 | 0 bis 2, vorzugsweise 1 oder 2, bevorzugt 1 |
| 2 * N-5 | Ar'-36 | 0 | 0 bis 2, vorzugsweise 1 oder 2, bevorzugt 1 |
| 2 * N-6 | Ar'-36 | 0 | 0 bis 2, vorzugsweise 1 oder 2, bevorzugt 1 |
| 2 * N-1 | Ar'-37 | 0 bis 6, vorzugsweise 0 bis 3, bevorzugt 0 oder 1 | 0 bis 4, vorzugsweise 1 oder 2, bevorzugt 1 |
| 2 * N-3 | Ar'-37 | 0 bis 6, vorzugsweise 0 bis 3, bevorzugt 0 oder 1 | 0 bis 4, vorzugsweise 1 oder 2, bevorzugt 1 |
| 2 * N-5 | Ar'-37 | 0 bis 6, vorzugsweise 0 bis 3, bevorzugt 0 oder 1 | 0 bis 4, vorzugsweise 1 oder 2, bevorzugt 1 |
| 2 * N-6 | Ar'-37 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 2 * N-1 | Ar'-37 | 0 | 0 bis 2, vorzugsweise 1 oder 2, bevorzugt 1 |
| 2 * N-3 | Ar'-37 | 0 | 0 bis 2, vorzugsweise 1 oder 2, bevorzugt 1 |
| 2 * N-5 | Ar'-37 | 0 | 0 bis 2, vorzugsweise 1 oder 2, bevorzugt 1 |
| 2 * N-6 | Ar'-37 | 0 | 0 bis 2, vorzugsweise 1 oder 2, bevorzugt 1 |
| 2 * N-1 | Ar'-38 | 0 bis 6, vorzugsweise 0 bis 3, bevorzugt 0 oder 1 | 0 bis 4, vorzugsweise 1 oder 2, bevorzugt 1 |
| 2 * N-3 | Ar'-38 | 0 bis 6, vorzugsweise 0 bis 3, bevorzugt 0 oder 1 | 0 bis 4, vorzugsweise 1 oder 2, bevorzugt 1 |
| 2 * N-5 | Ar'-38 | 0 bis 6, vorzugsweise 0 bis 3, bevorzugt 0 oder 1 | 0 bis 4, vorzugsweise 1 oder 2, bevorzugt 1 |
| 2 * N-6 | Ar' -38 | 0 bis 6, vorzugsweise 0 bis 3, bevorzugt 0 oder 1 | 0 bis 4, vorzugsweise 1 oder 2, bevorzugt 1 |
| 2 * N-1 | Ar'-38 | 0 | 0 bis 2, vorzugsweise 1 oder 2, bevorzugt 1 |
| 2 * N-3 | Ar'-38 | 0 | 0 bis 2, vorzugsweise 1 oder 2, bevorzugt 1 |
| 2 * N-5 | Ar'-38 | 0 | 0 bis 2, vorzugsweise 1 oder 2, bevorzugt 1 |
| 2 * N-6 | Ar'-38 | 0 | 0 bis 2, vorzugsweise 1 oder 2, bevorzugt 1 |
| 2 * N-1 | Ar'-39 | 0 bis 6, vorzugsweise 0 bis 3, bevorzugt 0 oder 1 | 0 bis 4, vorzugsweise 1 oder 2, bevorzugt 1 |
| 2 * N-3 | Ar'-39 | 0 bis 6, vorzugsweise 0 bis 3, bevorzugt 0 oder 1 | 0 bis 4, vorzugsweise 1 oder 2, bevorzugt 1 |
| 2 * N-5 | Ar'-39 | 0 bis 6, vorzugsweise 0 bis 3, bevorzugt 0 oder 1 | 0 bis 4, vorzugsweise 1 oder 2, bevorzugt 1 |
| 2 * N-6 | Ar'-39 | 0 bis 6, vorzugsweise 0 bis 3, bevorzugt 0 oder 1 | 0 bis 4, vorzugsweise 1 oder 2, bevorzugt 1 |
| 2 * N-1 | Ar'-39 | 0 | 0 bis 2, vorzugsweise 1 oder 2, bevorzugt 1 |
| 2 * N-3 | Ar'-39 | 0 | 0 bis 2, vorzugsweise 1 oder 2, bevorzugt 1 |
| 2 * N-5 | Ar'-39 | 0 | 0 bis 2, vorzugsweise 1 oder 2, bevorzugt 1 |
| 2 * N-6 | Ar'-39 | 0 | 0 bis 2, vorzugsweise 1 oder 2, bevorzugt 1 |
| 2 * N-1 | Ar'-40 | 0 bis 6, vorzugsweise 0 bis 3, bevorzugt 0 oder 1 | 0 bis 4, vorzugsweise 1 oder 2, bevorzugt 1 |
| 2 * N-3 | Ar'-40 | 0 bis 6, vorzugsweise 0 bis 3, bevorzugt 0 oder 1 | 0 bis 4, vorzugsweise 1 oder 2, bevorzugt 1 |
| 2 * N-5 | Ar'-40 | 0 bis 6, vorzugsweise 0 bis 3, bevorzugt 0 oder 1 | 0 bis 4, vorzugsweise 1 oder 2, bevorzugt 1 |
| 2 * N-6 | Ar'-40 | 0 bis 6, vorzugsweise 0 bis 3, bevorzugt 0 oder 1 | 0 bis 4, vorzugsweise 1 oder 2, bevorzugt 1 |
| 2 * N-1 | Ar'-40 | 0 | 0 bis 2, vorzugsweise 1 oder 2, bevorzugt 1 |
| 2 * N-3 | Ar'-40 | 0 | 0 bis 2, vorzugsweise 1 oder 2, bevorzugt 1 |
| 2 * N-5 | Ar'-40 | 0 | 0 bis 2, vorzugsweise 1 oder 2, bevorzugt 1 |
| 2 * N-6 | Ar'-40 | 0 | 0 bis 2, vorzugsweise 1 oder 2, bevorzugt 1 |
| 4 * N-1 | Ar'-41 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| 4 * N-3 | Ar'-41 | 0 bis 6, vorzugsweise 0 bis 3, bevorzugt 0 oder 1 | 0 bis 4, vorzugsweise 1 oder 2, bevorzugt 1 |
| 4 * N-5 | Ar'-41 | 0 bis 6, vorzugsweise 0 bis 3, bevorzugt 0 oder 1 | 0 bis 4, vorzugsweise 1 oder 2, bevorzugt 1 |
| 4 * N-6 | Ar'-41 | 0 bis 6, vorzugsweise 0 bis 3, bevorzugt 0 oder 1 | 0 bis 4, vorzugsweise 1 oder 2, bevorzugt 1 |
| 4 * N-1 | Ar'-41 | 0 | 0 bis 2, vorzugsweise 1 oder 2, bevorzugt 1 |
| 4 * N-3 | Ar'-41 | 0 | 0 bis 2, vorzugsweise 1 oder 2, bevorzugt 1 |
| 4 * N-5 | Ar'-41 | 0 | 0 bis 2, vorzugsweise 1 oder 2, bevorzugt 1 |
| 4 * N-6 | Ar'-41 | 0 | 0 bis 2, vorzugsweise 1 oder 2, bevorzugt 1 |
| 4 * N-1 | Ar'-42 | 0 bis 6, vorzugsweise 0 bis 3, bevorzugt 0 oder 1 | 0 bis 4, vorzugsweise 1 oder 2, bevorzugt 1 |
| 4 * N-3 | Ar'-42 | 0 bis 6, vorzugsweise 0 bis 3, bevorzugt 0 oder 1 | 0 bis 4, vorzugsweise 1 oder 2, bevorzugt 1 |
| 4 * N-5 | Ar'-42 | 0 bis 6, vorzugsweise 0 bis 3, bevorzugt 0 oder 1 | 0 bis 4, vorzugsweise 1 oder 2, bevorzugt 1 |
| 4 * N-6 | Ar'-42 | 0 bis 6, vorzugsweise 0 bis 3, bevorzugt 0 oder 1 | 0 bis 4, vorzugsweise 1 oder 2, bevorzugt 1 |
| 4 * N-1 | Ar'-42 | 0 | 0 bis 2, vorzugsweise 1 oder 2, bevorzugt 1 |
| 4 * N-3 | Ar'-42 | 0 | 0 bis 2, vorzugsweise 1 oder 2, bevorzugt 1 |
| 4 * N-5 | Ar'-42 | 0 | 0 bis 2, vorzugsweise 1 oder 2, bevorzugt 1 |
| 4 * N-6 | Ar'-42 | 0 | 0 bis 2, vorzugsweise 1 oder 2, bevorzugt 1 |
| 4 * N-1 | Ar'-43 | 0 bis 6, vorzugsweise 0 bis 3, bevorzugt 0 oder 1 | 0 bis 4, vorzugsweise 1 oder 2, bevorzugt 1 |
| 4 * N-3 | Ar'-43 | 0 bis 6, vorzugsweise 0 bis 3, bevorzugt 0 oder 1 | 0 bis 4, vorzugsweise 1 oder 2, bevorzugt 1 |
| 4 * N-5 | Ar'-43 | 0 bis 6, vorzugsweise 0 bis 3, bevorzugt 0 oder 1 | 0 bis 4, vorzugsweise 1 oder 2, bevorzugt 1 |
| 4 * N-6 | Ar'-43 | 0 bis 6, vorzugsweise 0 bis 3, bevorzugt 0 oder 1 | 0 bis 4, vorzugsweise 1 oder 2, bevorzugt 1 |
| 4 * N-1 | Ar' -43 | 0 | 0 bis 2, vorzugsweise 1 oder 2, bevorzugt 1 |
| 4 * N-3 | Ar' -43 | 0 | 0 bis 2, vorzugsweise 1 oder 2, bevorzugt 1 |
| 4 * N-5 | Ar'-43 | 0 | 0 bis 2, vorzugsweise 1 oder 2, bevorzugt 1 |
| 4 * N-6 | Ar'-43 | 0 | 0 bis 2, vorzugsweise 1 oder 2, bevorzugt 1 |
| N-2 | Ar'-44 | 0 bis 6, vorzugsweise 0 bis 3, bevorzugt 0 oder 1 | 0 bis 4, vorzugsweise 1 oder 2, bevorzugt 1 |
| N-4 | Ar'-44 | 0 bis 6, vorzugsweise 0 bis 3, bevorzugt 0 oder 1 | 0 bis 4, vorzugsweise 1 oder 2, bevorzugt 1 |
| N-2 | Ar'-44 | 0 | 0 bis 2, vorzugsweise 1 oder 2, bevorzugt 1 |
| N-4 | Ar'-44 | 0 | 0 bis 2, vorzugsweise 1 oder 2, bevorzugt 1 |
| N-2 | Ar'-45 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-4 | Ar'-45 | 0 bis 6, vorzugsweise 0 bis 3, bevorzugt 0 oder 1 | 0 bis 4, vorzugsweise 1 oder 2, bevorzugt 1 |
| N-2 | Ar'-45 | 0 | 0 bis 2, vorzugsweise 1 oder 2, bevorzugt 1 |
| N-4 | Ar'-45 | 0 | 0 bis 2, vorzugsweise 1 oder 2, bevorzugt 1 |
| N-2 | Ar'-46 | 0 bis 6, vorzugsweise 0 bis 3, bevorzugt 0 oder 1 | 0 bis 4, vorzugsweise 1 oder 2, bevorzugt 1 |
| N-4 | Ar'-46 | 0 bis 6, vorzugsweise 0 bis 3, bevorzugt 0 oder 1 | 0 bis 4, vorzugsweise 1 oder 2, bevorzugt 1 |
| N-2 | Ar'-46 | 0 | 0 bis 2, vorzugsweise 1 oder 2, bevorzugt 1 |
| N-4 | Ar'-46 | 0 | 0 bis 2, vorzugsweise 1 oder 2, bevorzugt 1 |
| N-2 | Ar'-47 | 0 bis 6, vorzugsweise 0 bis 3, bevorzugt 0 oder 1 | 0 bis 4, vorzugsweise 1 oder 2, bevorzugt 1 |
| N-4 | Ar'-47 | 0 bis 6, vorzugsweise 0 bis 3, bevorzugt 0 oder 1 | 0 bis 4, vorzugsweise 1 oder 2, bevorzugt 1 |
| N-2 | Ar'-47 | 0 | 0 bis 2, vorzugsweise 1 oder 2, bevorzugt 1 |
| N-4 | Ar'-47 | 0 | 0 bis 2, vorzugsweise 1 oder 2, bevorzugt 1 |
| 2 * N-2 | Ar'-48 | 0 bis 6, vorzugsweise 0 bis 3, bevorzugt 0 oder 1 | 0 bis 4, vorzugsweise 1 oder 2, bevorzugt 1 |
| 2 * N-4 | Ar'-48 | 0 bis 6, vorzugsweise 0 bis 3, bevorzugt 0 oder 1 | 0 bis 4, vorzugsweise 1 oder 2, bevorzugt 1 |
| 2 * N-2 | Ar'-48 | 0 | 0 bis 2, vorzugsweise 1 oder 2, bevorzugt 1 |
| 2 * N-4 | Ar'-48 | 0 | 0 bis 2, vorzugsweise 1 oder 2, bevorzugt 1 |
| 2 * N-2 | Ar'-49 | 0 bis 6, vorzugsweise 0 bis 3, bevorzugt 0 oder 1 | 0 bis 4, vorzugsweise 1 oder 2, bevorzugt 1 |
| 2 * N-4 | Ar'-49 | 0 bis 6, vorzugsweise 0 bis 3, bevorzugt 0 oder 1 | 0 bis 4, vorzugsweise 1 oder 2, bevorzugt 1 |
| 2 * N-2 | Ar'-49 | 0 | 0 bis 2, vorzugsweise 1 oder 2, bevorzugt 1 |
| 2 * N-4 | Ar'-49 | 0 | 0 bis 2, vorzugsweise 1 oder 2, bevorzugt 1 |
| 2 * N-2 | Ar'-50 | 0 bis 6, vorzugsweise 0 bis 3, bevorzugt 0 oder 1 | 0 bis 4, vorzugsweise 1 oder 2, bevorzugt 1 |
| 2 * N-4 | Ar'-50 | 0 bis 6, vorzugsweise 0 bis 3, bevorzugt 0 oder 1 | 0 bis 4, vorzugsweise 1 oder 2, bevorzugt 1 |
| 2 * N-2 | Ar'-50 | 0 | 0 bis 2, vorzugsweise 1 oder 2, bevorzugt 1 |
| 2 * N-4 | Ar'-50 | 0 | 0 bis 2, vorzugsweise 1 oder 2, bevorzugt 1 |
| N-1 | Ar'-51 | 0 bis 6, vorzugsweise 0 bis 3, bevorzugt 0 oder 1 | 0 bis 4, vorzugsweise 1 oder 2, bevorzugt 1 |
| N-3 | Ar'-51 | 0 bis 6, vorzugsweise 0 bis 3, bevorzugt 0 oder 1 | 0 bis 4, vorzugsweise 1 oder 2, bevorzugt 1 |
| N-5 | Ar'-51 | 0 bis 6, vorzugsweise 0 bis 3, bevorzugt 0 oder 1 | 0 bis 4, vorzugsweise 1 oder 2, bevorzugt 1 |
| N-6 | Ar'-51 | 0 bis 6, vorzugsweise 0 bis 3, bevorzugt 0 oder 1 | 0 bis 4, vorzugsweise 1 oder 2, bevorzugt 1 |

(fortgesetzt)

| Teilstruktur der Formel | Teilstruktur der Formel | Index v, t und s in den Teilstrukturen gemäß den Formeln (N-1) bis (N-6) | Summe der Indices o, n, m und l in den Teilstrukturen gemäß den Formeln (Ar'-1) bis (Ar'-53) |
|---|---|---|---|
| N-1 | Ar'-51 | 0 | 0 bis 2, vorzugsweise 1 oder 2, bevorzugt 1 |
| N-3 | Ar'-51 | 0 | 0 bis 2, vorzugsweise 1 oder 2, bevorzugt 1 |
| N-5 | Ar'-51 | 0 | 0 bis 2, vorzugsweise 1 oder 2, bevorzugt 1 |
| N-6 | Ar'-51 | 0 | 0 bis 2, vorzugsweise 1 oder 2, bevorzugt 1 |
| N-1 | Ar'-52 | 0 bis 6, vorzugsweise 0 bis 3, bevorzugt 0 oder 1 | 0 bis 4, vorzugsweise 1 oder 2, bevorzugt 1 |
| N-3 | Ar'-52 | 0 bis 6, vorzugsweise 0 bis 3, bevorzugt 0 oder 1 | 0 bis 4, vorzugsweise 1 oder 2, bevorzugt 1 |
| N-5 | Ar'-52 | 0 bis 6, vorzugsweise 0 bis 3, bevorzugt 0 oder 1 | 0 bis 4, vorzugsweise 1 oder 2, bevorzugt 1 |
| N-6 | Ar'-52 | 0 bis 6, vorzugsweise 0 bis 3, bevorzugt 0 oder 1 | 0 bis 4, vorzugsweise 1 oder 2, bevorzugt 1 |
| N-1 | Ar'-52 | 0 | 0 bis 2, vorzugsweise 1 oder 2, bevorzugt 1 |
| N-3 | Ar'-52 | 0 | 0 bis 2, vorzugsweise 1 oder 2, bevorzugt 1 |
| N-5 | Ar'-52 | 0 | 0 bis 2, vorzugsweise 1 oder 2, bevorzugt 1 |
| N-6 | Ar'-52 | 0 | 0 bis 2, vorzugsweise 1 oder 2, bevorzugt 1 |
| N-1 | Ar'-53 | 0 bis 6, vorzugsweise 0 bis 3, bevorzugt 0 oder 1 | 0 bis 4, vorzugsweise 1 oder 2, bevorzugt 1 |
| N-3 | Ar'-53 | 0 bis 6, vorzugsweise 0 bis 3, bevorzugt 0 oder 1 | 0 bis 4, vorzugsweise 1 oder 2, bevorzugt 1 |
| N-5 | Ar'-53 | 0 bis 6, vorzugsweise 0 bis 3, bevorzugt 0 oder 1 | 0 bis 4, vorzugsweise 1 oder 2, bevorzugt 1 |
| N-6 | Ar'-53 | 0 bis 6, vorzugsweise 0 bis 3, bevorzugt 0 oder 1 | 0 bis 4, vorzugsweise 1 oder 2, bevorzugt 1 |
| N-1 | Ar'-53 | 0 | 0 bis 2, vorzugsweise 1 oder 2, bevorzugt 1 |
| N-3 | Ar'-53 | 0 | 0 bis 2, vorzugsweise 1 oder 2, bevorzugt 1 |
| N-5 | Ar'-53 | 0 | 0 bis 2, vorzugsweise 1 oder 2, bevorzugt 1 |
| N-6 | Ar'-53 | 0 | 0 bis 2, vorzugsweise 1 oder 2, bevorzugt 1 |

Die Gruppe $R^1$ in obigen Kombinationen, wobei die Gruppe $R^1$ ungleich der Gruppe H ist, sind bevorzugt ausgewählt aus Lochleitergruppen und/oder Elektronenleitergruppen, wobei die Elektronenleitergruppen vorzugsweise mindestens 2 Stickstoffatome in einem Sechsring oder in zwei kondensierten Sechsringen umfassen, besonders bevorzugt ausgewählt sind aus Triazinen oder Pyrimidinen. Ferner sind Gruppen bevorzugt, die eine **TADF** (thermally activated delayed fluorescence) fördern, je nach Einsatzzweck der erfindungsgemäßen Verbindungen.

Von den zuvor dargelegten Verbindungen sind insbesondere Verbindungen bevorzugt, die kondensierte, besonders bevorzugt flächig kondensierte aromatische oder heteroaromatische Ringsysteme aufweisen, wie zum Beispiel Verbindungen mit Teilstrukturen der Formeln (Ar'-2) bis (Ar'-12), (Ar'-18) bis (Ar'-34) und (Ar'-44) bis (Ar'-53), besonders bevorzugt (Ar'-4) bis (Ar'-9), (Ar'-22) bis (Ar'-31) und (Ar'-45) bis (Ar'-53), speziell bevorzugt (Ar'-4), (Ar'-5), (Ar'-22) bis (Ar'-24), (Ar'-45) und (Ar'-48).

Gemäß einer weiteren Ausführungsform der vorliegenden Erfindung sind Verbindungen bevorzugt, die Fluoren-, Dibenzofuran, Dibenzothiofuran, Carbazol, Spirobifluoren und ähnlich Strukturen umfassen, besonders, wie zum Beispiel Verbindungen mit Teilstrukturen der Formeln (Ar'-10) bis (Ar'-15) und (Ar'-32) bis (Ar'-43), besonders bevorzugt (Ar'-13) bis (Ar'-15) und (Ar'-35) bis (Ar'-43).

**In** einer weiteren Ausgestaltung kann vorgesehen sein, dass die Verbindung, die einer organischen elektronischen Vorrichtung als aktive Verbindung einsetzbar ist, ein kondensiertes aromatisches oder heteroaromatisches

Ringsystem mit mindestens 2, vorzugsweise drei kondensierten Ringen umfasst, welches gegebenenfalls substituiert sein kann.

Gemäß einer weiteren Ausführungsform kann vorgesehen sein, dass die Verbindung, die in einer organischen elektronischen Vorrichtung als aktive Verbindung einsetzbar ist, eine Lochtransportgruppe umfasst, wobei vorzugsweise in einer Struktur gemäß den Formeln (I) bis (XVIII) und/oder den Formeln (Ia) bis (XVIIIa) die Gruppe Ar, die in einer Gruppe Y enthalten ist, oder eine Gruppe R eine Lochtransportgruppe umfasst, vorzugsweise darstellt oder in einer Struktur gemäß den Formeln (N-1) bis (N-6), (Ar-1) bis (Ar-54) und/oder (Ar'-1) bis (Ar'-53) eine Gruppe $R^1$ eine Lochtransportgruppe umfasst, vorzugsweise darstellt. Lochtransportgruppen sind in der Fachwelt bekannt, wobei diese vorzugsweise Triarylamin- oder Carbazolgruppen umfassen.

Vorzugsweise kann vorgesehen sein, dass die Lochtransportgruppe eine Gruppe umfasst, bevorzugt für eine Gruppe steht, die ausgewählt ist aus den Formeln (H-1) bis (H-3),

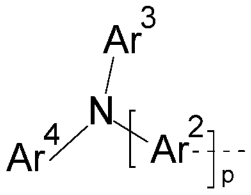

Formel (H-1)

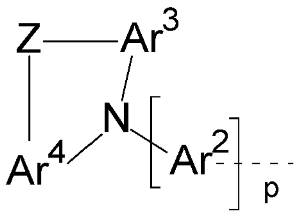

Formel (H-2)

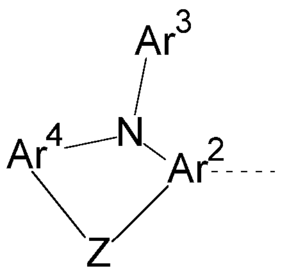

Formel (H-3)

wobei die gestrichelte Bindung die Anbindungsposition markiert und $Ar^2$, $Ar^3$, $Ar^4$ jeweils unabhängig eine Arylgruppe mit 6 bis 40 C-Atomen oder eine Heteroarylgruppe mit 3 bis 40 C-Atomen ist, welche jeweils durch einen oder mehrere Reste $R^1$ substituiert sein kann,

p 0 oder 1 ist, und

Z für eine Bindung oder $C(R^1)_2$, $Si(R^1)_2$, C=O, N-$R^1$, N-$Ar^1$, $BR^1$, $PR^1$, $PO(R^1)$, SO, $SO_2$, Se, O oder S, vorzugsweise für eine Bindung oder $C(R^1)_2$, N-$R^1$, O oder S steht, wobei die Symbole $Ar^1$ und $R^1$ die zuvor, insbesondere für Formeln (I) bis (XVIII) genannte Bedeutung aufweisen. Hierbei sind die Substituenten $R^1$ in den Strukturen der Formeln (H-1) bis (H-3) bei Strukturen gemäß Formeln (N-1) bis (N-6), (Ar-1) bis (Ar-54) und/oder (Ar'-1) bis (Ar'-53) durch Substituenten $R^2$ zu ersetzen. Ferner ist das Vorhandensein einer N-N-Bindung vorzugsweise ausgeschlossen.

Weiterhin kann vorgesehen sein, dass die Lochtransportgruppe eine Gruppe umfasst, bevorzugt für eine Gruppe steht, die ausgewählt ist aus den Formeln (H-4) bis (H-26),

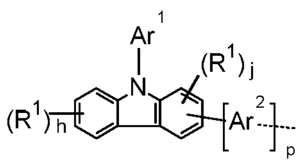

Formel (H-4)

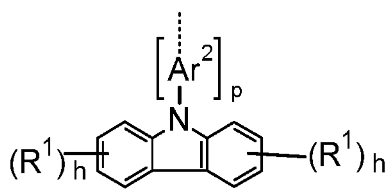

Formel (H-5)

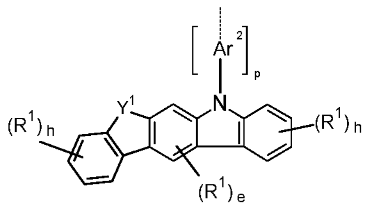

Formel (H-6)

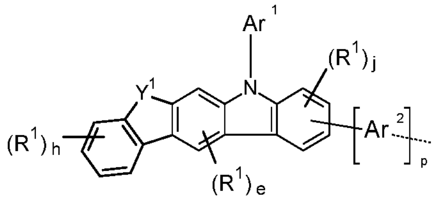

Formel (H-7)

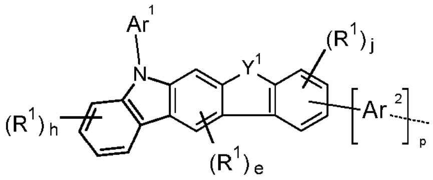

Formel (H-8)

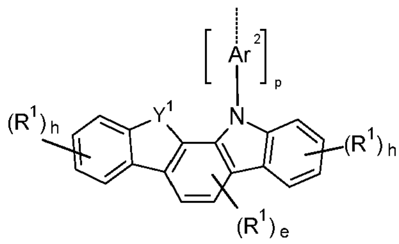

Formel (H-9)

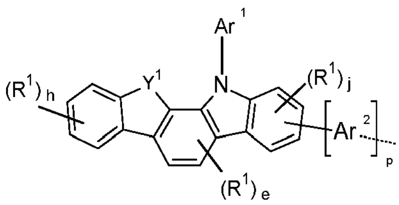

Formel (H-10)

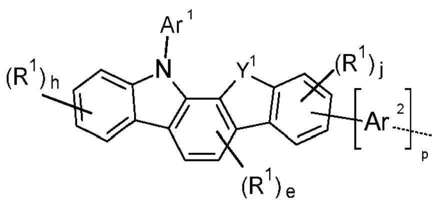

Formel (H-11)

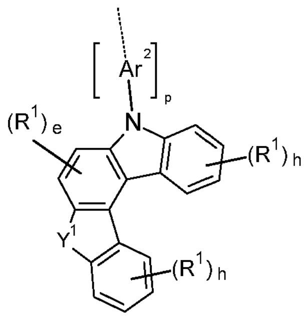

Formel (H-12)

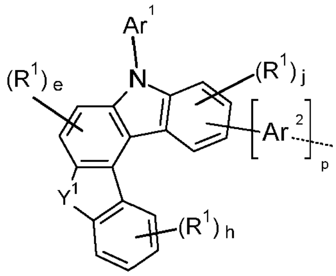

Formel (H-13)

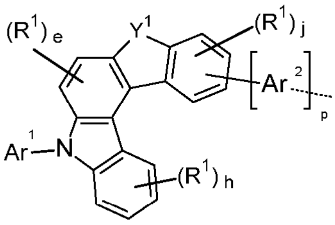

Formel (H-14)

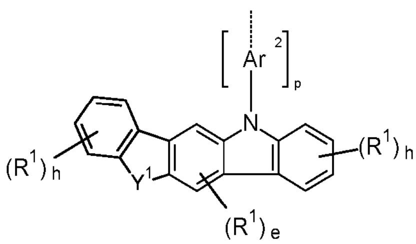

Formel (H-15)

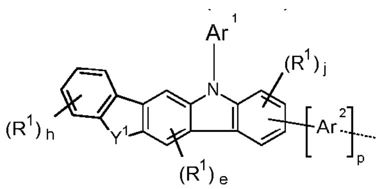

Formel (H-16)

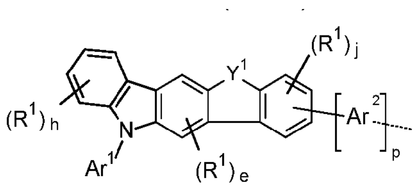

Formel (H-17)

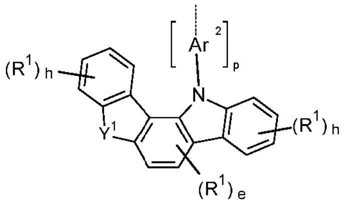

Formel (H-18)

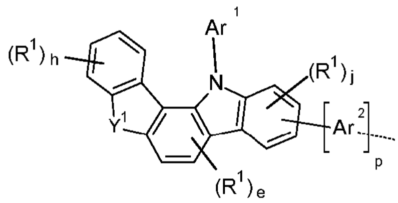

Formel (H-19)

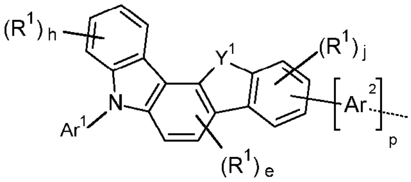

Formel (H-20)

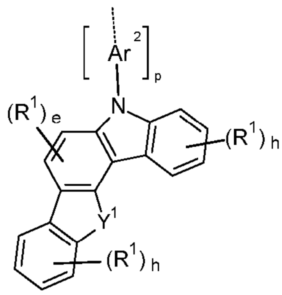

Formel (H-21)

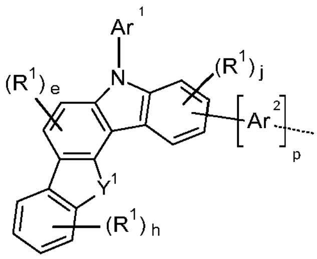

Formel (H-22)

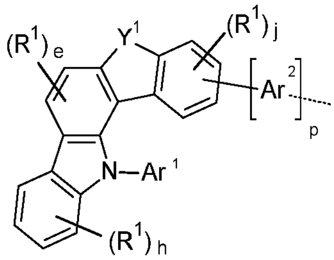

Formel (H-23)

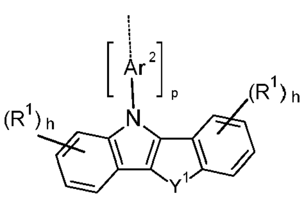

Formel (H-24)

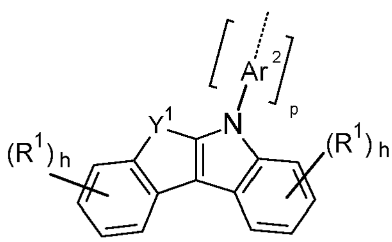

Formel (H-25)

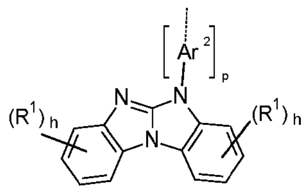

Formel (H-26)

wobei $Y^1$ O, S, $C(R^1)_2$, $NR^1$ oder $NAr^1$ darstellt, die gestrichelte Bindung die Anbindungsposition markiert, e 0, 1 oder 2 ist, j 0, 1, 2 oder 3 ist, h gleich oder verschieden bei jedem Auftreten 0, 1, 2, 3 oder 4 ist, p 0 oder 1 ist, $Ar^1$ und $R^1$ die zuvor, insbesondere für Formeln (I) bis (XVIII) und $Ar^2$ die zuvor, insbesondere für Formel (H-1) oder (H-2) genannten Bedeutungen aufweisen. Hierbei sind die Substituenten $R^1$ in den Strukturen der Formeln (H-3) bis (H-26) bei Strukturen gemäß Formeln (N-1) bis (N-6), (Ar-1) bis (Ar-54) und/oder (Ar'-1) bis (Ar'-53) durch Substituenten $R^2$ zu ersetzen.

Ferner ist das Vorhandensein einer N-N-Bindung vorzugsweise ausgeschlossen.

Die zuvor dargelegten Lochtransportgruppen der Formeln (H-1) bis (H-26) stellen bevorzugte Reste $R^1$ gemäß Formeln (I) bis (XVIII) oder bevorzugte Ausführungsformen dieser Formel dar, wobei in diesem Fall die in den Formeln (H-1) bis (H-26) dargelegten Gruppen $R^1$ durch Reste $R^2$ zu ersetzen sind.

Aus der obigen Formulierung ist ersichtlich, dass, falls der Index p = 0 ist, die entsprechende Gruppe $Ar^2$ nicht vorhanden ist und eine Bindung gebildet wird.

Bevorzugt kann die Gruppe $Ar^2$ mit dem aromatischen oder heteroaromatischen Rest oder dem Stickstoffatom, an den die Gruppe $Ar^2$ gemäß den Formeln (H-1) bis (H-26) gebunden sein kann, eine durchgängige Konjugation ausbilden.

In einer weiteren bevorzugten Ausführungsform der Erfindung steht $Ar^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formeln (I) bis (XVIII) genannte Bedeutung aufweisen kann. Besonders bevorzugt steht $Ar^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^1$ substituiert sein kann, bevorzugt aber unsubstituiert ist, wobei $R^1$ die zuvor, insbesondere für Formeln (I) bis (XVIII) genannte Bedeutung aufweisen kann.

Weiterhin bevorzugt steht das unter anderem in Formeln (H-1) bis (H-26) dargelegte Symbol $Ar^2$ für einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische

Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formeln (H-1) bis (H-26) dargelegte Gruppe $Ar^2$ ein aromatisches Ringsystem mit höchstens zwei kondensierten aromatischen und/oder heteroaromatischen 6-Ringen, vorzugsweise kein kondensiertes aromatisches oder heteroaromatisches Ringsystem mit kondensierten 6-Ringen umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt. Besonders bevorzugt sind Strukturen, die keine Kondensation aufweisen, wie beispielsweise Phenyl-, Biphenyl-, Terphenyl- und/oder Quaterphenyl-Strukturen.

Ferner kann vorgesehen sein, dass die unter anderem in Formeln (H-1) bis (H-26) dargelegte Gruppe $Ar^2$ höchstens 1 Stickstoffatom, bevorzugt höchstens 2 Heteroatome, insbesondere bevorzugt höchstens ein Heteroatom und besonders bevorzugt kein Heteroatom aufweist.

In einer weiteren bevorzugten Ausführungsform der Erfindung steht $Ar^3$ und/oder $Ar^4$ gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^1$ substituiert sein kann, bevorzugt aber unsubstituiert ist, wobei $R^1$ die zuvor, insbesondere in Formeln (I) bis (XVIII) dargestellte Bedeutung aufweisen kann.

In einer weiteren bevorzugten Ausführungsform kann vorgesehen sein, dass die Verbindung, die in einer organischen elektronischen Vorrichtung als aktive Verbindung einsetzbar ist, einen Elektronentransportgruppe-umfassenden Rest umfasst, wobei vorzugsweise in einer Struktur gemäß den Formeln (I) bis (XVIII) und/oder den Formeln (Ia) bis (XVIIIa) die

Gruppe Ar, die in einer Gruppe Y enthalten ist, oder eine Gruppe R einen Elektronentransportgruppe-umfassenden Rest umfasst, vorzugsweise darstellt, oder in einer Struktur gemäß den Formeln (N-1) bis (N-6), (Ar-1) bis (Ar-54)

und/oder (Ar'-1) bis (Ar'-53) eine Gruppe $R^1$ einen Elektronentransportgruppe-umfassenden Rest umfasst, vorzugsweise darstellt. Elektronentransportgruppen sind in der Fachwelt weithin bekannt und fördern die Fähigkeit von Verbindungen, Elektronen zu transportieren und/oder zu leiten.

Weiterhin zeigen Verbindungen, die in einer organischen elektronischen Vorrichtung als aktive Verbindung einsetzbar sind, überraschende Vorteile, die mindestens eine Struktur umfassen, die aus der Gruppe Pyridine, Pyrimidine, Pyrazine, Pyridazine, Triazine, Chinazoline, Chinoxaline, Chinoline, Isochinoline, Imidazole und/oder Benzimidazole ausgewählt ist, wobei Pyrimidine, Triazine und Chinazoline besonders bevorzugt sind. Diese Strukturen fördern im Allgemeinen die Fähigkeit von Verbindungen, Elektronen zu transportieren und/oder zu leiten.

In einer bevorzugten Ausgestaltung der vorliegenden Erfindung kann vorgesehen sein, dass der Elektronentransportgruppe-umfassende Rest für eine Gruppe steht, die durch die Formel (QL) darstellbar ist,

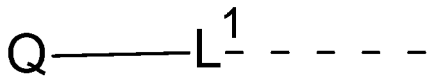

Formel (QL)

worin $L^1$ eine Bindung oder ein aromatisches oder heteroaromatisches Ringsystem mit 5 bis 40, bevorzugt 5 bis 30 aromatischen Ringatomen darstellt, welches durch einen oder mehrere Reste $R^1$ substituiert sein kann, Q eine Elektronentransportgruppe ist, wobei $R^1$ die zuvor, insbesondere für Formeln (I) bis (XVIII) genannte Bedeutung aufweist, und die Bindung die Anbindungsposition markiert. Hierbei sind die Substituenten $R^1$ in der Struktur der Formel (QL) bei Strukturen gemäß Formeln (N-1) bis (N-6), (Ar-1) bis (Ar-54) und/oder (Ar'-1) bis (Ar'-53) durch Substituenten $R^2$ zu ersetzen.

Bevorzugt kann die Gruppe $L^1$ mit der Gruppe Q und dem Atom, bevorzugt dem Kohlen- oder Stickstoffatom, an den die Gruppe $L^1$ gemäß Formel (QL) gebunden ist, eine durchgängige Konjugation ausbilden. Eine durchgängige Konjugation der aromatischen beziehungsweise heteroaromatischen Systeme wird ausgebildet, sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen der elektronentransportierenden Gruppe Q und dem Atom, über das die Gruppe der Formel (QL) an weitere Strukturelemente einer erfindungsgemäßen Verbindung bindet, liegt. Im Gegensatz hierzu kann bei einer zweiten Spirobifluorenstruktur eine durchgängige Konjugation ausgebildet werden, falls die Verbindung zwischen der Gruppe Q und dem aromatischen oder heteroaromatischen Rest, an den die Gruppe $L^1$ gemäß Formel (QL) gebunden ist, über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen der Gruppe Q und dem aromatischen oder heteroaromatischen Rest, an den die Gruppe $L^1$ gemäß Formel (QL) gebunden ist, über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen.

In einer weiteren bevorzugten Ausführungsform der Erfindung steht $L^1$ für eine Bindung oder für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor, insbesondere für Formeln (I) bis (XVIII) genannte Bedeutung aufweisen kann. Besonders bevorzugt steht L' für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor, insbesondere für Formeln (I) bis (XVIII) genannte Bedeutung aufweisen kann.

Weiterhin bevorzugt steht das unter anderem in Formel (QL) dargelegte Symbol L' gleich oder verschieden bei jedem Auftreten für eine Bindung oder einen Aryl- oder Heteroarylrest mit 5 bis 24 Ringatomen, vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die in Formel (QL) dargelegte Gruppe L' ein aromatisches Ringsystem mit höchstens zwei kondensierten aromatischen und/oder heteroaromatischen 6-Ringen, vorzugsweise kein kondensiertes aromatisches oder heteroaromatisches Ringsystem umfasst. Demgemäß sind Naphthylstrukturen gegenüber Anthracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme L’ sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen

oder mehrere Reste $R^1$ substituiert sein können, bevorzugt aber unsubstituiert sind.

Ferner kann vorgesehen sein, dass die unter anderem in Formel (QL) dargelegte Gruppe $L^1$ höchstens 1 Stickstoffatom, bevorzugt höchstens 2 Heteroatome, insbesondere bevorzugt höchstens ein Heteroatom und besonders bevorzugt kein Heteroatom aufweist.

Vorzugsweise kann die unter anderem in den Formel (QL) dargelegte Gruppe Q beziehungsweise die Elektronentransportgruppe ausgewählt sein aus Strukturen der Formeln (Q-1), (Q-2), (Q-4), (Q-4), (Q-5), (Q-6), (Q-7), (Q-8), (Q-9) und/oder (Q-10)

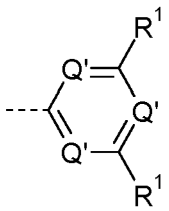

Formel (Q-1)

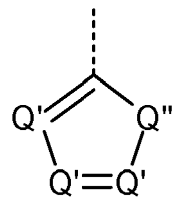

Formel (Q-2)

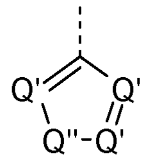

Formel (Q-3)

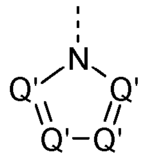

Formel (Q-4)

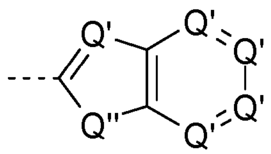

Formel (Q-5)

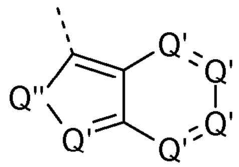

Formel (Q-6)

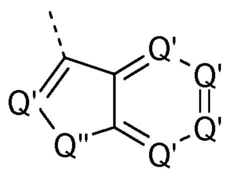

Formel (Q-7)

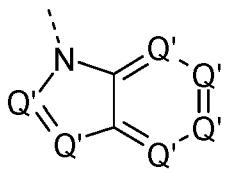

Formel (Q-8)

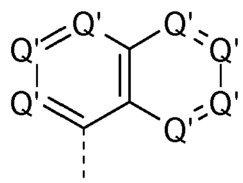

Formel (Q-9)

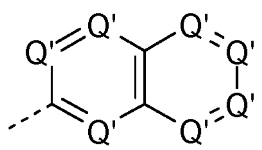

Formel (Q-10),

wobei die gestrichelte Bindung die Anbindungsposition markiert,

Q’ bei jedem Auftreten gleich oder verschieden $CR^1$ oder N darstellt, und
Q" $NR^1$, O oder S darstellt,

wobei wenigstens ein Q’ gleich N und

$R^1$ wie zuvor, insbesondere in Formeln (I) bis (XVIII) definiert ist.

Die Substituenten $R^1$ in den Strukturen der Formeln (Q-1) bis (Q-10) sind bei Strukturen gemäß Formeln (N-1) bis (N-6), (Ar-1) bis (Ar-54) und/oder (Ar'-1) bis (Ar'-53) durch Substituenten $R^2$ zu ersetzen.

Weiterhin kann die unter anderem in den Formel (QL) dargelegte Gruppe Q beziehungsweise die Elektronentransportgruppe vorzugsweise ausgewählt sein aus einer Struktur der Formeln (Q-11), (Q-12), (Q-13), (Q-14) und/oder (Q-15)

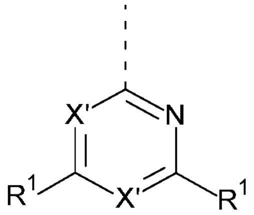

Formel (Q-11)

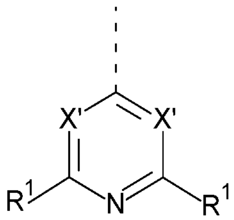

Formel (Q-12)

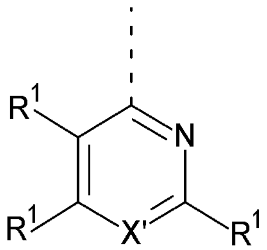

Formel (Q-13)

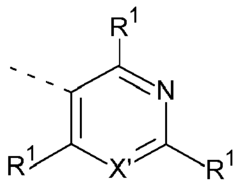

Formel (Q-14)

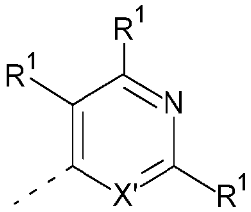

Formel (Q-15)

wobei das Symbol $R^1$ die zuvor unter anderem für Formeln (I) bis (XVIII) genannte Bedeutung aufweist, X' N oder $CR^1$ ist und die gestrichelte Bindung die Anbindungsposition markiert, wobei X' vorzugsweise ein Stickstoffatom darstellt. Die Substituenten $R^1$ in den Strukturen der Formeln (Q-11) bis (Q-15) sind bei Strukturen gemäß Formeln (N-1) bis (N-6), (Ar-1) bis (Ar-54) und/oder (Ar'-1) bis (Ar'-53) durch Substituenten $R^2$ zu ersetzen.

In einer weiteren Ausführungsform kann die unter anderem in den Formel (QL) dargelegte Gruppe Q beziehungsweise die Elektronentransportgruppe ausgewählt sein aus Strukturen der Formeln (Q-16), (Q-17), (Q-18), (Q-19), (Q-20), (Q-21) und/oder (Q-22)

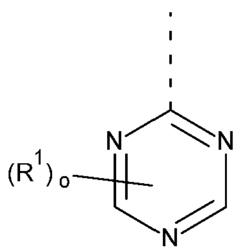

Formel (Q-16)

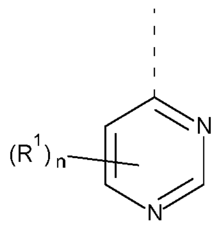

Formel (Q-17)

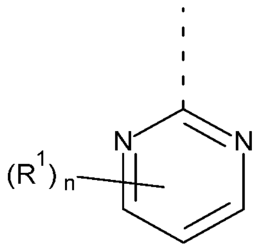

Formel (Q-18)

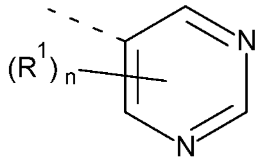

Formel (Q-19)

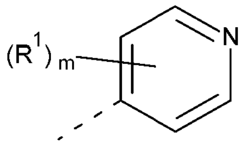

Formel (Q-20)

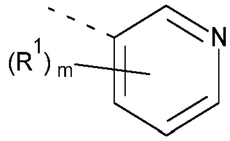

Formel (Q-21)

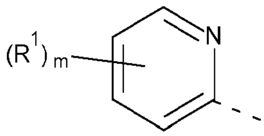

Formel (Q-22)

worin das Symbol $R^1$ die zuvor unter anderem für Formeln (I) bis (XVIII) dargelegte Bedeutung aufweist, die gestrichelte Bindung die Anbindungsposition markiert und m 0, 1, 2, 3 oder 4, vorzugsweise 0, 1 oder 2, n 0, 1, 2 oder 3, vorzugsweise 0, 1 oder 2 und o 0, 1 oder 2, vorzugsweise 1 oder 2 ist. Hierbei sind die Strukturen der Formeln (Q-16), (Q-17), (Q-18) und (Q-19) bevorzugt. Ferner sind die Substituenten $R^1$ in den Strukturen der Formeln (Q-16) bis (Q-22) bei Strukturen gemäß Formeln (N-1) bis (N-6), (Ar-1) bis (Ar-54) und/oder (Ar'-1) bis (Ar'-53) durch Substituenten $R^2$ zu ersetzen.

In einer weiteren Ausführungsform kann die unter anderem in den Formel (QL) dargelegte Gruppe Q beziehungsweise die Elektronentransportgruppe ausgewählt sein aus Strukturen der Formeln (Q-23), (Q-24) und/oder (Q-25),

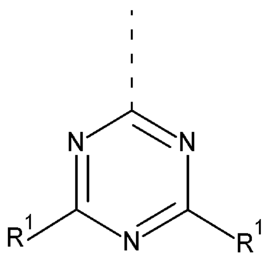

Formel (Q-23)

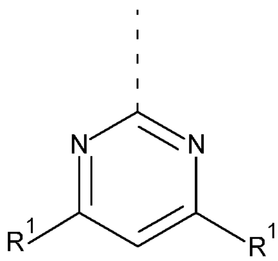

Formel (Q-24)

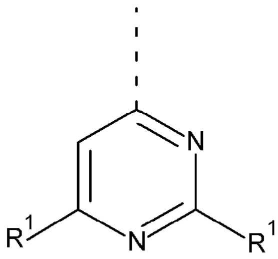

Formel (Q-25)

worin das Symbol $R^1$ die zuvor unter anderem für Formeln (I) bis (XVIII) dargelegte Bedeutung aufweist und die gestrichelte Bindung die Anbindungsposition markiert. Ferner sind die Substituenten $R^1$ in den Strukturen der Formeln (Q-23) bis (Q-25) bei Strukturen gemäß Formeln (N-1) bis (N-6), (Ar-1) bis (Ar-54) und/oder (Ar'-1) bis (Ar'-53) durch Substituenten $R^2$ zu ersetzen.

In einer weiteren Ausführungsform kann die unter anderem in den Formel (QL) dargelegte Gruppe Q beziehungsweise die Elektronentransportgruppe ausgewählt sein aus Strukturen der Formeln (Q-26), (Q-27), (Q-28), (Q-29) und/oder (Q-30),

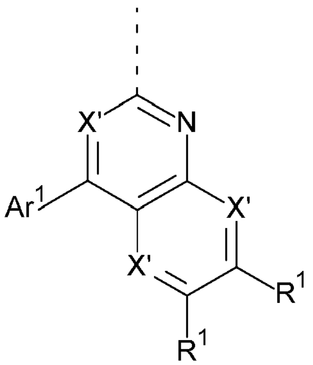

Formel (Q-26)

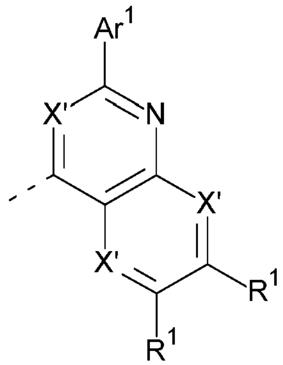

Formel (Q-27)

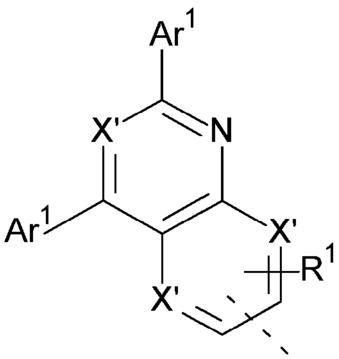

Formel (Q-28)

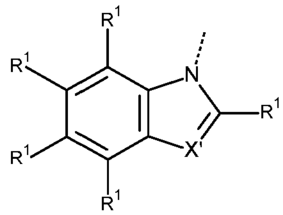

Formel (Q-29)

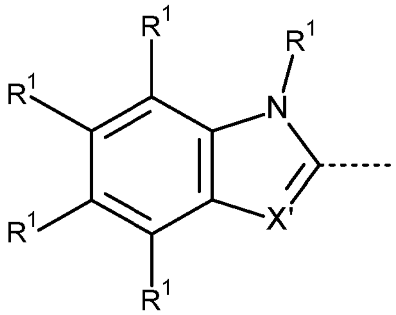

Formel (Q-30)

wobei Symbole $Ar^1$ und $R^1$ die zuvor zuvor unter anderem für Formeln (I) bis (XVIII) genannte Bedeutung aufweisen, X' N oder $CR^1$ ist und die gestrichelte Bindung die Anbindungsposition markiert. Vorzugsweise stellt in den Strukturen der Formeln (Q-26), (Q-27) und (Q-28) genau ein X' ein Stickstoffatom dar. Ferner sind die Substituenten $R^1$ in den Strukturen der Formeln (Q-26) bis (Q-30) bei Strukturen gemäß Formeln (N-1) bis (N-6), (Ar-1) bis (Ar-54) und/oder (Ar'-1) bis (Ar'-53) durch Substituenten $R^2$ zu ersetzen.

Vorzugsweise kann die unter anderem in den Formel (QL) dargelegte Gruppe Q beziehungsweise die Elektronentransportgruppe ausgewählt sein aus Strukturen der Formeln (Q-31), (Q-32), (Q-33), (Q-34), (Q-35), (Q-36), (Q-37), (Q-38), (Q-39), (Q-40), (Q-41), (Q-42), (Q-43) und/oder (Q-44),

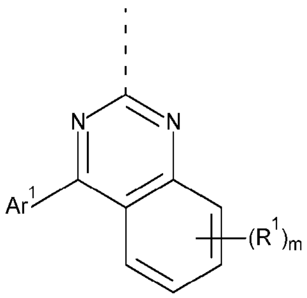

Formel (Q-31)

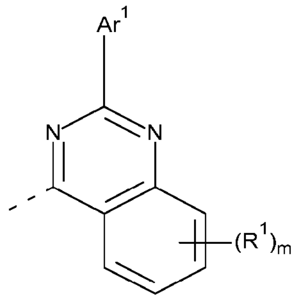

Formel (Q-32)

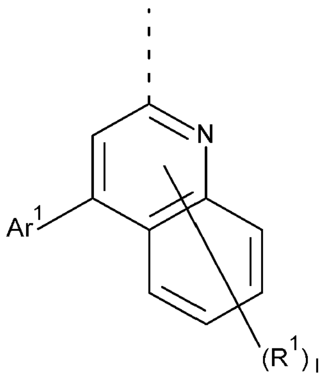

Formel (Q-33)

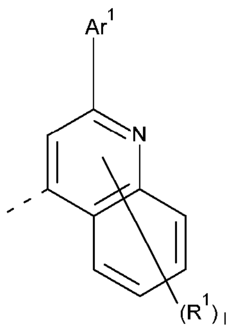

Formel (Q-34)

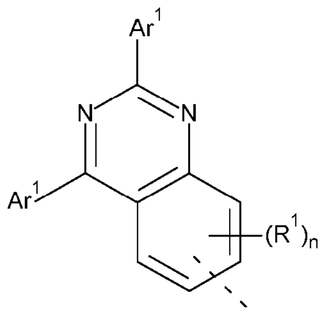

Formel (Q-35)

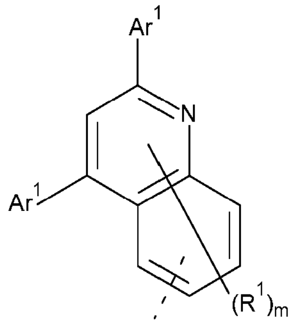

Formel (Q-36)

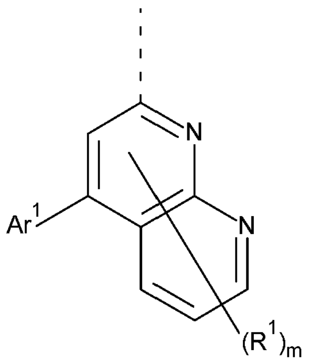

Formel (Q-37)

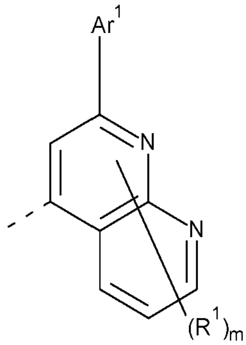

Formel (Q-38)

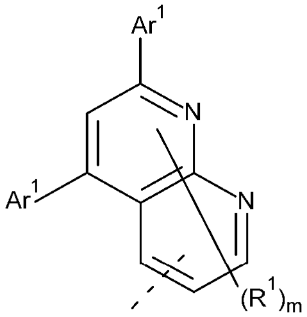

Formel (Q-39)

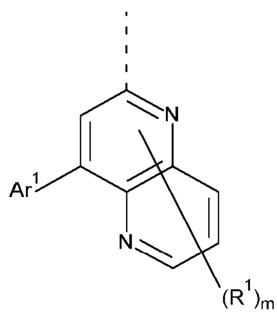

Formel (Q-40)

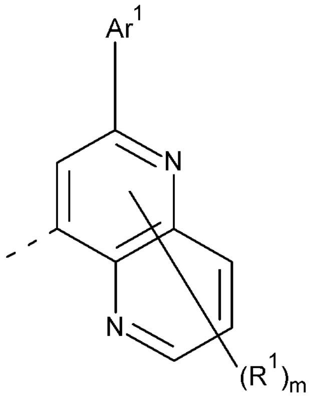

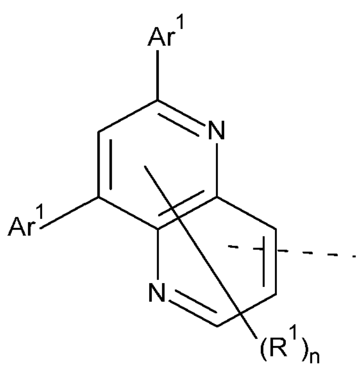

Formel (Q-41)

Formel (Q-42)

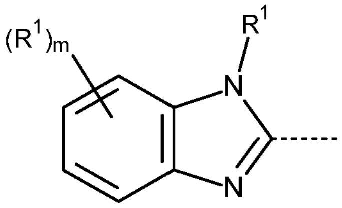

Formel (Q-43)

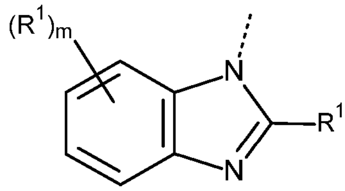

Formel (Q-44)

worin die Symbole $Ar^1$ und $R^1$ die zuvor unter anderem für Formeln (I) bis (XVIII) dargelegte Bedeutung aufweisen, die gestrichelte Bindung die Anbindungsposition markiert und m 0, 1, 2, 3 oder 4, vorzugsweise 0, 1 oder 2, n 0, 1, 2 oder 3, vorzugsweise 0 oder 1, n 0, 1, 2 oder 3, vorzugsweise 0, 1 oder 2 und l 1, 2, 3, 4 oder 5, vorzugsweise 0, 1 oder 2 ist. Hierbei sind die Substituenten $R^1$ in den Strukturen der Formeln (Q-31) bis (Q-44) bei Strukturen gemäß Formeln (N-1) bis (N-6), (Ar-1) bis (Ar-54) und/oder (Ar'-1) bis (Ar'-53) durch Substituenten $R^2$ zu ersetzen.

In einer weiteren bevorzugten Ausführungsform der Erfindung steht $Ar^1$ gleich oder verschieden bei jedem Auftreten für ein aromatisches oder heteroaromatisches Ringsystem, vorzugsweise einen Aryl- oder Heteroarylrest mit 5 bis 24 aromatischen Ringatomen, bevorzugt mit 6 bis 18 aromatischen Ringatomen, besonders bevorzugt für ein aromatisches Ringsystem, vorzugsweise einen Arylrest mit 6 bis 12 aromatischen Ringatomen bzw. ein heteroaromatisches Ringsystem, vorzugsweise eine Heteroarylgruppe mit 5 bis 13 aromatischen Ringatomen, das jeweils durch einen oder mehrere Reste $R^2$ substituiert sein kann, bevorzugt aber unsubstituiert ist, wobei $R^2$ die zuvor, insbesondere in Formeln (I) bis (XVIII) dargestellte Bedeutung aufweisen kann.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist, beispielweise ein C- oder N-Atom der zuvor dargestellten Gruppen (H-1) bis (H-26) oder (Q-26) bis (Q-44).

Mit Vorteil stellt $Ar^1$ in den Formeln (H-1) bis (H-26) oder (Q-26) bis (Q-44) ein aromatisches Ringsystem mit 6 bis 12 aromatischen Ringatomen dar, welches mit einem oder mehreren Resten $R^2$ substituiert sein kann, vorzugsweise aber unsubstituiert ist, wobei $R^2$ die zuvor, insbesondere für Formeln (I) bis (XVIII) dargestellte Bedeutung aufweisen kann.

Bevorzugt bilden die Reste $R^1$ oder $R^2$ in den Formeln (H-1) bis (H-26) oder (Q-1) bis (Q-44) mit den Ringatomen der Arylgruppe oder Heteroarylgruppe $Ar^1$, $Ar^2$, $Ar^3$ und/oder $Ar^4$, an die die Reste $R^1$ oder $R^2$ gebunden sind, kein kondensiertes Ringsystem. Dies schließt die Bildung eines kondensierten Ringsystems mit möglichen Substituenten $R^2$, $R^3$ ein, die an die Reste $R^1$ oder $R^2$ gebunden sein können.

Ferner kann vorgesehen sein, dass die Gruppe Ar, $Ar^1$, $Ar^2$, $Ar^3$ und/oder $Ar^4$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imimdazolyl, Benzimidazolyl, Benzoxazolyl, Benzthiazolyl, 1-, 2-, 3- oder 4-Carbazolyl, Indenocarbazolyl, 1- oder 2-Napthyl, Anthracenyl, vorzugsweise 9-Anthracenyl, Phenanthrenyl und/oder Triphenylenyl, die jeweils durch einen oder mehrere Reste $R^1$ und/oder $R^2$ substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen-Gruppen besonders bevorzugt sind.

Wenn X oder $X^1$ für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten $R^2$ substituiert sein kann, wobei eine oder mehrere nicht-benachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die vorzugsweise an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann, wobei die Gruppe $Ar^1$ die zuvor, insbesondere für Formeln (I) bis (XVIII) genannte Bedeutung aufweist.

Besonders bevorzugt sind diese Substituenten $R^1$ ausgewählt aus der Gruppe bestehend aus H, D, F, CN,

$N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, vorzugsweise die an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber

unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, 1-, 2-, 3- oder 4-Carbazolyl und Indenocarbazolyl, die jeweils durch einen oder mehrere Reste $R^2$ substituiert sein können, bevorzugt aber unsubstituiert sind.

Weiterhin kann vorgesehen sein, dass die Substitutenten $R^1$ des heteroaromatischen Ringssystems gemäß den Formeln (I) bis (XVIII), (Ia) bis (XVIIIa), (N-1) bis (N-6), (Ar-1) bis (Ar-54) und/oder (Ar'-1) bis (Ar'-53) mit den Ringatomen des aromatischen oder heteroaromatischen Ringsystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können.

Weiterhin kann vorgesehen sein, dass in einer Struktur gemäß Formel (I) bis (XVIII), (Ia) bis (XVIIIa), (N-1) bis (N-6), (Ar-1) bis (Ar-54) und/oder (Ar'-1) bis (Ar'-53) mindestens ein Rest $R^1$ oder $Ar^1$ für eine Gruppe steht, die ausgewählt ist aus den Formeln ($R^1$-1) bis ($R^1$- 92), beziehungsweise in einer Struktur gemäß Formel (H-1) bis (H-26), (Q-1) bis (Q-44) mindestens ein Rest $Ar^1$ oder $R^1$ für eine Gruppe steht, die ausgewählt ist aus den Formeln ($R^1$-1) bis ($R^1$- 92)

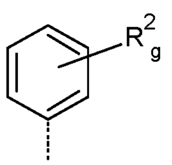

Formel ($R^1$-1)

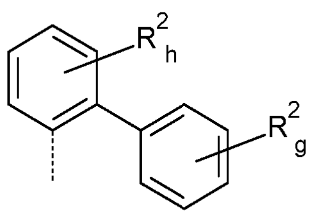

Formel ($R^1$-2)

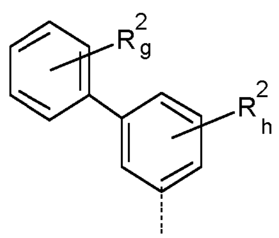

Formel ($R^1$-3)

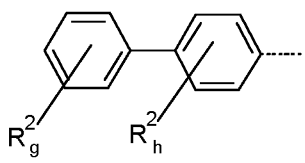

Formel ($R^1$-4)

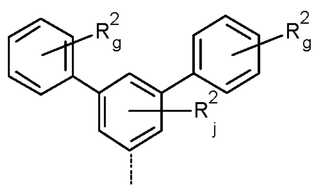

Formel ($R^1$-5)

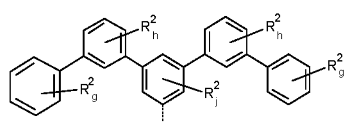

Formel ($R^1$-6)

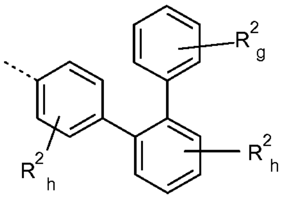

Formel ($R^1$-7)

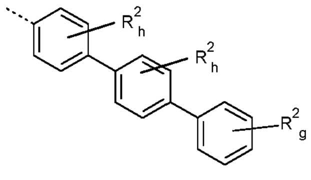

Formel ($R^1$-8)

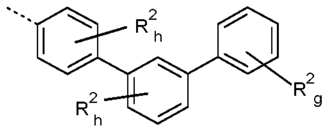

Formel ($R^1$-9)

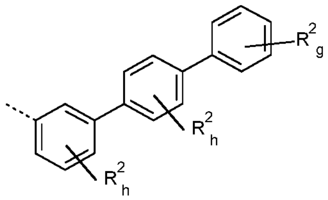

Formel ($R^1$-10)

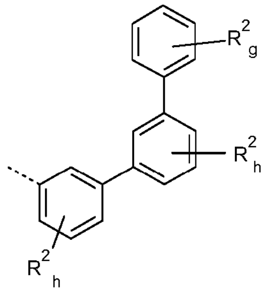

Formel ($R^1$-11)

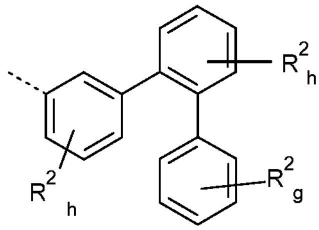

Formel ($R^1$-12)

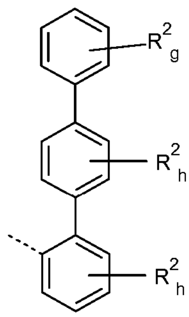

Formel ($R^1$-13)

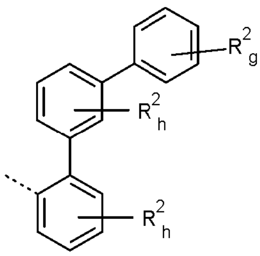

Formel ($R^1$-14)

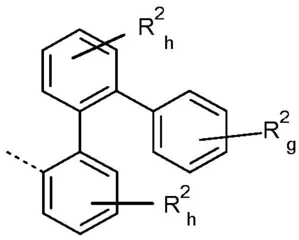

Formel ($R^1$-15)

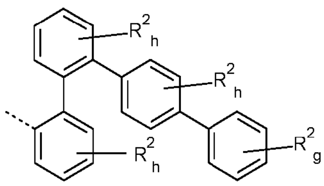

Formel ($R^1$-16)

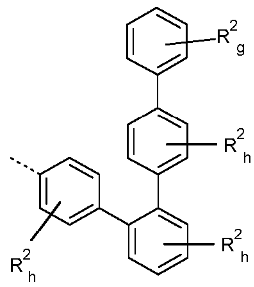

Formel ($R^1$-17)

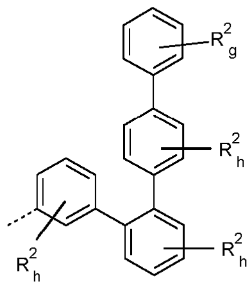

Formel ($R^1$-18)

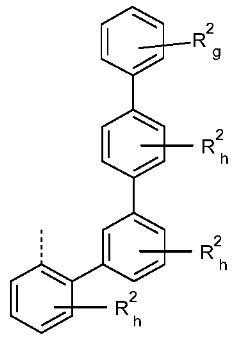

Formel ($R^1$-19)

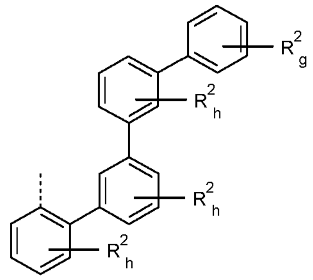

Formel ($R^1$-20)

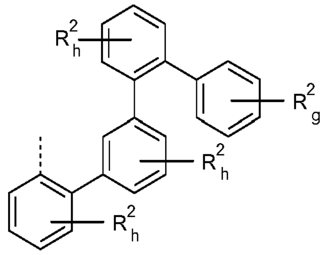

Formel ($R^1$-21)

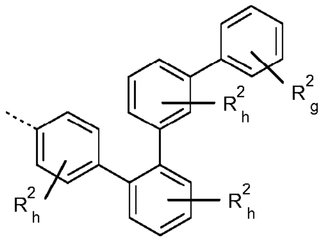

Formel ($R^1$-22)

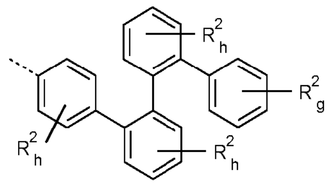

Formel ($R^1$-23)

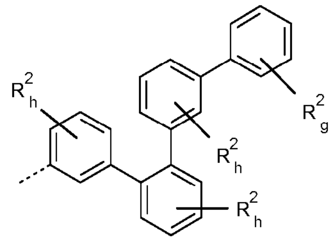

Formel ($R^1$-24)

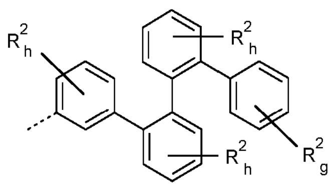

Formel ($R^1$-25)

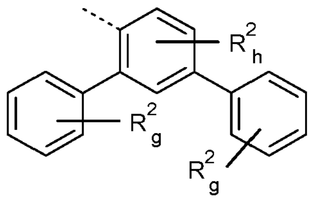

Formel ($R^1$-26)

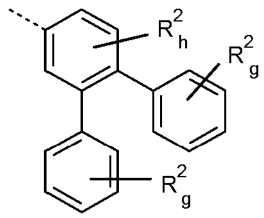

Formel ($R^1$-27)

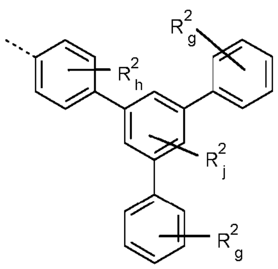

Formel ($R^1$-28)

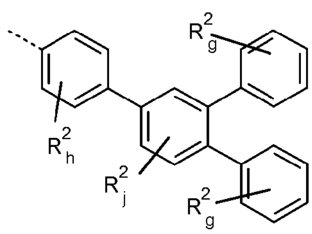

Formel ($R^1$-29)

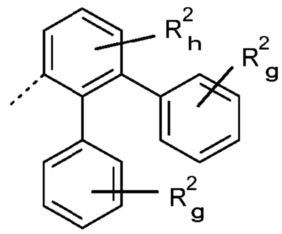

Formel ($R^1$-30)

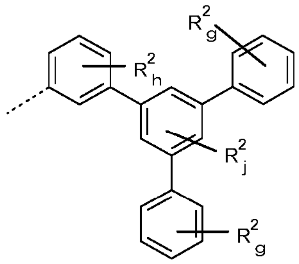

Formel ($R^1$-31)

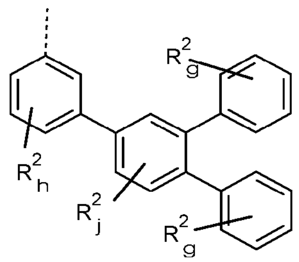

Formel ($R^1$-32)

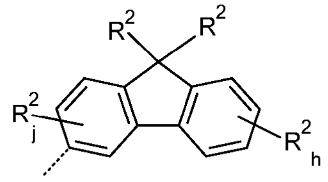

Formel ($R^1$-33)

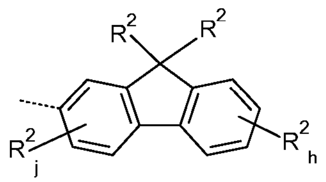

Formel ($R^1$-34)

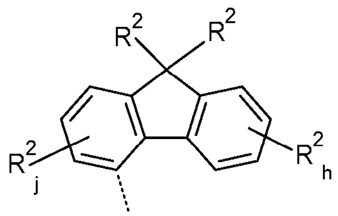

Formel ($R^1$-35)

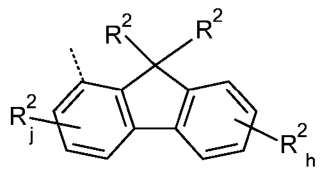

Formel ($R^1$-36)

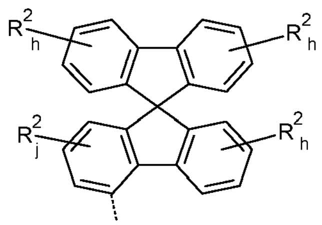

Formel ($R^1$-37)

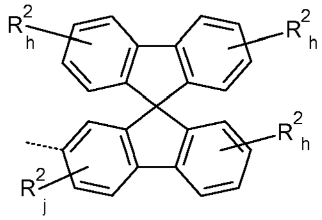

Formel ($R^1$-38)

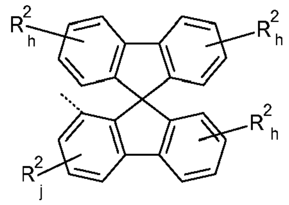

Formel ($R^1$-39)

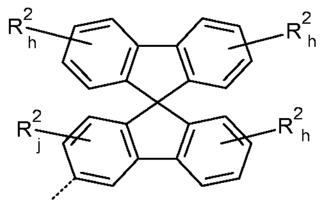

Formel ($R^1$-40)

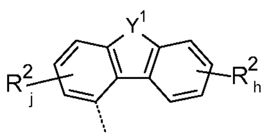

Formel ($R^1$-41)

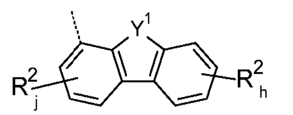

Formel ($R^1$-42)

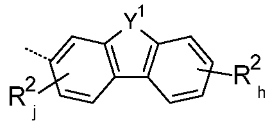

Formel ($R^1$-43)

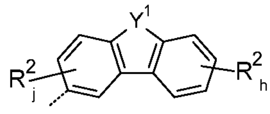

Formel ($R^1$-44)

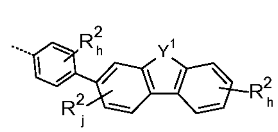

Formel ($R^1$-45)

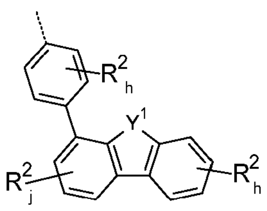

Formel ($R^1$-46)

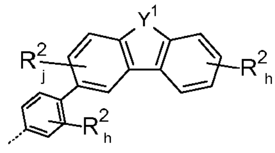

Formel ($R^1$-47)

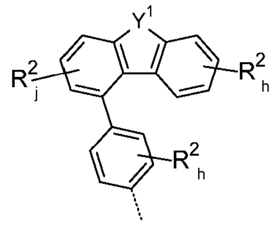

Formel ($R^1$-48)

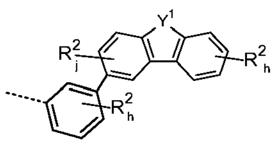

Formel ($R^1$-49)

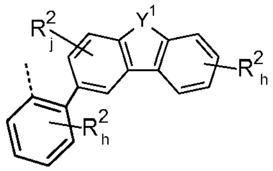

Formel ($R^1$-50)

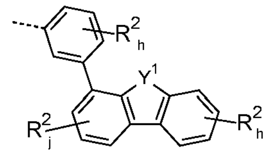

Formel ($R^1$-51)

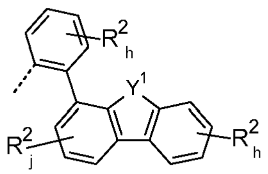

Formel ($R^1$-52)

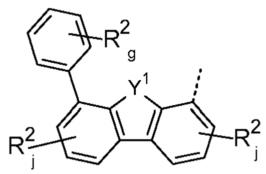

Formel ($R^1$-53)

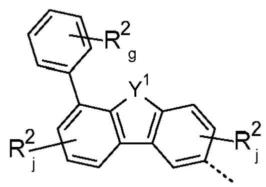

Formel ($R^1$-54)

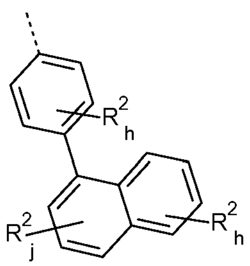

Formel ($R^1$-55)

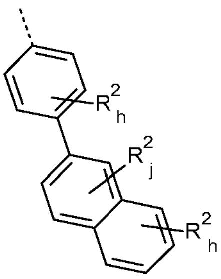

Formel ($R^1$-56)

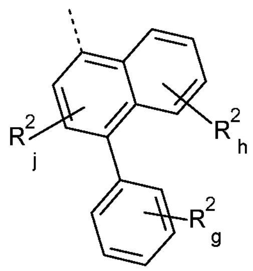

Formel ($R^1$-57)

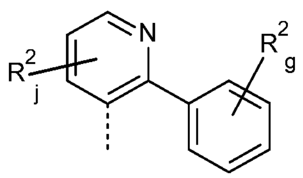

Formel ($R^1$-58)

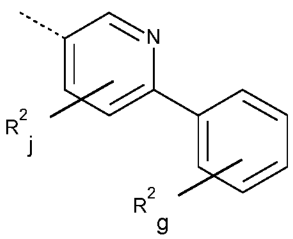

Formel ($R^1$-59)

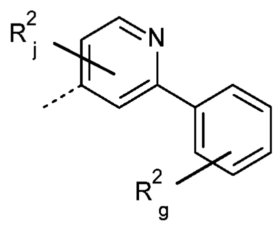

Formel ($R^1$-60)

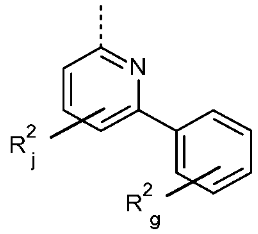
Formel ($R^1$-61)

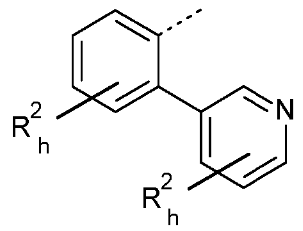
Formel ($R^1$-62)

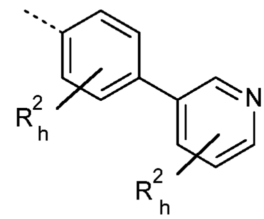
Formel ($R^1$-63)

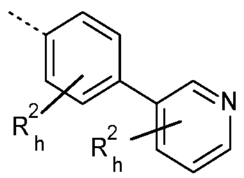
Formel ($R^1$-64)

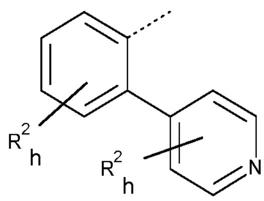
Formel ($R^1$-65)

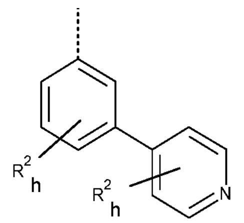
Formel ($R^1$-66)

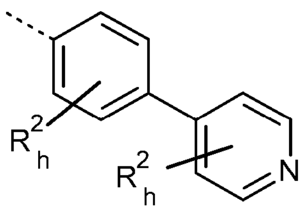
Formel ($R^1$-67)

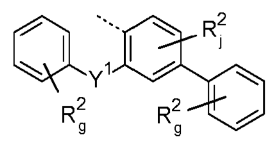
Formel ($R^1$-68)

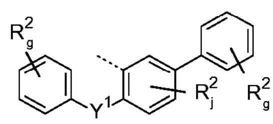
Formel ($R^1$-69)

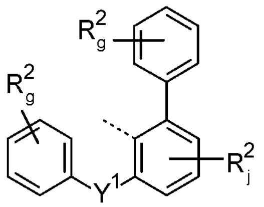
Formel ($R^1$-70)

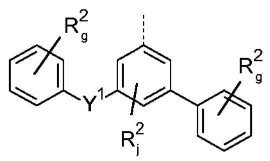
Formel ($R^1$-71)

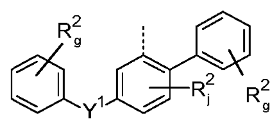
Formel ($R^1$-72)

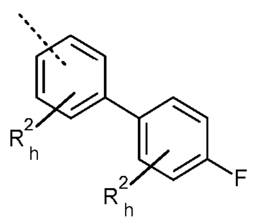
Formel ($R^1$-73)

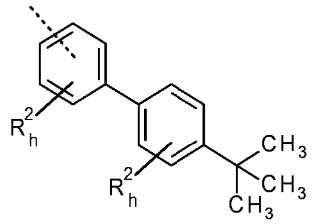
Formel ($R^1$-74)

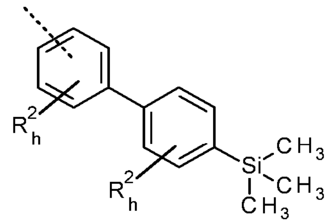
Formel ($R^1$-75)

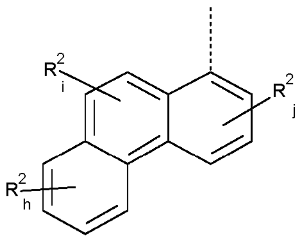
Formel ($R^1$-76)

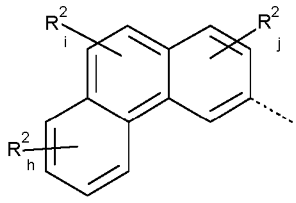
Formel ($R^1$-77)

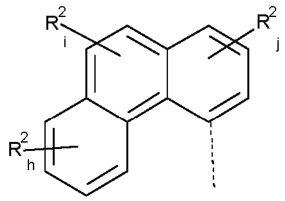
Formel ($R^1$-78)

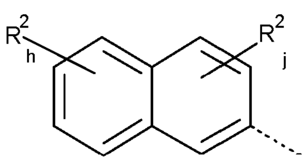
Formel ($R^1$-79)

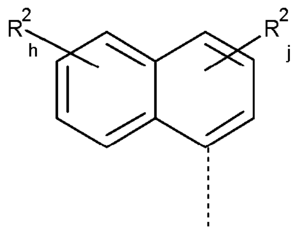
Formel ($R^1$-80)

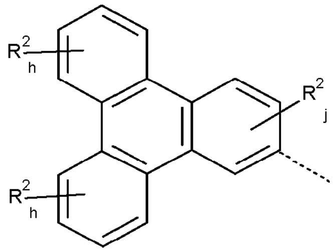
Formel ($R^1$-81)

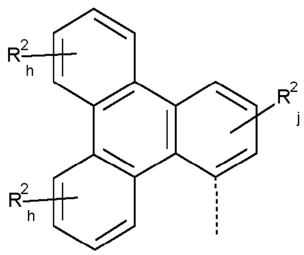
Formel ($R^1$-82)

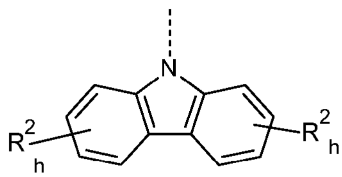
Formel ($R^1$-83)

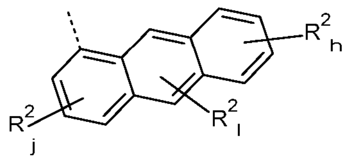
Formel ($R^1$-84)

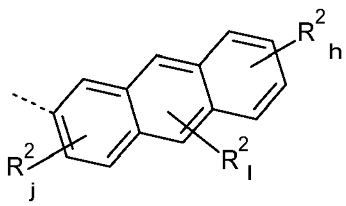
Formel ($R^1$-85)

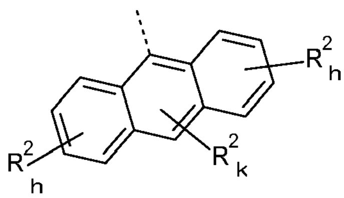
Formel ($R^1$-86)

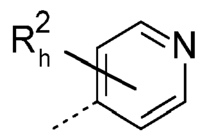
Formel ($R^1$-87)

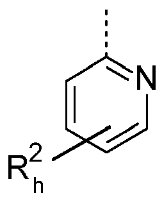
Formel ($R^1$-88)

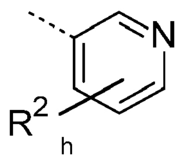
Formel ($R^1$-89)

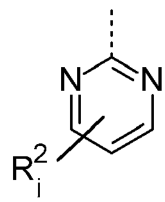
Formel ($R^1$-90)

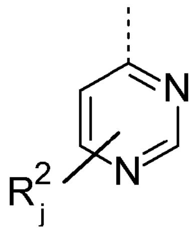

Formel ($R^1$-91)

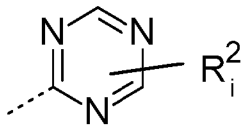

Formel ($R^1$-92)

wobei für die verwendeten Symbole gilt:

$Y^1$ ist O, S oder $NR^2$, vorzugsweise O oder S;
k ist bei jedem Auftreten unabhängig 0 oder 1;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5;
$R^2$ kann die zuvor genannte, insbesondere für Formeln (I) bis (XVIII) genannte Bedeutung aufweisen und

die gestrichelte Bindung markiert die Anbindungsposition.

Hierbei sind die Gruppen der Formeln $R^1$-1 bis $R^1$-54 bevorzugt, wobei die Gruppen R'-1, R'-3, R'-5, R'-6, R'-15, R'-29, R'-30, R'-31, R'-32, R'-33, $R^1$-38, $R^1$-39, $R^1$-40, $R^1$-41, $R^1$-42, $R^1$-43, $R^1$-44 und/oder $R^1$-45 besonders bevorzugt sind.

Vorzugsweise kann vorgesehen sein, dass die Summe der Indices k, i, j, h und g in den Strukturen der Formel ($R^1$-1) bis ($R^1$-92) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-92) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Die zuvor dargelegten Reste der Formeln ($R^1$-1) bis ($R^1$-92) stellen bevorzugte Reste Ar gemäß Formeln (I) bis (XVIII) beziehungsweise $Ar^3$, $Ar^4$ gemäß Formeln (H-1) bis (H-3) oder bevorzugte Ausführungsformen dieser Formeln dar, wobei in diesem Fall die in den Formeln ($R^1$-1) bis ($R^1$-92) dargelegten Gruppen $R^2$ durch Reste $R^1$ zu ersetzen sind. Die zuvor dargelegten Bevorzugungen hinsichtlich der Formeln ($R^1$-1) bis ($R^1$-92) gelten entsprechend.

Bevorzugt sind Verbindungen, umfassend mindestens eine Struktur der Formeln (H-1) bis (H-26), in denen die Gruppe $Ar^2$ für eine Gruppe steht, die ausgewählt ist aus den Formeln ($L^1$-1) bis ($L^1$-108), und/oder Verbindungen, umfassend Strukturen der Formel (QL), in denen die Gruppe $L^1$ für eine Bindung oder für eine Gruppe steht, die ausgewählt ist aus den Formeln ($L^1$-1) bis ($L^1$-108)

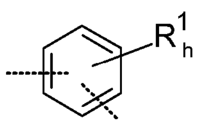

Formel ($L^1$-1)

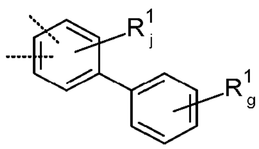

Formel ($L^1$-2)

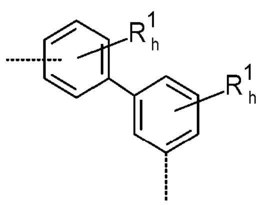

Formel ($L^1$-3)

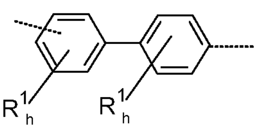

Formel ($L^1$-4)

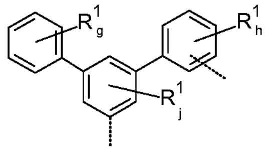

Formel ($L^1$-5)

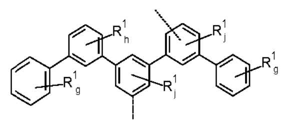

Formel ($L^1$-6)

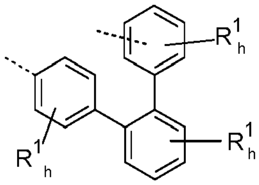

Formel ($L^1$-7)

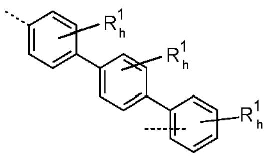

Formel ($L^1$-8)

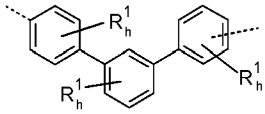

Formel ($L^1$-9)

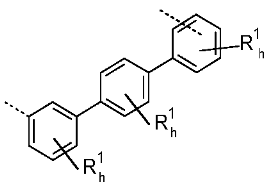

Formel ($L^1$-10)

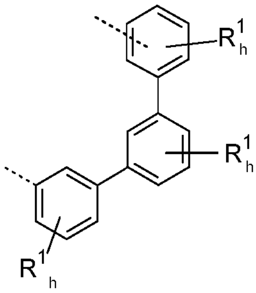

Formel ($L^1$-11)

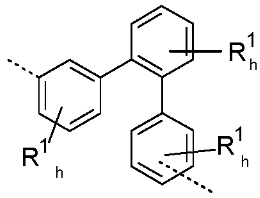

Formel ($L^1$-12)

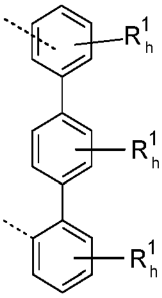

Formel ($L^1$-13)

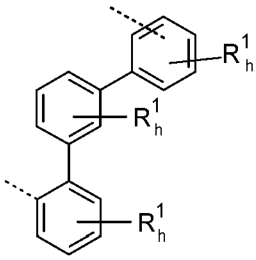

Formel ($L^1$-14)

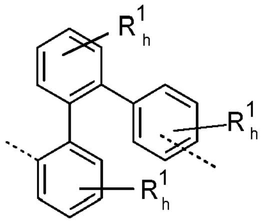

Formel ($L^1$-15)

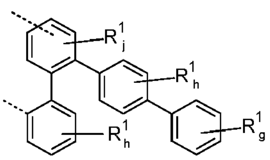

Formel ($L^1$-16)

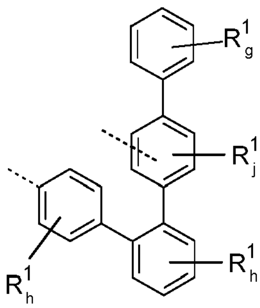

Formel ($L^1$-17)

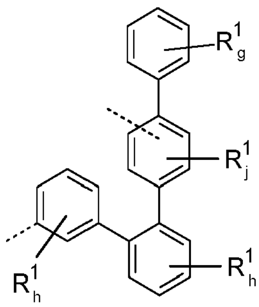

Formel ($L^1$-18)

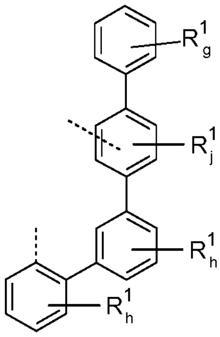
Formel ($L^1$-19)

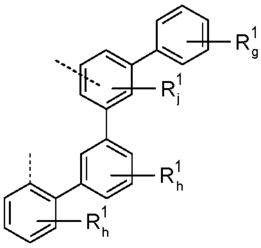
Formel ($L^1$-20)

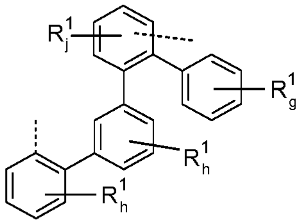
Formel ($L^1$-21)

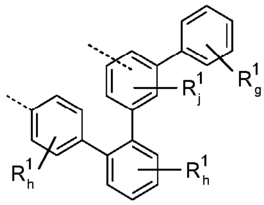
Formel ($L^1$-22)

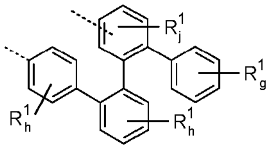
Formel ($L^1$-23)

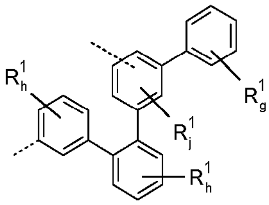
Formel ($L^1$-24)

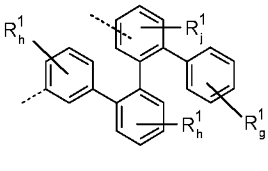
Formel ($L^1$-25)

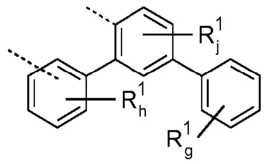
Formel ($L^1$-26)

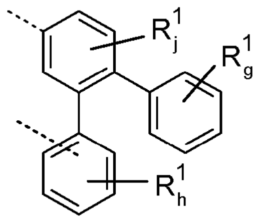
Formel ($L^1$-27)

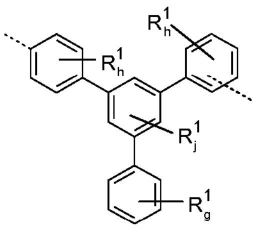
Formel ($L^1$-28)

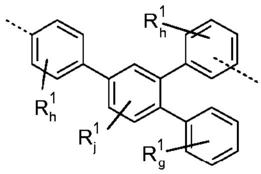
Formel ($L^1$-29)

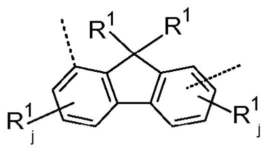
Formel ($L^1$-30)

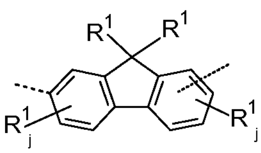
Formel ($L^1$-31)

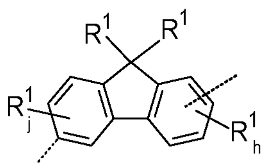
Formel ($L^1$-32)

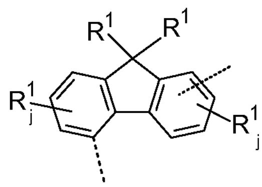
Formel ($L^1$-33)

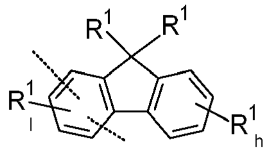

Formel ($L^1$-34)

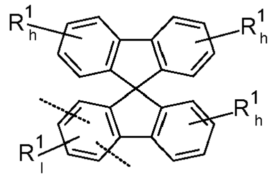

Formel ($L^1$-35)

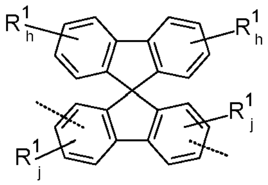

Formel ($L^1$-36)

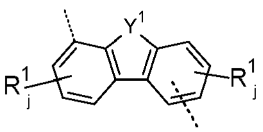

Formel ($L^1$-37)

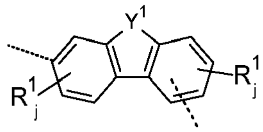

Formel ($L^1$-38)

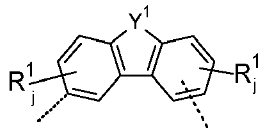

Formel ($L^1$-39)

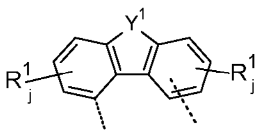

Formel ($L^1$-40)

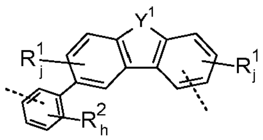

Formel ($L^1$-41)

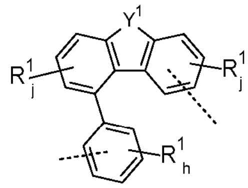

Formel ($L^1$-42)

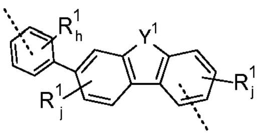

Formel ($L^1$-43)

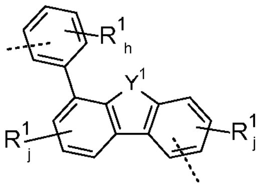

Formel ($L^1$-44)

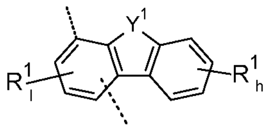

Formel ($L^1$-45)

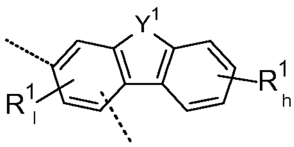

Formel ($L^1$-46)

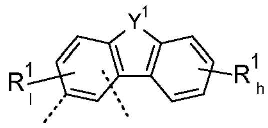

Formel ($L^1$-47)

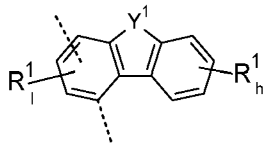

Formel ($L^1$-48)

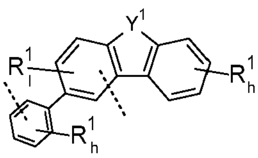

Formel ($L^1$-49)

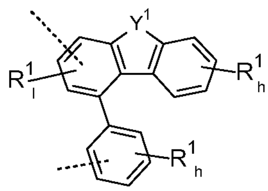

Formel ($L^1$-50)

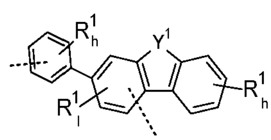

Formel ($L^1$-51)

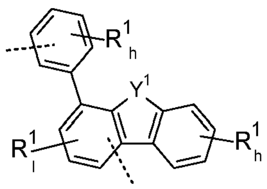

Formel ($L^1$-52)

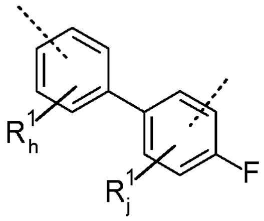

Formel ($L^1$-53)

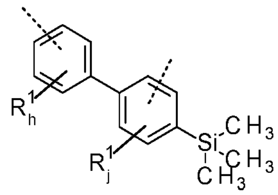

Formel ($L^1$-54)

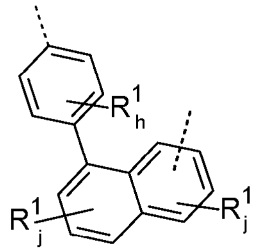

Formel ($L^1$-55)

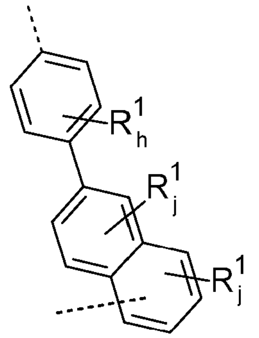

Formel ($L^1$-56)

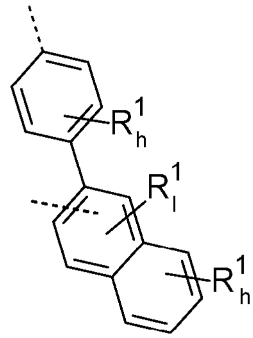

Formel ($L^1$-57)

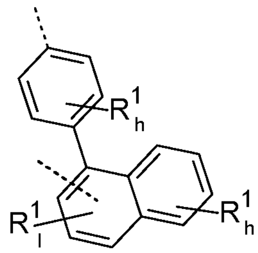

Formel ($L^1$-58)

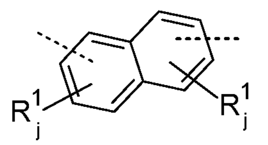

Formel ($L^1$-59)

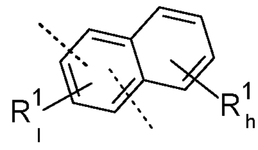

Formel ($L^1$-60)

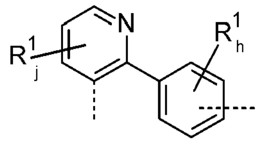

Formel ($L^1$-61)

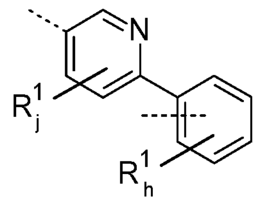

Formel ($L^1$-62)

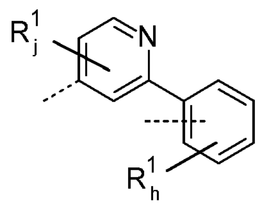

Formel ($L^1$-63)

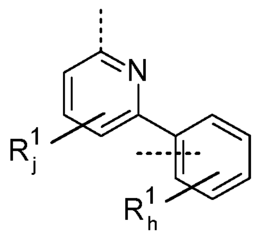

Formel ($L^1$-64)

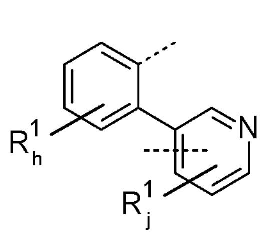

Formel ($L^1$-65)

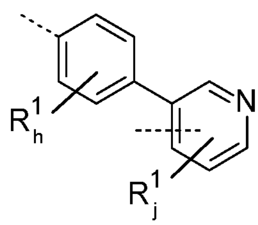

Formel ($L^1$-66)

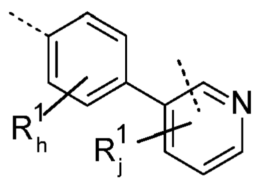
Formel ($L^1$-67)

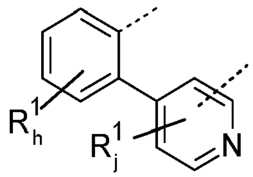
Formel ($L^1$-68)

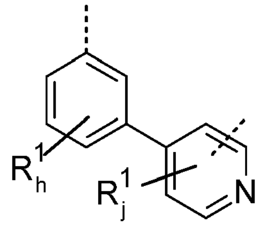
Formel ($L^1$-69)

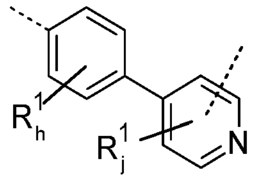
Formel ($L^1$-70)

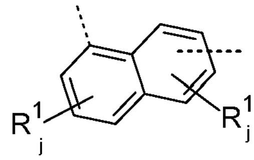
Formel ($L^1$-71)

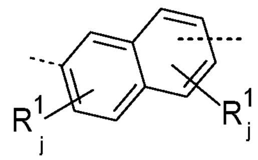
Formel ($L^1$-72)

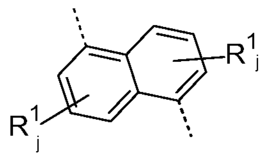
Formel ($L^1$-73)

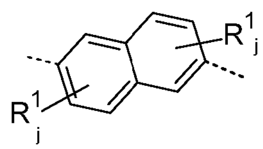
Formel ($L^1$-74)

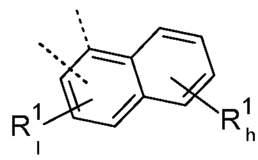
Formel ($L^1$-75)

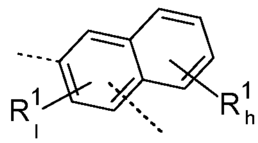
Formel ($L^1$-76)

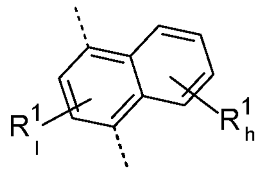
Formel ($L^1$-77)

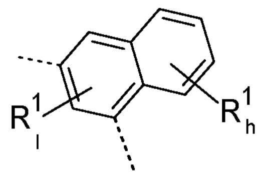
Formel ($L^1$-78)

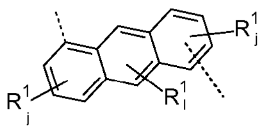
Formel ($L^1$-79)

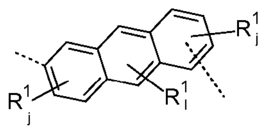
Formel ($L^1$-80)

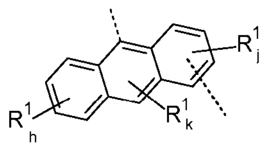
Formel ($L^1$-81)

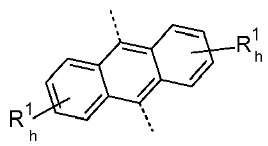
Formel ($L^1$-82)

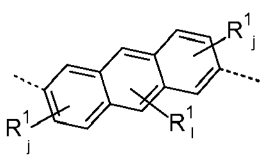
Formel ($L^1$-83)

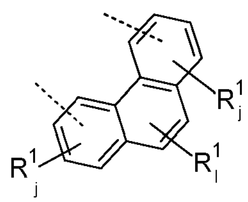
Formel ($L^1$-84)

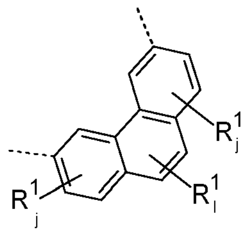

Formel ($L^1$-85)

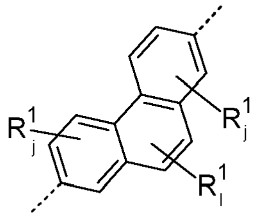

Formel ($L^1$-86)

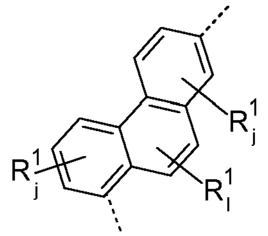

Formel ($L^1$-87)

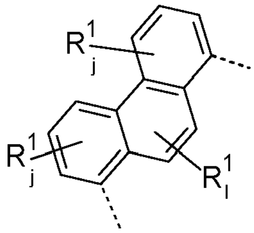

Formel ($L^1$-88)

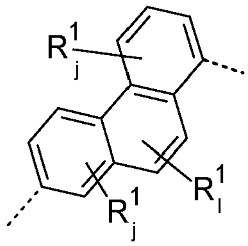

Formel ($L^1$-89)

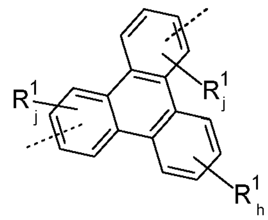

Formel ($L^1$-90)

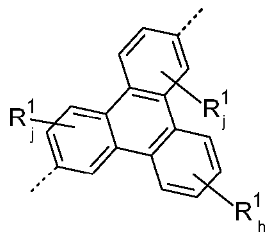

Formel ($L^1$-91)

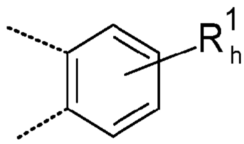

Formel ($L^1$-92)

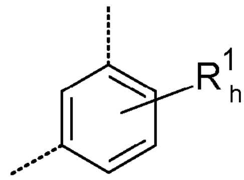

Formel ($L^1$-93)

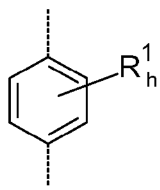

Formel ($L^1$-94)

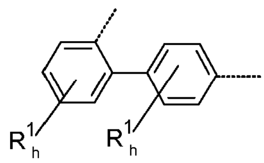

Formel ($L^1$-95)

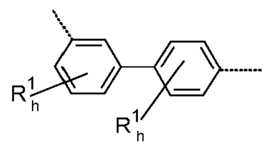

Formel ($L^1$-96)

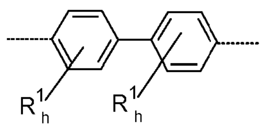

Formel ($L^1$-97)

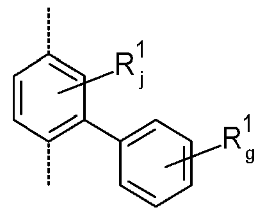

Formel ($L^1$-98)

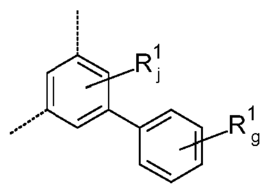

Formel ($L^1$-99)

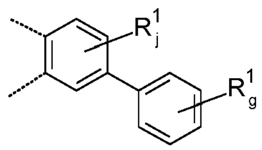

Formel ($L^1$-100)

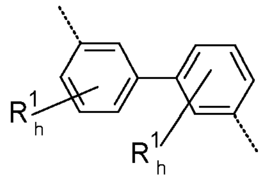

Formel ($L^1$-101)

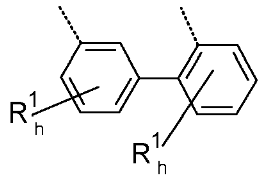

Formel ($L^1$-102)

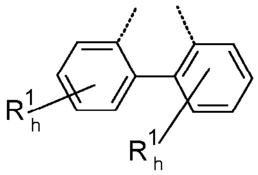

Formel ($L^1$-103)

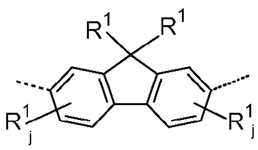

Formel ($L^1$-104)

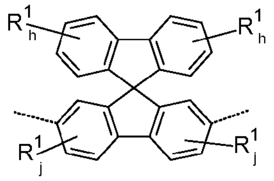

Formel ($L^1$-105)

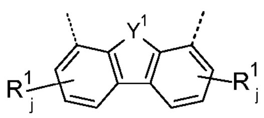

Formel ($L^1$-106)

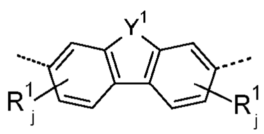

Formel ($L^1$-107)

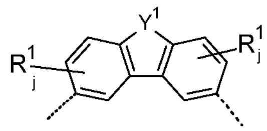

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol $Y^1$ O, S oder $NR^1$, vorzugsweise O oder S ist; und das Symbol $R^1$ die zuvor, insbesondere für Formeln (I) bis (XVIII) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Bevorzugte erfindungsgemäße Verbindungen mit einer Gruppe der Formeln (H-1) bis (H-26) umfassen eine Gruppe $Ar^2$, die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugte erfindungsgemäße Verbindungen mit einer Gruppe der Formel (QL) umfassen eine Gruppe $L^1$, die eine Bindung darstellt oder die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Gemäß einer bevorzugten Ausgestaltung sind erfindungsgemäße Verbindungen, die in einer organischen elektronischen Vorrichtung als aktive Verbindung einsetzbar sind, ausgewählt aus der Gruppe der Phenyle, Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene, Anthracene, Benzanthracene, Fluoradene.

Gemäß einer bevorzugten Ausgestaltung sind erfindungsgemäße Verbindungen durch Strukturen gemäß den Formeln (I) bis (XVIII) und/oder den Formeln (Ia) bis (XVIIIa) oder durch eine Kombination der Teilstrukturen gemäß den Formeln (N-1) bis (N-6), (Ar-1) bis (Ar-54) und/oder (Ar'-1) bis (Ar'-53) darstellbar. Vorzugsweise weisen Verbindungen, die in einer organischen elektronischen Vorrichtung als aktive Verbindung einsetzbar sind, bevorzugt Verbindungen,

umfassend Strukturen gemäß den Formeln (I) bis (XVIII) und/oder den Formeln (Ia) bis (XVIIIa) oder die durch eine Kombination der Teilstrukturen gemäß den Formeln (N-1) bis (N-6), (Ar-1) bis (Ar-54) und/oder (Ar'-1) bis (Ar'-53) erhältlichen Verbindungen, ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Bei Ausgestaltung der erfindungsgemäßen Verbindungen, die in einer organischen elektronischen Vorrichtung als aktive Verbindung einsetzbar ist, zur Verwendung als fluoreszierender Emitter oder als blaue OLED-Materialien können bevorzugte Verbindungen entsprechende Gruppen, beispielsweise Fluoren-, Anthracen- und/oder Pyren-Gruppen enthalten, die mit Gruppen $R^1$ oder $R^2$ substituiert sein können oder die durch entsprechende Substituition der Gruppen ($R^1$-1) bis ($R^1$-95), vorzugsweise ($R^1$-33) bis (R'-57) und ($R^1$-76) bis ($R^1$-86), oder ($L^1$-1) bis ($L^1$-109), vorzugsweise ($L^1$-30) bis ($L^1$-60) und ($L^1$-71) bis ($L^1$-91), mit den Substituenten $R^2$ gebildet werden.

In einer weiteren bevorzugten Ausführungsform der Erfindung ist $R^2$, beispielsweise bei einer Struktur gemäß Formeln (I) bis (XVIII) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Bevorzugt bilden die Reste $R^2$ mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^3$, beispielsweise bei einer Struktur gemäß Formeln (I) bis (XVIII) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren Ausgestaltung der vorliegenden Erfindung sind Verbindungen bevorzugt, die durch eine Kombination der Teilstrukturen N-1 und Ar-1 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-1' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden

Eigenschaften aufweisen, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-4 erhalten werden, wobei insgesamt höchstens 6, vorzugsweise höchstens 4 und speziell bevorzugt höchstens 2 Reste der Formel X' unleich CH oder CD sind welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-4' erhalten werden, wobei die Summe der Indices v, o und m höchstens 5, vorzugsweise höchstens 3 und speziell bevorzugt 2 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-5 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |

(fortgesetzt)

| $R^1$, ungleich H oder D | vorzugsweise | Mindestens ein $R^1$ | vorzugsweise |
|---|---|---|---|
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-5' erhalten werden, wobei die Summe der Indices v, o und m höchstens 5, vorzugsweise höchstens 3 und speziell bevorzugt 2 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-10 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination der Teilstrukturen N-1 und Ar-10 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-10 erhalten werden, wobei der Rest U für $C(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination der Teilstrukturen N-1 und Ar'-10 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |

(fortgesetzt)

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar-10 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination der Teilstrukturen N-1 und Ar-10 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-10 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination der Teilstrukturen N-1 und Ar'-10 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine

Kombination der Teilstrukturen N-1 und Ar-11 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination der Teilstrukturen N-1 und Ar-11 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-11 erhalten werden, wobei der Rest U für $C(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination der Teilstrukturen N-1 und Ar'-11 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar-11 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination der Teilstrukturen N-1 und Ar-11 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |

(fortgesetzt)

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-11 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination der Teilstrukturen N-1 und Ar'-11 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar-12 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination der Teilstrukturen N-1 und Ar-12 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-12 erhalten werden, wobei der Rest U für $C(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch

eine Kombination der Teilstrukturen N-1 und Ar'-12 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar-10 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination der Teilstrukturen N-1 und Ar-10 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar'-12 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination der Teilstrukturen N-1 und Ar'-12 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |

(fortgesetzt)

| $R^1$, ungleich H oder D | vorzugsweise | bevorzugt ein $R^1$ | besonders bevorzugt |
|---|---|---|---|
| $R^1$-1 bis $R^1$-4 | $R^1$-1 | - | - |

In einer weiteren Ausgestaltung der vorliegenden Erfindung sind Verbindungen bevorzugt, die durch eine Kombination der Teilstrukturen N-1 und Ar-13 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-13' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-14 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-14' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden

Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-15 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination der Teilstrukturen N-1 und Ar-15' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-23 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |

(fortgesetzt)

| $R^1$, ungleich H oder D | vorzugsweise | Mindestens ein $R^1$ | vorzugsweise |
|---|---|---|---|
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-22’ erhalten werden, wobei die Summe der Indices v, o und m höchstens 5, vorzugsweise höchstens 3 und speziell bevorzugt 2 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-24 erhalten werden, wobei insgesamt höchstens 6, vorzugsweise höchstens 4 und speziell bevorzugt höchstens 2 Reste der Formel X’ ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-23’ erhalten werden, wobei die Summe der Indices v, o und m höchstens 5, vorzugsweise höchstens 3 und speziell bevorzugt 2 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-25 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-24' erhalten werden, wobei die Summe der Indices v, o und m höchstens 5, vorzugsweise höchstens 3 und speziell bevorzugt 2 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-33 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur Ar-33 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-32 erhalten werden, wobei der Rest U für $C(R^1)_2$ steht und die Summe der Indices v, o und m höchstens 5, vorzugsweise höchstens 3 und speziell bevorzugt 2 ist, wobei der

Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur Ar'-32 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-33 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination von zwei Teilstrukturen N-1 und einer Teilstruktur Ar-33 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-32 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination von zwei Teilstrukturen N-1 und einer Teilstruktur Ar'-32 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

(fortgesetzt)

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-34 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur Ar-34 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-33 erhalten werden, wobei der Rest U für $C(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur Ar'-33 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-34 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination von zwei Teilstrukturen N-1 und einer Teilstruktur Ar-34 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-33 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination von zwei Teilstrukturen N-1 und einer Teilstruktur Ar'-33 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-35 erhalten werden, wobei der Rest U für $C(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur Ar-35 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-34 erhalten werden, wobei der Rest U für $C(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise von R'-2 abgeleitet sind und zusammen ein Ringsystem bilden, an welches bevorzugt wiederum ein Strukturelement gebundes ist, welches durch eine Kombination von zwei Teilstrukturen N-1 und einer Teilstruktur N-1 und Ar'-34 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-35 erhalten werden, wobei der Rest U für $Si(R^1)_2$ steht und insgesamt höchstens 6, vorzugsweise höchstens 4 und speziell bevorzugt höchstens 2 Reste der Formel X' ungleich CH oder CD sind, wobei die durch die zwei Reste $R^1$ der durch U definierten Gruppe vorzugsweise ein Strukturelement bilden, welches durch eine Kombination von zwei Teilstrukturen N-1 und einer Teilstruktur Ar-35 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar'-34 erhalten werden, wobei der Rest U für $Si(R^1)_2$ steht und die Summe der Indices v, o und m höchstens 5, vorzugsweise höchstens 3 und speziell bevorzugt 2 ist, wobei der Index v speziell bevorzugt 0 ist, wobei die durch die zwei Reste $R^1$ der durch U definierten Gruppe vorzugsweise ein Strukturelement bilden, welches durch eine Kombination von zwei Teilstrukturen N-1 und einer Teilstruktur N-1 und Ar'-34 erhalten werden kann, wobei diese Verbindungen die folgenden Eigenschaften aufweisen:

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |

(fortgesetzt)

| $R^1$, ungleich H oder D | vorzugsweise | bevorzugt ein $R^1$ | besonders bevorzugt |
|---|---|---|---|
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |
| $R^1$-1 bis $R^1$-95 | $R^1$-1 bis $R^1$-54 | - | - |
| $R^1$-1 bis $R^1$-4 | $R^1$-1 | - | - |

In einer weiteren Ausgestaltung der vorliegenden Erfindung sind Verbindungen bevorzugt, die durch eine Kombination von zwei Teilstrukturen N-1 und einer Teilstruktur Ar-36 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-35' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-37 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-36' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-38 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-37' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-39 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |

(fortgesetzt)

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-38' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-40 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von zwei Teilstrukturen N-1 und einer Teilstruktur Ar-39' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |

(fortgesetzt)

| $R^1$, ungleich H oder D | vorzugsweise | Mindestens ein $R^1$ | vorzugsweise |
|---|---|---|---|
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-41 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-40' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-42 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-41' erhalten werden, wobei die Summe der Indices v, o und m höchstens 5, vorzugsweise höchstens 3 und speziell bevorzugt 1 ist, wobei der Index v speziell bevorzugt 0 ist,

welche die folenden Eienschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-43 erhalten werden, wobei insgesamt höchstens 6, vorzugsweise höchstens 4 und speziell bevorzugt höchstens 2 Reste der Formel X' ungleich CH oder CD sind, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

In einer weiteren Ausgestaltung der vorliegenden Erfindung sind Verbindungen bevorzugt, die durch eine Kombination von vier Teilstrukturen N-1 und einer Teilstruktur Ar-42' erhalten werden, wobei die Summe der Indices v, o und m höchstens 5, vorzugsweise höchstens 3 und speziell bevorzugt 1 ist, wobei der Index v speziell bevorzugt 0 ist, welche die folgenden Eigenschaften aufweisen:

| $R^1$, ungleich H oder D | vorzugsweise | Mindestens ein $R^1$ | vorzugsweise |
|---|---|---|---|
| $R^1$-1 bis $R^1$-95 | $R^1$-1 bis $R^1$-54 | H-1 bis H-26 | H1 |
| $R^1$-1 bis $R^1$-95 | $R^1$-1 bis $R^1$-54 | H-1 bis H-26 | H4 oder H-5 |
| $R^1$-1 bis $R^1$-4 | $R^1$-1 | $R^1$-1 bis $R^1$-54 | H1 |
| $R^1$-1 bis $R^1$-4 | $R^1$-1 | H-1 bis H-26 | H4 oder H-5 |
| $R^1$-1 bis $R^1$-95 | $R^1$-1 bis $R^1$-54 | QL | Q-11 bis Q-19, Q-23 bis Q-28, Q-31 bis Q-42 |
| $R^1$-1 bis $R^1$-4 | $R^1$-1 | QL | Q-11 bis Q-19 |

Ferner kann vorgesehen sein, dass die Verbindung, die in einer organischen elektronischen Vorrichtung als aktive Verbindung einsetzbar ist, nicht in unmittelbaren Kontakt mit einem Metallatom steht, vorzugsweise kein Ligand für einen Metallkomplex darstellt.

Beispiele für geeignete erfindungsgemäße Verbindungen sind die nachstehend gezeigten Strukturen gemäß den folgenden Formeln 1 bis 108:

| | | |
|---|---|---|
| 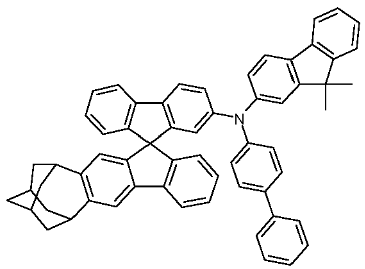 | 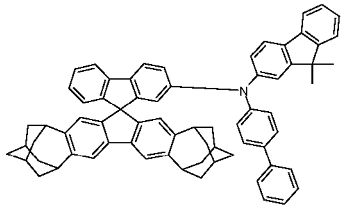 | 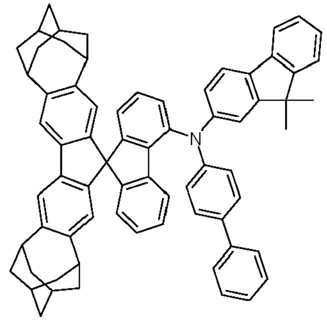 |
| Formel 1 | Formel 2 | Formel 3 |
| 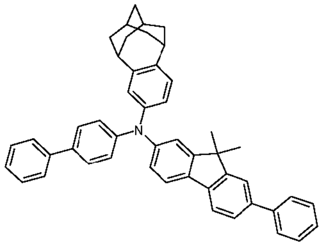 | 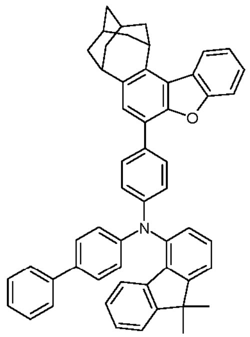 | 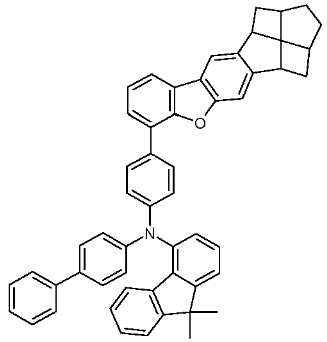 |
| Formel 4 | Formel 5 | Formel 6 |

(fortgesetzt)

| | | | | | |
|---|---|---|---|---|---|
| 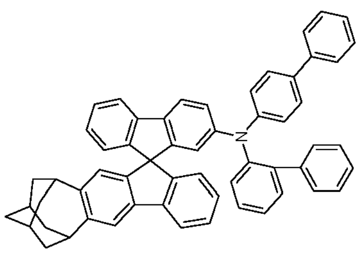 | Formel 7 | 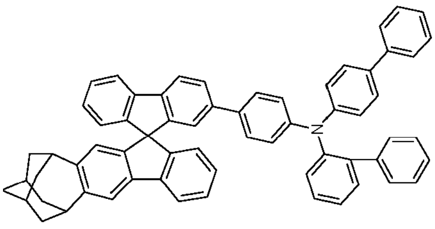 | Formel 8 | 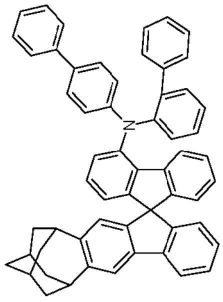 | Formel 9 |
| 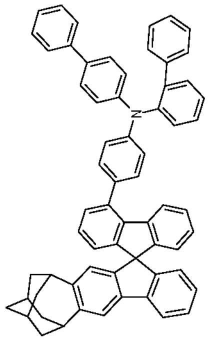 | Formel 10 | 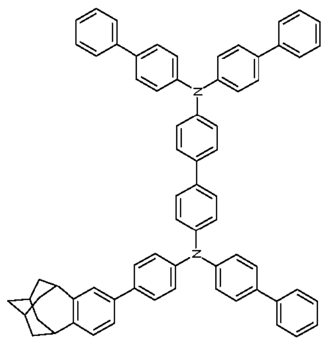 | Formel 11 | 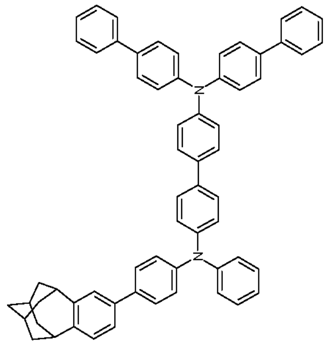 | Formel 12 |

(fortgesetzt)

| | | |
|---|---|---|
| 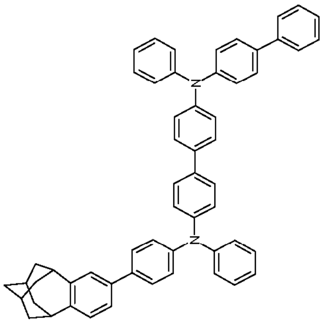 | 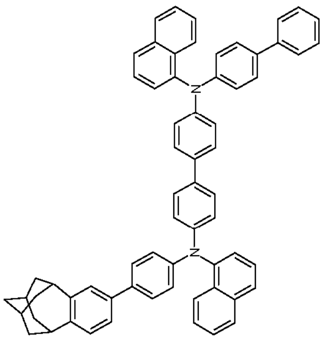 | 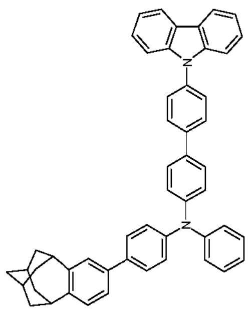 |
| Formel 13 | Formel 14 | Formel 15 |
| 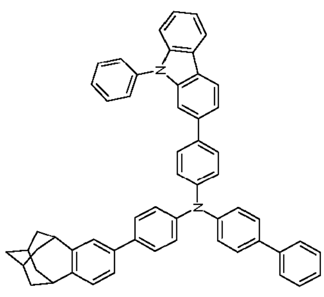 | 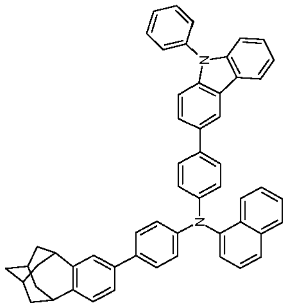 | 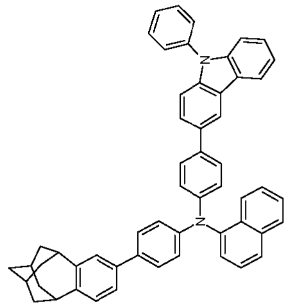 |
| Formel 16 | Formel 17 | Formel 18 |
| 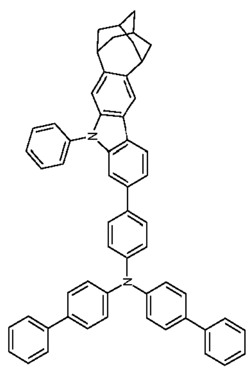 | 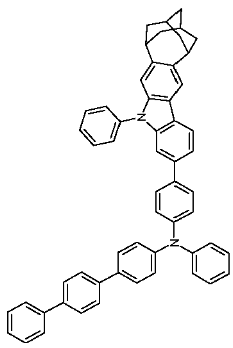 | 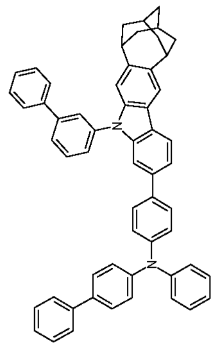 |
| Formel 19 | Formel 20 | Formel 21 |

(fortgesetzt)

| | | |
|---|---|---|
| 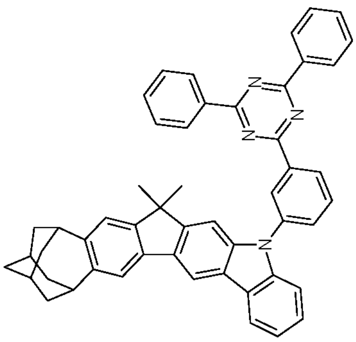 | 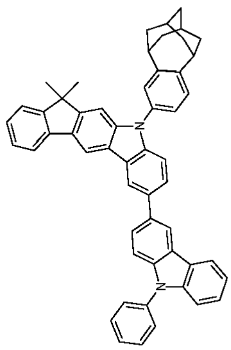 | 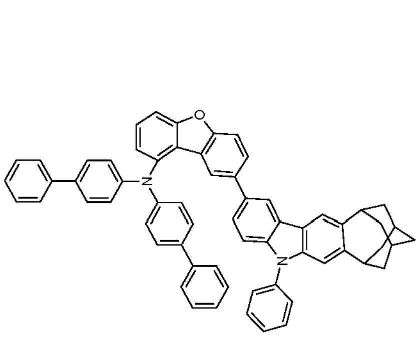 |
| Formel 22 | Formel 23 | Formel 24 |
| 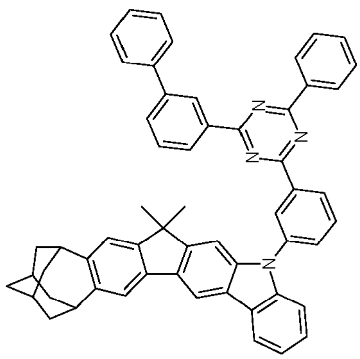 | 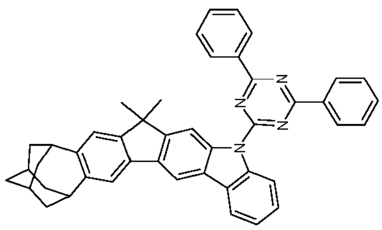 | 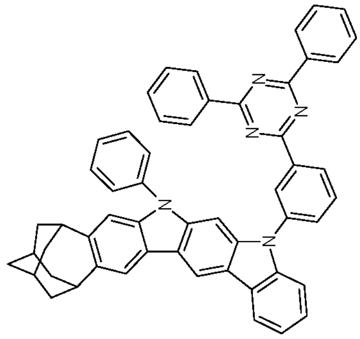 |
| Formel 25 | Formel 26 | Formel 27 |

(fortgesetzt)

| | | |
|---|---|---|
| 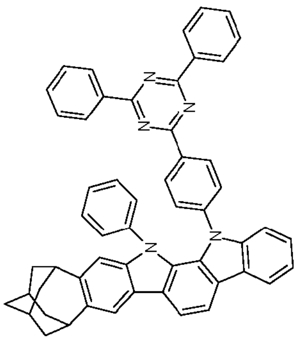 | 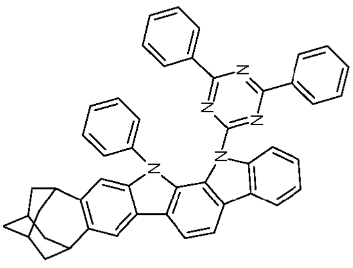 | 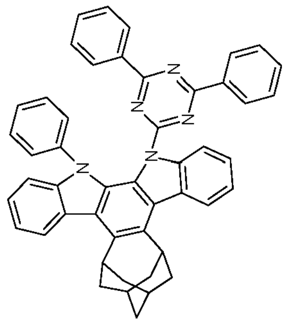 |
| Formel 28 | Formel 29 | Formel 30 |
| 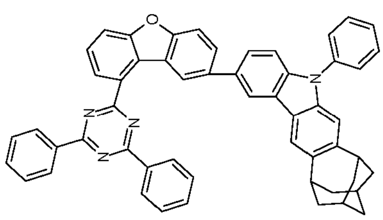 | 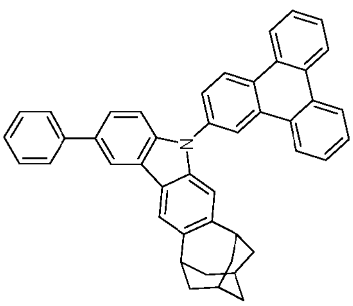 | 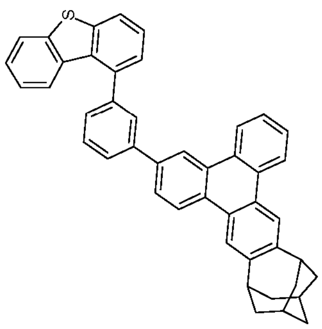 |
| Formel 31 | Formel 32 | Formel 33 |
| 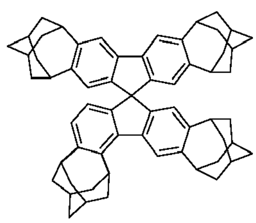 | 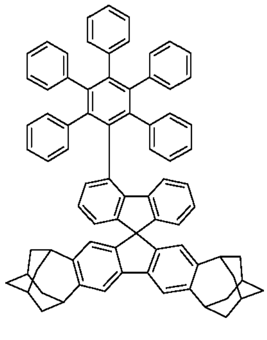 | 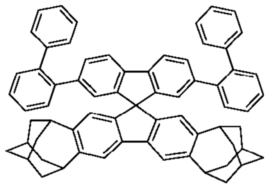 |
| Formel 34 | Formel 35 | Formel 36 |

(fortgesetzt)

| | | |
|---|---|---|
| 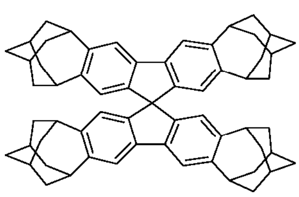 | 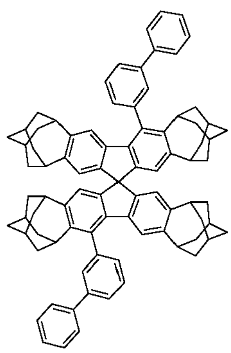 | 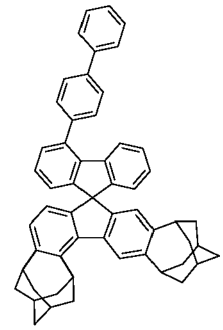 |
| Formel 37 | Formel 38 | Formel 39 |
| 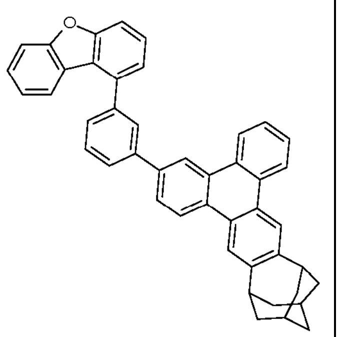 | 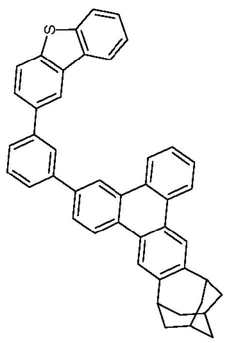 | 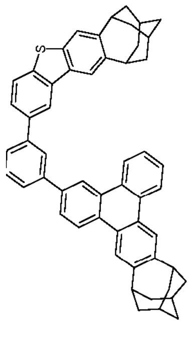 |
| Formel 40 | Formel 41 | Formel 42 |
| 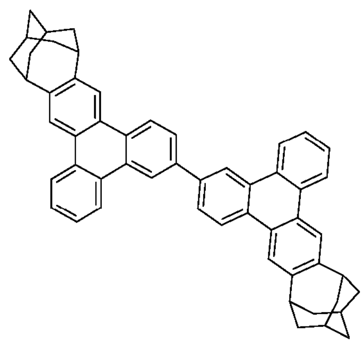 | 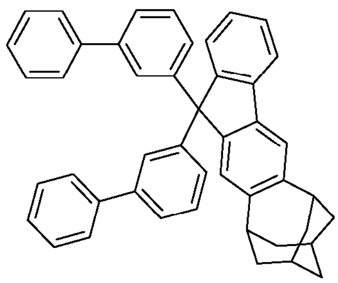 | 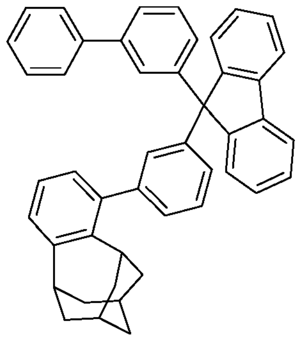 |
| Formel 43 | Formel 44 | Formel 45 |

(fortgesetzt)

| | | |
|---|---|---|
| 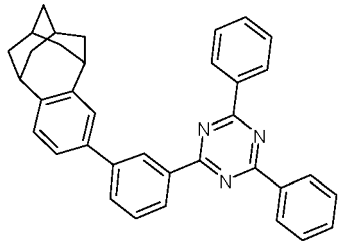 | 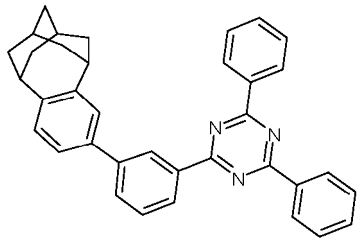 | 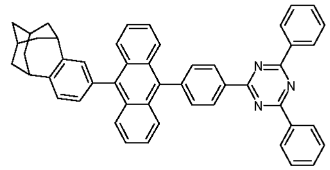 |
| Formel 46 | Formel 47 | Formel 48 |
| 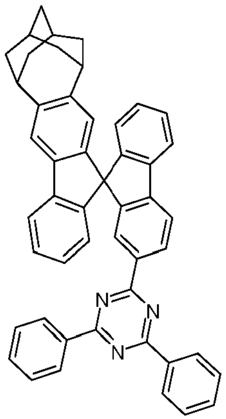 | 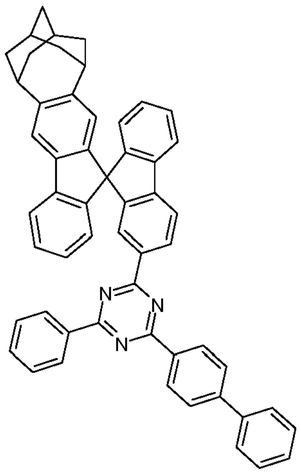 | 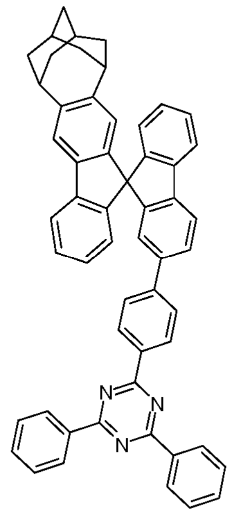 |
| Formel 49 | Formel 50 | Formel 51 |

(fortgesetzt)

| | | |
|---|---|---|
| 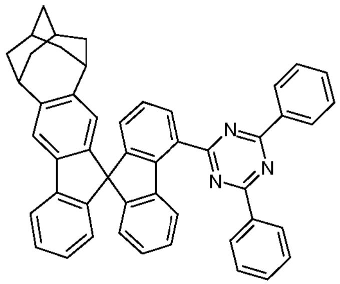 | 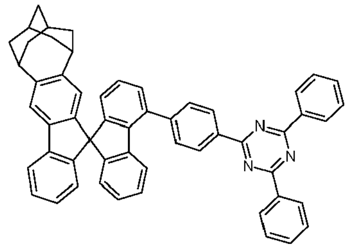 | 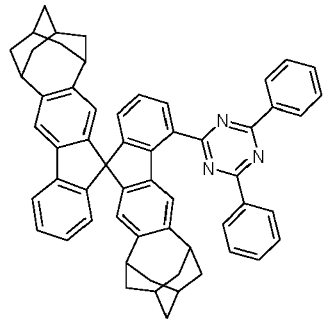 |
| Formel 52 | Formel 53 | Forme 54 |
| 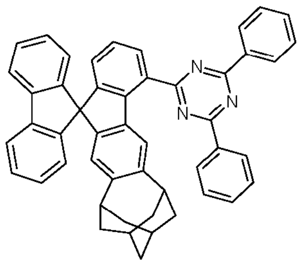 | 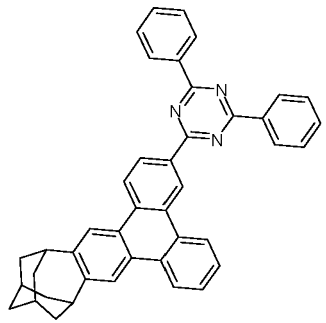 | 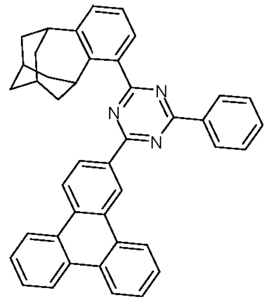 |
| Formel 55 | Formel 56 | Formel 57 |
| 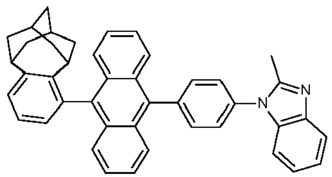 | 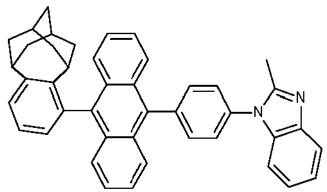 | 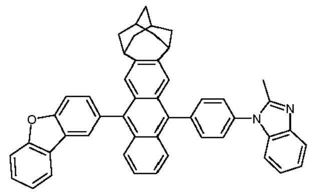 |
| Formel 58 | Formel 59 | Formel 60 |

(fortgesetzt)

| | | |
|---|---|---|
| 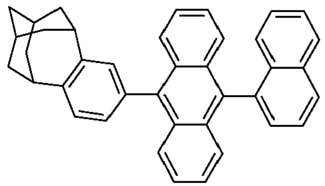 | 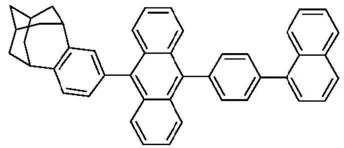 | 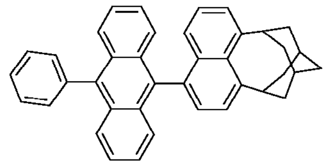 |
| Formel 61 | Formel 62 | Formel 63 |
| 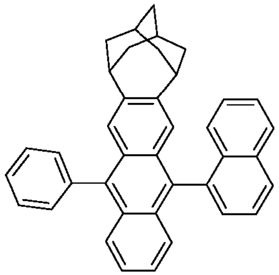 | 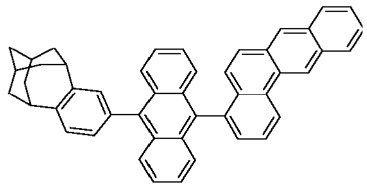 | 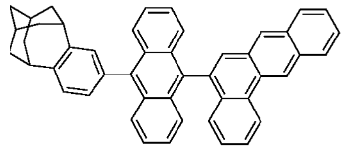 |
| Formel 64 | Formel 65 | Formel 66 |
| 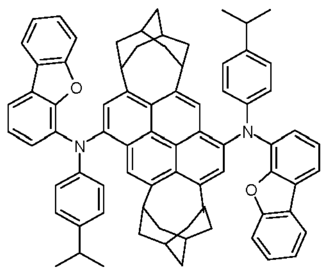 | 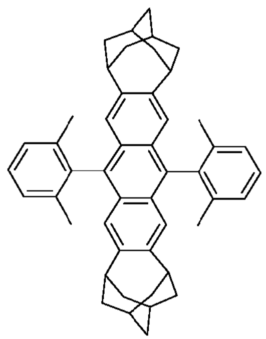 | 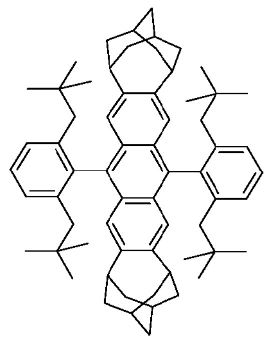 |
| Formel 67 | Formel 68 | Formel 69 |

(fortgesetzt)

| | | |
|---|---|---|
| 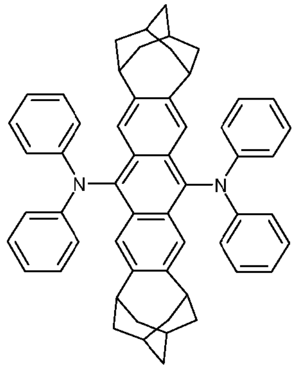 | 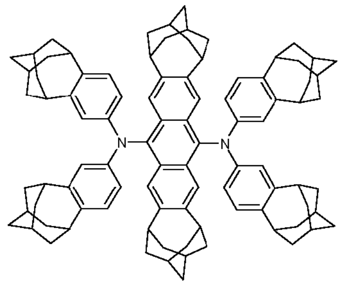 | 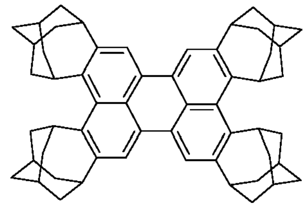 |
| Formel 70 | Formel 71 | Formel 72 |
| 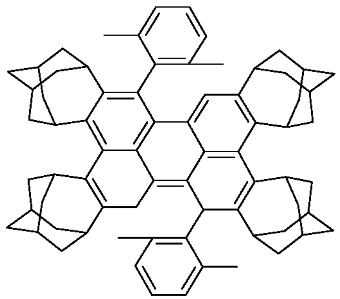 | 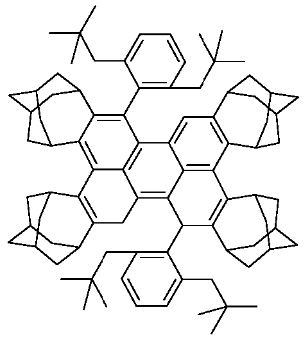 | 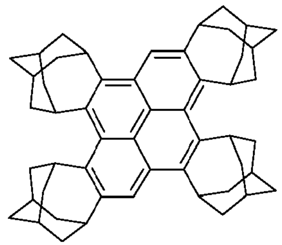 |
| Formel 73 | Formel 74 | Formel 75 |

(fortgesetzt)

| | | |
|---|---|---|
| 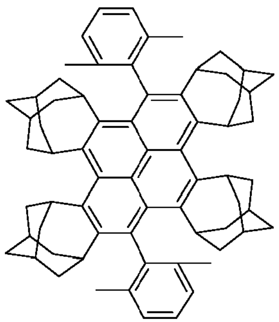 | 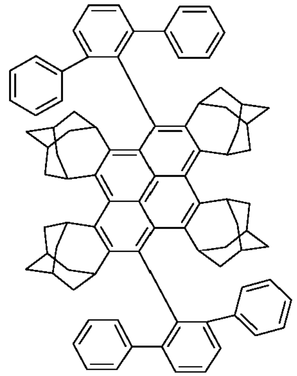 | 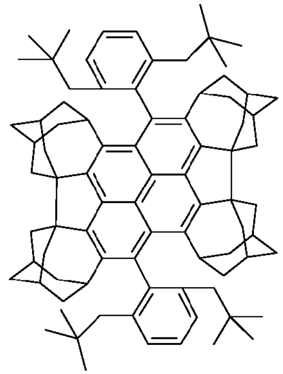 |
| Formel 76 | Formel 77 | Formel 78 |
| 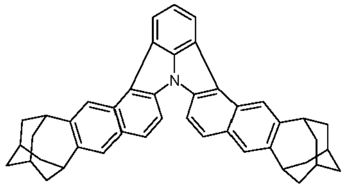 | 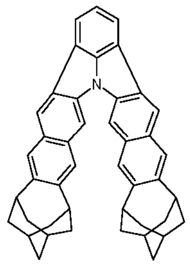 | 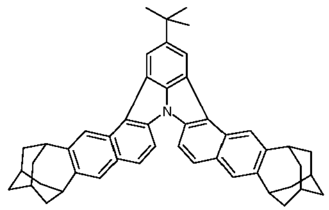 |
| Formel 79 | Formel 80 | Formel 81 |

(fortgesetzt)

| | |
|---|---|
| Formel 82 | 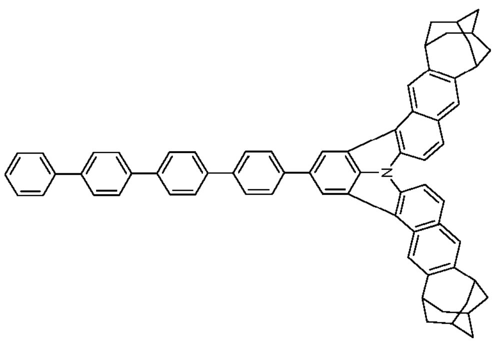 |
| Formel 83 | 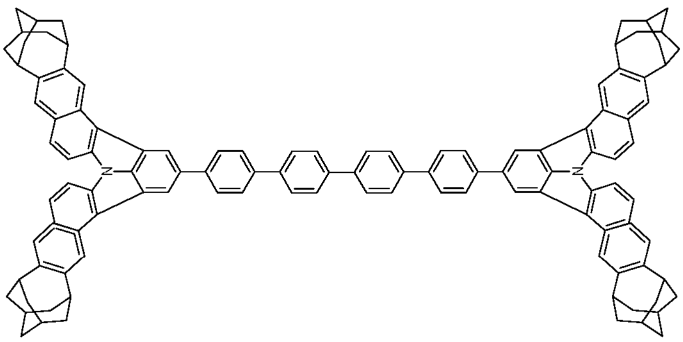 |
| Formel 84 | 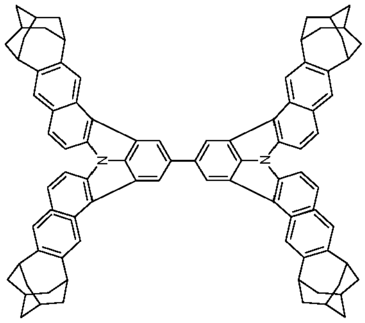 |

(fortgesetzt)

| Formel 85 | Formel 86 | Formel 87 |
|---|---|---|
| 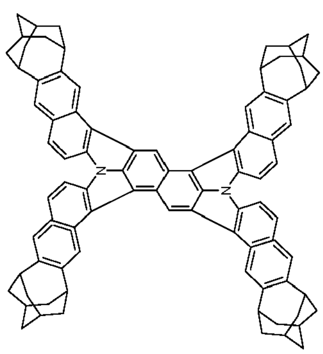 | 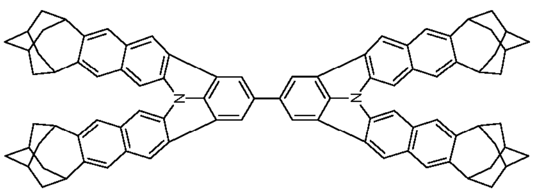 | 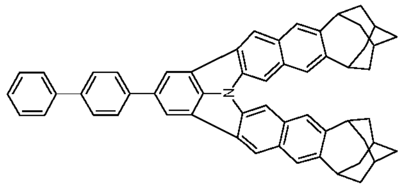 |
| Formel 88 | Formel 89 | Formel 90 |
| 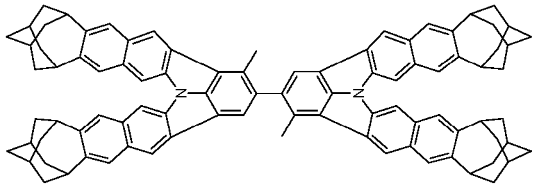 | 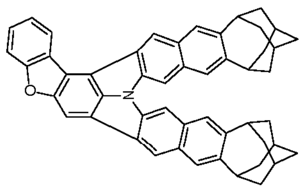 | 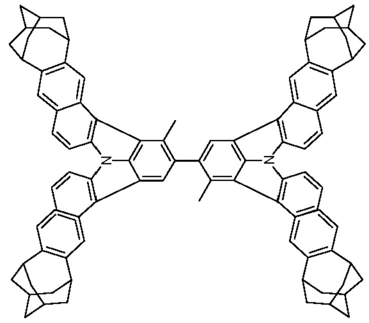 |

(fortgesetzt)

| | | | | | |
|---|---|---|---|---|---|
| 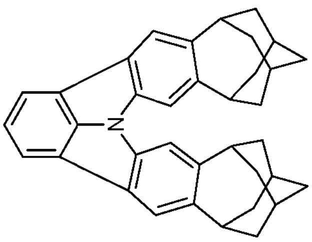 | Formel 91 | 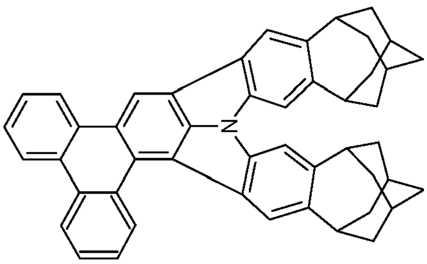 | Formel 92 | 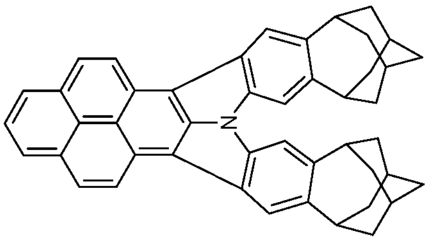 | Formel 93 |
| 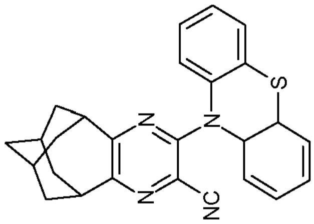 | Formel 94 | 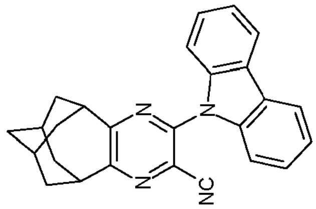 | Formel 95 | 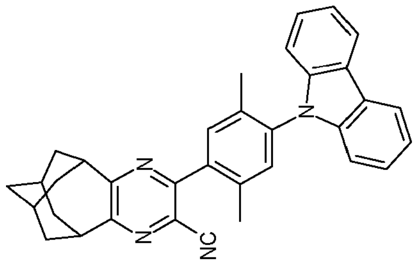 | Formel 96 |

(fortgesetzt)

| | | |
|---|---|---|
| 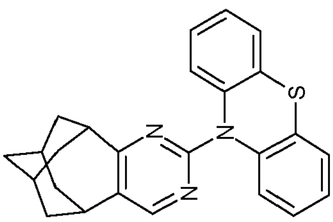 | 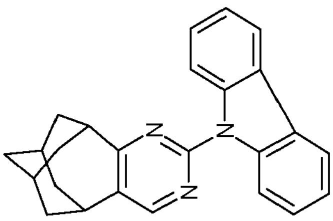 | 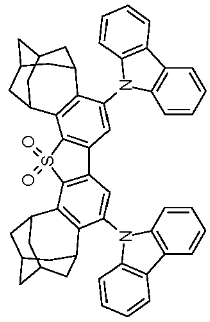 |
| Formel 97 | Formel 98 | Formel 99 |
| 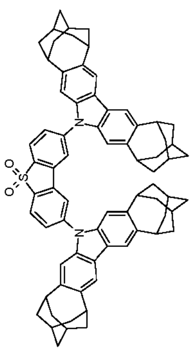 | 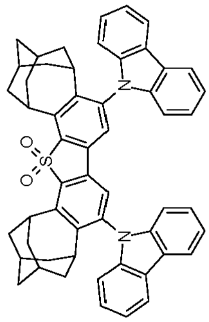 | 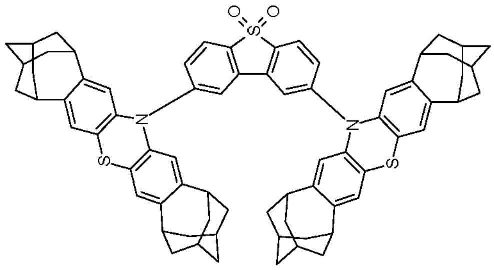 |
| Formel 100 | Formel 101 | Formel 102 |

(fortgesetzt)

| | | |
|---|---|---|
| 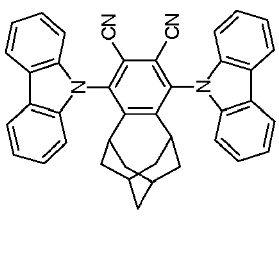 | 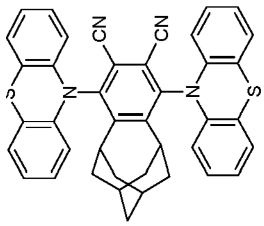 | 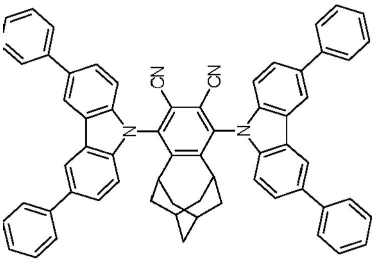 |
| Formel 103 | Formel 104 | Formel 105 |
| 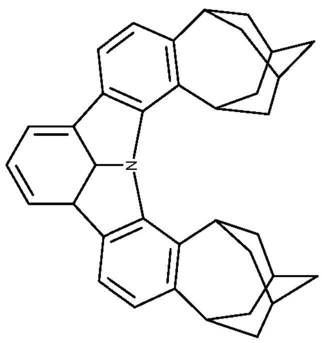 | 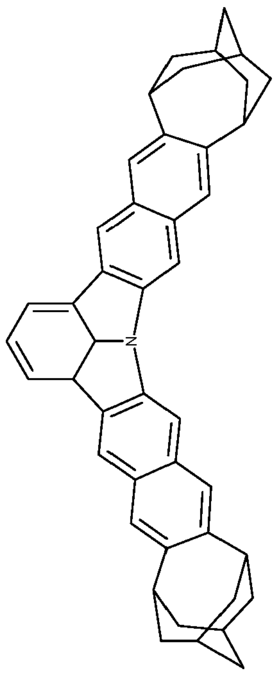 | |
| Formel 106 | Formel 107 | |

(fortgesetzt)

| | | |
|---|---|---|
| 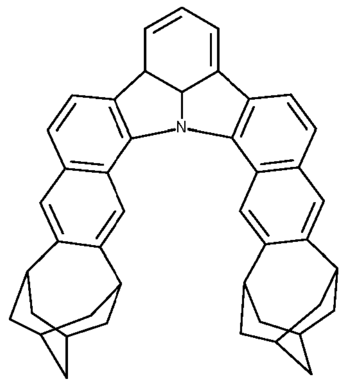 | | |
| Formel 108 | | |

Die Strukturen der Formeln 1 bis 18 eignen sich unter anderem als Lochleitermaterialien (HTM) und gegebenenfalls als Elektronenblockiermaterialien (EMB). Die Strukturen der Formeln 1 bis 33 eignen sich unter anderem als Tripletmatrixmaterial (TMM), insbesondere für phosphoreszierende Emitter und als Matrixmaterial für TADF-Verbindungen. Die Strukturen der Formeln 34 bis 45 eignen sich insbesondere als Wide-Band-Gap-Materialien. Die Strukturen der Formeln 46 bis 60 eignen sich insbesondere als Elektronentransportmaterialien (ETM) bzw. als elektronenleitendes Tripletmatrixmaterial (eTMM). Die Strukturen der Formeln 61 bis 67 eignen sich insbesondere als Matrixmaterialen fluoreszierende Emitter (SMB). Die Strukturen der Formeln 67 bis 93 eignen sich insbesondere als fluoreszierender Emitter (SEB). Die Strukturen der Formeln 94 bis 105 eignet sich insbesondere als Emitter, der TADF (thermally activated delayed fluorescence) zeigt und als TADF-Verbindung in Hyperfluoreszenzen Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Verbindungen, bevorzugt Verbindungen umfassend Strukturen der Formeln (I) bis (XVIII), bei dem in einer Kupplungsreaktion eine Verbindung, umfassend mindestens ein aliphatisches polycyclisches Ringsystem mit mindestens 3 Ringen, mit einer Verbindung, umfassend mindestens eine aromatische oder heteroaromatische Gruppe, verbunden wird.

Geeignete Verbindungen, umfassend mindestens ein aliphatisches polycyclisches Ringsystem mit mindestens 3 Ringen, können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Verbindungen, umfassend mindestens eine aromatische oder heteroaromatische Gruppe, umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formeln (I) bis (XVIII) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, die eine Löslichkeit in gängigen organischen Lösemitteln bewirken, so dass die Verbindungen beispielsweise in Toluol oder Xylol bei Raumtemperatur in ausreichender Konzentration löslich sind, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formeln (I) bis (XVIII) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formeln (I) bis (XVIII) oder erfindungsgemäße Verbindungen, wobei ein oder mehrere Bindungen der erfindungsgemäßen Verbindungen oder der Strukturen der Formeln (I) bis (XVIII) zum Polymer, Oligomer oder Dendrimer vorhanden sind. Je nach Verknüpfung der Strukturen der Formeln (I) bis (XVIII) bzw.

der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formeln (I) bis (XVIII) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen, die in einer organischen elektronischen Vorrichtung als aktive Verbindung einsetzbar sind, bevorzugt Verbindungen, umfassend Strukturen gemäß den Formeln (I) bis (XVIII) und/oder den Formeln (Ia) bis (XVIIIa) oder die durch eine Kombination der Teilstrukturen gemäß den Formeln (N-1) bis (N-6), (Ar-1) bis (Ar-54) und/oder (Ar'-1) bis (Ar'-53) erhältlichen Verbindungen bzw. die zuvor und nachfolgend ausgeführten bevorzugten Ausführungsformen bevorzugt, die eine Glasübergangstemperatur von mindestens 70 °C, besonders bevorzugt von mindestens 110 °C, ganz besonders bevorzugt von mindestens 125 °C und insbesondere bevorzugt von mindestens 150 °C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, beispielsweise ein fluoreszierender Dotand, ein phosphoreszierender Dotand oder eine Verbindung, die TADF (thermally activated delayed fluorescence) zeigt, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine erfindungsgemäße Verbindung, bevorzugt eine Verbindung umfassend Strukuren gemäß Formeln (I) bis (XVIII) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine erfindungsgemäße Verbindung, bevorzugt eine Verbindung umfassend mindestens eine Struktur gemäß Formeln (I) bis (XVIII) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31 G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\mathrm{HOMO(eV)} = ((\mathrm{HEh}*27.212)-0.9899)/1.1206$$

$$\mathrm{LUMO(eV)} = ((\mathrm{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formeln (I) bis (XVIII) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder

Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2016124304, WO 2017032439, WO 2018019687, WO 2018019688, WO 2018041769, WO 2018054798, WO 2018069196, WO 2018069197, WO 2018069273 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

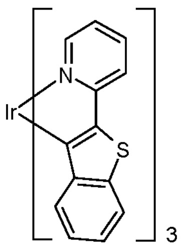
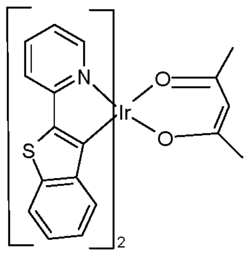
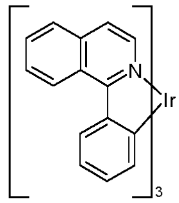

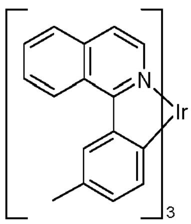
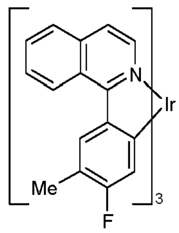
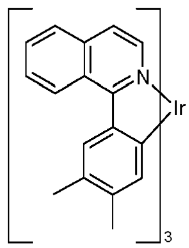

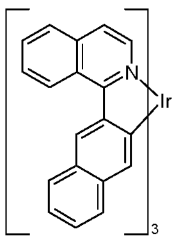
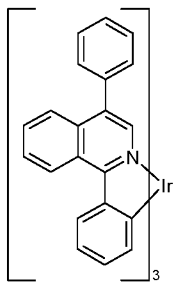
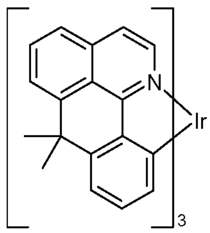

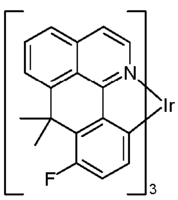
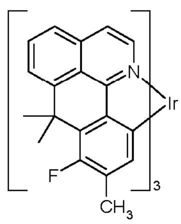
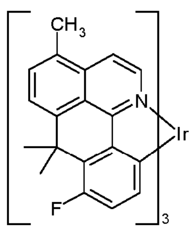

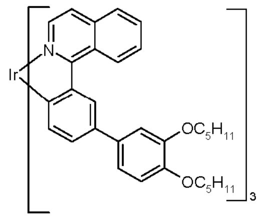 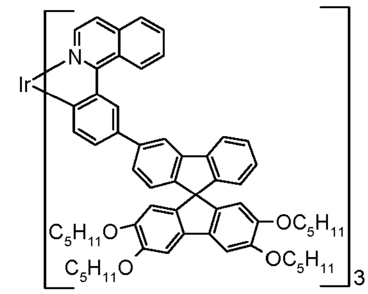 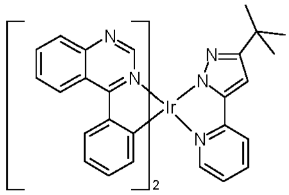

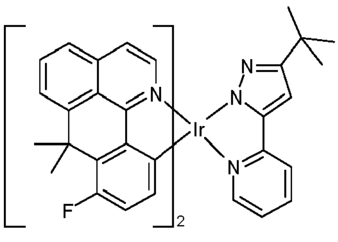 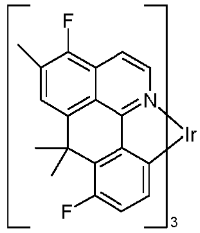 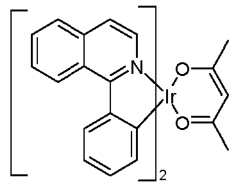

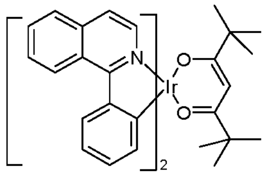 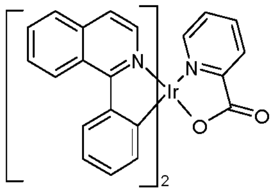 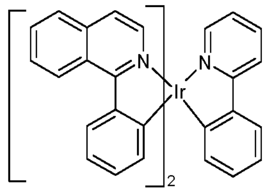

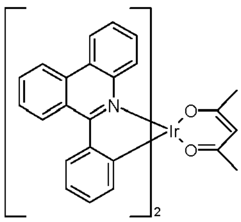 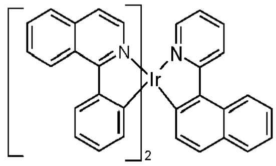 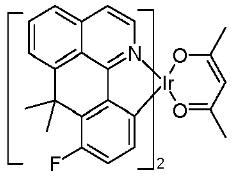

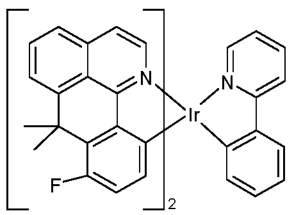 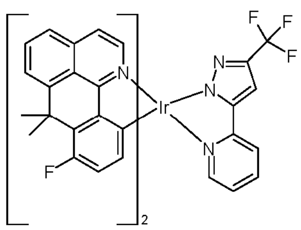 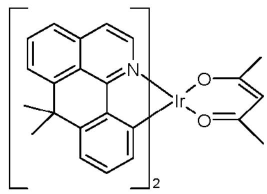

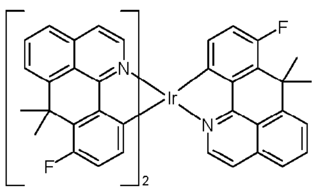 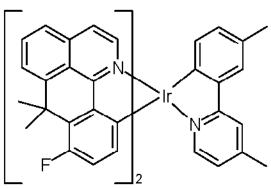 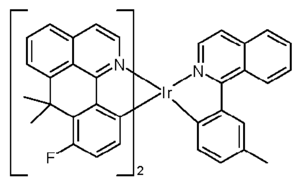

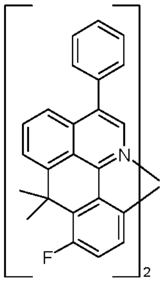

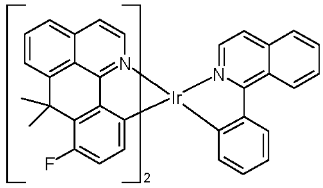

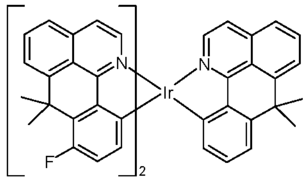

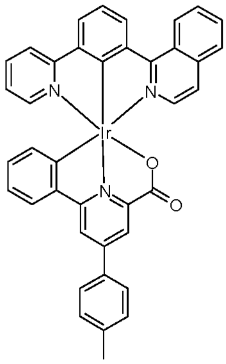

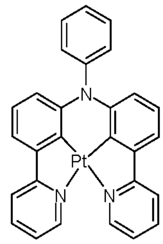

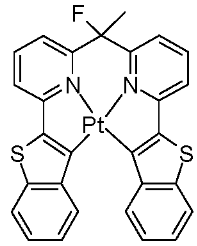

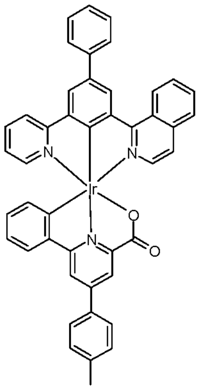

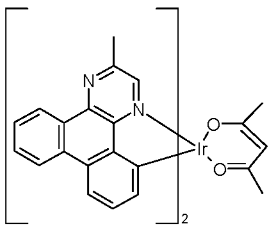

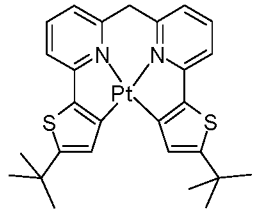

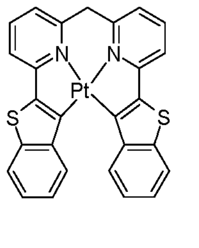

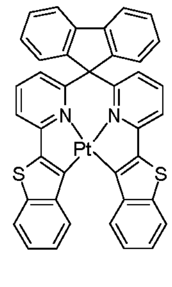

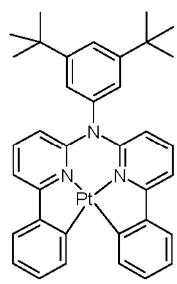

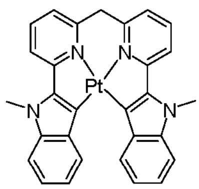

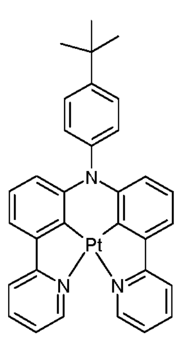

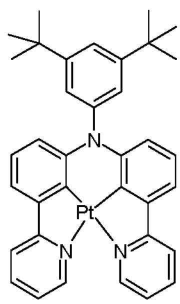

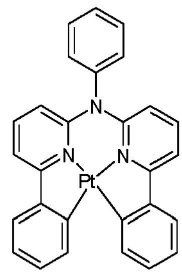

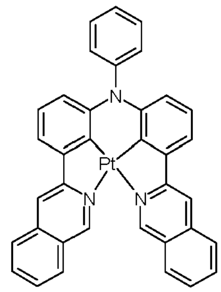

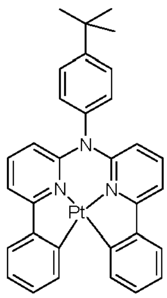

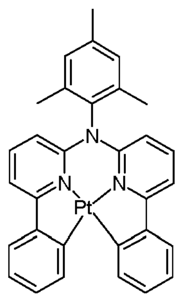

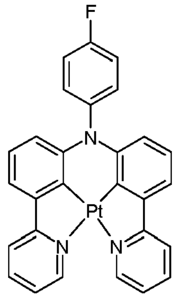

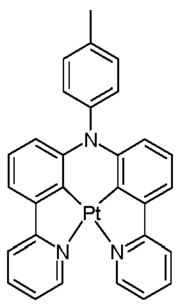

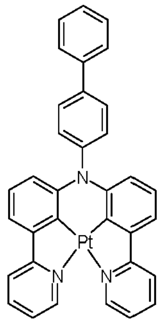

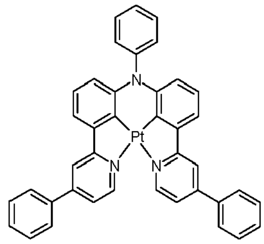

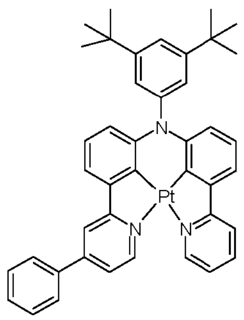

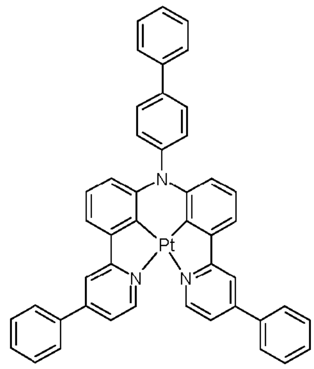

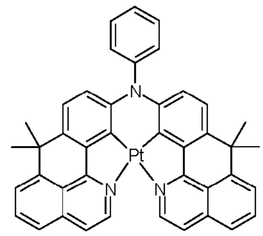

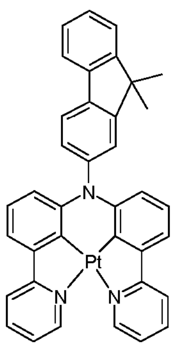

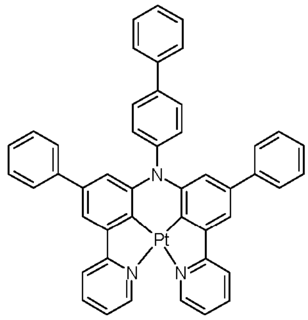

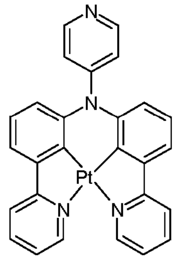

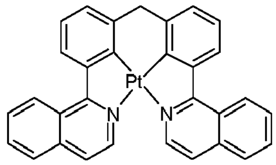
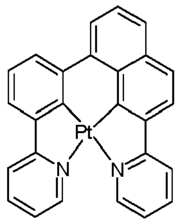
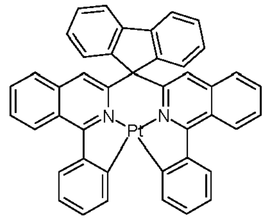

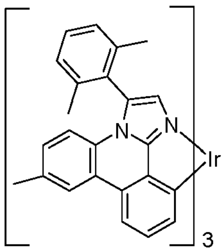
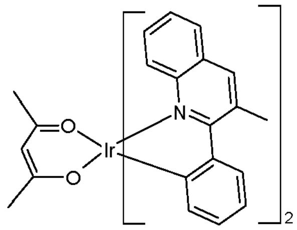
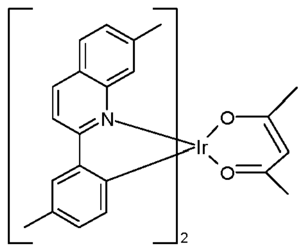

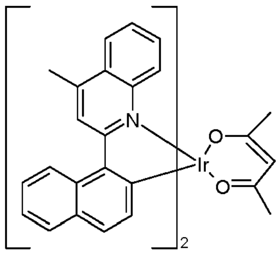
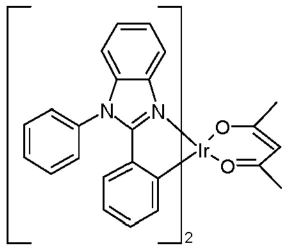
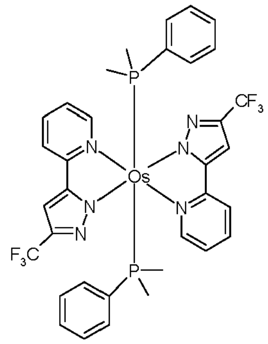

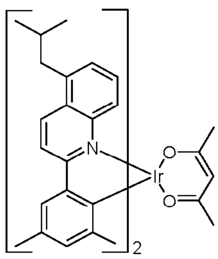
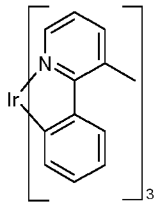
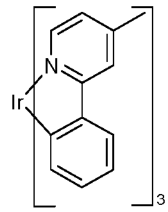

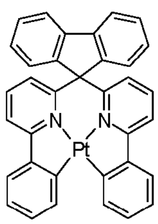
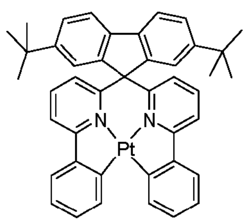
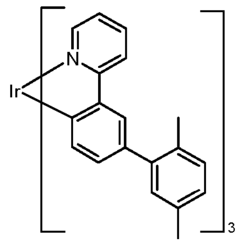

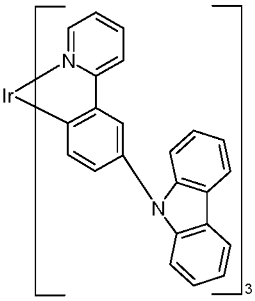
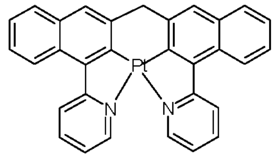
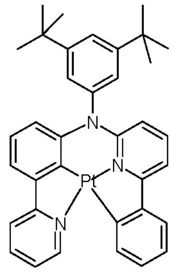
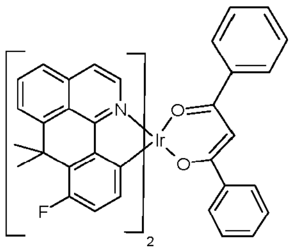
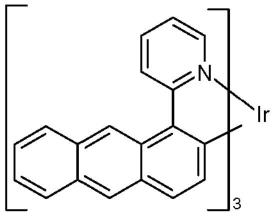
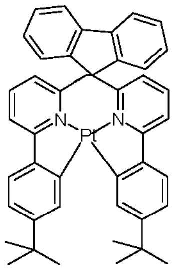
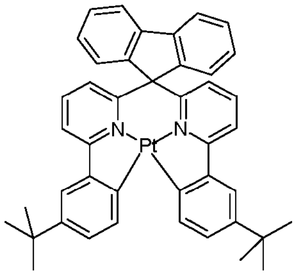
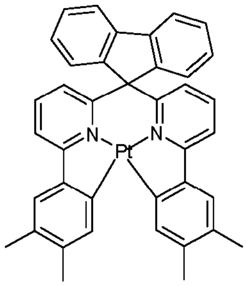
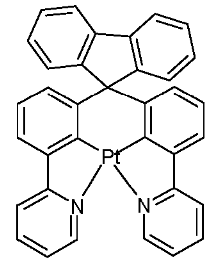
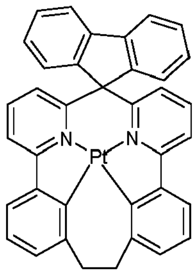
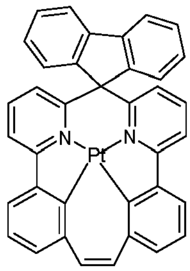
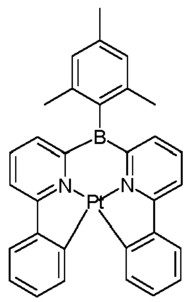
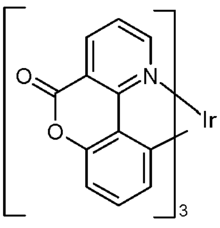
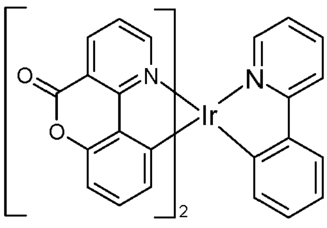
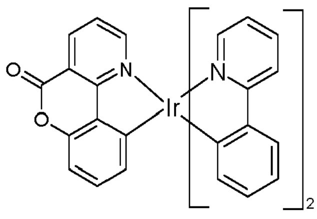

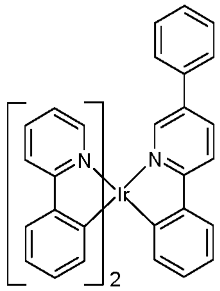
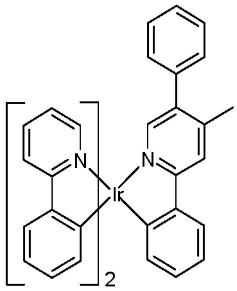
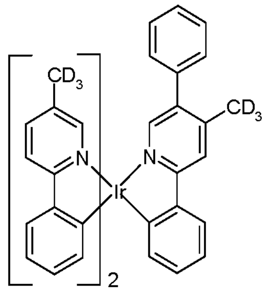

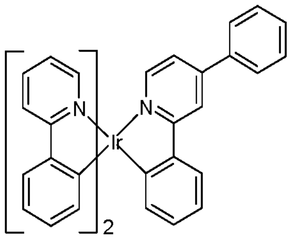
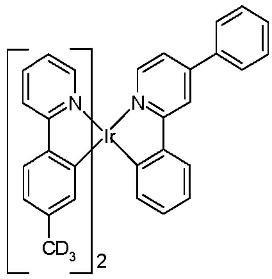
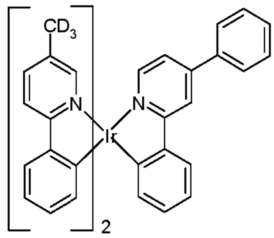

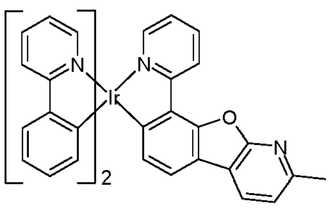
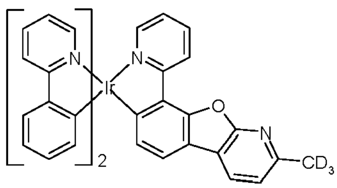
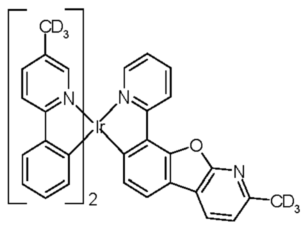

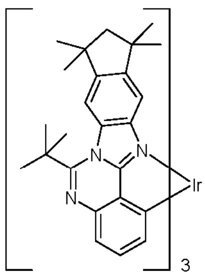
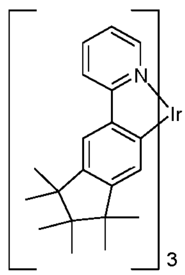
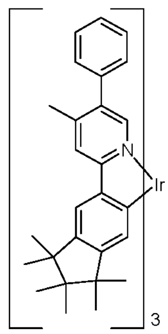

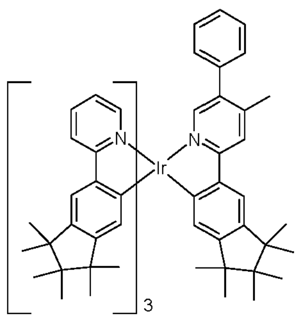
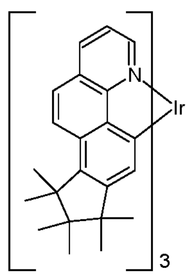
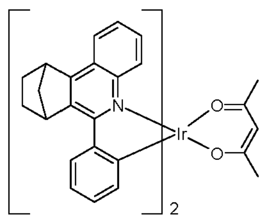

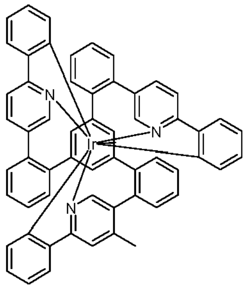
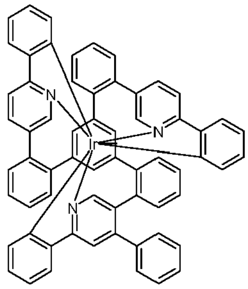
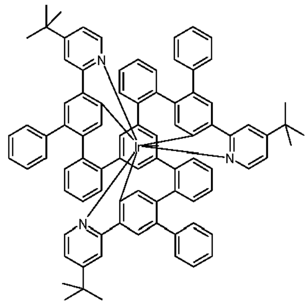

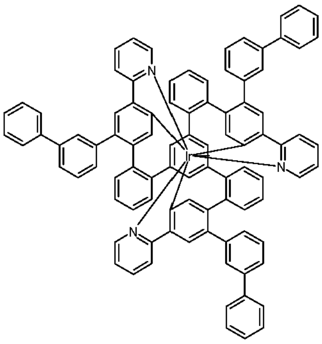
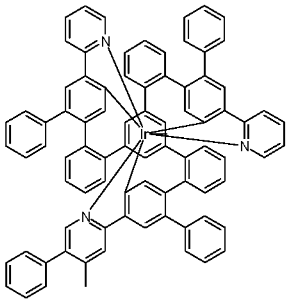
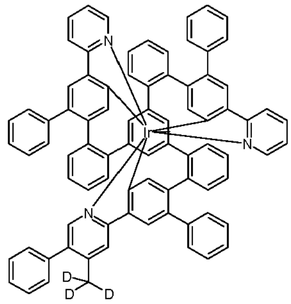

Die oben beschriebenen Verbindungen, umfassend Strukturen der Formeln (I) bis (XVIII) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr

als drei emittierende Schichten aufweisen. Weiterhin bevorzugt sind auch Tandem-OLEDs. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die efindungsgemäße Verbindung, vorzugsweise eine Verbindung umfassend Strukturen gemäß Formeln (I) bis (XVIII) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

In einer weiterhin besonders bevorzugten Ausführungsform der vorliegenden Erfindung umfasst eine erfindungsgemäße organische Elektrolumineszenzvorrichtung die efindungsgemäße Verbindung, vorzugsweise eine Verbindung umfassend Strukturen gemäß Formeln (I) bis (XVIII) bzw. die oben aufgeführten bevorzugten Ausführungsformen in einer Lochleitschicht oder einer Elektronenleitschicht.

Geeignete Matrixmaterialien, welche in Kombination mit den Verbindungen gemäß Formeln (I) bis (XVIII) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, 4-SpirocarbazolDerivate, z. B. gemäß WO 2014/094963 oder WO 2015/192939, oder Dibenzofuran-Derivate, z. B. gemäß WO 2015/169412, WO 2016/015810, WO 2016/023608 oder den noch nicht offen gelegten Anmeldungen EP 16158460.2 und EP 16159829.7. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

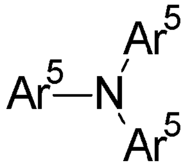

Formel (TA-1)

wobei $Ar^5$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formeln (I) bis (XVIII) genannte Bedeutung aufweist. Vorzugsweise stellt $Ar^5$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^5$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl,

Indenocarbazolyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^5$ gleich oder verschieden bei jedem Auftreten ausgewählt aus den oben genannten Gruppen $R^1$-1 bis R'-92, besonders bevorzugt $R^1$-1 bis R'-54.

In einer bevorzugten Ausführungsform der Verbindungen der Formel (TA-1) ist mindestens eine Gruppe $Ar^5$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^5$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^5$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^5$ ausgewählt aus einer Fluoren- oder Spirobifluorengruppe, insbesondere einer 4-Fluoren- bzw. 4-Spirobifluorengruppe, und eine Gruppe $Ar^5$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^5$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

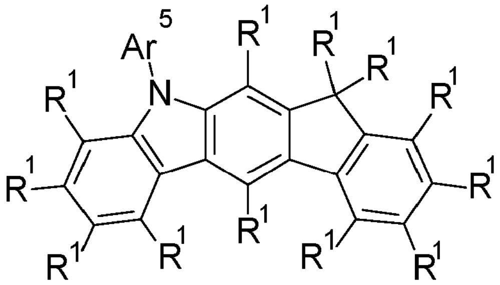

Formel (TA-2)

wobei $Ar^5$ und $R^1$ die oben insbesondere für Formeln (I) und/oder (TA-1) aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis $R^1$-54.

Eine bevorzugte Ausführungsform der Verbindungen der Formel (TA-2) sind die Verbindungen der folgenden Formel (TA-2a),

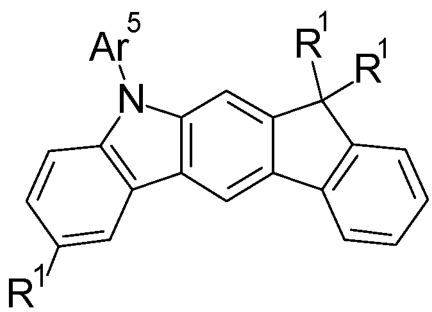

Formel (TA-2a)

wobei $Ar^5$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das

Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

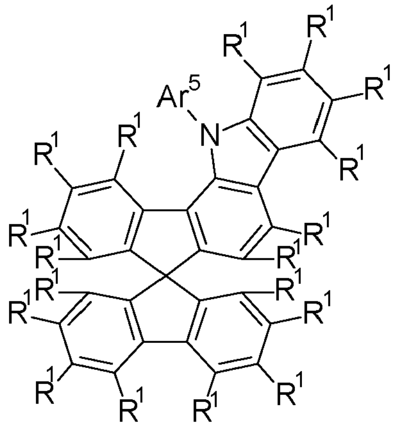

Formel (TA-3)

wobei $Ar^5$ und $R^1$ die oben, insbesondere für Formeln (I) und/oder (TA-1), aufgeführten Bedeutungen aufweisen. Dabei sind bevorzugte Ausführungsformen der Gruppe $Ar^5$ die oben aufgeführten Strukturen $R^1$-1 bis $R^1$-92, besonders bevorzugt $R^1$-1 bis R'-54.

Eine bevorzugte Ausführungsform der Verbindungen der Formel (TA-3) sind die Verbindungen der folgenden Formel (TA-3a),

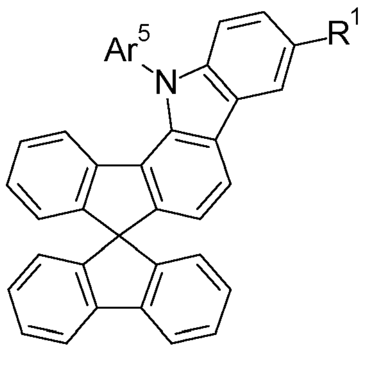

Formel (TA-3a)

wobei $Ar^5$ und $R^1$ die oben, insbesondere für Formeln (I) und/oder (TA-1), aufgeführten Bedeutungen Bedeutungen aufweisen. Dabei sind bevorzugte Ausführungsformen der Gruppe $Ar^5$ die oben aufgeführten Strukturen $R^1$-1 bis R'-92, besonders bevorzugt $R^1$-1 bis R'-54.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

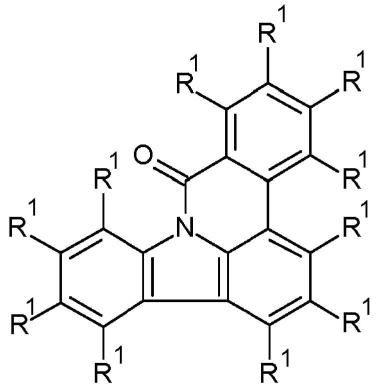

Formel (LAC-1)

wobei $R^1$ die oben, insbesondere für Formeln (I) aufgeführte Bedeutung aufweist.

Eine bevorzugte Ausführungsform der Verbindungen der Formel (LAC-1) sind die Verbindungen der folgenden Formel (LAC-1a),

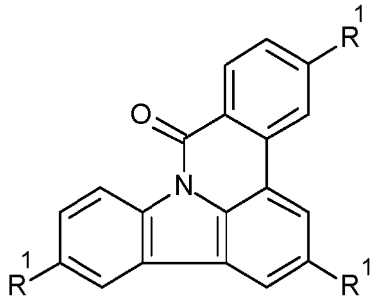

Formel (LAC-1a)

wobei $R^1$ die oben, insbesondere für Formeln (I) bis (XVIII) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formeln (I) bis (XVIII) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis R'-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formeln (I) bis (XVIII) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren gemäß Formeln (I) bis (XVIII) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen umfassend Strukuren gemäß Formeln (I) bis (XVIII) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch

verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formeln (I) bis (XVIII) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formeln (I) bis (XVIII) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend erfindungsgemäße Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen, insbesondere als elektronenleitende Materialien und/oder Lochleitermaterialien oder als Matrixmaterialien, weisen eine sehr gute Lebensdauer auf.

2. Elektronische Vorrichtungen, insbesondere organische Elektrolumineszenzvorrichtungen enthaltend erfindungsgemäße Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen insbesondere als Elektronentransport-Materialien, Lochleitermaterialien und/oder als Hostmaterialien weisen eine hervorragende Effizienz auf. Insbesondere ist die Effizienz deutlich höher gegenüber analogen Verbindungen, die kein aliphatisches polycyclisches Ringsystem mit mindestens 3 Ringen enthalten, welches an ein aromatisches oder heteroaromatisches Ringsystem mit 5 bis 60 Kohlenstoffaromen kondensiert ist. Hierbei bewirken die erfindungsgemäßen Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen eine geringe Betriebsspannung bei Verwendung in elektronischen Vorrichtungen. Hierbei bewirken diese Verbindungen insbesondere einen geringen Roll-off, d.h. einen geringen Abfall der Leistungseffizienz der Vorrichtung bei hohen Leuchtdichten.

3. Elektronische Vorrichtungen, insbesondere organische Elektrolumineszenzvorrichtungen enthaltend Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen als Elektronentransport-Materialien, Lochleitermaterialien und/oder als Hostmaterialien weisen eine hervorragende Farbreinheit auf.

4. Die erfindungsgemäßen Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen zeigen eine sehr hohe thermische und photochemische Stabilität und führen zu Verbindungen mit einer sehr hohen Lebensdauer.

5. Mit Verbindungen, Oligomeren, Polymeren oder Dendrimere, die in einer organischen elektronischen Vorrichtung als aktive Verbindung einsetzbar sind, bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

6. Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen weisen eine ausgezeichnete Glasfilmbildung auf.

7. Verbindungen, Oligomere, Polymere oder Dendrimere, die in einer organischen elektronischen Vorrichtung als aktive Verbindung einsetzbar sind, bzw. die zuvor und nachfolgend ausgeführten bevorzugten Ausführungsformen bilden aus Lösungen sehr gute Filme.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als fluoreszierenden Emitter, Emitter, der TADF (thermally activated delayed fluorescence) zeigt, Hostmaterial, Elektronentransportmaterial, Elektroneninjektionsmaterial, Lochleitermaterial, Loch-injektionsmaterial, Elektronenblockiermaterial, Lochblockiermaterial und/oder Wide-Band-Gap-Material, vorzugsweise als fluoreszierenden Emitter (Singulet-Emitter), Hostmaterial, Lochleitermaterial und/oder Elektronentransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen, die in einer organischen elektronischen Vorrichtung als aktive Verbindung einsetzbar sind, bevorzugt Verbindungen, umfassend Strukturen gemäß den Formeln (I) bis (XVIII) und/oder den Formeln (Ia) bis (XVIIIa) oder die durch eine Kombination der Teilstrukturen gemäß den Formeln (N-1) bis (N-6), (Ar-1)

bis (Ar-54) und/oder (Ar'-1) bis (Ar'-53) erhältlichen Verbindungen bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbindungen die mehrere tautomere Formen ausweisen können wird eine tautomere Form stellvertretend gezeigt.

### 1) Synthese der Synthone S:

### Beispiel S1:

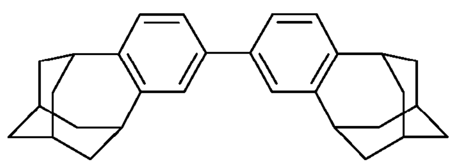

Ein gut gerührtes Gemisch aus 27.2 g (100 mmol) 2-Brom-6,7,8,9,10,11-hexahydro-5,9:7,11-dimethano-5H-benzocyclononen [1801624-97-4], 32.4 g (100 mmol) 2-(6,7,8,9,10,11-Hexahydro-5,9:7,11-dimethano-5H-benzocyclononen-2-yl)-4,4,5,5-tetramethyl-1,3,2-dioxaborolan [1801624-63-4], 63.7 g (300 mmol) Trikaliumphosphat, 1.83 g (6 mmol) Tri-o-tolyl-phosphin, 225 mg (1 mmol) Palladium(II)acetat, 350 ml Toluol, 80 ml Dioxan und 300 ml Wasser wird 16 h unter Rückfluss erhitzt. Nach Erkalten trennt man von der wässrigen Phase ab, wäscht die organische Phase einmal mit 300 ml Wasser, einmal mit 300 ml ges. Kochsalzlösung und trocknet dann über Magnesiumsulfat. Man filtriert vom Trockenmittel über ein mit Toluol vorgeschlämmtes Kieselgel-Bett ab und engt das Filtrat zur Trockne ein. Der glasartige Rückstand wird aus iso-Propanol umkristallisiert. Ausbeute: 30.8 g (78 mmol) 78 %. Reinheit n. $^1$H-NMR ca. 97 %.

Analog können folgende Verbindungen dargestellt werden:

| Bsp. | Edukte Bromid Keton / Elektrophil | Produkt | Ausbeute |
|---|---|---|---|
| S10 | 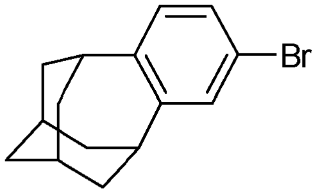 [1801624-97-4] 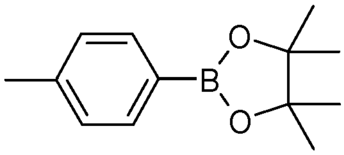 [195062-57-8] | 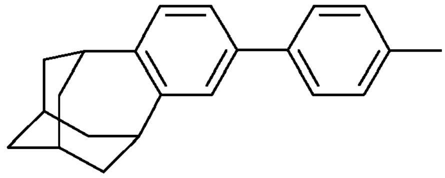 | 81 % |
| S11 | 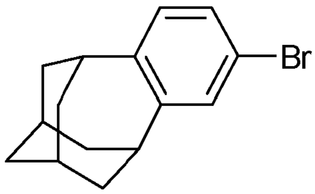 [1801624-97-4] 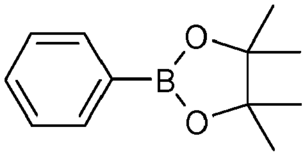 [24388-23-6] | 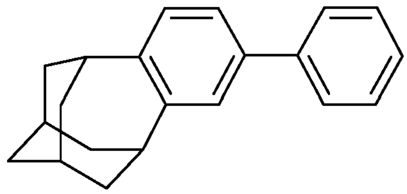 | 78 % |

**Beispiel S2:**

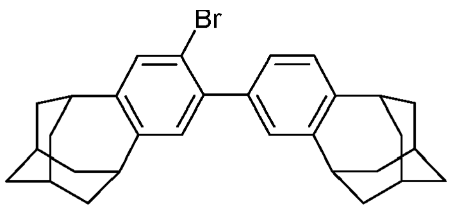

Eine gut gerührte Lösung von 39.5 g (100 mmol) S1 in 500 ml Dichlormethan wird unter Lichtausschluss tropfenweise während 3 h mit einem Gemisch aus 3.1 ml (120 mmol) Brom und 100 ml Dichlormethan versetzt. Nach beendeter Zugabe rührt man 4 h unter Rückfluss und 8 h bei Raumtemperatur nach. Man versetzt mit 200 ml ges. Natriumsulfit-Lösung, um überschüssiges Brom zu vernichten, trennt die org. Phase ab, wäscht diese mit 500 ml Wasser und 300 ml ges.

Natriumhydogencarbonat-Lösung und trocknet über Masgnesiumsulfat. Man filtriert von Trockenmittel ab, engt das Filtrat zur Trockene ein und kristallisiert den roten zähen Rückstand aus ca. 500 ml iso-Propanol um. Ausbeute: 32.7 g (69 mmol) 69 %. Reinheit n. $^{1}$H-NMR ca. 95 %.

Analog kann folgende Verbindung dargestellt werden:

| Bsp. | Edukte Bromid Keton / Elektrophil | Produkt | Ausbeute |
|---|---|---|---|
| S12 | S10 | 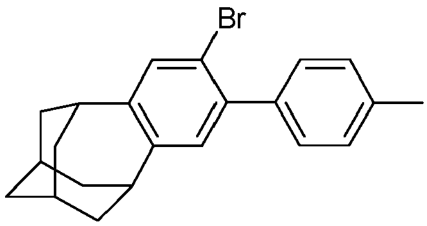 | 46 % |

**Beispiel S3:**

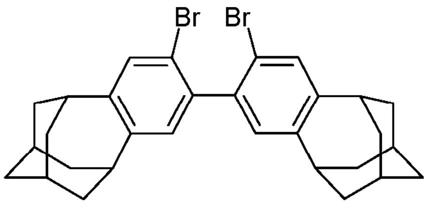

Darstellung analog S2, jedoch werden 6.4 ml (240 mmol) Brom eingesetzt. Außerdem wird der Lösung von S1 in Dichlormethan 100 mg Eisenpulver zugesetzt. Ausbeute: 33.7 g (61 mmol) 61 %. Reinheit n. $^{1}$H-NMR ca. 97 %.

Analog kann folgende Verbindung dargestellt werden:

| Bsp. | Edukte Bromid Keton / Elektrophil | Produkt | Ausbeute |
|---|---|---|---|
| S13 | S11 | 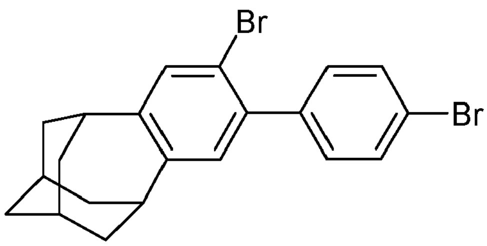 | 43% |

**Beispiel S4:**

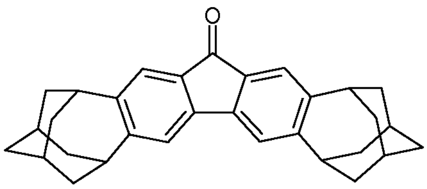

Eine auf -78 °C gekühlte, gut gerührte Lösung von 27.6 g (50 mmol) S3 in 500 ml THF wird tropfenweise mit 65.6 ml (105 mmol) n-BuLi in Hexan, 1.6 M versetzt und 30 min. nachgerührt. Dann tropft man ein Gemisch aus 5.1 ml (55 mmol) Dimethylcarbamoylchlorid [79-44-7] (Vorsicht: Giftig, Cancerogen) und 50 ml THF langsam zu, rührt 30 min. nach und lässt dann langsam auf Raumtemperatur erwärmen. Nach 2 h bei Raumtemperatur gibt man 200 ml ges. Ammoniumchlorid-Lösung zu, erweitert mit 300 ml Ethylacetat, trennt die wässrige Phase ab und engt die organische zur Trockene ein. Man nimmt den Rückstand in 250 ml Dichlormethan (DCM) auf, wäscht dieses dreimal mit 300 ml Wasser, einmal mit 300 ml ges. Kochsalz-Lösung und trocknet über Magnesiumsulfat. Man filtriert vom Trockenmittel ab, engt das Filtrat im Vakuum zur Trockene ein und kristallisiert den Rückstand aus Acetonitril um. Ausbeute: 18.1 g (43 mmol) 85 %. Reinheit n. $^{1}$H-NMR ca. 97 %.

**Beispiel S5:**

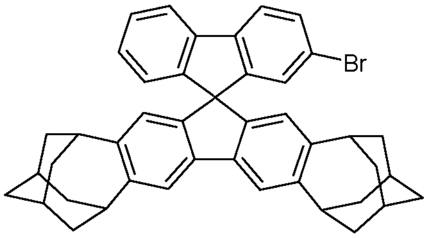

Eine auf -78 °C gekühlte, gut gerührte Lösung von 47.4 g (100 mmol) S2 in 500 ml THF wird tropfenweise mit 65.6 ml (105 mmol) n-BuLi in Hexan, 1.6 M versetzt und 3 h nachgerührt. Dann tropft man eine Lösung von 27.2 g (105 mmol) 2-Brom-9-fluorenon [3096-56-3] in 300 ml THF langsam zu, rührt 30 min. nach und lässt dann langsam auf Raumtemperatur erwärmen. Nach 2 h bei Raumtemperatur entfernt man das THF im Vakuum, nimmt den Rückstand in 500 ml Eisessig auf, gibt 30 ml konz. Salzsäure zu und erhitzt 3 h unter Rückfluss. Man lässt auf 80 °C abkühlen, tropft langsam 500 ml Wasser zu, saugt noch warm vom ausgefallenen Produkt ab, wäscht diese mit 100 ml Wasser und dann dreimal mit je 100 ml Methanol und trocknet im Vakuum. Ausbeute: 56.6 g (89 mmol) 89 %. Reinheit n. $^{1}$H-NMR ca. 97 %.

Analog können folgende Verbindungen dargestellt werden.

| Bsp. | Edukte Bromid Keton / Elektrophil | Produkt | Ausbeute |
|---|---|---|---|
| S6 | S2 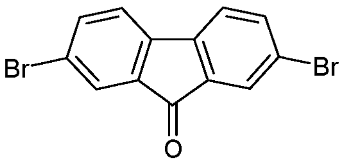 [14348-75-5] | 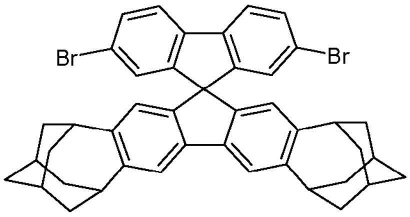 | 86 % |
| S7 | 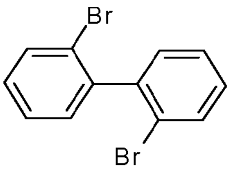 [13029-09-9] S4 | 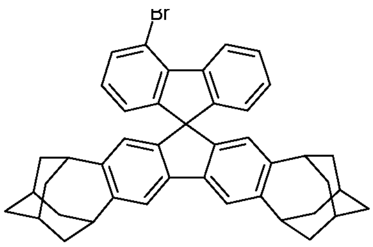 | 79 % |
| S8 | S3 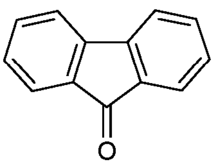 [486-25-9] | 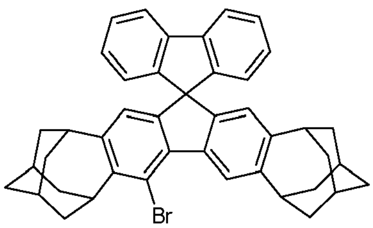 | 83 % |
| S9 | S3 S4 | 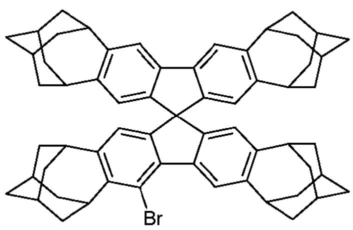 | 80 % |

(fortgesetzt)

| Bsp. | Edukte Bromid Keton / Elektrophil | Produkt | Ausbeute |
|---|---|---|---|
| S15 | S12 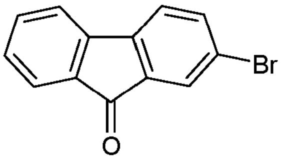 [3096-56-3] | 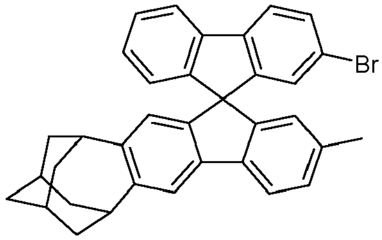 | 78 % |

**Beispiel S20:**

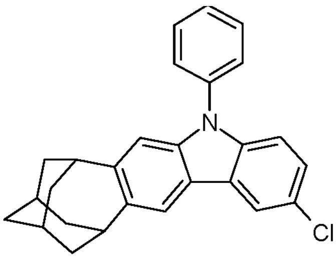

Ein gut gerührtes Gemisch aus 28.3 g (100 mmol) (2-Brom-4-chlorphenyl)phenylamin [2149611-39-0], 32.4 g (100 mmol) 2-(6,7,8,9,10,11-Hexahydro-5,9:7,11-dimethano-5H-benzocyclononen-2-yl)-4,4,5,5-tetramethyl-1,3,2-dioxaborolan [1801624-63-4], 63.7 g (300 mmol) Trikaliumphosphat, 1.83 g (6 mmol) Tri-o-tolylphosphin, 225 mg (1 mmol) Palladium(II)acetat, 350 ml Toluol, 60 ml Dioxan und 300 ml Wasser wird 16 h unter Rückfluss erhitzt. Nach Erkalten trennt man von der wässrigen Phase ab, wäscht die organische Phase einmal mit 300 ml Wasser, einmal mit 300 ml ges. Kochsalzlösung und trocknet dann über Magnesiumsulfat. Man filtriert vom Trockenmittel über ein mit Toluol vorgeschlämmtes Kieselgel-Bett ab und engt das Filtrat zur Trockne ein. Der Rückstand wird aus Acetonitril unter Zusatz von etwas Ethylacetat umkristallisiert. Das so erhaltene sec. Amin wird in 300 ml DMF gelöst, mit 45.4 g (250 mmol) Kupfer(II) acetat und 2.24 g (10 mmol) Palladium(II)acetat versetzt und 4 h bei 140 °C gerührt. Man entfernt das DMF weitgehend im Vakuum, nimmt den Rückstand in 500 ml DCM auf, versetzt mit 300 ml konz. Ammoniak-Lösung, rührt 1 h bei Raumtemperatur, trennt die org. Phase ab, wäscht diese dreimal mit 100 ml konz. Ammoniak-Lösung, einmal mit ges. Kochsalz-Lösung und trocknet über Magnesiumsulfat. Man filtriert vom Magnesiumsulfat über ein mit DCM vorgeschlämmtes Kieselgel-Bett ab, engt das Filtrat zur Trockene ein und kristallisiert den Rückstand aus Acetonitril/Ethylacetat um. Ausbeute: 18.7 g (47 mmol) 47 %. Reinheit n. $^{1}$H-NMR ca. 95 %.

**Beispiel S25:**

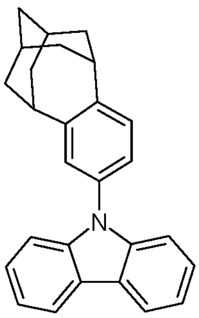

Ein gut gerührtes Gemisch aus 27.2 g (100 mmol) 2-Brom-6,7,8,9,10,11-hexahydro-5,9:7,11-dimethano-5H-benzocyclononen [1801624-97-4], 18.4 g (110 mmol) Carbazol [86-74-8], 41.5 g (300 mmol) Kaliumcarbonat, 1.9 g (10

mmol) Kupfer(I)iodid [7681-65-4], 100 g Glaskugeln (3 mm Durchmesser) und 300 ml Dimethylacetamid wird 30 h unter Rückfluss erhitzt. Man saugt noch warm von den Salzen über ein mit Dimethylacetamid vorgeschlämmtes Celite-Bett ab, engt das Filtrat zur Trockene ein, nimmt den Rückstand in 300 ml DCM auf, filtiriert über eine Kieselgelsäule (10 x 30 cm) und schneidet die Kernfraktion heraus. Das Eluat wird im Vakuum vom DCM befreit, der Rückstand wird aus Acetonitril umkristallisiert. Ausbeute: 33.4 g (88 mmol) 88 %. Reinheit n. $^{1}$H-NMR ca. 99 %.

**Beispiel S26:**

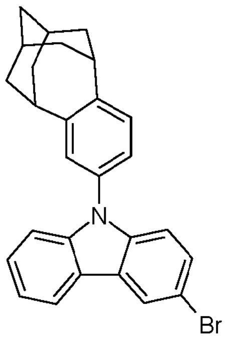

Eine gut gerührte, auf 0 °C gekühlte Lösung von 38.0 g (100 mmol) S25 in 500 ml DCM wird unter Lichtausschluss tropfenweise mit einer Lösung von 17.8 g (100 mmol) N-Bromsuccinimid in 300 ml Dichlormethan versetzt und dann 12 h bei Raumtemperatur nachgerührt. Man wäscht die Reaktionslösung einmal mit 200 ml ges. Natriumhydrogencarbonat-Lösung, dreimal mit je 200 ml Wasser, einmal mit 200 ml ges. Kochsalz-Lösung und trocknet dann über Magnesiumsulfat. Man filtriert vom Trockenmittel ab, engt das Filtrat ein und flash-chromatographiert (Combi-Flash Torrent der Fa. A. Semrau) den Rückstand. Ausbeute: 33.0 g (72 mmol) 72 %. Reinheit n. $^{1}$H-NMR ca. 95 %.

**Beispiel S30:**

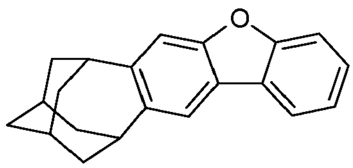

Ein gut gerührtes Gemisch aus 17.3 g (100 mmol) 2-Bromphenol [95-96-7], 32.4 g (100 mmol) 2-(6,7,8,9,10,11-Hexahydro-5,9:7,11-dimethano-5H-benzocyclononen-2-yl)-4,4,5,5-tetramethyl-1,3,2-dioxaborolan [1801624-63-4], 63.7 g (300 mmol) Trikaliumphosphat, 1.83 g (6 mmol) Tri-o-tolylphosphin, 225 mg (1 mmol) Palladium(II)acetat, 350 ml Toluol, 60 ml Ethanol und 300 ml Wasser wird 16 h unter Rückfluss erhitzt. Nach Erkalten fügt man 60 ml 10N wässrige HCl zu, trennt von der wässrigen Phase ab, wäscht die organische Phase einmal mit 300 ml Wasser, einmal mit 300 ml ges. Kochsalzlösung und trocknet dann über Magnesiumsulfat. Man filtriert vom Trockenmittel über ein mit Toluol vorgeschlämmtes Kieselgel-Bett ab und engt das Filtrat zur Trockne ein. Der Rückstand wird aus iso-Propanol unter Zusatz von etwas Ethylacetat umkristallisiert. Das so erhaltene Phenol wird in 800 ml Mesitylen gelöst, die gut gerührte Lösung wird mit 27.6 g (200 mmol) Kaliumcarbonat, 100 g Molsieb 3A, 9.3 g (50 mmol) Natrium-2,4,6-trimethylbenzoat [32642-28-7], 1.82 g (10 mmol) 4,5-Diazafluoren-9-on [50890-67-0], 4.26 g (10 mmol) 1,3-Bis[2, 6-bis(1-methylethyl)phenyl]-1H-imidazoliumchlorid [250285-32-6] und 1.12 g (5 mmol) Palladium(II)acetat versetzt und dann 16 h unter Einleiten eines schwachen Luftstroms auf 120 °C erhitzt. Man saugt noch warm über ein mit Mesitylen vorgeschlämmtes Kieselgelbett ab, entfernt das Mesitylen im Vakuum und kristallisiert den Rückstand aus Acetonitril um. Ausbeute: 15.6 g (54 mmol) 54 %. Reinheit n. $^{1}$H-NMR ca. 95 %.

**Beispiel S31:**

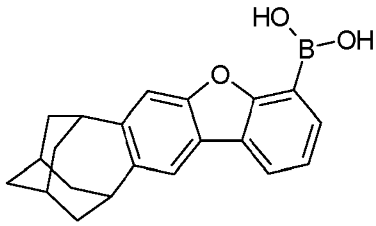

Ein gut gerührtes, auf -78 °C gekühltes Gemisch aus 33.2 g (100 mmol) S30 und 500 ml THF wird tropfenweise mit 65.6 ml (105 mmol) n-BuLi in Hexan, 1.6 M versetzt, 60 min. bei -78 °C und dann 30 min. bei -40°C nachgerührt. Nach erneuten Abkülen auf -78 °C gibt man unter gutem Rühren zügig ein Gemisch von 20.7 g (110 mmol) Tri-iso-propylborat [5419-55-6] und 50 ml THF zu und rührt 30 min. nach. Man lässt die Reaktionsmischung auf Raumtemperatur erwärmen, fügt 100 ml ges. Ammoniumchlorid-Lösung zu, rührt 15 min. nach, trennt die org. Phase ab, erweitert diese mit 500 ml Ethylacetat, wächt dreimal mit je 300 ml Wasser und einmal mit 200 ml ges. Kochsalz-Lösung. Man engt die org. Phase im Vakuum zur Trockene ein und kristallisiert den Rückstand aus Acetonitril unter Zusatz von etwas Wasser um. Ausbeute: 18.9 g ( 57 mmol) 57 %. Reinheit n. $^{1}$H-NMR ca. 95 %.

**Beispiel S35:**

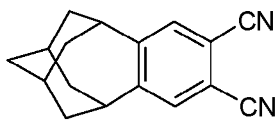

Ein gut gerührtes Gemisch aus 35.6 g (100 mmol) 2,3-Dibrom-6,7,8,9,10, 11-hexahydro-5,9:7,11-dimethano-5H-benzocyclononen [1801624-66-7], 26.9 g (300 mmol) Kupfer(I)cyanid, 50 g Glaskugeln (3 mm Durchmesser) und 300 NMP wird 18 h auf 170 °C erhitzt. Man saugt noch warm über ein mit NMP vorgeschlämmtes Celite-Bett ab, engt das Filtrat im Vakuum zur Trockene ein und rührt den Rückstand in 300 ml siedendem MeOH aus. Das Rohprodukt wird zweimal mit Acetonitril heißextrahiert. Ausbeute: 15.0 g (60 mmol) 60 %. Reinheit n. $^{1}$H-NMR ca. 95 %.

**Beispiel S36:**

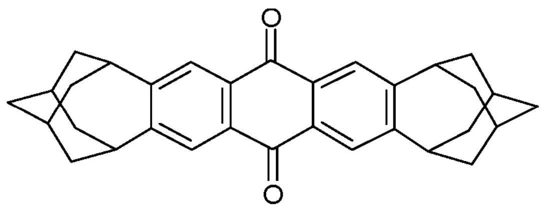

Eine gut gerührte auf -100 °C gekühlte Lösung von 35.6 g (100 mmol) 2,3-Dibrom-6,7,8,9,10,11-hexahydro-5,9:7,11-dimethano-5H-benzocyclononen [1801624-66-7] in 1000 ml THF wird tropfenweise mit 235 ml (400 mmol) t-BuLi, 1.7 M in Pentan versetzt und 30 min nachgerührt. Dann tropft man eine Lösung von 24.8 g (100 mmol) S35 in 300 ml THF langsam zu, rührt 1 h nach lässt auf Raumtemperatur erwärmen und quenscht durch Zugabe von 50 ml Methanol. Man entfernt das THF im Vakuum, nimmt den Rückstand in 300 ml NMP auf, versetzt mit 30 ml konz. wässriger Salzsäure und erhitzt 4 h auf ca. 150 °C. Nach Erkalten erweitert man mit 500 ml Ethylacetat, wäscht die org. Phase dreimal mit je 500 ml Wasser, einmal mit 300 ml ges. Kochsalz-Lösung und trocknet über Magnesiumsulfat. Man filtriert vom Trockenmittel ab, engt das Filtrat im Vakuum zur Trochene ein und kristallisiert den gelben Rückstand zweimal aus Acetonitril um. Ausbeute: 19.4 g (43 mmol) 43 %. Reinheit n. $^{1}$H-NMR ca. 95 %.

Analog können folgende Verbindungen dargestellt werden.

| Bsp. | Edukte | Produkt | Ausbeute |
|---|---|---|---|
| S37 | S35 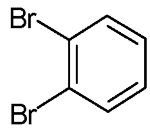 [583-53-9] | 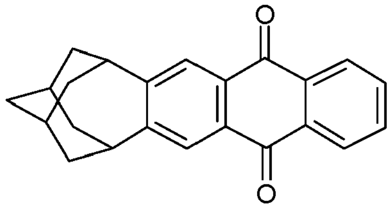 | 47 % |

**Beispiel S38:**

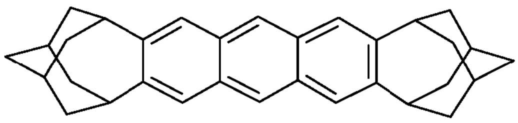

Eine Suspension von 44.9 g (100 mmol) S36 in 500 ml Eisessig wird mit 100 ml wässriger Jodwasserstoffsäure (57 Gew.-%) und 200 ml wässriger Hypophosphoriger Säure (50 Gew.-%) versetzt und 18 h unter Rückfluss erhitzt. Man saugt vom ausgefallenen Feststoff ab, wäscht diesen mit fünfmal mit je 300 ml heißem Wasser aus, rührt dann mit 300 ml heißem Ethanol aus, saugt ab, wäscht mit 300 ml heißem Ethanol nach und trocknet im Vakuum. Ausbeute: 38.6 g (92 mmol) 92 %. Reinheit n. $^1$H-NMR ca. 95 %.

Analog kann folgende Verbindungen dargestellt werden.

| Bsp. | Edukte | Produkt | Ausbeute |
|---|---|---|---|
| S39 | S37 | 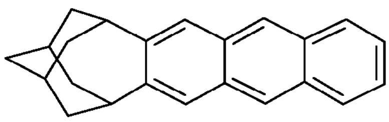 | 72 % |

**Beispiel S40:**

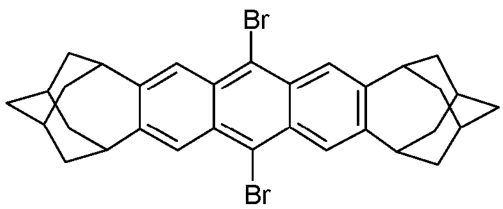

Ein Lösung von 41.9 g (100 mmol) S38 in 500 ml Dichlormethan wird unter gutem Rühren und Lichtausschluss portionsweise mit 19.6 g (110 mmol) N-Bromsuccinimid versetzt und 6 h bei Raumtemperatur gerührt. Man wäscht die Reaktionsmischung einmal mit 300 ml ges. Natriumhydrogencarbonat-Lösung, dreimal mit je 300 ml Wasser, einmal mit 300 ml ges. Kochsalz-lösung und trocknet über Magnesiumsulfat. Man filtriert vom Trockenmittel ab, engt das Filtrat zur Tockene ein und kristallisiert aus Acetonitril / Ethylacetat um. Ausbeute: 50.2 g (87 mmol) 87 %. Reinheit n. $^1$H-NMR ca. 98 %.

Analog kann folgende Verbindungen dargestellt werden.

| Bsp. | Edukte | Produkt | Ausbeute |
|---|---|---|---|
| S41 | S37 | 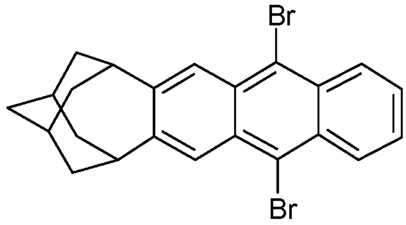 | 84 % |

**2) Synthese der Amine A:**

**Beispiel A1**

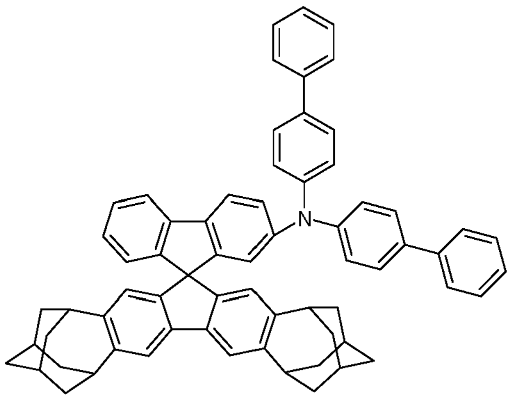

Eine Lösung von 63.6 g (100 mmol) S5 und 38.6 g (120 mmol) Bis-p-biphenylamin [102113-98-4] in 500 ml Toluol wird mit 4.0 ml (4.0 mmol) einer 1.0 M Tri-tert-butylphosphin Lösung in Toluol, 449 mg (2 mmol) Palladiumacetat und 16.0 g Natrium-tert-butanolat (166 mmol) versetzt und 3 h unter Rückfluss erhitzt. Das Reaktionsgemisch wird auf Raumtemperatur abgekühlt, mit Toluol erweitert und über Celite-Bett filtriert. Das Filtrat wird im Vakuum eingeengt und der Rückstand aus Essigsäureethylester/n-Heptan kristallisiert. Das Rohprodukt wird dreimal

mit Acetonitril heißextrahiert und durch zweimalige Zonensublimation im Vakuum (p ~ $10^{-5}$ mbar, T ~ 310 °C) gereinigt. Ausbeute: 63.1 g ( 72 mmol) 72 %. Reinheit n. HPLC >99.9 %.

Analog können folgende Verbindungen dargestellt werden.

| Bsp. | Edukte | Produkt | Ausbeute |
|---|---|---|---|
| A2 | S5 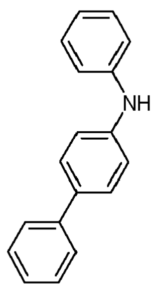 [32228-99-2] | 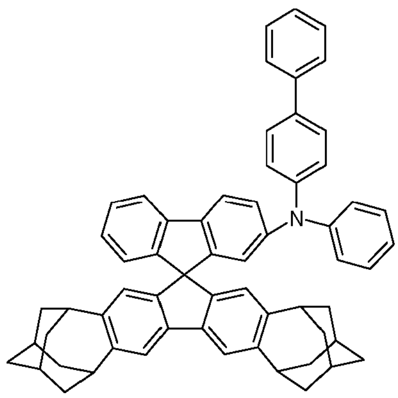 | 70 % |

(fortgesetzt)

| Bsp. | Edukte | Produkt | Ausbeute |
|---|---|---|---|
| A3 | S5 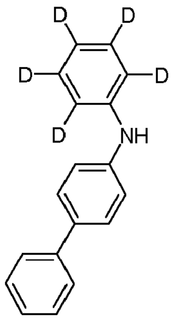 [1322090-81-2] | 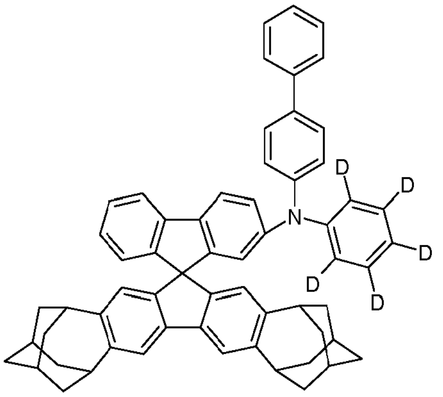 | 68 % |
| A4 | S5 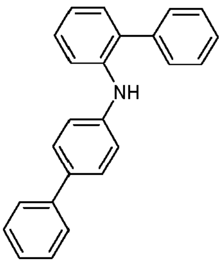 [1372775-52-4] | 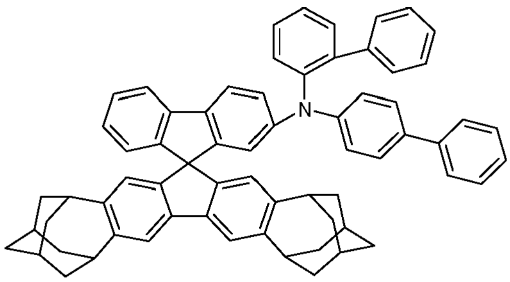 | 75 % |
| A5 | S5 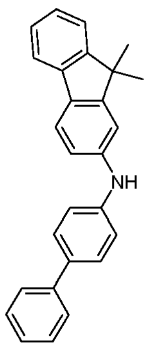 [897671-69-1] | 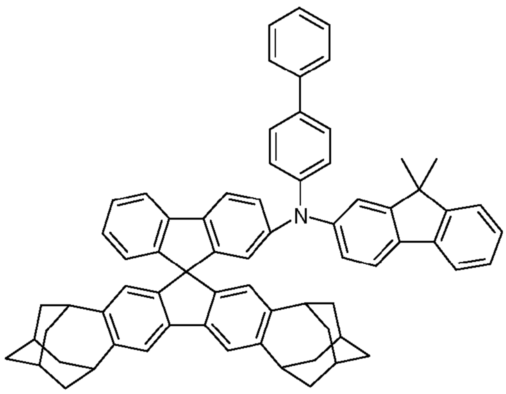 | 73 % |
| A6 | S5 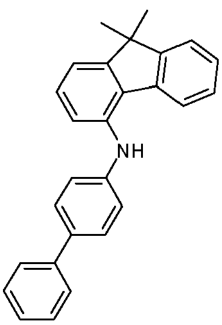 [1421789-16-3] | 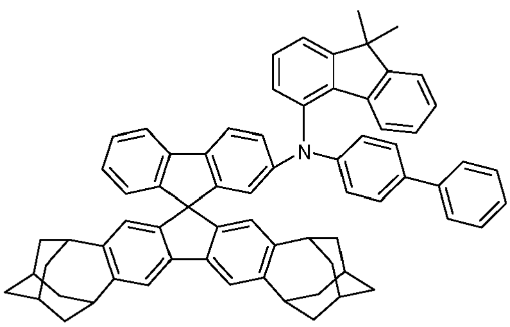 | 65 % |

(fortgesetzt)

| Bsp. | Edukte | Produkt | Ausbeute |
|---|---|---|---|
| A7 | S5 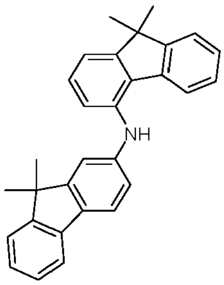 [1644054-07-8] | 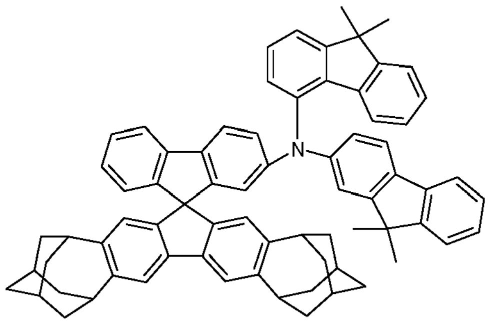 | 69 % |
| A8 | S5 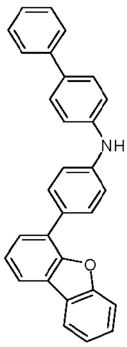 [955959-89-4] | 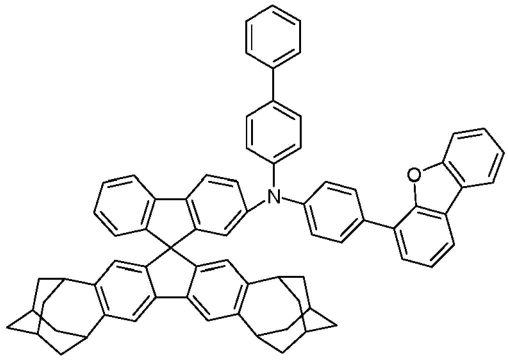 | 74 % |
| A9 | S5 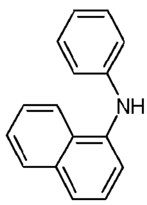 [90-30-2] | 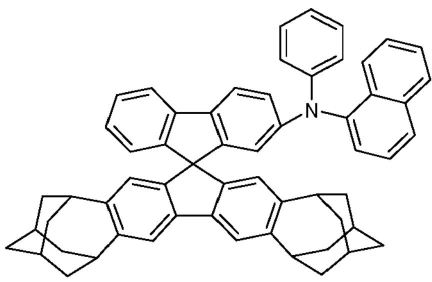 | 74 % |
| A10 | S5 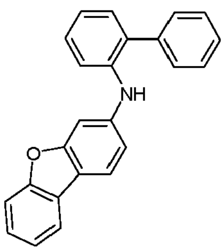 [1427556-44-2] | 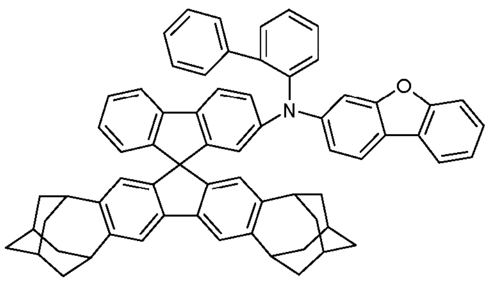 | 70 % |

(fortgesetzt)

| Bsp. | Edukte | Produkt | Ausbeute |
|---|---|---|---|
| A11 | S6 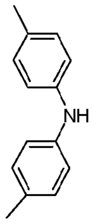 [620-93-9] | 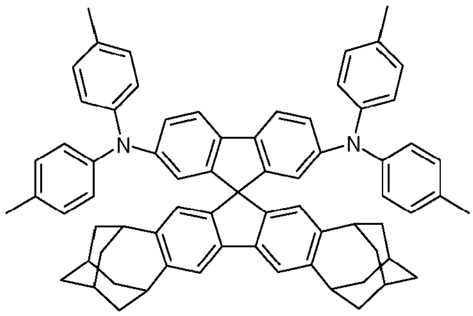 | 66 % |
| A12 | S6 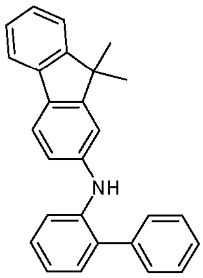 [1198395-24-2] | 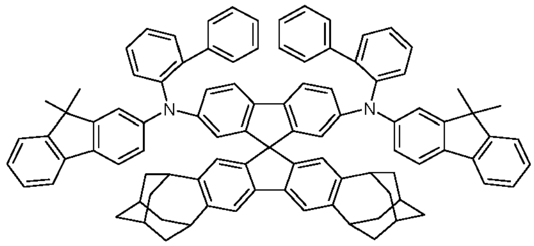 | 61 % |
| A13 | S7 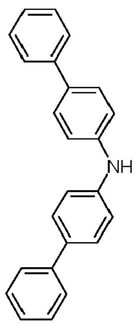 | 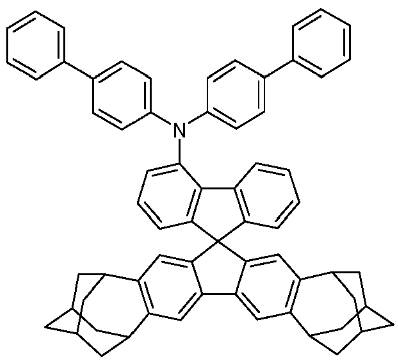 | 78 % |
| | [102113-98-4] | | |
| A14 | S7 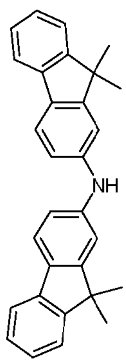 [500717-23-7] | 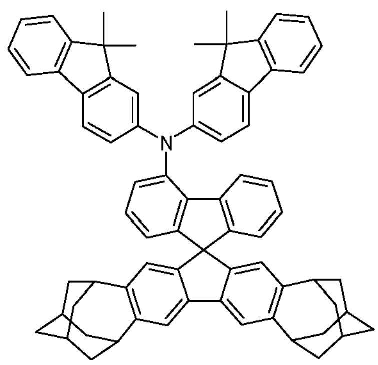 | 75 % |

(fortgesetzt)

| Bsp. | Edukte | Produkt | Ausbeute |
|---|---|---|---|
| A15 | S7 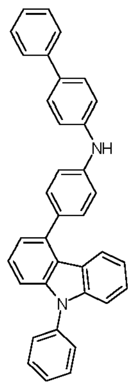 [1629995-09-0] | 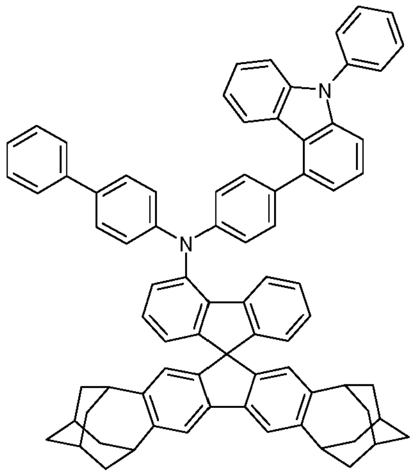 | 71 % |
| A16 | S7 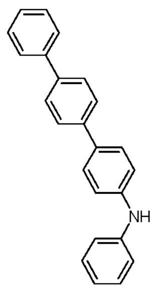 [897671-81-7] | 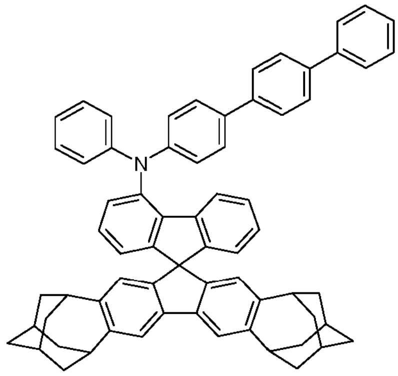 | 72 % |
| A17 | S7 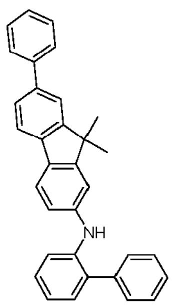 [1372775-93-9] | 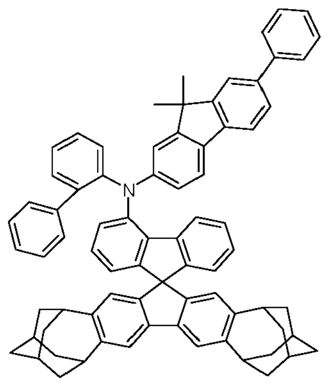 | 70 % |

(fortgesetzt)

| Bsp. | Edukte | Produkt | Ausbeute |
|---|---|---|---|
| A18 | S7 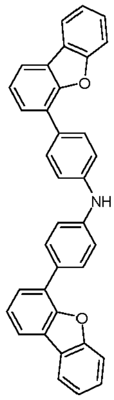 [955959-91-8] | 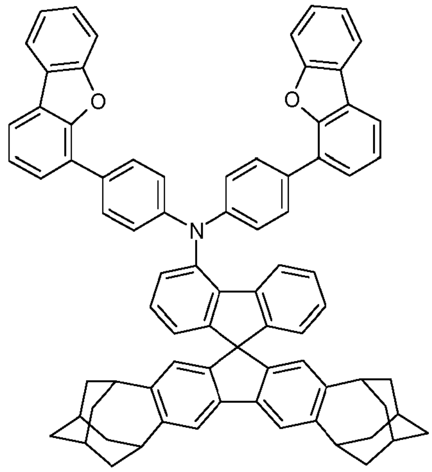 | 76 % |
| A19 | S8 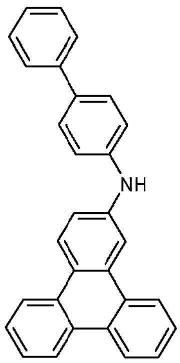 [1314527-06-4] | 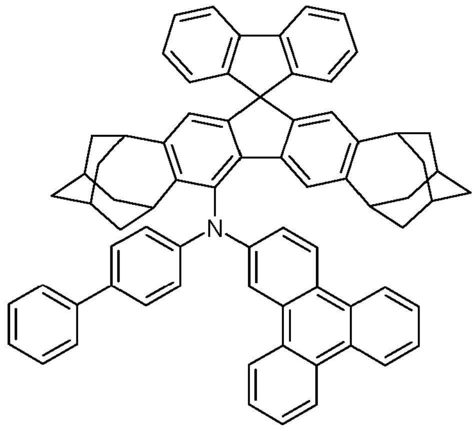 | 44 % |
| A20 | S8 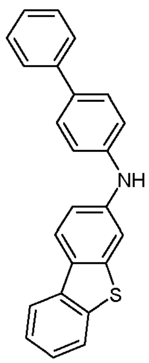 [1290037-87-0] | 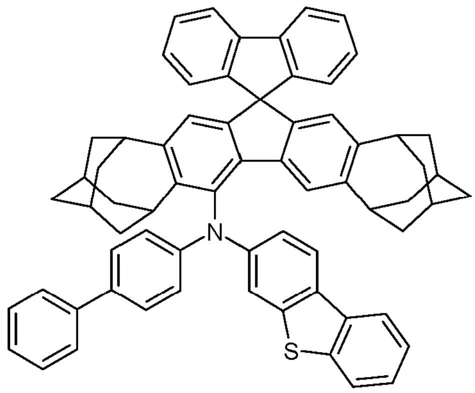 | 41 % |

(fortgesetzt)

| Bsp. | Edukte | Produkt | Ausbeute |
|---|---|---|---|
| A21 | S9 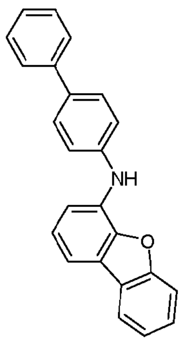 [1318338-47-4] | 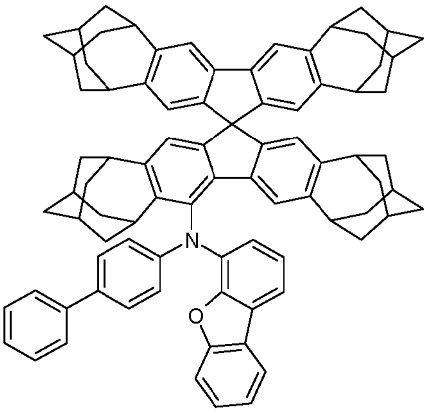 | 48 % |
| A30 | S15 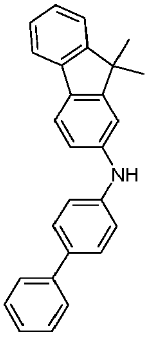 [897671-69-1] | 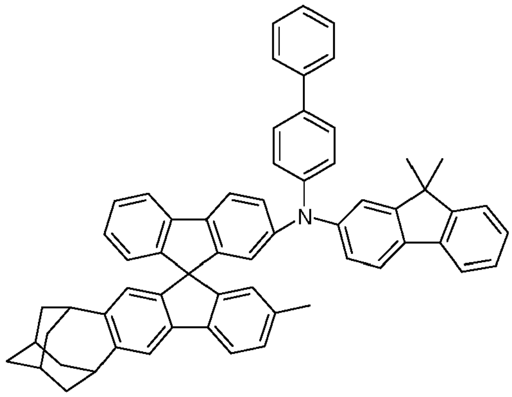 | 67 % |
| A31 | S15 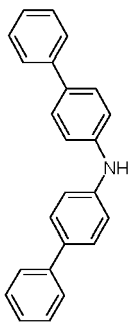 [102113-98-4] | 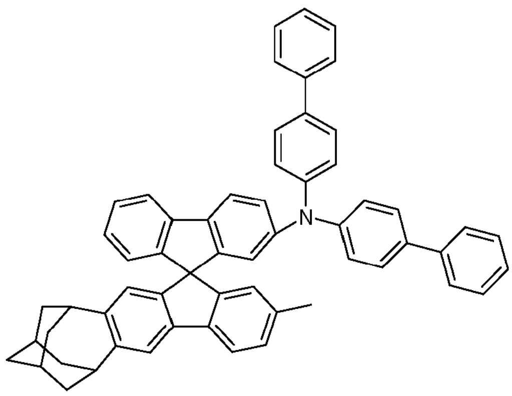 | 65 % |
| A40 | S13 (50 mmol) 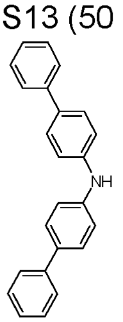 [102113-98-4] | 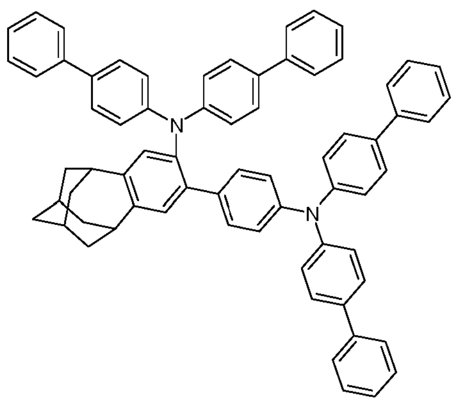 | 56 % |

**Beispiel A22:**

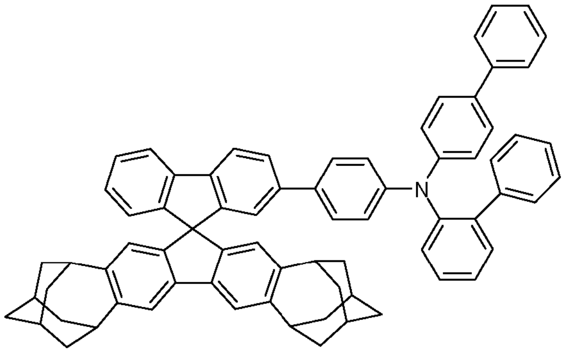

Ein gut gerührtes Gemisch aus 63.6 g (100 mmol) S5, 57.6 g (110 mmol) N-[1,1'-Biphenyl]-2-yl-N-[4-(4,4,5,5-tetramethyl-1,3,2-dioxaborolan-2-yl) phenyl]- [1,1'-biphenyl]-4-amine [1608462-54-9], 63.7 g (300 mmol) Trikaliumphosphat, 1.83 g (6 mmol) Tri-o-tolyl-phosphin, 225 mg (1 mmol) Palladium(II)acetat, 500 ml Toluol, 100 ml Dioxan und 400 ml Wasser wird 16 h unter Rückfluss erhitzt. Nach Erkalten trennt man von der wässrigen Phase ab, wäscht die organische Phase einmal mit 300 ml Wasser, einmal mit 300 ml ges. Kochsalzlösung und trocknet dann über Magnesiumsulfat. Man filtriert vom Trockenmittel über ein mit Toluol vorgeschlämmtes Kieselgel-Bett ab und engt das Filtrat zur Trockne ein. Der glasartige Rückstand wird aus Ethylacetat / iso-Propanol umkristallisiert. Das Rohprodukt wird dreimal mit Toluol heißextrahiert und durch zweimalige Zonensublimation im Vakuum (p ~ $10^{-5}$ mbar, T ~ 330 °C) gereinigt. Ausbeute: 66.8 g ( 70 mmol) 70 %. Reinheit n. HPLC >99.9 %.

Analog können folgende Verbindungen dargestellt werden.

| Bsp. | Edukte | Produkt | Ausbeute |
|---|---|---|---|
| A23 | S5 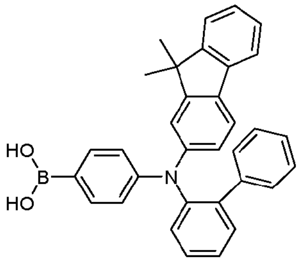 [1959599-90-6] | 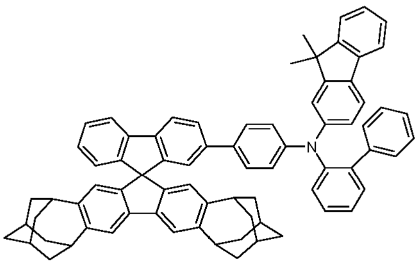 | 68 % |
| A24 | S5 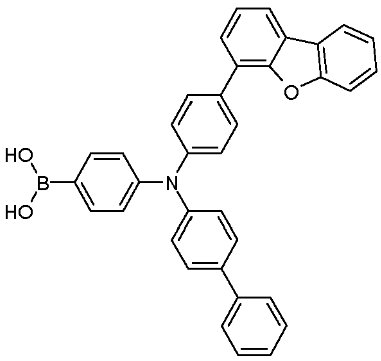 [1609381-12-5] | 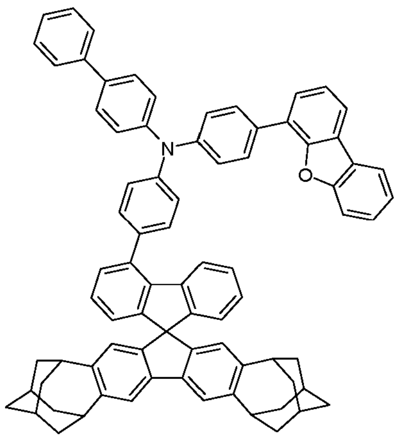 | 71% |

(fortgesetzt)

| Bsp. | Edukte | Produkt | Ausbeute |
|---|---|---|---|
| A25 | S5 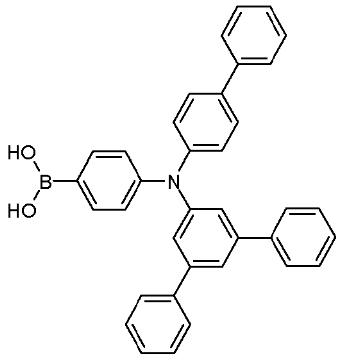 [1942032-55-4] | 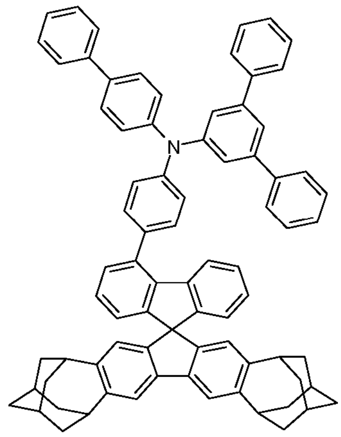 | 75 % |
| A26 | S8 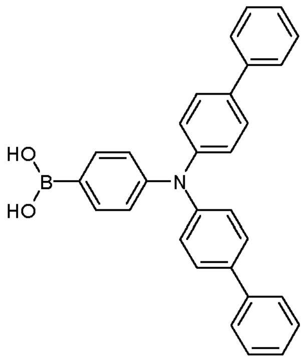 [943836-24-6] | 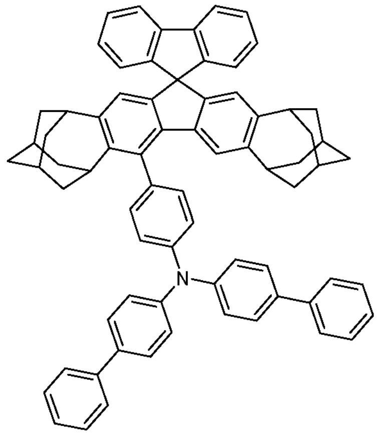 | 73 % |
| A27 | S15 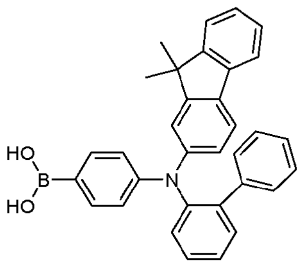 [1959599-90-6] | 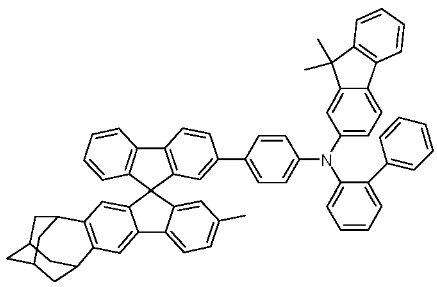 | 69 % |
| A28 | S15 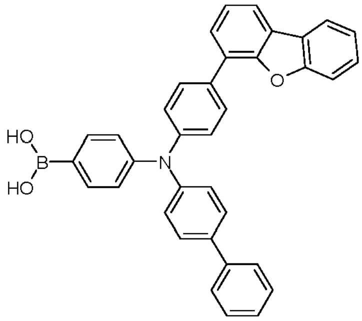 [1609381-12-5] | 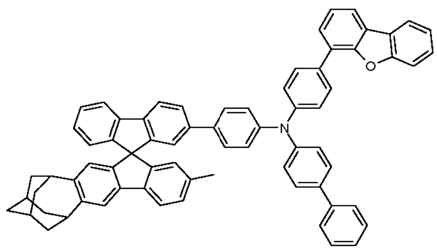 | 73 % |

**3) Synthese der Carbazole C:**

**Beispiel C1:**

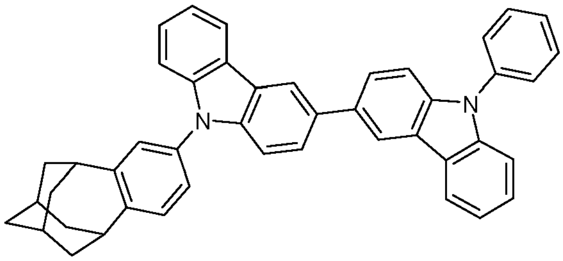

Ein gut gerührtes Gemisch aus 44.2 g (100 mmol) S26, 36.9 g (100 mmol) 9-Phenyl-3-(4,4,5,5-tetramethyl-1,3,2-dioxaborolan-2-yl)- 9H-carbazole [1126522-69-7], 63.7 g (300 mmol) Trikaliumphosphat, 1.83 g (6 mmol) Tri-o-tolylphosphin, 225 mg (1 mmol) Palladium(II)acetat, 350 ml Toluol, 80 ml Dioxan und 300 ml Wasser wird 16 h unter Rückfluss erhitzt. Nach Erkalten trennt man von der wässrigen Phase ab und engt die org. Phase zur Trockene ein. Man nimmt den Rückstand in 500 ml DCM auf, wäscht die organische Phase einmal mit 300 ml Wasser, einmal mit 300 ml ges. Kochsalzlösung und trocknet dann über Magnesiumsulfat. Man filtriert vom Trockenmittel über ein mit DCM vorgeschlämmtes Kieselgel-Bett ab und engt das Filtrat zur Trockne ein. Der Rückstand wird mit Butylacetat/iso-Propanol heiß ausgerührt, dann dreimal mit Toluol heißextrahiert und durch Zonensublimation im Vakuum (p ~ $10^{-5}$ mbar, T ~ 320 °C) gereinigt. Ausbeute: 40.0 g ( 66 mmol) 66 %. Reinheit n. HPLC >99.9 %.

Analog können folgende Verbindungen dargestellt werden.

| Bsp. | Edukte | Produkt | Ausbeute |
|---|---|---|---|
| C2 | S26 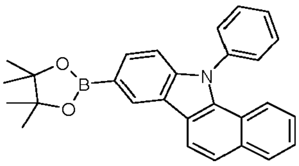 [1493715-55-1] | 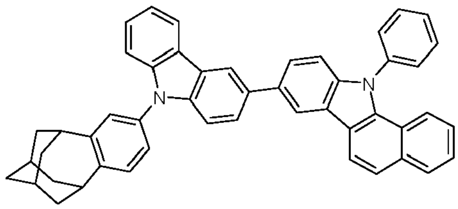 | 64 % |
| C3 | S26 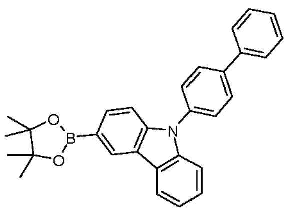 [1391729-66-0] | 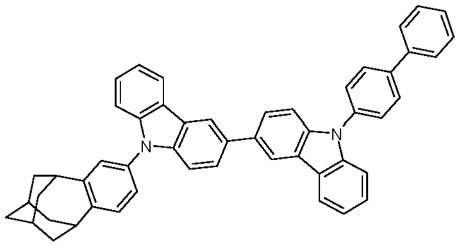 | 70 % |
| C4 | S26 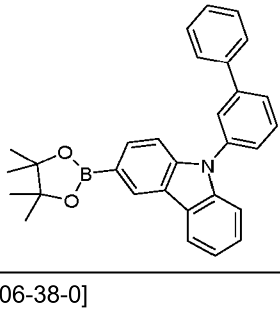 | 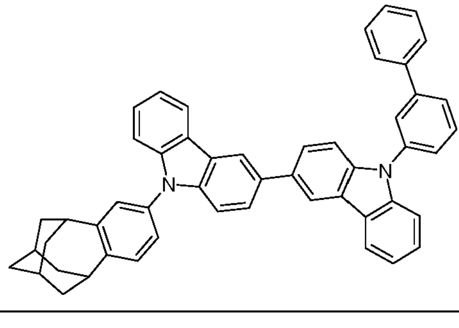 | 65 % |
| | [1533406-38-0] | | |

(fortgesetzt)

| Bsp. | Edukte | Produkt | Ausbeute |
|---|---|---|---|
| C5 | S26<br>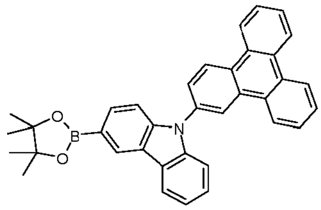<br>[1351870-14-8] | 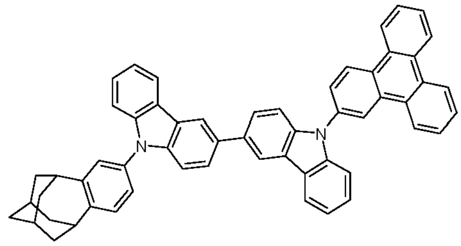 | 73 % |
| C6 | S26<br>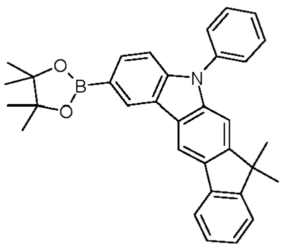<br>[1357150-79-8] | 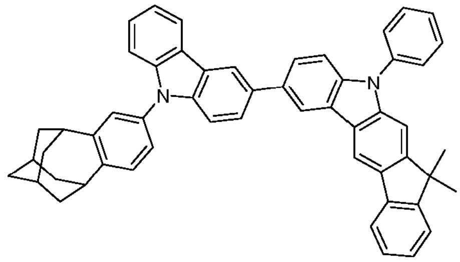 | 71 % |
| C7 | S26<br>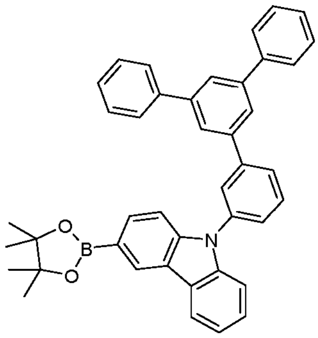<br>[1846559-20-3] | 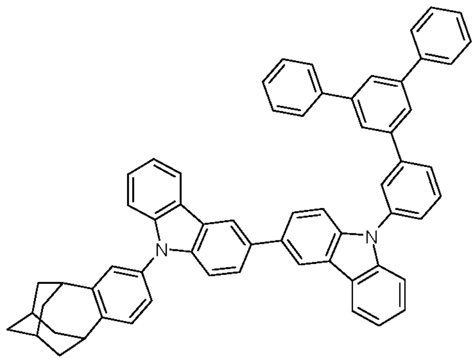 | 51 % |
| C8 | S20<br>Einsatz von 2 mmol S-Phos anstelle des Tri-o-tolylphosphins<br>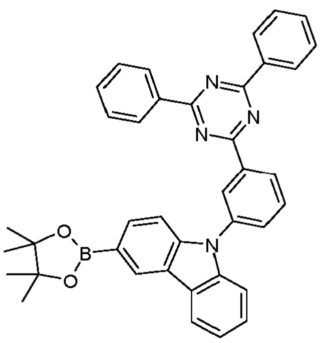<br>[1622875-90-4] | 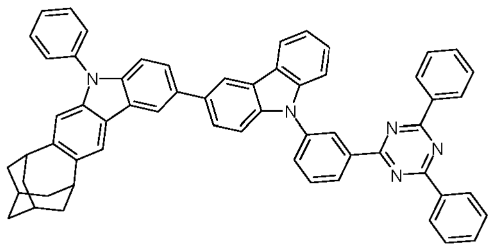 | 57 % |

(fortgesetzt)

| Bsp. | Edukte | Produkt | Ausbeute |
|---|---|---|---|
| C9 | S20<br>Einsatz von 2 mmol S-Phos anstelle des Tri-o-tolylphosphins<br>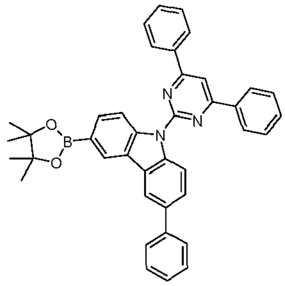<br>[1446005-91-9] | 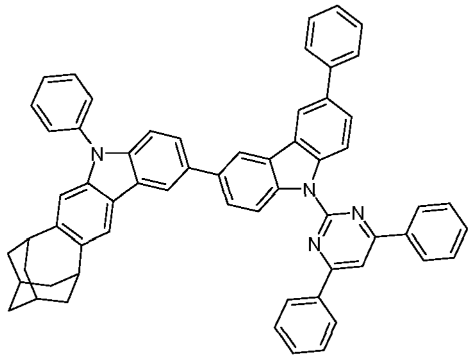 | 64 % |
| C10 | S20<br>Einsatz von 2 mmol S-Phos anstelle des Tri-o-tolylphosphins<br>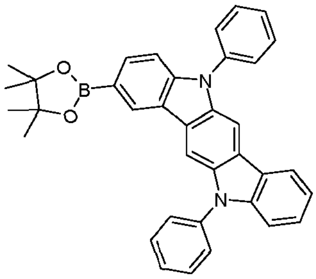<br>[1338068-90-8] | 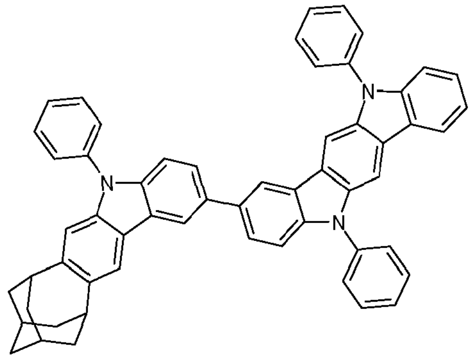 | 70 % |
| C11 | S20<br>Einsatz von 2 mmol S-Phos anstelle des Tri-o-tolylphosphins<br>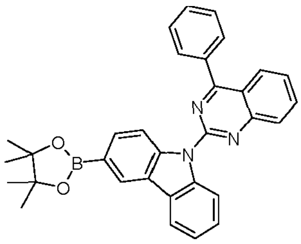<br>[1656982-71-6] | 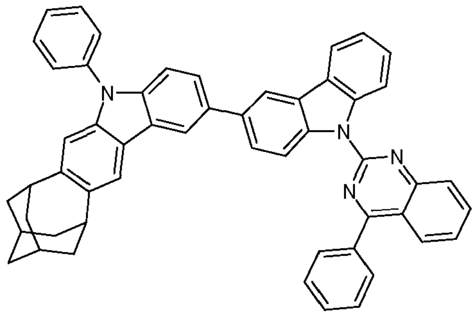 | 68 % |
| C12 | S20<br>Einsatz von 2 mmol S-Phos anstelle des Tri-o-tolylphosphins<br>S31 | 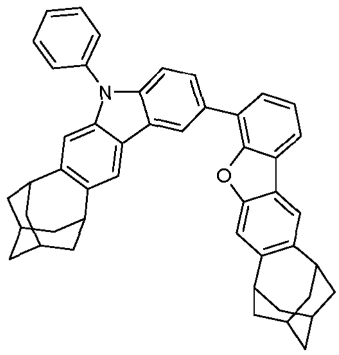 | 59 % |

**4) Synthese der Triazine T:**

**Beispiel T1:**

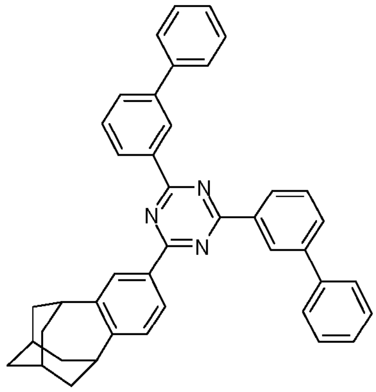

Ein gut gerührtes Gemisch aus 42.0 g (100 mmol) 2,4-Bis([1,1'-biphenyl]-3-yl)-6-chlor-1,3,5-triazin [1205748-61-3], 32.4 g (100 mmol) 2-(6,7,8,9,10, 11-Hexahydro-5,9:7,11-dimethano-5H-benzocyclononen-2-yl)-4,4,5,5-tetramethyl-1,3,2-dioxaborolan [1801624-63-4], 31.9 g (150 mmol) Trikaliumphosphat, 821 mg (2 mmol) S-Phos, 225 mg (1 mmol) Palladium(II)acetat, 400 ml Toluol, 80 ml Dioxan und 300 ml Wasser wird 16 h unter Rückfluss erhitzt. Nach Erkalten trennt man von der wässrigen Phase ab, wäscht die organische Phase einmal mit 300 ml Wasser, einmal mit 300 ml ges. Kochsalzlösung und trocknet dann über Magnesiumsulfat. Man filtriert vom Trockenmittel über ein mit Toluol vorgeschlämmtes Kieselgel-Bett ab und engt das Filtrat zur Trockne ein. Der Rückstand wird mit iso-Propanol heiß ausgerührt, dann fünfmal mit Acetonitril heißextrahiert und durch Zonensublimation im Vakuum (p ~ $10^{-5}$ mbar, T ~ 310 °C) gereinigt. Ausbeute: 37.8 g (65 mmol) 65 %. Reinheit n. HPLC >99.9 %.

Analog können folgende Verbindungen dargestellt werden.

| Bsp. | Edukte | Produkt | Ausbeute |
|---|---|---|---|
| T2 | S26 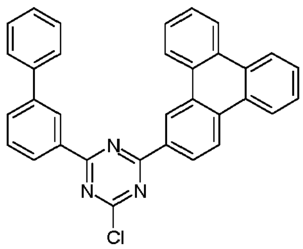 [2170382-97-3] | 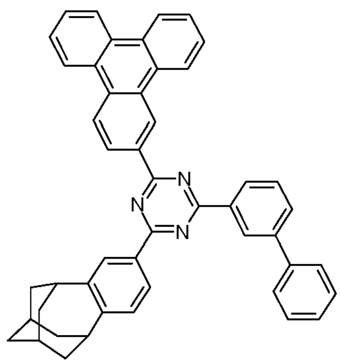 | 64 % |
| T3 | S26 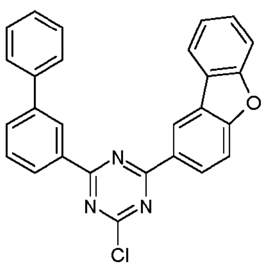 [1699739-82-6] | 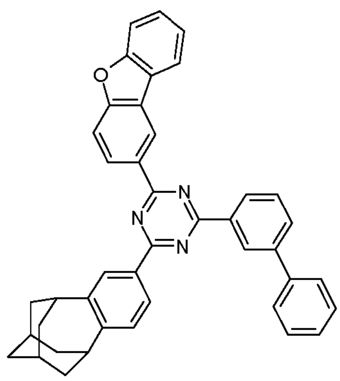 | 55 % |

(fortgesetzt)

| Bsp. | Edukte | Produkt | Ausbeute |
|---|---|---|---|
| T4 | S26 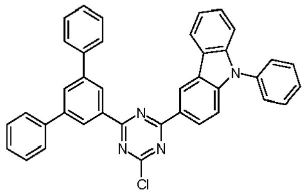 [2047632-07-6] | 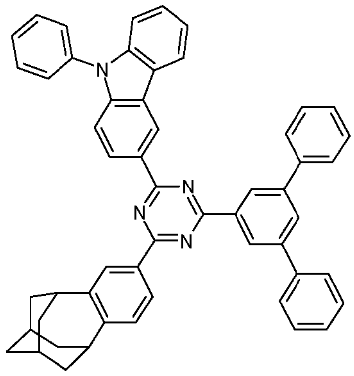 | 57 % |
| T5 | S26 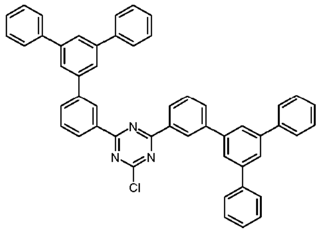 [1233200-61-7] | 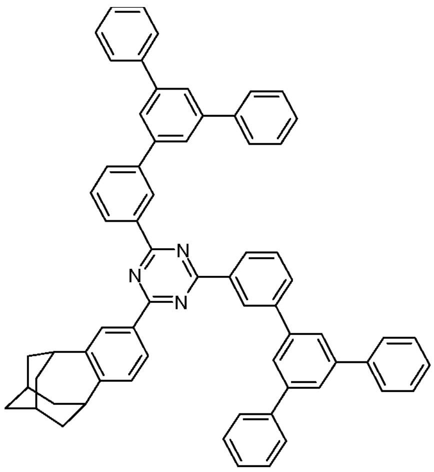 | 49 % |

**5) Synthese der Hostmaterialien für Singlet Emitter SH:**

**Beispiel SH1:**

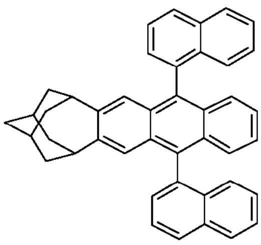

Ein gut gerührtes Gemisch aus 45.6 g (100 mmol) S41, 37.8 g (220 mmol) 1-Naphthylboronsäure [13922-41-3], 63.7 g (300 mmol) Trikaliumphosphat, 1.83 g (6 mmol) Tri-o-tolylphosphin, 225 mg (1 mmol) Palladium(II)acetat, 400 ml Toluol, 80 ml Dioxan und 300 ml Wasser wird 16 h unter Rückfluss erhitzt. Nach Erkalten trennt man von der wässrigen Phase ab und engt die org. Phase zur trockene ein. Man nimmt den Rückstand in 500 ml DCM auf, wäscht die organische Phase einmal mit 300 ml Wasser, einmal mit 300 ml ges. Kochsalzlösung und trocknet dann über Magnesiumsulfat. Man filtriert vom Trockenmittel über ein mit DCM vorgeschlämmtes Kieselgel-Bett ab und engt das Filtrat zur Trockne ein. Der Rückstand wird mit Butylacetat/iso-Propanol heiß ausgerührt, dann fünfmal mit Toluol heißextrahiert und durch Zonensublimation im Vakuum (p ~ $10^{-5}$ mbar, T ~ 320 °C) gereinigt. Ausbeute: 31.9 g (58 mmol) 58 %, Sny- / Anti-Isomerengemisch. Reinheit n. HPLC >99.9 %.

**6) Synthese der Singlet Emitter S:**

**Beispiel SE1:**

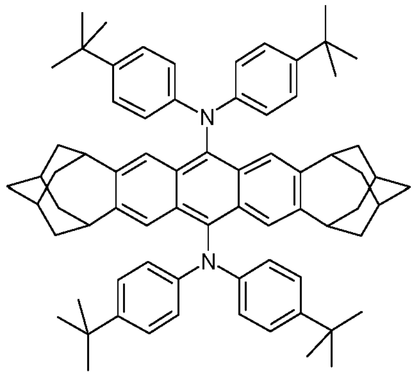

Durchführung analog zu A1, wobei anstelle von S5 28.8 g (50 mmol) S40 und 31.0 g (110 mmol) 4-(1,1-Dimethylethyl)-N-[4-(1,1-dimethylethyl) phenyl]-phenylamin [4627-22-9] eingestzt werden. Reinigung durch fünfmalige Heißextraktion mit Cyclohexan und Zonensublimation im Vakuum (p ~ $10^{-5}$ mbar, T ~ 330 °C). Ausbeute: 26.0 g (53 mmol) 53 %. Reinheit n. HPLC >99.9 %.

Analog kann folgende Verbindungen dargestellt werden.

| Bsp. | Edukte | Produkt | Ausbeute |
|---|---|---|---|
| SE2 | S40 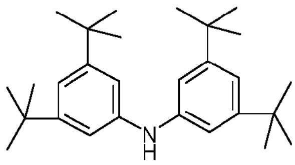 [37055-49-5] | 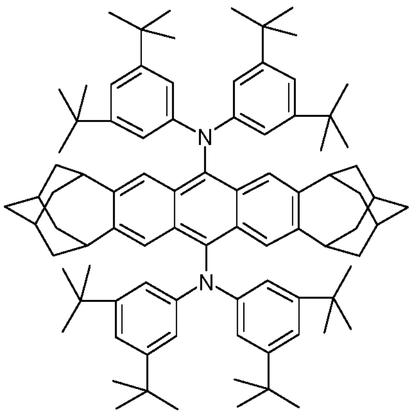 | 48 % |

**7) Synthese der Spiromaterialen H:**

Analog zu S5 können folgende Verbindungen dargestellt werden.

| Bsp. | Edukte Bromid Keton / Elektrophil | Produkt | Ausbeute |
|---|---|---|---|
| H1 | S2<br>S4 | 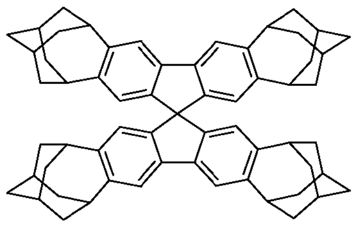 | 91 % |

(fortgesetzt)

| Bsp. | Edukte Bromid Keton / Elektrophil | Produkt | Ausbeute |
|---|---|---|---|
| H2 | S2<br>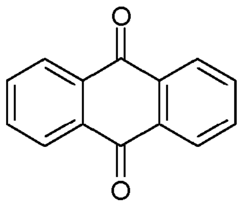<br>50 mmol [84-65-1] | 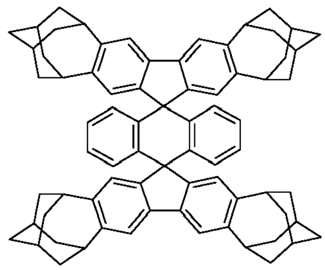 | 78 % |
| H3 | S3, 25 mmol<br>$SiCl_4$, 12 mmol [10026-04-7] | 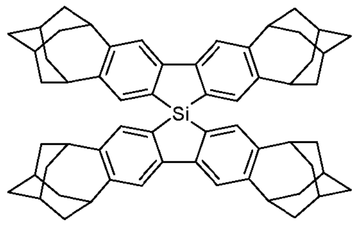 | 54 % |

**Herstellung der OLED-Devices**

**1) Vakuum-prozessierte Devices:**

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylenedioxythiophene) poly(styrenesulfonate), bezogen als CLEVIOS™ P VP Al 4083 von Heraeus Precious Metals GmbH Deutschland, aus wässriger Lösung aufgeschleudert) und anschließend bei 180°C 10 min. lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochinjektionsschicht 1 (HIL1) bestehend aus HTM1 dotiert mit 5 % NDP-9 (kommerziell erhältlich von der Fa. Novaled), 20 nm / Lochstransportschicht 1 (HTL1) / Lochtransportschicht 2 (HTL2) / Emissionsschicht (EML) / Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuum-prozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie TMM1:TMM2:Ir(L1) (55%:35%:10%) bedeutet hierbei, dass das Material TMM1 in einem Volumenanteil von 55%, TMM2 in einem Anteil von 35% und Ir(L1) in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 4 gezeigt.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Als Lebensdauer LD90 wird die Zeit definiert, nach der die Leuchtdichte bei Betrieb mit einer Starthelligkeit von 10000 cd/$m^2$ auf 90% der Startleuchtdichte abgesunken ist.

Die OLEDs können initital auch bei anderen Startleuchtdichten betrieben werden. Die Werte für die Lebensdauer können dann mit Hilfe dem Fachmann bekannten Umrechnungsformeln auf eine Angabe für andere Startleuchtdichten umgerechnet werden.

**Verwendung von erfindungsgemäßen Verbindungen als Materialien in phosphoreszierenden OLEDs**

Die erfindungsgemäßen Verbindungen lassen sich unter anderem als HTM (hole transport material), TMM (triplet matrix material), ETM (electron transport material) sowie als Emittermaterialien in der Emissionsschicht in OLEDs einsetzen. Als Vergleich gemäß dem Stand der Technik werden die Verbindungen gemäß Tabelle 4 verwendet. Die Ergebnisse der OLEDs sind in Tabelle 2 zusammengefasst.

**Tabelle 1: Aufbau der OLEDs**

| Bsp. | HTL1 Dicke | HTL2 Dicke | EML Dicke | HBL Dicke | ETL Dicke |
|---|---|---|---|---|---|
| Ref.D1 | HTM1 210 nm | HTM2 10 nm | TMM1:TMM2:Ir-Ref.1 (45%:40%:15%) 30 nm | ETM1 10nm | ETM1:ETM2 (50%:50%) 30 nm |
| Ref. D2 | HTM1 210 nm | HTM2 10 nm | TMM1:TMM2:Ir-Ref.2 (40%:50%:10%) 30 nm | ETM1 10nm | ETM1:ETM2 (50%:50%) 30 nm |
| D1 | HTM1 210 nm | A1 10 nm | TMM1:TMM2:Ir-Ref.1 (45%:40%:15%) 30 nm | ETM1 10nm | ETM1:ETM2 (50%:50%) 30 nm |
| D2 | HTM1 210 nm | A2 10 nm | TMM1:TMM2:Ir-Ref.1 (45%:40%:15%) 30 nm | ETM1 10nm | ETM1:ETM2 (50%:50%) 30 nm |
| D3 | HTM1 210 nm | A3 10 nm | TMM1:TMM2:Ir-Ref.1 (45%:40%:15%) 30 nm | ETM1 10nm | ETM1:ETM2 (50%:50%) 30 nm |
| D4 | HTM1 210 nm | A4 10 nm | TMM1:TMM2:Ir-Ref.1 (45%:40%:15%) 30 nm | ETM1 10nm | ETM1:ETM2 (50%:50%) 30 nm |
| D5 | HTM1 210 nm | A5 10 nm | TMM1:TMM2:Ir-Ref.1 (45%:40%:15%) 30 nm | ETM1 10nm | ETM1:ETM2 (50%:50%) 30 nm |
| D6 | HTM1 210 nm | A6 10 nm | TMM1:TMM2:Ir-Ref.1 (45%:40%:15%) 30 nm | ETM1 10nm | ETM1:ETM2 (50%:50%) 30 nm |
| D7 | HTM1 210 nm | A7 10 nm | TMM1:TMM2:Ir-Ref.1 (45%:40%:15%) 30 nm | ETM1 10nm | ETM1:ETM2 (50%:50%) 30 nm |
| D8 | HTM1 210 nm | A8 10 nm | TMM1:TMM2:Ir-Ref.1 (45%:40%:15%) 30 nm | ETM1 10nm | ETM1:ETM2 (50%:50%) 30 nm |
| D9 | HTM1 210 nm | A10 10 nm | TMM1:TMM2:Ir-Ref.1 (45%:40%:15%) 30 nm | ETM1 10nm | ETM1:ETM2 (50%:50%) 30 nm |
| D10 | HTM1 210 nm | A13 10 nm | TMM1:TMM2:Ir-Ref.1 (45%:40%:15%) 30 nm | ETM1 10nm | ETM1:ETM2 (50%:50%) 30 nm |
| D11 | HTM1 210 nm | A14 10 nm | TMM1:TMM2:Ir-Ref.1 (45%:40%:15%) 30 nm | ETM1 10nm | ETM1:ETM2 (50%:50%) 30 nm |
| D12 | HTM1 210 nm | A16 10 nm | TMM1:TMM2:Ir-Ref.1 (45%:40%:15%) 30 nm | ETM1 10nm | ETM1:ETM2 (50%:50%) 30 nm |
| D13 | HTM1 210 nm | A17 10 nm | TMM1:TMM2:Ir-Ref.1 (45%:40%:15%) 30 nm | ETM1 10nm | ETM1:ETM2 (50%:50%) 30 nm |
| D14 | HTM1 210 nm | A19 10 nm | TMM1:TMM2:Ir-Ref.1 (45%:40%:15%) 30 nm | ETM1 10nm | ETM1:ETM2 (50%:50%) 30 nm |
| D15 | HTM1 210 nm | A20 10 nm | TMM1:TMM2:Ir-Ref.1 (45%:40%:15%) 30 nm | ETM1 10nm | ETM1:ETM2 (50%:50%) 30 nm |
| D16 | HTM1 210 nm | A30 10 nm | TMM1:TMM2:Ir-Ref.1 (45%:40%:15%) 30 nm | ETM1 10nm | ETM1:ETM2 (50%:50%) 30 nm |
| D17 | HTM1 210 nm | A31 10 nm | TMM1:TMM2:Ir-Ref.1 (45%:40%:15%) 30 nm | ETM1 10nm | ETM1:ETM2 (50%:50%) 30 nm |
| D18 | A9 210 nm | HTM2 10 nm | TMM1:TMM2:Ir-Ref.1 (45%:40%:15%) 30 nm | ETM1 10nm | ETM1:ETM2 (50%:50%) 30 nm |

(fortgesetzt)

| Bsp. | HTL1 Dicke | HTL2 Dicke | EML Dicke | HBL Dicke | ETL Dicke |
|---|---|---|---|---|---|
| D19 | A11 210 nm | HTM2 10 nm | TMM1:TMM2:Ir-Ref.1 (45%:40%:15%) 30 nm | ETM1 10nm | ETM1:ETM2 (50%:50%) 30 nm |
| D20 | A12 210 nm | HTM2 10 nm | TMM1:TMM2:Ir-Ref.1 (45%:40%:15%) 30 nm | ETM1 10nm | ETM1:ETM2 (50%:50%) 30 nm |
| D21 | A40 210 nm | HTM2 10 nm | TMM1:TMM2:Ir-Ref.1 (45%:40%:15%) 30 nm | ETM1 10nm | ETM1:ETM2 (50%:50%) 30 nm |
| D22 | HTM1 210 nm | A22 10 nm | TMM1:TMM2:Ir-Ref.2 (40%:50%:10%) 30 nm | ETM1 10nm | ETM1:ETM2 (50%:50%) 30 nm |
| D23 | HTM1 210 nm | A23 10 nm | TMM1:TMM2:Ir-Ref.2 (40%:50%:10%) 30 nm | ETM1 10nm | ETM1:ETM2 (50%:50%) 30 nm |
| D24 | HTM1 210 nm | A24 10 nm | TMM1:TMM2:Ir-Ref.2 (40%:50%:10%) 30 nm | ETM1 10nm | ETM1:ETM2 (50%:50%) 30 nm |
| D25 | HTM1 210 nm | A25 10 nm | TMM1:TMM2:Ir-Ref.2 (40%:50%:10%) 30 nm | ETM1 10nm | ETM1:ETM2 (50%:50%) 30 nm |
| D26 | HTM1 210 nm | A26 10 nm | TMM1:TMM2:Ir-Ref.2 (40%:50%:10%) 30 nm | ETM1 10nm | ETM1:ETM2 (50%:50%) 30 nm |
| D27 | HTM1 210 nm | A27 10 nm | TMM1:TMM2:Ir-Ref.2 (40%:50%:10%) 30 nm | ETM1 10nm | ETM1:ETM2 (50%:50%) 30 nm |
| D28 | HTM1 210 nm | A28 10 nm | TMM1:TMM2:Ir-Ref.2 (40%:50%:10%) 30 nm | ETM1 10nm | ETM1:ETM2 (50%:50%) 30 nm |
| D29 | HTM1 210 nm | HTM2 10 nm | TMM1:C1:Ir-Ref.1 (45%:40%:15%) 30 nm | ETM1 10nm | ETM1:ETM2 (50%:50%) 30 nm |
| D30 | HTM1 210 nm | HTM2 10 nm | TMM1:C3:Ir-Ref.1 (45%:40%:15%) 30 nm | ETM1 10nm | ETM1:ETM2 (50%:50%) 30 nm |
| D31 | HTM1 210 nm | HTM2 10 nm | TMM1:C4:Ir-Ref.1 (45%:40%:15%) 30 nm | ETM1 10nm | ETM1:ETM2 (50%:50%) 30 nm |
| D32 | HTM1 210 nm | HTM2 10 nm | TMM1:C5:Ir-Ref.1 (45%:40%:15%) 30 nm | ETM1 10nm | ETM1:ETM2 (50%:50%) 30 nm |
| D33 | HTM1 210 nm | A13 10 nm | TMM1:Cl2:Ir-Ref.1 (45%:40%:15%) 30 nm | ETM1 10nm | ETM1:ETM2 (50%:50%) 30 nm |
| D34 | HTM1 210 nm | HTM2 10 nm | TMM1:TMM2:Ir-Ref.1 (45%:40%:15%) 30 nm | T1 10nm | T1:ETM2 (50%:50%) 30 nm |
| D35 | HTM1 210 nm | HTM2 10 nm | TMM1:TMM2:Ir-Ref.1 (45%:40%:15%) 30 nm | T2 10nm | T2:ETM2 (50%:50%) 30 nm |
| D36 | HTM1 210 nm | HTM2 10 nm | TMM1:TMM2:Ir-Ref.1 (45%:40%:15%) 30 nm | T3 10nm | T3: ETM2 (50%:50%) 30 nm |
| D37 | HTM1 210 nm | HTM2 10 nm | TMM1:TMM2:Ir-Ref.1 (45%:40%:15%) 30 nm | ETM1 10nm | T4:ETM2 (50%:50%) 30 nm |
| D38 | HTM1 210 nm | HTM2 10 nm | TMM1:TMM2:Ir-Ref.1 (45%:40%:15%) 30 nm | T5 10nm | T5: ETM2 (50%:50%) 30 nm |
| D39 | HTM1 160 nm | HTM2 10 nm | SH1:BD-Ref.1 (94%:6%) 20 nm | --- | ETM1:ETM2 (50%:50%) 30 nm |
| D40 | HTM1 200 nm | HTM2 10 nm | SH1:SE1 (94%:6%) 25 nm | --- | ETM1:ETM2 (50%:50%) 30 nm |

**Tabelle 2: Ergebnisse der Vakuum-prozessierten OLEDs**

| Bsp. | EQE (%) 1000 cd/m² | Spannung (V) 1000 cd/m² | CIE x/y 1000 cd/m² | LD90 (h) 10000 cd/m² |
|---|---|---|---|---|
| Grüne und Gelbe OLEDs | | | | |
| Ref.D1 | 19.2 | 3.3 | 0.33/0.62 | 310 |
| Ref. D2 | 18.1 | 3.2 | 0.41/0.58 | 440 |
| D1 | 19.4 | 3.3 | 0.33/0.62 | 330 |
| D2 | 20.0 | 3.2 | 0.32/0.62 | 310 |
| D3 | 19.5 | 3.2 | 0.33/0.62 | 340 |
| D4 | 19.0 | 3.1 | 0.33/0.63 | 330 |
| D5 | 19.4 | 3.3 | 0.33/0.62 | 350 |
| D6 | 20.3 | 3.3 | 0.33/0.62 | 300 |
| D7 | 18.7 | 3.1 | 0.32/0.61 | 360 |
| D8 | 19.7 | 3.1 | 0.33/0.62 | 330 |
| D9 | 19.5 | 3.3 | 0.33/0.62 | 340 |
| D10 | 20.0 | 3.1 | 0.32/0.62 | 330 |
| D11 | 19.2 | 3.0 | 0.33/0.62 | 350 |
| D12 | 19.4 | 3.2 | 0.33/0.62 | 340 |
| D13 | 19.2 | 3.3 | 0.33/0.62 | 370 |
| D14 | 19.7 | 3.2 | 0.32/0.61 | 320 |
| D15 | 18.9 | 3.3 | 0.33/0.62 | 350 |
| D16 | 20.1 | 2.9 | 0.33/0.62 | 380 |
| D17 | 19.7 | 3.0 | 0.33/0.62 | 390 |
| D18 | 19.3 | 3.5 | 0.33/0.62 | 320 |
| D19 | 20.0 | 2.8 | 0.33/0.62 | 310 |
| D20 | 20.4 | 2.9 | 0.33/0.62 | 380 |
| D21 | 20.2 | 2.8 | 0.32/0.62 | 370 |
| D22 | 18.2 | 3.0 | 0.41/0.58 | 450 |
| D23 | 18.0 | 2.8 | 0.41/0.58 | 470 |
| D24 | 19.1 | 2.9 | 0.41/0.58 | 510 |
| D25 | 18.7 | 2.9 | 0.41/0.58 | 470 |
| D26 | 19.0 | 3.0 | 0.41/0.58 | 450 |
| D27 | 19.3 | 2.8 | 0.41/0.58 | 460 |
| D28 | 19.2 | 2.9 | 0.41/0.58 | 500 |
| D29 | 19.7 | 3.1 | 0.33/0.62 | 390 |
| D30 | 19.6 | 3.1 | 0.33/0.62 | 380 |
| D31 | 19.7 | 3.1 | 0.33/0.62 | 370 |
| D32 | 20.0 | 3.2 | 0.32/0.62 | 420 |
| D33 | 19.3 | 3.1 | 0.32/0.62 | 360 |
| D34 | 19.4 | 3.2 | 0.33/0.62 | 340 |
| D35 | 19.3 | 3.1 | 0.33/0.62 | 350 |
| D36 | 19.4 | 3.2 | 0.33/0.62 | 340 |

(fortgesetzt)

| Bsp. | EQE (%) 1000 cd/m² | Spannung (V) 1000 cd/m² | CIE x/y 1000 cd/m² | LD90 (h) 10000 cd/m² |
|---|---|---|---|---|
| **Grüne und Gelbe OLEDs** | | | | |
| D37 | 19.5 | 3.4 | 0.33/0.62 | 360 |
| D38 | 19.7 | 3.2 | 0.33/0.62 | 370 |
| **Blaue und Grüne OLEDs** | | | | |
| **Bsp.** | **EQE (%) 1000 cd/m²** | **Spannung (V) 1000 cd/m²** | **CIE x/y 1000 cd/m²** | **LD90 (h) 10000 cd/m²** |
| D39 | 7.2 | 4.2 | 0.15/0.09 | 300h |
| D40 | 8.6 | 3.7 | 0.29/0.62 | --- |

**2. Lösungs-prozessierte Devices:**

**A: Aus niedermolekularen löslichen Funktionsmaterialien**

Die erfindungsgemäßen Materialien können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / Lochinjektionsschicht (60 nm) / Interlayer (20 nm) / Emissionsschicht (60 nm) / Lochblockierschicht (10 nm) / Elektronentransportschicht (40 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum eine 20 nm Lochinjektionsschicht durch Spin-Coating aufgebracht. Die benötigte Spinrate hängt vom Verdünnungsgrad und der spezifischen Spin-Coater-Geometrie ab. Um Restwasser aus der Schicht zu entfernen, werden die Substrate für 30 Minuten bei 200 °C auf einer Heizplatte ausgeheizt. Die verwendete Interlayer dient dem Lochtransport, in diesem Fall wird HL-X von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Triplettemitter zusammen mit den Matrixmaterialien in Toluol oder Chlorbenzol gelöst. Der typische Feststoffgehalt solcher Lösungen liegt zwischen 16 und 25 g/L, wenn, wie hier, die für eine Device typische Schichtdicke von 60 nm mittels Spincoating erzielt werden soll. Die lösungsprozessierten Devices enthalten eine Emissionsschicht aus Matrix1:Matrix2:Ir-Ref.3 und gegebenenfalls Ir-Ref.4. Gegebenenfalls wird zusätzlich eine Matrix3 verwendet (s. Tabelle 3). Die Emissionsschicht wird in einer Inertgasatmosphäre, im vorliegenden Fall Argon, aufgeschleudert und 10 min bei 160 °C ausgeheizt. Darüber wird die Lochblockierschicht (10nm ETM1) und die Elektronentransportschicht (40nm ETM1 (50%) / ETM2 (50%)) aufgedampft (Aufdampfanlagen von Lesker o.a., typischer Aufdampfdruck 5 x $10^{-6}$ mbar). Zuletzt wird eine Kathode aus Aluminium (100 nm) (hochreines Metall von Aldrich) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen. Als Lebensdauer LD50 wird die Zeit definiert, nach der die Leuchtdichte bei Betrieb mit einer Starthelligkeit von 1000 cd/m² auf 50% der Startleuchtdichte absinkt.

**Tabelle 3: Ergebnisse mit aus Lösung orozessierten Materialien**

| Bsp. | Matrix1<br>Matrix2<br>Matrix3<br>Ir-Ref.3 | EQE (%) 1000 cd/m² | Spannung (V) 1000 cd/m² | CIE x/y | LD50 (h) 1000 cd/m² |
|---|---|---|---|---|---|
| Sol-D1 | TMM3 (20%)<br>TMM4 (60%)<br>---<br>Ir-Ref.3 (20%) | 21.0 | 4.3 | 0.34/0.62 | 310000 |

(fortgesetzt)

| Bsp. | Matrix1<br>Matrix2<br>Matrix3<br>Ir-Ref.3 | EQE (%) 1000 cd/$m^2$ | Spannung (V) 1000 cd/$m^2$ | CIE x/y | LD50 (h) 1000 cd/$m^2$ |
|---|---|---|---|---|---|
| Sol-D2 | TMM3 (26%)<br>TMM4 (50%)<br>A15 (8%)<br>Ir-Ref.3 (16%) | 20.9 | 3.9 | 0.34/0.62 | 330000 |
| Sol-D3 | TMM3 (30%)<br>TMM4 (48%)<br>A18 (6%)<br>Ir-Ref.3 (16%) | 21.2 | 3.9 | 0.35/0.62 | 350000 |
| Sol-D4 | TMM3 (30%)<br>TMM4 (48%)<br>A21 (6%)<br>Ir-Ref.3 (16%) | 21.5 | 3.8 | 0.34/0.62 | 330000 |
| Sol-D5 | TMM3 (30%)<br>TMM4 (48%)<br>C6 (6%)<br>Ir-Ref.3 (16%) | 21.3 | 4.2 | 0.35/0.62 | 340000 |
| Sol-D6 | TMM3 (30%)<br>TMM4 (48%)<br>C7 (6%)<br>Ir-Ref.3 (16%) | 21.1 | 4.1 | 0.35/0.62 | 330000 |
| Sol-D7 | C8 (20%)<br>TMM4 (56%)<br>---<br>Ir-Ref.3 (24%) | 21.4 | 4.1 | 0.35/0.62 | 370000 |
| Sol-D8 | C9 (20%)<br>TMM4 (56%)<br>---<br>Ir-Ref.3 (24%) | 21.0 | 4.0 | 0.34/0.62 | 350000 |
| Sol-D9 | TMM3 (38%)<br>TMM4 (40%)<br>C10 (6%)<br>Ir-Ref.3 (16%) | 20.8 | 3.9 | 0.35/0.62 | 340000 |
| Sol-D10 | TMM3 (20%)<br>TMM4 (52%)<br>H1 (8%)<br>Ir-Ref.3 (20%) | 21.6 | 4.2 | 0.34/0.62 | 360000 |
| Sol-D11 | TMM3 (10%)<br>C11 (20%)<br>TMM4 (34%)<br>Ir-Ref.3 (30%)<br>Ir-Ref.4 (6%) | 18.1 | 5.7 | 0.67/0.33 | 300000 |

**Tabelle 4: Strukturformeln der verwendeten Materialien**

| | |
|---|---|
| 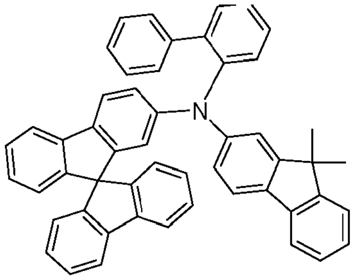 HTM1 [136463-07-5] | 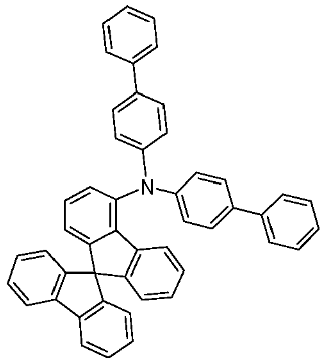 HTM2 [1450933-43-3] |
| 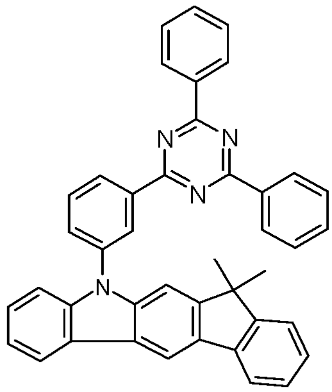 TMM1 [1257248-13-7] | 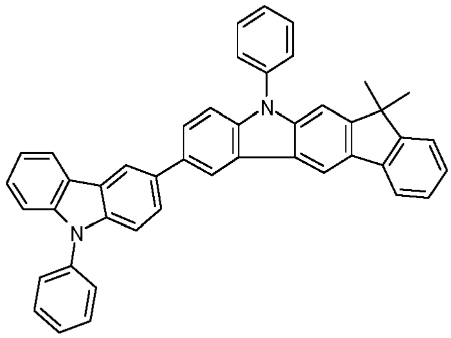 TMM2 [1357150-54-9] |
| 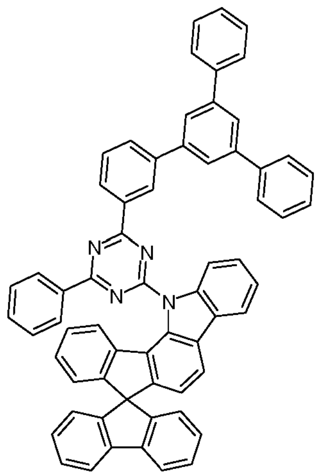 TMM3 [1616231-60-7] | 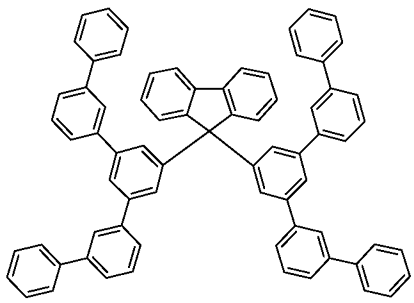 TMM4 [1246496-85-4] |

(fortgesetzt)

| | |
|---|---|
| 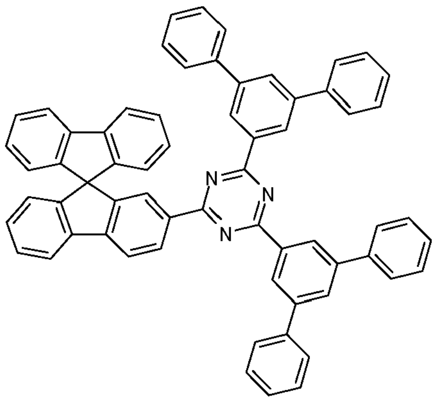 ETM1 = M10 [1233200-52-6] | 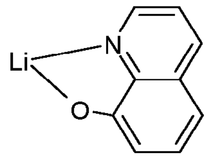 ETM2 [25387-93-3] |
| 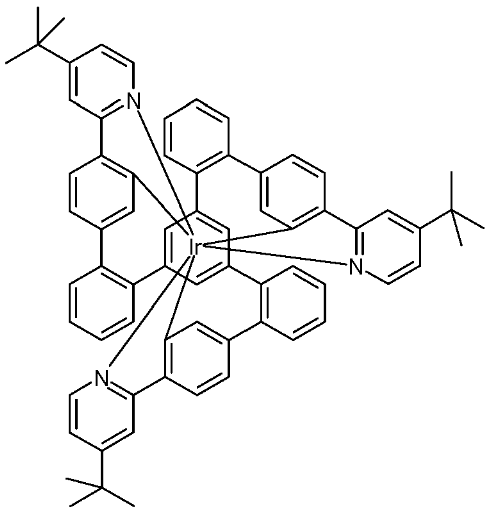 Ir-Ref.1 [1989606-01-0] | 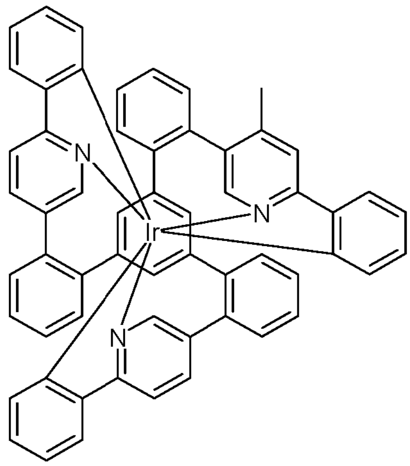 Ir-Ref.2 [1989605-56-2] |

(fortgesetzt)

| | |
|---|---|
| 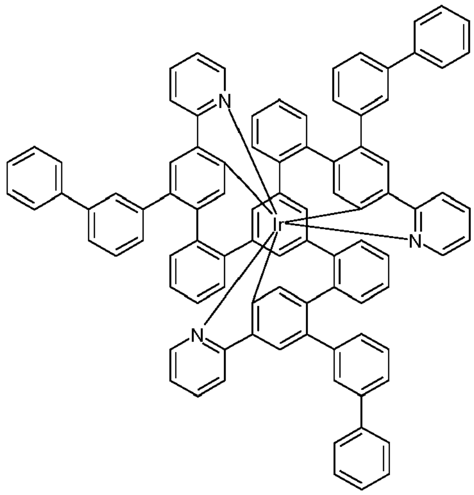 Ir-Ref.3 [2170173-12-1] | 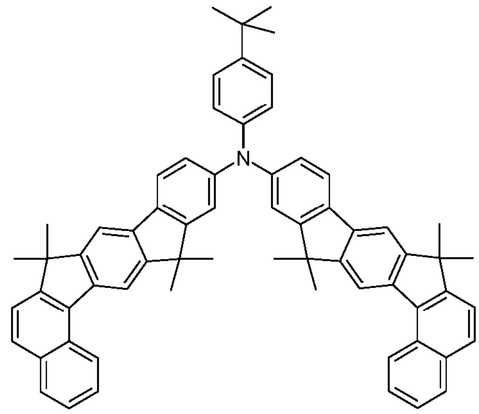 BD-Ref.1 [1883835-29-7] |
| 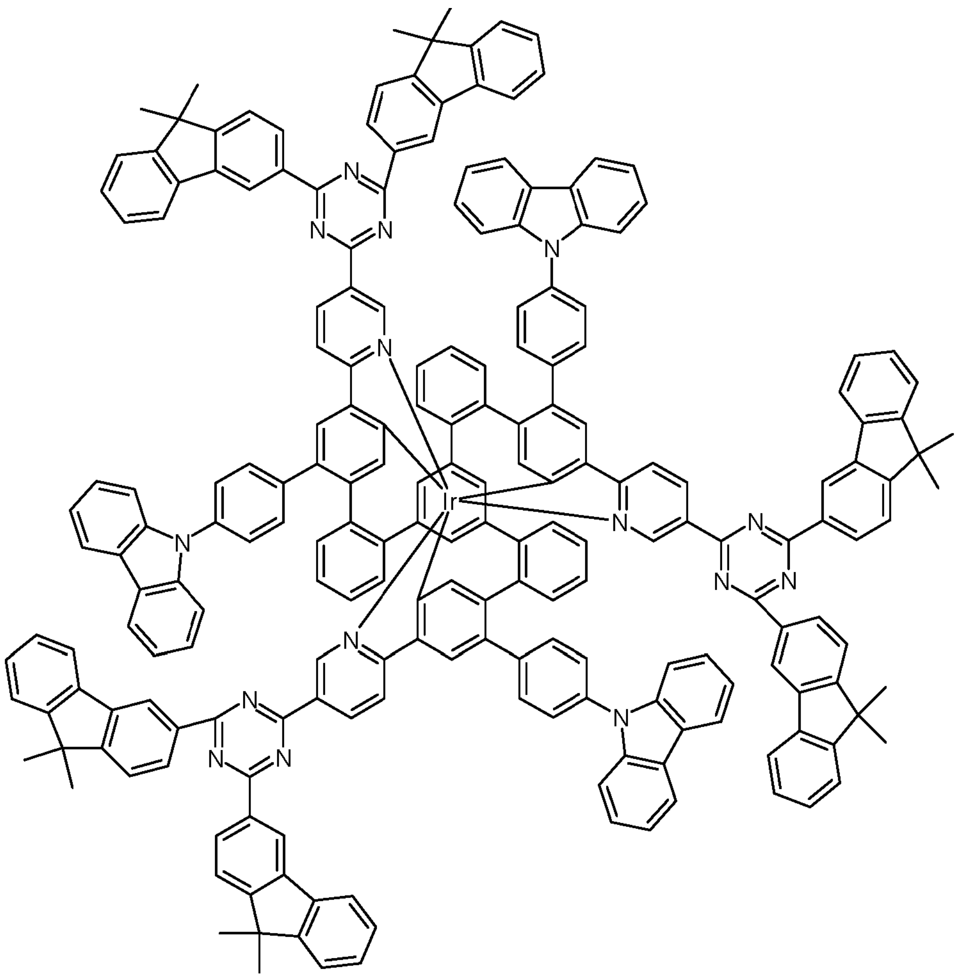 Ir-Ref.4 [1989604-92-3] | |

## Patentansprüche

1. Verbindung, die vorzugsweise in einer organischen elektronischen Vorrichtung als aktive Verbindung einsetzbar ist, **dadurch gekennzeichnet, dass** die Verbindung mindestens ein aromatisches oder heteroaromatisches Ringsystem mit 5 bis 60 Kohlenstoffatomen aufweist, welches an ein aliphatisches polycyclisches Ringsystem mit mindestens 3 Ringen kondensiert ist,
**dadurch gekennzeichnet, dass** das aliphatische polycyclische Ringsystem mit mindestens 3 Ringen welches an ein aromatisches oder heteroaromatisches Ringsystem mit 5 bis 60 Kohlenstoffatomen kondensiert ist, eine Teilstruktur der Formeln (N-1) bis (N-6) bildet,

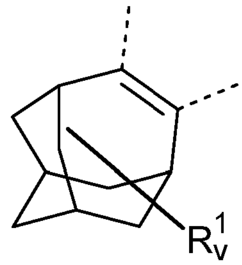

Formel (N-1)

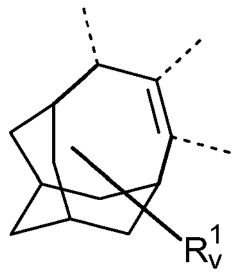

Formel (N-2)

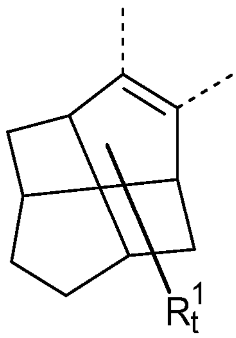

Formel (N-3)

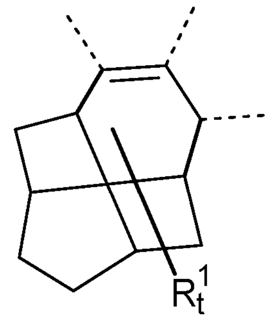

Formel (N-4)

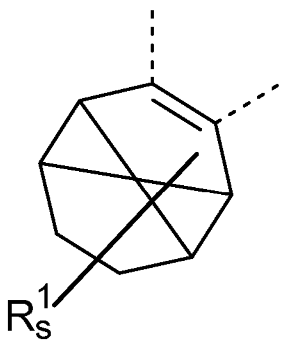

Formel (N-5)

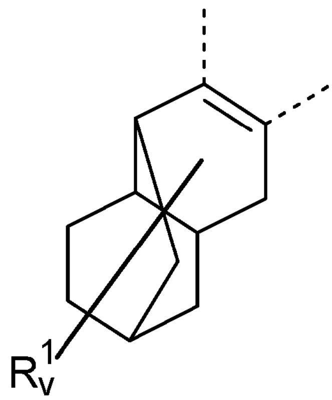

Formel (N-6)

wobei die gestrichelten Linien die Verknüpfungen des aromatischen oder des heteroaromatischen Ringsystems mit 5 bis 60 Kohlenstoffatomen darstellen, an welches das aliphatische polycyclische Ringsystem mit mindestens 3 Ringen kondensiert ist, und wobei für die Symbole $R^1$, v, t und s gilt:

$R^1$ ist bei jedem Auftreten gleich oder verschieden H, D, OH, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $B(OR^2)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen oder einer Alkenyl- oder Alkinylgruppe mit 2 bis 40 C-Atomen, die jeweils mit einem

oder mehreren Resten $R^2$ substituiert sein kann, wobei eine oder mehrere nicht benachbarte $CH_2$-Gruppen durch $-R^2C=CR^2-$, -C≡C-, $Si(R^2)_2$, $Ge(R^2)_2$, $Sn(R^2)_2$, C=O, C=S, C=Se, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann, oder eine Kombination dieser Systeme;dabei können zwei oder mehrere, vorzugsweise benachbarte Reste $R^1$ miteinander ein mono- oder polycyclisches, aliphatisches oder aromatisches oder heteroaromatisches Ringsystem bilden;

der Index s ist 0, 1, 2, 3, 4, 5 oder 6, vorzugsweise 0, 1, 2, 3, oder 4, besonders bevorzugt 0, 1 oder 2;

der Index t ist 0, 1, 2, 3, 4, 5, 6, 7 oder 8, vorzugsweise 0, 1, 2, 3, oder 4, besonders bevorzugt 0, 1 oder 2;

der Index v ist 0, 1, 2, 3, 4, 5, 6, 7, 8 oder 9, vorzugsweise 0, 1, 2, 3, oder 4, besonders bevorzugt 0, 1 oder 2 und **dadurch gekennzeichnet, dass** das aromatische oder heteroaromatische Ringsystem mit 5 bis 60 Kohlenstoffatomen, an welches an ein aliphatisches polycyclisches Ringsystem mit mindestens 3 Ringen kondensiert ist, eine Teilstruktur der Formel (Ar-1) bildet,

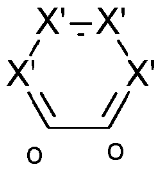

(Ar-1)

wobei X' N oder $CR^1$, vorzugsweise $CR^1$ ist, wobei $R^1$ die zuvor dargelegte Bedeutung hat und das aliphatische polycyclische Ringsystem mit mindestens 3 Ringen jeweils an den durch o gekennzeichneten Positionen an das aromatische oder heteroaromatische Ringsystem mit 5 bis 60 Kohlenstoffaromen unter Bildung eines Ringes bindet.

2. Verbindung gemäß Anspruch 1, **dadurch gekennzeichnet, dass** das aromatische oder heteroaromatische Ringsystem mit 5 bis 60 Kohlenstoffaromen, an welches an ein aliphatisches polycyclisches Ringsystem mit mindestens 3 Ringen kondensiert ist, eine Teilstruktur der Formel (Ar'-1) bildet

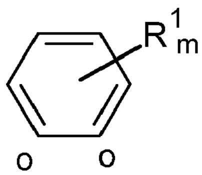

(Ar'-1)

wobei $R^1$ die in Anspruch 1 dargelegte Bedeutung aufweist, der Index m 0, 1, 2, 3 oder 4, vorzugsweise 0, 1 oder 2 ist, und das aliphatische polycyclische Ringsystem mit mindestens 3 Ringen jeweils an den durch o gekennzeichneten Positionen an das aromatische oder heteroaromatische Ringsystem mit 5 bis 60 Kohlenstoffaromen unter Bildung eines Ringes bindet..

3. Verbindung gemäß Anspruch 1 oder 2, **dadurch gekennzeichnet, dass** die Verbindung eine Lochtransportgruppe umfasst, wobei vorzugsweise in einer Struktur gemäß den Formeln (N-1) bis (N-6), (Ar-1) und/oder (Ar'-1) eine Gruppe $R^1$ eine Lochtransportgruppe umfasst, vorzugsweise darstellt.

4. Verbindung gemäß mindestens einem der vorhergehenden Ansprüche, **dadurch gekennzeichnet, dass** die Verbindung eine Elektronentransportgruppe umfasst, wobei vorzugsweise in einer Struktur gemäß den Formeln (N-1) bis (N-6), (Ar-1) und/oder (Ar'-1) eine Gruppe $R^1$ eine Elektronentransportgruppe umfasst, vorzugsweise darstellt, wobei die Elektronenleitergruppen vorzugsweise mindestens 2 Stickstoffatome in einem Sechsring oder in zwei kondensierten Sechsringen umfassen.

5. Verbindung gemäß mindestens einem der vorhergehenden Ansprüche 1, 3 und 4 , **dadurch gekennzeichnet, dass**

der Ring, über den das aliphatische polycyclische Ringsystem mit mindestens 3 Ringen an das aromatische oder heteroaromatische Ringsystem mit 5 bis 60 Kohlenstoffaromen kondensiert ist, sechs Ringatome und mindestens zwei nicht benachbarte Stickstoffatome umfasst.

**6.** Verbindung gemäß mindestens einem der vorhergehenden Ansprüche 1 2 und 4, **dadurch gekennzeichnet, dass** der Ring, über den das aliphatische polycyclische Ringsystem mit mindestens 3 Ringen an das aromatische oder heteroaromatische Ringsystem mit 5 bis 60 Kohlenstoffaromen kondensiert ist, sechs Ringatome und mindestens ein Stickstoffatom umfasst und an diesen Ring kein weiteres Ringsystem kondensiert ist.

**7.** Oligomer, Polymer oder Dendrimer enthaltend eine oder mehrere Verbindungen nach einem der Ansprüche 1 bis 6, wobei statt eines Wasserstoffatoms oder eines Substituenten ein oder mehrere Bindungen der Verbindungen zum Polymer, Oligomer oder Dendrimer vorhanden sind.

**8.** Zusammensetzung enthaltend wenigstens eine Verbindung nach einem oder mehreren der Ansprüche 1 bis 6 oder ein Oligomer, Polymer oder Dendrimer nach Anspruch 7 und wenigstens eine weitere Verbindung ausgewählt aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Elektroneninjektionsmaterialien, Lochleitermaterialien, Lochinjektionsmaterialien, Elektronenblockiermaterialien und Lochblockiermaterialien.

**9.** Formulierung, enthaltend mindestens eine Verbindung nach einem oder mehreren der Ansprüche 1 bis 6 oder ein Oligomer, Polymer oder Dendrimer nach Anspruch 7 oder eine Zusammensetzung nach Anspruch 8 und mindestens ein Lösemittel.

**10.** Verwendung einer Verbindung nach einem oder mehreren der Ansprüche 1 bis 6, eines Oligomers, Polymers oder Dendrimers nach Anspruch 7 oder einer Zusammensetzung nach Anspruch 9 in einer elektronischen Vorrichtung fluoreszierenden Emitter, Emitter, der TADF (thermally activated delayed fluorescence) zeigt, Hostmaterial, Elektronentransportmaterial, Elektroneninjektionsmaterial, Lochleitermaterial, Loch-injektionsmaterial, Elektronenblockiermaterial, Lochblockiermaterial und/oder Wide-Band-Gap-Material, vorzugsweise als fluoreszierenden Emitter (Singulet-Emitter), Hostmaterial, Lochleitermaterial und/oder Elektronentransportmaterial.

**11.** Verfahren zur Herstellung einer Verbindung nach einem oder mehreren der Ansprüche 1 bis 6 oder eines Oligomers, Polymers und/oder Dendrimers nach Anspruch 7, **dadurch gekennzeichnet, dass** in einer Kupplungsreaktion eine Verbindung, umfassend mindestens ein aliphatisches polycyclisches Ringsystem mit mindestens 3 Ringen, mit einer Verbindung, umfassend mindestens eine aromatische oder heteroaromatische Gruppe, verbunden wird.

**12.** Elektronische Vorrichtung, enthaltend mindestens eine Verbindung nach einem oder mehreren der Ansprüche 1 bis 6, ein Oligomer, ein Oligomer, Polymer oder Dendrimer nach Anspruch 7 oder eine Zusammensetzung gemäß Anspruch 8, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4539507 A
- US 5151629 A
- EP 0676461 A
- WO 9827136 A
- WO 2014094960 A1
- WO 2014094961 A1
- WO 2015104045 A1
- WO 2015117718 A1
- WO 2016124304 A
- WO 2015036078 A
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016015815 A
- WO 2017032439 A
- WO 2018019687 A
- WO 2018019688 A
- WO 2018041769 A
- WO 2018054798 A
- WO 2018069196 A
- WO 2018069197 A
- WO 2018069273 A
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A

- WO 2011137951 A
- WO 2013064206 A
- WO 2014094963 A
- WO 2015192939 A
- WO 2015169412 A
- WO 2016015810 A
- WO 2016023608 A
- EP 16158460
- EP 16159829
- WO 2010108579 A
- WO 2005053051 A
- WO 2009030981 A
- WO 2004058911 A
- WO 2004037887 A


**In der Beschreibung aufgeführte Nicht-Patentliteratur**


- **D. M. KOLLER et al.** *Nature Photonics*, 2008, 1-4
- **B. M. S. ARNOLD et al.** *Appl. Phys. Lett.*, 2008, vol. 92, 053301